# EUROPEAN PATENT SPECIFICATION

(11) **EP 2 251 695 B1**
(45) Date of publication and mention of the grant of the patent: **17.07.2013**
(21) Application number: 10169645.8
(22) Date of filing: 21.12.2004
(51) Int. Cl.: G01N 33/574

(54) **MARKERS ASSOCIATED WITH ENDOMETRIAL DISEASE**
MIT ENDOMETRIALER KRANKHEIT ASSOZIIERTE MARKER
MARQUEURS ASSOCIEES A LA MALADIE DE L'ENDOMETRE

(30) Priority: 23.12.2003 US 532601 P; 24.11.2004 US 630990 P
(43) Date of publication of application: 17.11.2010
(62) Divisional of application: 04802347.7
(73) Proprietor: Mount Sinai Hospital Corporation, Toronto, Ontario M5G 1X5 (CA); University Health Network, Toronto, Ontario M5G 2M9 (CA); Siu, Michael K. W., Toronto ON M2J 2X6 (CA)
(72) Inventor: Siu, Michael K.W., Toronto, Ontario M2J 2X6 (CA); Colgan, Terence J., Toronto, Ontario M2P 1A3 (CA); Romaschin, Alexander D., Toronto, Ontario M9C 1L4 (CA); Yang, Eric C.C., Toronto, Ontario M2R 1C5 (CA); Desouza, Leroi, North York, Ontario M6A 1N9 (CA); Diehl, George, D-79176 Weil am Rhein (DE); Gou, Jingzhong, Saint-Laurent, Québec H4M 2M1 (CA)
(74) Representative: Schiweck, Wolfram

(56) References cited:
- US-B1- 6 228 596
- SHAARAWY M ET AL: "BIOMARKERS OF INTRINSIC AND ANTI-ANGIOGENIC ACTIVITY IN PATIENTS WITH ENDOMETRIAL HYPERPLASIA AND ENDOMETRIAL CANCER", ACTA ONCOLOGICA, INFORMA HEALTHCARE, LONDON, GB LNKD- DOI:10.1080/028418601750288253, vol. 40, no. 4, 1 January 2001 (2001-01-01), pages 513-518, XP008071359, ISSN: 0284-186X
- DESMOND JULIAN C ET AL: "Identification of genes differentially expressed in endometrial carcinoma.", PROCEEDINGS OF THE AMERICAN ASSOCIATION FOR CANCER RESEARCH ANNUAL MEETING, vol. 44, July 2003 (2003-07), page 1198, XP002465479, & 94TH ANNUAL MEETING OF THE AMERICAN ASSOCIATION FOR CANCER RESEARCH; WASHINGTON, DC, USA; JULY 11-14, 2003 ISSN: 0197-016X
- DATABASE BIOSIS [Online] BIOSCIENCES INFORMATION SERVICE, PHILADELPHIA, PA, US; March 2003 (2003-03), HARDT P D ET AL: "Measurement of fecal Pyruvate Kinase type M2 (Tumor M2-PK) concentrations in patients with gastric cancer, colorectal cancer, colorectal adenomas and controls.", XP002598448, Database accession no. PREV200300403239 & ANTICANCER RESEARCH, vol. 23, no. 2A, March 2003 (2003-03), pages 851-854, ISSN: 0250-7005
- DESOUZA LEROI ET AL: "Search for cancer markers from endometrial tissues using differentially labeled tags iTRAQ and clCAT with multidimensional liquid chromatography and tandem mass spectrometry", JOURNAL OF PROTEOME RESEARCH, ACS, WASHINGTON, DC, US LNKD- DOI:10.1021/PR049821J, vol. 4, no. 2, 1 March 2005 (2005-03-01), pages 377-386, XP002411144, ISSN: 1535-3893
- GRY ET AL.: "Correlations between RNA and protein expression profiles in 23 human cell lines.", BMC GENOMICS, vol. 10, 365, 2009,

### SEQUENCE LISTING

<110> MOUNT SINAI HOSPITAL
<120> ENDOMETRIAL MARKERS
<130> 064016-355354
<140> PCT/CA2004/002172
   <141> 2004-12-21
<150> US 60/532,601
   <151> 2003-12-23
<150> US 60/630,990
   <151> 2004-11-24
<160> 52
<170> PatentIn version 3.3
<210> 1
   <211> 102
   <212> PRT
   <213> Homo sapiens
<300>
   <308> Q04984
   <309> 2003-09-15
   <313> (1)..(102)
<300>
   <308> AAH23518
   <309> 2004-06-29
   <313> (1)..(102)
<400> 1
   Met Ala Gly Gln Ala Phe Arg Lys Phe Leu Pro Leu Phe Asp Arg Val
   1               5                   10                  15
   Leu Val Glu Arg Ser Ala Ala Glu Thr Val Thr Lys Gly Gly Ile Met
               20                  25                  30
   Leu Pro Glu Lys Ser Gln Gly Lys Val Leu Gln Ala Thr Val Val Ala
           35                  40                  45
   Val Gly Ser Gly Ser Lys Gly Lys Gly Gly Glu Ile Gln Pro Val Ser
       50                  55                  60
   Val Lys Val Gly Asp Lys Val Leu Leu Pro Glu Tyr Gly Gly Thr Lys
   65                  70                  75                  80
   Val Val Leu Asp Asp Lys Asp Tyr Phe Leu Phe Arg Asp Gly Asp Ile
                   85                  90                  95
   Leu Gly Lys Tyr Val Asp
               100
<210> 2
   <211> 538
   <212> DNA
   <213> Homo sapiens
<300>
   <308> NM_002157
   <309> 2004-12-18
   <313> (1)..(538)
<400> 2
   gctacactag agcagagtac gagtctgagg cggagggagt aatggcagga caagcgttta      60
   gaaagtttct tccactcttt gaccgagtat tggttgaaag gagtgctgct gaaactgtaa     120
   ccaaaggagg cattatgctt ccagaaaaat ctcaaggaaa agtattgcaa gcaacagtag     180
   tcgctgttgg atcgggttct aaaggaaagg gtggagagat tcaaccagtt agcgtgaaag     240
   ttggagataa agttcttctc ccagaatatg gaggcaccaa agtagttcta gatgacaagg     300
   attatttcct atttagagat ggtgacattc ttggaaagta cgtagactga aataagtcac     360
   tattgaaatg gcatcaacat gatgctgccc attccactga agttctgaaa tctttcgtca     420
   tgtaaataat ttccatattt ctcttttata ataaactaat gataactaat gacatccagt     480
   gtctccaaaa ttgtttcctt gtactgatat aaacacttcc aaataaaaat atgtaaat       538
<210> 3
   <211> 93
   <212> PRT
   <213> Homo sapiens
<300>
   <308> P05109
   <309> 2004-10-25
   <313> (1)..(93)
<400> 3
   Met Leu Thr Glu Leu Glu Lys Ala Leu Asn Ser Ile Ile Asp Val Tyr
   1               5                   10                  15
   His Lys Tyr Ser Leu Ile Lys Gly Asn Phe His Ala Val Tyr Arg Asp
               20                  25                  30
   Asp Leu Lys Lys Leu Leu Glu Thr Glu Cys Pro Gln Tyr Ile Arg Lys
           35                  40                  45
   Lys Gly Ala Asp Val Trp Phe Lys Glu Leu Asp Ile Asn Thr Asp Gly
       50                  55                  60
   Ala Val Asn Phe Gln Glu Phe Leu Ile Leu Val Ile Lys Met Gly Val
   65                  70                  75                  80
   Ala Ala His Lys Lys Ser His Glu Glu Ser His Lys Glu
                   85                  90
<210> 4
   <211> 4205
   <212> DNA
   <213> Homo sapiens
<300>
   <308> A12027
   <309> 1994-11-30
   <313> (1)..(4205)
<400> 4
   cttgggttgc ttccaccttt tggctcttgt aaataatgct gctatgaaca tgaatgtaca 60
   aacatctgtt tgaatccctg cattcaattc ttttgcatat atacccagga gcagaatgat 120
   ggatcatatg gtaattctgt gtttatttat ttgaggaaca aacttgccgt tttccataac 180
   agctgcacta ttttacattc ccactaacag tgcattaggc ttccaattct ctatgccctc 240
   accaacactt gttttctggg ttttaaaaga agtagtagtc atccttgtag gtgtcaggtg 300
   gtatctcatt gtcgttttgc ttcatgtttt cctaaagatt agtaattttc atatgcttat 360
   tgaccatttg tatatcttct tcggagaagt gtctatttga gtctttcccc aattttgatt 420
   ggtttgtttg ttttttgttg ttgagttgta gggattcttt tatattctgg atattaatcc 480
   cttatcagat atttgtttta caaatatttt ctttgtaaca acagaaacac accacagtct 540
   tcaaggttgg aagccagtta atctgagtag cattttgtta gtggtgggga gaggatttgt 600
   tcctcctgaa atcctgggga attggccacc tcctcttctc ctcttaggca tgaagcgcgt 660
   ctggcttctc caaagaactc ttcccctcca ctacctcaga gttagcttcc tctcttcagc 720
   cagtgatcct ggggtcccag acacaataat taaccaagag agggtgaaag gctccctgct 780
   gtgtttatgc aatggctcag gcccttgtga agtgccgagg gaccccaagc agcctccatc 840
   tcccagggca tggtccatcc ccagctttca cagaacagga aagctgtgga ggagtgtggg 900
   cagcagggta ggaatggata tagcccttgg caacaacaca tttccccaca aagcacccac 960
   ccaaaagaac aacaacgata gttttagttt ttagtaatga gaacaatagt tctcatgact 1020
   aaaagccatc agccaggaca ctgttctcaa cccttttgcg gtctttggac cctttgaaac 1080
   tctgacagaa gccatggagg aatgttctca ctgagtgcat gcactcaaaa tgatgcattc 1140
   aacttcaatt cagtttcagg gatgtatggc ctgaccacca atgcagggga ttagcaatcg 1200
   caatagtgga gagggcatgg gagtgggaat ctggctggat caagcaagtg gatgccagca 1260
   gcccagaaaa agagcccccc tacctgcttt ttccttcctg ggcactattg cccagcaaat 1320
   gccttcctct ttccgcttct cctacctccc cacccaaaat tttcattctg cacagtgatt 1380
   gccacattca ctggttgaga aacagagact gtagcaactc tggcagggag aagctgtctc 1440
   tgatggcctg aagctgtggg cagctggcca agcctaaccg ctataaaaag gagctgcctc 1500
   tcagccctgc atgtctcttg tcagctgtct ttcagaagac ctggtaagtg ggactgtctg 1560
   ggttggcccc gcactttggg cttctcttgg ggagggtcag ggaagtggag cagccttcct 1620
   gagagaggag agagaaagct cagggaggtc tggagcaaag atactcctgg aggtggggag 1680
   tgaggcaggg ataaggaagg agagtatcct ccagcacctt ccagtgggta agggcacatt 1740
   gtctcctagg ctggactttt cttgagcaga gggtggggtg gtaaggaaag tctacgggcc 1800
   cccgtgtgtg tgcacatgtc tctgtgtgaa tggacccttc cccttcccac acgtgtatcc 1860
   ctatcatccc acccttccca ccagaggcca tagccatctg ctggtttggt tatttgagag 1920
   tgcaggccag gacaaggcca tcgcttgggg catgaatcct ctgcgtactg ccctggccag 1980
   atgcaaattc cctgccatgg gattccccag aaggttctgt ttttcaggtg gggcaagttc 2040
   cgtgggcatc atgttgaccg agctggagaa agccttgaac tctatcatcg acgtctacca    2100
   caagtactcc ctgataaagg ggaatttcca tgccgtctac agggatgacc tgaagaaatt    2160
   gctagagacc gagtgtcctc agtatatcag ggtgaggagg ggctgggtgt ggcgggggct    2220
   ctctgcctgg tcctggggct gccctgggcc agcggtcctc cctgccaccc ttcatagatg    2280
   ctatgcctcg gctctctctg agatctttaa actctggctt cttcctcctc aatcttgaca    2340
   gaaaaagggt gcagacgtct ggttcaaaga gttggatatc aacactgatg gtgcagttaa    2400
   cttccaggag ttcctcattc tggtgataaa gatgggcgtg gcagcccaca aaaaaagcca    2460
   tgaagaaagc cacaaagagt agctgagtta ctgggcccag aggctgggcc cctggacatg    2520
   tacctgcaga ataataaagt catcaatacc tcatgcctct ctcttatgct tttgtggaat    2580
   gaggttcctc ggtgtggagg gagggttgga aaacccaaag gaagaaaaag aaatctatgt    2640
   tatcccaccc tacctctcac aagcctttcc tgctttaccc ctcacctggc ctctgcccca    2700
   cattccttca gcccctcatt tcgagcattg gatttgaggc ttaaggattc aaaaagtcgt    2760
   catgaatata gctgatgatt ttatagtggt tctgaaatgg gtcggggatt tgggaacagg    2820
   gtggtagtat aagaacaact gatactgttc tctaagctaa atcttagctt ccagctacct    2880
   gtcttagatg tggctcttgg gaaccttaga gtgatagcta catagaagtg tgtgggtgtg    2940
   tgtgtgtgtg tctgtgtgtg tgtgtgtgag agagagacag acagaaagag agcaagagag    3000
   ggaagggggg agaggctgat tgtgtgtgtg gtgtgatgta ggtggacaat gttcagagtc    3060
   ctccattaac aggataatcc tcacacctgt ccacatacct gtagtttgtc cttggggatt    3120
   ttgaaaattt ttcctccctc tccactccca aactcccaac tcaattaaat gataaaggaa    3180
   taggcaaata ggaaaataaa ttagtaaaac ttaagtcaaa gaataggtta ttcatacgct    3240
   gcctatggga ttctatgctt tgtgatcaga aaattatcta aaaaatactt cccaagggct    3300
   ggtacaaggg aggccagaag acgagtggtt cttctctgag gtggacatta aaaaaagaag    3360
   aaaatgaagg ggaacctttt gacaagaatg tcaccccaaa ctggattttc atgctgtggt    3420
   gtggggaatt ttctgttgtc ctcacttagg tgctggggca gtggtgttag tgatgggtaa    3480
   aaaggtagga agctgtcaca gaatcactaa accagggttc ttaacttgtc tgtctataca    3540
   tctctgaaat tgggttgaag ttgtgtgcat cattttgagt gacgcactga gaacattcct    3600
   ccacggcttc catcgagagt ctcgaaaagg cccaacacct caaaaaggtt aagaacactt    3660
   gtcctgctta ctggttttta gtaacaaatg gcagagtatt tctctctgtc tctctctctt    3720
   tttttttttt tttttttgag acacagggtc ttgtctgtca cgtggactag agtacaatgg    3780
   gcatgatcat gggctcactg tagcctcgaa cacctgggct caagtaatcc tcccacctca    3840
   gcctctttag tagctgggac tacagcatga gccactgccc ttggctaatt tttaaattat    3900
   ttttttgtag agatggaaac ttgctatgtt gcccaggcta gtctcaaact cctggactca    3960
   agcgatcctc ctaccttggc ctcccaaagt gctgagatta cagtgtgatc cacaccacac    4020
   ctggccaaag attggagtat ttttattgct attgttgtgc tgggtgggtg ggtgggtgta    4080
   tgctttgtgg ggacgtgtgt tgttgccaag ggctaaatca gttcctaccc tgctgcccac    4140
   agtcctccac agctttcctg ctctgtgaag ctaaggatac accccgatga taagctgtca 4200
   acata 4205
<210> 5
   <211> 428
   <212> DNA
   <213> Homo sapiens
<300>
   <308> NM_002964
   <309> 2004-10-26
   <313> (1)..(428)
<400> 5
   atgtctcttg tcagctgtct ttcagaagac ctggtggggc aagtccgtgg gcatcatgtt 60
   gaccgagctg gagaaagcct tgaactctat catcgacgtc taccacaagt actccctgat 120
   aaaggggaat ttccatgccg tctacaggga tgacctgaag aaattgctag agaccgagtg 180
   tcctcagtat atcaggaaaa agggtgcaga cgtctggttc aaagagttgg atatcaacac 240
   tgatggtgca gttaacttcc aggagttcct cattctggtg ataaagatgg gcgtggcagc 300
   ccacaaaaaa agccatgaag aaagccacaa agagtagctg agttactggg cccagaggct 360
   gggcccctgg acatgtacct gcagaataat aaagtcatca atacctcaaa aaaaaaaaaa 420
   aaaaaaaa 428
<210> 6
   <211> 114
   <212> PRT
   <213> Homo sapiens
<300>
   <308> P06702
   <309> 1993-09-12
   <313> (1)..(114)
<400> 6
   Met Thr Cys Lys Met Ser Gln Leu Glu Arg Asn Ile Glu Thr Ile Ile
   1               5                   10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
   65                  70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 7
   <211> 462
   <212> DNA
   <213> Homo sapiens
<300>
   <308> X06233
   <309> 1993-09-12
   <313> (1)..(462)
<400> 7
   aaaacactct gtgtggctcc tcggctttga cagagtgcaa gacgatgact tgcaaaatgt 60
   cgcagctgga acgcaacata gagaccatca tcaacacctt ccaccaatac tctgtgaagc 120
   tggggcaccc agacaccctg aaccaggggg aattcaaaga gctggtgcga aaagatctgc 180
   aaaattttct caagaaggag aataagaatg aaaaggtcat agaacacatc atggaggacc 240
   tggacacaaa tgcagacaag cagctgagct tcgaggagtt catcatgctg atggcgaggc 300
   taacctgggc ctcccacgag aagatgcacg agggtgacga gggccctggc caccaccata 360
   agccaggcct cggggagggc accccctaag accacagtgg ccaagatcac agtggccacg 420
   gccacggcca cagtcatggt ggccacggcc acagccaccc at 462
<210> 8
   <211> 4439
   <212> DNA
   <213> Homo sapiens
<300>
   <308> M21064
   <309> 1993-04-27
   <313> (1)..(4439)
<400> 8
   atcactgtgg agtaggggaa gggcactcct ggggtggcaa ggtgggaggt gggccctgtg      60
   ttcccacagt gggcagggag gtagtgaaag ggaagctggc cggacaggaa gggccattcc     120
   aagagggctt tgtgcgcagg gctaagccaa gctttctcca taggcaatgg ggagcaactg     180
   gaggttcgta gcaggagaag gacacatcaa gcccaccagg aggctaagta aaaacagttg     240
   tctcccaagt tataagttcc tggaaccctt gctgggagca ggatttagaa aaatgatgct     300
   gagagatgct agaaacatat tcgccctgag gctctctcac tcagactgca agaggaaggt     360
   atcatcagaa ttgcccttaa ccaggaacca gaatagctgg gtccccttcc tgccaagtca     420
   gcaaccagct atgtgacctt gctcaggtcc atctccgggt gtcagtttct tcatctacaa     480
   tgcaagaggg ttgcccacct ctgagaaccc ttctaacccc aaatctcacc ctatgaatct     540
   aagaacacaa cccctcgcca tcctaagtat cacagagcca ggcaagcatg ggtgagagct     600
   cagaccatcc ttgttggact aaaaggaagg ggcagactgc catggggggc agccgagagg     660
   gtcaggcccc cataggtcct cagcctgctt caacctcaaa ggggatgggg ggctgagtgg     720
   tgccagagga gcagcaggct cgctcgggga gagtagggcc ttaggataga agggaaatga 780
   actaaacaac cagcttcctg caaaccagtt tcaggccagg gctgggaatt tcacaaaaaa 840
   gcagaaggcg ctctgtgaac atttcctgcc ccgccccagc ccccttcctg gcagcattag 900
   cacactgctc acctgtgaag caatcttccg gagacagggc caaagggcaa gtgccccagt 960
   caggagctgc ctataaatgc cgagcctgca cagctctggc aaacactctg tgtggctcct 1020
   cggctttggt aagtgagctg ccagcttccc caggcagaag cctgcctgcc gattccttct 1080
   ttccttccct gacccaactt ccttccaaat cctcctccta gaagccctcc ttggttggcc 1140
   ctgcctactt taaagcttct ttcacatttt cttaggtcat gttcccctgg ggcctcctgc 1200
   cctcaaatgc tttgcttttt ggcactctgt agatattcta aaaaatcatt ttgtacatgt 1260
   gtgtgacagg ccatctccca gttaagttgc agcctgtgct ttctttttat tttgcacttc 1320
   ccccactatt tctgtgagtg cttagtagga agtgtcaaag aagcttgaca gcattttctt 1380
   ctaagtgtcc caactcttgg ttttccatta cacagacaga gtgcaagacg atgacttgca 1440
   aaatgtcgca gctggaacgc aacatagaga ccatcatcaa caccttccac caatactctg 1500
   tgaagctggg gcacccagac accctgaacc agggggaatt caaagagctg gtgcgaaaag 1560
   atctgcaaaa ttttctcaag gtagggctgg actctggcag gtctgaccca gcctcaccgc 1620
   agtttgggtt gacaagggag gatgggagta tgggctacag caatcaaggg gaagatttga 1680
   gctcctggag cccagcccca agacgcagcg agtgtcctgt tatacagggc aggtgctcac 1740
   agttacacag gacgacaggg tcaagaaatt gctcaattga acacctgcta tttgtcgggc 1800
   cctgttctgg gcagagggat gtagtggtaa atgggagccc actattccat gaggagacac 1860
   acagtaaagt tgttggccaa taaagagcac agataaagcc aaatgccaat aagtgcctgg 1920
   aagaaaatga gatagagtgc gctgtgggca atggggctgg gtggggtgga ggtgaccagt 1980
   tagggtacat gagaagggcc tctttgagga ggtaacattt gagctgagcc ccgaatgttg 2040
   gggagggaag cccctgagga tgacacttgg cacaaagctg aggagaccct aagcctcagg 2100
   gcgaacttgg ggtggaagac ttgggggctt ttctaatcct aagggtctgc ggtggaaaat 2160
   gaatgcataa agagcacatg gagagcacct gcacagcact cagggaactg ggaggttttt 2220
   cccccgctcc aaaaatgatt aggcagttct aagaaaaagg ctgagcactt ccaacagcct 2280
   ttttgttttc ttttcaaatt tggggaaagt cgggaaacag aggcctgcat taagaagggt 2340
   ggaacacatg ggtctcagtc tcagttccag tcccggagcc agacatcctg gggtaggtcc 2400
   ccagccctcc cagtgcccct ccctccgcct tggtaaggtg gagaattgca gccttcagag 2460
   ttaggggccc tgacagctct ccataggtgg aggcctcagg caggcaggat gctgggtggg 2520
   gtaggcaaga aagggcccag cagagaggcc gcatcggaaa actatcctcc atgtgacccc 2580
   ctatgcccgc ttcacccccc acctgacatc ccccaccaga agcaaagcga tgctgtggga 2640
   aaggaagcag agcctcatgg atgggctgca caggagagtg ctcgcattgg ctgggtaccc 2700
   cacaggttct gggaggggac ttagcgaggt gactcagtgc ctcggcctcc caaagtgctg 2760
   ggattacaag catgagccac cctgtccgac catctcccct tttatacttt atcacaccct 2820
   tgaggtcagc ggagcacata ctctgctctc tgaccctcca tctcccctgc ccacacctag     2880
   gtttttctag tgtttccccg ttgtattggt tgaaataagt ttcactaatt ggtaacctcc     2940
   agagggaagg gaagggaggg caggggaagg agtgaagtgc agaggggtag cagagtggaa     3000
   ctggcctcta agtcagatct gaatttgcat gccctcaata gtcaagcctg tgaaaactaa     3060
   tgaccctctc taggactggt ttcaagtctt cctccaggaa gataccattc ctagctgtta     3120
   aagttgttat aaggaccaaa tgaggtgaca tttccaggct tactcatgcc atgaccaggg     3180
   caagaccctg gaactcagct tcctcttcta taaatagaga atcagcaccc aagtcacagg     3240
   gtcatggagg gaataaactg gagagcgttt ggtatgtgct cagtgtctgc tccattgtgc     3300
   gcactcagcc tatggtcatt tttaattttt aaatccagcc ccagggtcga ggcttccttg     3360
   tacatttgcc agctggtcat ttactgtgct cccagtcccc acctctggcc acacccagct     3420
   ctcacagcct tctctcccca cccgcagaag gagaataaga atgaaaaggt catagaacac     3480
   atcatggagg acctggacac aaatgcagac aagcagctga gcttcgagga gttcatcatg     3540
   ctgatggcga ggctaacctg ggcctcccac gagaagatgc acgagggtga cgagggccct     3600
   ggccaccacc ataagccagg cctcggggag ggcaccccct aagaccacag tggccaagat     3660
   cacagtggcc acggccacgg ccacagtcat ggtggccacg gccacaggcc actaatcagg     3720
   aggccaggcc accctgcctc tacccaacca gggccccggg gctgttatgt caaactgtct     3780
   tggctgtggg gctaggggct ggggcaaata agtctcttcc tccaagtcag tgctctgtgt     3840
   gcttcttcca cctcttctcc aaccctgcct tcccagggct ctggcattta gacagccctg     3900
   tccttatctg tgactcagcc ccctcattca gtattaacaa aatgagaagc agcaaaacat     3960
   gggtctgtgc tgggcccctt ggctcacctc cctgaccatg tcctcacctc tgacttcagg     4020
   ccccactgtt cagatcccag gctccctgcc ccatctcaga caccctgtcc agcctgtcca     4080
   gcctgacaaa tggcccttgt cactgtacac tgtagaaagc aaaaaggcat atctctaccc     4140
   cttgatatgc ctgctacctc accaaccagc cccaagcctg tcttcaccca tcactgtcta     4200
   cacagccctc tctctctcct aacagaattc tattcctctg aaagtcttca gaaactggac     4260
   ctagatagtg ccatgtctgg ggaggaatat ggcaccaggc agtggaaaca aggacagatc     4320
   ggtgtgttat ctcacatttg atcagagagc atgatctctc ttaacagacc tgccacccta     4380
   atcaacggga gtgctcacac aagtgggagt ctgagagctt agccctatgc ccaccctgg      4439
<210> 9
   <211> 764
   <212> PRT
   <213> Homo sapiens
<300>
   <308> P01833
   <309> 2004-06-15
   <313> (1)..(764)
<400> 9
   Met Leu Leu Phe Val Leu Thr Cys Leu Leu Ala Val Phe Pro Ala Ile
   1               5                   10                  15
   Ser Thr Lys Ser Pro Ile Phe Gly Pro Glu Glu Val Asn Ser Val Glu
   20 25 30
   Gly Asn Ser Val Ser Ile Thr Cys Tyr Tyr Pro Pro Thr Ser Val Asn
   35 40 45
   Arg His Thr Arg Lys Tyr Trp Cys Arg Gln Gly Ala Arg Gly Gly Cys
   50 55 60
   Ile Thr Leu Ile Ser Ser Glu Gly Tyr Val Ser Ser Lys Tyr Ala Gly
   65 70 75 80
   Arg Ala Asn Leu Thr Asn Phe Pro Glu Asn Gly Thr Phe Val Val Asn
   85 90 95
   Ile Ala Gln Leu Ser Gln Asp Asp Ser Gly Arg Tyr Lys Cys Gly Leu
   100 105 110
   Gly Ile Asn Ser Arg Gly Leu Ser Phe Asp Val Ser Leu Glu Val Ser
   115 120 125
   Gln Gly Pro Gly Leu Leu Asn Asp Thr Lys Val Tyr Thr Val Asp Leu
   130 135 140
   Gly Arg Thr Val Thr Ile Asn Cys Pro Phe Lys Thr Glu Asn Ala Gln
   145 150 155 160
   Lys Arg Lys Ser Leu Tyr Lys Gln Ile Gly Leu Tyr Pro Val Leu Val
   165 170 175
   Ile Asp Ser Ser Gly Tyr Val Asn Pro Asn Tyr Thr Gly Arg Ile Arg
   180 185 190
   Leu Asp Ile Gln Gly Thr Gly Gln Leu Leu Phe Ser Val Val Ile Asn
   195 200 205
   Gln Leu Arg Leu Ser Asp Ala Gly Gln Tyr Leu Cys Gln Ala Gly Asp
   210 215 220
   Asp Ser Asn Ser Asn Lys Lys Asn Ala Asp Leu Gln Val Leu Lys Pro
   225 230 235 240
   Glu Pro Glu Leu Val Tyr Glu Asp Leu Arg Gly Ser Val Thr Phe His
   245 250 255
   Cys Ala Leu Gly Pro Glu Val Ala Asn Val Ala Lys Phe Leu Cys Arg
   260 265 270
   Gln Ser Ser Gly Glu Asn Cys Asp Val Val Val Asn Thr Leu Gly Lys
   275 280 285
   Arg Ala Pro Ala Phe Glu Gly Arg Ile Leu Leu Asn Pro Gln Asp Lys
       290                 295                 300
   Asp Gly Ser Phe Ser Val Val Ile Thr Gly Leu Arg Lys Glu Asp Ala
   305                 310                 315                 320
   Gly Arg Tyr Leu Cys Gly Ala His Ser Asp Gly Gln Leu Gln Glu Gly
                   325                 330                 335
   Ser Pro Ile Gln Ala Trp Gln Leu Phe Val Asn Glu Glu Ser Thr Ile
               340                 345                 350
   Pro Arg Ser Pro Thr Val Val Lys Gly Val Ala Gly Ser Ser Val Ala
           355                 360                 365
   Val Leu Cys Pro Tyr Asn Arg Lys Glu Ser Lys Ser Ile Lys Tyr Trp
       370                 375                 380
   Cys Leu Trp Glu Gly Ala Gln Asn Gly Arg Cys Pro Leu Leu Val Asp
   385                 390                 395                 400
   Ser Glu Gly Trp Val Lys Ala Gln Tyr Glu Gly Arg Leu Ser Leu Leu
                   405                 410                 415
   Glu Glu Pro Gly Asn Gly Thr Phe Thr Val Ile Leu Asn Gln Leu Thr
               420                 425                 430
   Ser Arg Asp Ala Gly Phe Tyr Trp Cys Leu Thr Asn Gly Asp Thr Leu
           435                 440                 445
   Trp Arg Thr Thr Val Glu Ile Lys Ile Ile Glu Gly Glu Pro Asn Leu
       450                 455                 460
   Lys Val Pro Gly Asn Val Thr Ala Val Leu Gly Glu Thr Leu Lys Val
   465                 470                 475                 480
   Pro Cys His Phe Pro Cys Lys Phe Ser Ser Tyr Glu Lys Tyr Trp Cys
                   485                 490                 495
   Lys Trp Asn Asn Thr Gly Cys Gln Ala Leu Pro Ser Gln Asp Glu Gly
               500                 505                 510
   Pro Ser Lys Ala Phe Val Asn Cys Asp Glu Asn Ser Arg Leu Val Ser
           515                 520                 525
   Leu Thr Leu Asn Leu Val Thr Arg Ala Asp Glu Gly Trp Tyr Trp Cys
       530                 535                 540
   Gly Val Lys Gln Gly His Phe Tyr Gly Glu Thr Ala Ala Val Tyr Val
   545                 550                 555                 560
   Ala Val Glu Glu Arg Lys Ala Ala Gly Ser Arg Asp Val Ser Leu Ala
                   565                 570                 575
   Lys Ala Asp Ala Ala Pro Asp Glu Lys Val Leu Asp Ser Gly Phe Arg
   580 585 590
   Glu Ile Glu Asn Lys Ala Ile Gln Asp Pro Arg Leu Phe Ala Glu Glu
   595 600 605
   Lys Ala Val Ala Asp Thr Arg Asp Gln Ala Asp Gly Ser Arg Ala Ser
   610 615 620
   Val Asp Ser Gly Ser Ser Glu Glu Gln Gly Gly Ser Ser Arg Ala Leu
   625 630 635 640
   Val Ser Thr Leu Val Pro Leu Gly Leu Val Leu Ala Val Gly Ala Val
   645 650 655
   Ala Val Gly Val Ala Arg Ala Arg His Arg Lys Asn Val Asp Arg Val
   660 665 670
   Ser Ile Arg Ser Tyr Arg Thr Asp Ile Ser Met Ser Asp Phe Glu Asn
   675 680 685
   Ser Arg Glu Phe Gly Ala Asn Asp Asn Met Gly Ala Ser Ser Ile Thr
   690 695 700
   Gln Glu Thr Ser Leu Gly Gly Lys Glu Glu Phe Val Ala Thr Thr Glu
   705 710 715 720
   Ser Thr Thr Glu Thr Lys Glu Pro Lys Lys Ala Lys Arg Ser Ser Lys
   725 730 735
   Glu Glu Ala Glu Met Ala Tyr Lys Asp Phe Leu Leu Gln Ser Ser Thr
   740 745 750
   Val Ala Ala Glu Ala Gln Asp Gly Pro Gln Glu Ala
   755 760
<210> 10
   <211> 4266
   <212> DNA
   <213> Homo sapiens
<300>
   <308> NM_002644
   <309> 2004-10-27
   <313> (1)..(4266)
<400> 10
   agagtttcag ttttggcagc agcgtccagt gccctgccag tagctcctag agaggcaggg 60
   gttaccaact ggccagcagg ctgtgtccct gaagtcagat caacgggaga gaaggaagtg 120
   gctaaaacat tgcacaggag aagtcggcct gagtggtgcg gcgctcggga cccaccagca 180
   atgctgctct tcgtgctcac ctgcctgctg gcggtcttcc cagccatctc cacgaagagt 240
   cccatatttg gtcccgagga ggtgaatagt gtggaaggta actcagtgtc catcacgtgc 300
   tactacccac ccacctctgt caaccggcac acccggaagt actggtgccg gcagggagct 360
   agaggtggct gcataaccct catctcctcg gagggctacg tctccagcaa atatgcaggc 420
   agggctaacc tcaccaactt cccggagaac ggcacatttg tggtgaacat tgcccagctg 480
   agccaggatg actccgggcg ctacaagtgt ggcctgggca tcaatagccg aggcctgtcc 540
   tttgatgtca gcctggaggt cagccagggt cctgggctcc taaatgacac taaagtctac 600
   acagtggacc tgggcagaac ggtgaccatc aactgccctt tcaagactga gaatgctcaa 660
   aagaggaagt ccttgtacaa gcagataggc ctgtaccctg tgctggtcat cgactccagt 720
   ggttatgtaa atcccaacta tacaggaaga atacgccttg atattcaggg tactggccag 780
   ttactgttca gcgttgtcat caaccaactc aggctcagcg atgctgggca gtatctctgc 840
   caggctgggg atgattccaa tagtaataag aagaatgctg acctccaagt gctaaagccc 900
   gagcccgagc tggtttatga agacctgagg ggctcagtga ccttccactg tgccctgggc 960
   cctgaggtgg caaacgtggc caaatttctg tgccgacaga gcagtgggga aaactgtgac 1020
   gtggtcgtca acaccctggg gaagagggcc ccagcctttg agggcaggat cctgctcaac 1080
   ccccaggaca aggatggctc attcagtgtg gtgatcacag gcctgaggaa ggaggatgca 1140
   gggcgctacc tgtgtggagc ccattcggat ggtcagctgc aggaaggctc gcctatccag 1200
   gcctggcaac tcttcgtcaa tgaggagtcc acgattcccc gcagccccac tgtggtgaag 1260
   ggggtggcag gaggctctgt ggccgtgctc tgcccctaca accgtaagga aagcaaaagc 1320
   atcaagtact ggtgtctctg ggaaggggcc cagaatggcc gctgccccct gctggtggac 1380
   agcgaggggt gggttaaggc ccagtacgag ggccgcctct ccctgctgga ggagccaggc 1440
   aacggcacct tcactgtcat cctcaaccag ctcaccagcc gggacgccgg cttctactgg 1500
   tgtctgacca acggcgatac tctctggagg accaccgtgg agatcaagat tatcgaagga 1560
   gaaccaaacc tcaaggtacc agggaatgtc acggctgtgc tgggagagac tctcaaggtc 1620
   ccctgtcact ttccatgcaa attctcctcg tacgagaaat actggtgcaa gtggaataac 1680
   acgggctgcc aggccctgcc cagccaagac gaaggcccca gcaaggcctt cgtgaactgt 1740
   gacgagaaca gccggcttgt ctccctgacc ctgaacctgg tgaccagggc tgatgagggc 1800
   tggtactggt gtggagtgaa gcagggccac ttctatggag agactgcagc cgtctatgtg 1860
   gcagttgaag agaggaaggc agcggggtcc cgcgatgtca gcctagcgaa ggcagacgct 1920
   gctcctgatg agaaggtgct agactctggt tttcgggaga ttgagaacaa agccattcag 1980
   gatcccaggc tttttgcaga ggaaaaggcg gtggcagata caagagatca agccgatggg 2040
   agcagagcat ctgtggattc cggcagctct gaggaacaag gtggaagctc cagagcgctg 2100
   gtctccaccc tggtgcccct gggcctggtg ctggcagtgg gagccgtggc tgtgggggtg 2160
   gccagagccc ggcacaggaa gaacgtcgac cgagtttcaa tcagaagcta caggacagac 2220
   attagcatgt cagacttcga gaactccagg gaatttggag ccaatgacaa catgggagcc 2280
   tcttcgatca ctcaggagac atccctcgga ggaaaagaag agtttgttgc caccactgag 2340
   agcaccacag agaccaaaga acccaagaag gcaaaaaggt catccaagga ggaagccgag 2400
   atggcctaca aagacttcct gctccagtcc agcaccgtgg ccgccgaggc ccaggacggc   2460
   ccccaggaag cctagacggt gtcgccgcct gctccctgca cccatgacaa tcaccttcag   2520
   aatcatgtcg atcctggggc cctcagctcc tggggacccc actccctgct ctaacacctg   2580
   cctaggtttt tcctactgtc ctcagaggcg tgctggtccc ctcctcagtg acatcaaagc   2640
   ctggcctaat tgttcctatt ggggatgagg gtggcatgag gaggtcccac ttgcaacttc   2700
   tttctgttga gagaacctca ggtacggaga agaatagagg tcctcatggg tcccttgaag   2760
   gaagagggac cagggtggga gagctgattg cagaaaggag agacgtgcag cgcccctctg   2820
   cacccttatc atgggatgtc aacagaattt ttccctccac tccatccctc cctcccgtcc   2880
   ttcccctctt cttctttcct tccatcaaaa gatgtatttg aattcatact agaattcagg   2940
   tgctttgcta gatgctgtga caggtatgcc accaacactg ctcacagcct ttctgaggac   3000
   accagtgaaa gaagccacag ctcttcttgg cgtatttata ctcactgagt cttaactttt   3060
   caccaggggt gctcacctct gcccctattg ggagaggtca taaaatgtct cgagtcctaa   3120
   ggccttaggg gtcatgtatg atgagcatac acacaggtaa ttataaaccc acattcttac   3180
   catttcacac ataagaaaat tgaggtttgg aagagtgaag cgtttttctt tttctttttt   3240
   ttttttgaga cggagtctct cactgtcgcc caggctggag tgcagtggcg caatctcggc   3300
   tcactgcaac ctccgcctcc caggttgaca ccattctcct gcctcaccct cccaagtagc   3360
   tgggactaca ggcgcctgcc agcacgcctg gctaattttt tgtattttta gtagagacag   3420
   ggtttcaccg tgttagccag gatggtctcg atctcctgac ctcgtgatcc gcctgcctct   3480
   gcctcccaaa gtgctgggat tacaggcgtg agccaccgcg tccggcctct ttttttcttt   3540
   tctttttttt gagacaaagt ctcactgtgt cacccagact ggaatgcagt gacacaatct   3600
   cggctcactg aaacctctgc cttccaggtt caagctattc tcatgcctca gcctctcaag   3660
   tagctgggac tacagatgtg ggccaccatg tctggctaat tttttttttt tttttttttt   3720
   tttgtagaga cagggtttcg ccatgttgac gagactggtc tcgaactcct ggcctcaagt   3780
   gatctgccgc ctcagcttct caaagtactg ggattatata ggcatgagcc actgagcctg   3840
   gccctgaagc gtttttctca aaggccctca gtgagataaa ttagatttgg catctcctgt   3900
   cctgggccag ggatctctct acaagagccc ctgcccctct gttggaggca cagttttaga   3960
   ataaggagga ggagggagaa gagaaaatgt aaaggaggga gatctttccc aggccgcacc   4020
   atttctgtca ctcacatgga cccaagataa aagaatggcc aaaccctcac aacccctgat   4080
   gtttgaagag ttccaagttg aagggaaaca aagaagtgtt tgatggtgcc agagaggggc   4140
   tgctctccag aaagctaaaa tttaatttct tttttcctct gagttctgta cttcaaccag   4200
   cctacaagct ggcacttgct aacaaatcag aaatatgaca attaatgatt aaagactgtg   4260
   attgcc                                                              4266
<210> 11
   <211> 187
   <212> PRT
   <213> Homo sapiens
<300>
   <308> P30086
   <309> 2004-10-25
   <313> (1)..(187)
<400> 11
   Met Pro Val Asp Leu Ser Lys Trp Ser Gly Pro Leu Ser Leu Gln Glu
   1               5                   10                  15
   Val Asp Glu Gln Pro Gln His Pro Leu His Val Thr Tyr Ala Gly Ala
               20                  25                  30
   Ala Val Asp Glu Leu Gly Lys Val Leu Thr Pro Thr Gln Val Lys Asn
           35                  40                  45
   Arg Pro Thr Ser Ile Ser Trp Asp Gly Leu Asp Ser Gly Lys Leu Tyr
       50                  55                  60
   Thr Leu Val Leu Thr Asp Pro Asp Ala Pro Ser Arg Lys Asp Pro Lys
   65                  70                  75                  80
   Tyr Arg Glu Trp His His Phe Leu Val Val Asn Met Lys Gly Asn Asp
                   85                  90                  95
   Ile Ser Ser Gly Thr Val Leu Ser Asp Tyr Val Gly Ser Gly Pro Pro
               100                 105                 110
   Lys Gly Thr Gly Leu His Arg Tyr Val Trp Leu Val Tyr Glu Gln Asp
           115                 120                 125
   Arg Pro Leu Lys Cys Asp Glu Pro Ile Leu Ser Asn Arg Ser Gly Asp
       130                 135                 140
   His Arg Gly Lys Phe Lys Val Ala Ser Phe Arg Lys Lys Tyr Glu Leu
   145                 150                 155                 160
   Arg Ala Pro Val Ala Gly Thr Cys Tyr Gln Ala Glu Trp Asp Asp Tyr
                   165                 170                 175
   Val Pro Lys Leu Tyr Glu Gln Leu Ser Gly Lys
               180                 185
<210> 12
   <211> 1507
   <212> DNA
   <213> Homo sapiens
<300>
   <308> NM_002567
   <309> 2004-10-27
   <313> (1)..(1507)
<400> 12
   tgggcggcgg ctgaggcgcg tgctctcgcg tggtcgctgg gtctgcgtct tcccgagcca      60
   gtgtgctgag ctctccgcgt cgcctctgtc gcccgcgcct ggcctaccgc ggcactcccg     120
   gctgcacgct ctgcttggcc tcgccatgcc ggtggacctc agcaagtggt ccgggccctt     180
   gagcctgcaa gaagtggacg agcagccgca gcacccgctg catgtcacct acgccggggc     240
   ggcggtggac gagctgggca aagtgctgac gcccacccag gttaagaata gacccaccag     300
   catttcgtgg gatggtcttg attcagggaa gctctacacc ttggtcctga cagacccgga     360
   tgctcccagc aggaaggatc ccaaatacag agaatggcat catttcctgg tggtcaacat     420
   gaagggcaat gacatcagca gtggcacagt cctctccgat tatgtgggct cggggcctcc     480
   caagggcaca ggcctccacc gctatgtctg gctggtttac gagcaggaca ggccgctaaa     540
   gtgtgacgag cccatcctca gcaaccgatc tggagaccac cgtggcaaat tcaaggtggc     600
   gtccttccgt aaaaagtatg agctcagggc cccggtggct ggcacgtgtt accaggccga     660
   gtgggatgac tatgtgccca aactgtacga gcagctgtct gggaagtagg gggttagctt     720
   ggggacctga actgtcctgg aggccccaag ccatgttccc cagttcagtg ttgcatgtat     780
   aatagatttc tcctcttcct gccccccttg gcatgggtga gacctgacca gtcagatggt     840
   agttgagggt gacttttcct gctgcctggc ctttataatt ttactcactc actctgattt     900
   atgttttgat caaatttgaa cttcattttg gggggtattt tggtactgtg atggggtcat     960
   caaattatta atctgaaaat agcaacccag aatgtaaaaa agaaaaaact ggggggaaaa    1020
   agaccaggtc tacagtgata gagcaaagca tcaaagaatc tttaagggag gtttaaaaaa    1080
   aaaaaaaaaa aaaaagattg gttgcctctg cctttgtgat cctgagtcca gaatggtaca    1140
   caatgtgatt ttatggtgat gtcactcacc tagacaacca gaggctggca ttgaggctaa    1200
   cctccaacac agtgcatctc agatgcctca gtaggcatca gtatgtcact ctggtccctt    1260
   taaagagcaa tcctggaaga agcaggaggg agggtggctt tgctgttgtt gggacatggc    1320
   aatctagacc ggtagcagcg ctcgctgaca gcttgggagg aaacctgaga tctgtgtttt    1380
   ttaaattgat cgttcttcat gggggtaaga aaagctggtc tggagttgct gaatgttgca    1440
   ttaattgtgc tgtttgcttg tagttgaata aaaatagaaa cctgaatgaa gaaaaaaaaa    1500
   aaaaaaa                                                              1507
<210> 13
   <211> 249
   <212> PRT
   <213> Homo sapiens
<300>
   <308> P39687
   <309> 2004-06-15
   <313> (1)..(249)
<400> 13
   Met Glu Met Gly Arg Arg Ile His Leu Glu Leu Arg Asn Arg Thr Pro
   1               5                   10                  15
   Ser Asp Val Lys Glu Leu Val Leu Asp Asn Ser Arg Ser Asn Glu Gly
               20                  25                  30
   Lys Leu Glu Gly Leu Thr Asp Glu Phe Glu Glu Leu Glu Phe Leu Ser
           35                  40                  45
   Thr Ile Asn Val Gly Leu Thr Ser Ile Ala Asn Leu Pro Lys Leu Asn
       50                  55                  60
   Lys Leu Lys Lys Leu Glu Leu Ser Asp Asn Arg Val Ser Gly Gly Leu
   65                  70                  75                  80
   Glu Val Leu Ala Glu Lys Cys Pro Asn Leu Thr His Leu Asn Leu Ser
                   85                  90                  95
   Gly Asn Lys Ile Lys Asp Leu Ser Thr Ile Glu Pro Leu Lys Lys Leu
               100                 105                 110
   Glu Asn Leu Lys Ser Leu Asp Leu Phe Asn Cys Glu Val Thr Asn Leu
           115                 120                 125
   Asn Asp Tyr Arg Glu Asn Val Phe Lys Leu Leu Pro Gln Leu Thr Tyr
       130                 135                 140
   Leu Asp Gly Tyr Asp Arg Asp Asp Lys Glu Ala Pro Asp Ser Asp Ala
   145                 150                 155                 160
   Glu Gly Tyr Val Glu Gly Leu Asp Asp Glu Glu Glu Asp Glu Asp Glu
                   165                 170                 175
   Glu Glu Tyr Asp Glu Asp Ala Gln Val Val Glu Asp Glu Glu Asp Glu
               180                 185                 190
   Asp Glu Glu Glu Glu Gly Glu Glu Glu Asp Val Ser Gly Glu Glu Glu
           195                 200                 205
   Glu Asp Glu Glu Gly Tyr Asn Asp Gly Glu Val Asp Asp Glu Glu Asp
       210                 215                 220
   Glu Glu Glu Leu Gly Glu Glu Glu Arg Gly Gln Lys Arg Lys Arg Glu
   225                 230                 235                 240
   Pro Glu Asp Glu Gly Glu Asp Asp Asp
                   245
<210> 14
   <211> 1136
   <212> DNA
   <213> Homo sapiens
<300>
   <308> NM_006305
   <309> 2004-10-27
   <313> (1)..(1136)
<400> 14
   cgggtgctgg gggctcgaga accgagcgga gctggttgag ccttcaaagt cctaaaacgc     60
   gcggccgtgg gttcggggtt tattgattga attccgccgg cgcgggagcc tctgcagaga    120
   gagagcgcga gagatggaga tgggcagacg gattcattta gagctgcgga acaggacgcc    180
   ctctgatgtg aaagaacttg tcctggacaa cagtcggtcg aatgaaggca aactcgaagg    240
   cctcacagat gaatttgaag aactggaatt cttaagtaca atcaacgtag gcctcacctc    300
   aatcgcaaac ttaccaaagt taaacaaact taagaagctt gaactaagcg ataacagagt    360
   ctcagggggc ctggaagtat tggcagaaaa gtgtccgaac ctcacgcatc taaatttaag    420
   tggcaacaaa attaaagacc tcagcacaat agagccactg aaaaagttag aaaacctcaa    480
   gagcttagac cttttcaatt gcgaggtaac caacctgaac gactaccgag aaaatgtgtt    540
   caagctcctc ccgcaactca catatctcga cggctatgac cgggacgaca aggaggcccc    600
   tgactcggat gctgagggct acgtggaggg cctggatgat gaggaggagg atgaggatga    660
   ggaggagtat gatgaagatg ctcaggtagt ggaagacgag gaggacgagg atgaggagga    720
   ggaaggtgaa gaggaggacg tgagtggaga ggaggaggag gatgaagaag gttataacga    780
   tggagaggta gatgacgagg aagatgaaga agagcttggt gaagaagaaa ggggtcagaa    840
   gcgaaaacga gaacctgaag atgagggaga agatgatgac taagtggaat aacctatttt    900
   gaaaaattcc tattgtgatt tgactgtttt tacccatatc ccctctcccc cccccctcca     960
   atcctgcccc ctgaaactta tttttttctg attgtaacgt tgctgtggga acgagagggg   1020
   aagagtgtac tgggggttgc ggggggaggg atggcgggtg ggggtggaat aaaatactat    1080
   ttttactgcc actctttaaa aaaaaaaaaa aaaaaaaaaa aaaaaaaaaa aaaaaa       1136
<210> 15
   <211> 643
   <212> PRT
   <213> Homo sapiens
<300>
   <308> P17066
   <309> 2003-09-15
   <313> (1)..(643)
<400> 15
   Met Gln Ala Pro Arg Glu Leu Ala Val Gly Ile Asp Leu Gly Thr Thr
   1               5                   10                  15
   Tyr Ser Cys Val Gly Val Phe Gln Gln Gly Arg Val Glu Ile Leu Ala
               20                  25                  30
   Asn Asp Gln Gly Asn Arg Thr Thr Pro Ser Tyr Val Ala Phe Thr Asp
           35                  40                  45
   Thr Glu Arg Leu Val Gly Asp Ala Ala Lys Ser Gln Ala Ala Leu Asn
       50                  55                  60
   Pro His Asn Thr Val Phe Asp Ala Lys Arg Leu Ile Gly Arg Lys Phe
   65                  70                  75                  80
   Ala Asp Thr Thr Val Gln Ser Asp Met Lys His Trp Pro Phe Arg Val
                   85                  90                  95
   Val Ser Glu Gly Gly Lys Pro Lys Val Arg Val Cys Tyr Arg Gly Glu
   100 105 110
   Asp Lys Thr Phe Tyr Pro Glu Glu Ile Ser Ser Met Val Leu Ser Lys
   115 120 125
   Met Lys Glu Thr Ala Glu Ala Tyr Leu Gly Gln Pro Val Lys His Ala
   130 135 140
   Val Ile Thr Val Pro Ala Tyr Phe Asn Asp Ser Gln Arg Gln Ala Thr
   145 150 155 160
   Lys Asp Ala Gly Ala Ile Ala Gly Leu Asn Val Leu Arg Ile Ile Asn
   165 170 175
   Glu Pro Thr Ala Ala Ala Ile Ala Tyr Gly Leu Asp Arg Arg Gly Ala
   180 185 190
   Gly Glu Arg Asn Val Leu Ile Phe Asp Leu Gly Gly Gly Thr Phe Asp
   195 200 205
   Val Ser Val Leu Ser Ile Asp Ala Gly Val Phe Glu Val Lys Ala Thr
   210 215 220
   Ala Gly Asp Thr His Leu Gly Gly Glu Asp Phe Asp Asn Arg Leu Val
   225 230 235 240
   Asn His Phe Met Glu Glu Phe Arg Arg Lys His Gly Lys Asp Leu Ser
   245 250 255
   Gly Asn Lys Arg Ala Leu Arg Arg Leu Arg Thr Ala Cys Glu Arg Ala
   260 265 270
   Lys Arg Thr Leu Ser Ser Ser Thr Gln Ala Thr Leu Glu Ile Asp Ser
   275 280 285
   Leu Phe Glu Gly Val Asp Phe Tyr Thr Ser Ile Thr Arg Ala Arg Phe
   290 295 300
   Glu Glu Leu Cys Ser Asp Leu Phe Arg Ser Thr Leu Glu Pro Val Glu
   305 310 315 320
   Lys Ala Leu Arg Asp Ala Lys Leu Asp Lys Ala Gln Ile His Asp Val
   325 330 335
   Val Leu Val Gly Gly Ser Thr Arg Ile Pro Lys Val Gln Lys Leu Leu
   340 345 350
   Gln Asp Phe Phe Asn Gly Lys Glu Leu Asn Lys Ser Ile Asn Pro Asp
   355 360 365
   Glu Ala Val Ala Tyr Gly Ala Ala Val Gln Ala Ala Val Leu Met Gly
       370                 375                 380
   Asp Lys Cys Glu Lys Val Gln Asp Leu Leu Leu Leu Asp Val Ala Pro
   385                 390                 395                 400
   Leu Ser Leu Gly Leu Glu Thr Ala Gly Gly Val Met Thr Thr Leu Ile
                   405                 410                 415
   Gln Arg Asn Ala Thr Ile Pro Thr Lys Gln Thr Gln Thr Phe Thr Thr
               420                 425                 430
   Tyr Ser Asp Asn Gln Pro Gly Val Phe Ile Gln Val Tyr Glu Gly Glu
           435                 440                 445
   Arg Ala Met Thr Lys Asp Asn Asn Leu Leu Gly Arg Phe Glu Leu Ser
       450                 455                 460
   Gly Ile Pro Pro Ala Pro Arg Gly Val Pro Gln Ile Glu Val Thr Phe
   465                 470                 475                 480
   Asp Ile Asp Ala Asn Gly Ile Leu Ser Val Thr Ala Thr Asp Arg Ser
                   485                 490                 495
   Thr Gly Lys Ala Asn Lys Ile Thr Ile Thr Asn Asp Lys Gly Arg Leu
               500                 505                 510
   Ser Lys Glu Glu Val Glu Arg Met Val His Glu Ala Glu Gln Tyr Lys
           515                 520                 525
   Ala Glu Asp Glu Ala Gln Arg Asp Arg Val Ala Ala Lys Asn Ser Leu
       530                 535                 540
   Glu Ala His Val Phe His Val Lys Gly Ser Leu Gln Glu Glu Ser Leu
   545                 550                 555                 560
   Arg Asp Lys Ile Pro Glu Glu Asp Arg Arg Lys Met Gln Asp Lys Cys
                   565                 570                 575
   Arg Glu Val Leu Ala Trp Leu Glu His Asn Gln Leu Ala Glu Lys Glu
               580                 585                 590
   Glu Tyr Glu His Gln Lys Arg Glu Leu Glu Gln Ile Cys Arg Pro Ile
           595                 600                 605
   Phe Ser Arg Leu Tyr Gly Gly Pro Gly Val Pro Gly Gly Ser Ser Cys
       610                 615                 620
   Gly Thr Gln Ala Arg Gln Gly Asp Pro Ser Thr Gly Pro Ile Ile Glu
   625                 630                 635                 640
   Glu Val Asp
<210> 16
   <211> 2664
   <212> DNA
   <213> Homo sapiens
<300>
   <308> NMR_002155
   <309> 2004-10-28
   <313> (1)..(2664)
<400> 16
   agagccagcc cggaggagct agaaccttcc ccgcatttct ttcagcagcc tgagtcagag     60
   gcgggctggc ctggcgtagc cgcccagcct cgcggctcat gccccgatct gcccgaacct    120
   tctcccgggg tcagcgccgc gccgcgccac ccggctgagt cagcccgggc gggcgagagg    180
   ctctcaactg ggcgggaagg tgcgggaagg tgcggaaagg ttcgcgaaag ttcgcggcgg    240
   cgggggtcgg gtgaggcgca aaaggataaa aagcccgtgg aagcggagct gagcagatcc    300
   gagccgggct ggctgcagag aaaccgcagg gagagcctca ctgctgagcg cccctcgacg    360
   gcggagcggc agcagcctcc gtggcctcca gcatccgaca agaagcttca gccatgcagg    420
   ccccacggga gctcgcggtg ggcatcgacc tgggcaccac ctactcgtgc gtgggcgtgt    480
   ttcagcaggg ccgcgtggag atcctggcca acgaccaggg caaccgcacc acgcccagct    540
   acgtggcctt caccgacacc gagcggctgg tcggggacgc ggccaagagc caggcggccc    600
   tgaaccccca caacaccgtg ttcgatgcca agcggctgat cgggcgcaag ttcgcggaca      660
   ccacggtgca gtcggacatg aagcactggc ccttccgggt ggtgagcgag ggcggcaagc    720
   ccaaggtgcg cgtatgctac cgcggggagg acaagacgtt ctaccccgag gagatctcgt    780
   ccatggtgct gagcaagatg aaggagacgg ccgaggcgta cctgggccag cccgtgaagc    840
   acgcagtgat caccgtgccc gcctatttca atgactcgca gcgccaggcc accaaggacg    900
   cgggggccat cgcggggctc aacgtgttgc ggatcatcaa tgagcccacg gcagctgcca    960
   tcgcctatgg gctggaccgg cggggcgcgg gagagcgcaa cgtgctcatt tttgacctgg   1020
   gtgggggcac cttcgatgtg tcggttctct ccattgacgc tggtgtcttt gaggtgaaag   1080
   ccactgctgg agatacccac ctgggaggag aggacttcga caaccggctc gtgaaccact   1140
   tcatggaaga attccggcgg aagcatggga aggacctgag cgggaacaag cgtgccctgc   1200
   gcaggctgcg cacagcctgt gagcgcgcca agcgcaccct gtcctccagc acccaggcca   1260
   ccctggagat agactccctg ttcgagggcg tggacttcta cacgtccatc actcgtgccc   1320
   gctttgagga actgtgctca gacctcttcc gcagcaccct ggagccggtg gagaaggccc   1380
   tgcgggatgc caagctggac aaggcccaga ttcatgacgt cgtcctggtg gggggctcca   1440
   ctcgcatccc caaggtgcag aagttgctgc aggacttctt caacggcaag gagctgaaca   1500
   agagcatcaa ccctgatgag gctgtggcct atggggctgc tgtgcaggcg gccgtgttga   1560
   tgggggacaa atgtgagaaa gtgcaggatc tcctgctgct ggatgtggct cccctgtctc   1620
   tggggctgga gacagcaggt ggggtgatga ccacgctgat ccagaggaac gccactatcc   1680
   ccaccaagca gacccagact ttcaccacct actcggacaa ccagcctggg gtcttcatcc   1740
   aggtgtatga gggtgagagg gccatgacca aggacaacaa cctgctgggg cgttttgaac 1800
   tcagtggcat ccctcctgcc ccacgtggag tcccccagat agaggtgacc tttgacattg 1860
   atgctaatgg catcctgagc gtgacagcca ctgacaggag cacaggtaag gctaacaaga 1920
   tcaccatcac caatgacaag ggccggctga gcaaggagga ggtggagagg atggttcatg 1980
   aagccgagca gtacaaggct gaggatgagg cccagaggga cagagtggct gccaaaaact 2040
   cgctggaggc ccatgtcttc catgtgaaag gttctttgca agaggaaagc cttagggaca 2100
   agattcccga agaggacagg cgcaaaatgc aagacaagtg tcgggaagtc cttgcctggc 2160
   tggagcacaa ccagctggca gagaaggagg agtatgagca tcagaagagg gagctggagc 2220
   aaatctgtcg ccccatcttc tccaggctct atggggggcc tggtgtccct gggggcagca 2280
   gttgtggcac tcaagcccgc cagggggacc ccagcaccgg ccccatcatt gaggaggttg 2340
   attgaatggc ccttcgtgat aagtcagctg tgactgtcag ggctatgcta tgggccttct 2400
   agactgtctt ctatgatcct gcccttcaga gatgaacttt ccctccaaag ctagaacttt 2460
   cttcccagga taactgaagt cttttgactt tttgggggga gggcggttca tcctcttctg 2520
   cttcaaataa aaagtcatta atttattaaa acttgtgtgg cactttaaca ttgctttcac 2580
   ctatattttg tgtactttgt tacttgcatg tatgaatttt gttatgtaaa atatagttat 2640
   agacctaaat aaaaaaaaaa aaaa 2664
<210> 17
   <211> 2492
   <212> DNA
   <213> Homo sapiens
<300>
   <308> X51757
   <309> 1998-11-13
   <313> (1)..(2492)
<400> 17
   cccgggcggg cgagaggctc tcaactgggc gggaaggtgc gggaaggtgc ggaaaggttc     60
   gcgaaagttc gcggcggcgg gggtcgggtg aggcgcaaaa ggataaaaag cccgtggaag    120
   cggagctgag cagatccgag ccgggctggc tgcagagaca ccgcagggag agcctcactg    180
   ctgagcgccc ctcgacggcg gacgggcagc agcctccgtg gcctccagca tccgacaaga    240
   agcttcagcc atgcaggccc cacgggagct cgcggtgggc atcgacctgg gcaccaccta    300
   ctcgtgcgtg ggcgtgtttc agcagggccg cgtggagatc ctggccaacg accagggcaa    360
   ccgcaccacg cccagctacg tggccttcac cgacaccgag cggctggtcg gggacgcggc    420
   caagagccag gcggccctga acccccacaa caccgtgttc gatgccaagc ggctgatcgg    480
   gcgcaagttc gcggacacca cggtgcagtc ggacatgaag cactggccct tccgggtggt    540
   gagcgagggc ggcaagccca aggtgccggt atcgtaccgc ggggaggaca agacgttcta    600
   ccccgaggag atctcgtcca tggtgctgag caagatgaag gagacggccg aggcgtacct    660
   gggccagccc gtgaagcacg cagtgatcac cgtgcccgcc tatttcaatg actcgcagcg    720
   ccaggccacc aaggacgcgg gggccatcgc ggggctcaac gtgttgcgga tcatcaatga    780
   gcccacggca gctgccatcg cctatgggct ggaccggcgg ggcgcgggag agcgcaacgt 840
   gctcattttt gacctgggtg ggggcacctt cgatgtgtcg gttctctcca ttgacgctgg 900
   tgtctttgag gtgaaagcca ctgctggaga tacccacctg ggaggagagg acttcgacaa 960
   ccggctcgtg aaccacttca tggaagaatt ccggcggaag catgggaagg acctgagcgg 1020
   gaacaagcgt gccctcggca ggctgcgcac agcctgtgag cgcgccaagc gcaccctgtc 1080
   ctccagcacc caggccaccc tggagataga ctccctgttc gagggcgtgg acttctacac 1140
   gtccatcact cgtgcccgct ttgaggaact gtgctcagac ctcttccgca gcaccctgga 1200
   gccggtggag aaggccctgc gggatgccaa gctggacaag gcccagattc atgacgtcgt 1260
   cctggtgggg ggctccactc gcatccccaa ggtgcagaag ttgctgcagg acttcttcaa 1320
   cggcaaggag ctgaacaaga gcatcaaccc tgatgaggct gtggcctatg gggctgctgt 1380
   gcaggcggcc gtgttgatgg gggacaaatg tgagaaagtg caggatctcc tgctgctgga 1440
   tgtggctccc ctgtctctgg ggctggagac agcaggtggg gtgatgacca cgctgatcca 1500
   gaggaacgcc actatcccca ccaagcagac ccagactttc accacctact cggacaacca 1560
   gcctggggtc ttcatccagg tgtatgaggg tgagagggcc atgaccaagg acaacaacct 1620
   gctggggcgt tttgaactca gtggcatccc tcctgcccca cgtggagtcc cccagataga 1680
   ggtgaccttt gacattgatg ctaatggcat cctgagcgtg acagccactg acaggagcac 1740
   aggtaaggct aacaagatca ccatcaccaa tgacaagggc cggctgagca aggaggaggt 1800
   ggagaggatg gttcatgaag ccgagcagta caaggctgag gatgaggccc agagggacag 1860
   agtggctgcc aaaaactcgc tggaggccca tgtcttccat gtgaaaggtt ctttgcaaga 1920
   ggaaagcctt agggacaaga ttcccgaaga ggacaggcgc aaaatgcaag acaagtgtcg 1980
   ggaagtcctt gcctggctgg agcacaacca gctggcagag aaggaggagt atgagcatca 2040
   gaagagggag ctggagcaaa tctgtcgccc catcttctcc aggctctatg gggggcctgg 2100
   tgtccctggg ggcagcagtt gtggcactca agcccgccag ggggacccca gcaccggccc 2160
   catcattgag gaggttgatt gaatggccct tcgtgataag tcagctgtga ctgtcagggc 2220
   tatgctatgg gccttctaga ctgtcttcta tgatcctgcc cttcagagat gaactttccc 2280
   tccaaagcta gaactttctt cccaggataa ctgaagtctt ttgacttttt gcggggaggg 2340
   cggttcatcc tcttctgctt caaataaaaa gtcattaatt tattaaaact tgtgtggcac 2400
   tttaacattg ctttcaccta tattttgtgt actttgttac ttgcatgtat gaattttgtt 2460
   atgtaaaata tagttataga cctaaataag ct 2492
<210> 18
   <211> 115
   <212> PRT
   <213> Homo sapiens
<300>
   <308> P14174
   <309> 2005-01-25
   <313> (1)..(115)
<400> 18
   Met Pro Met Phe Ile Val Asn Thr Asn Val Pro Arg Ala Ser Val Pro
   1               5                   10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 19
   <211> 561
   <212> DNA
   <213> Homo sapiens
<300>
   <308> NM_002415
   <309> 2004-10-26
   <313> (1)..(561)
<400> 19
   accacagtgg tgtccgagaa gtcaggcacg tagctcagcg gcggccgcgg cgcgtgcgtc      60
   tgtgcctctg cgcgggtctc ctggtccttc tgccatcatg ccgatgttca tcgtaaacac     120
   caacgtgccc cgcgcctccg tgccggacgg gttcctctcc gagctcaccc agcagctggc     180
   gcaggccacc ggcaagcccc cccagtacat cgcggtgcac gtggtcccgg accagctcat     240
   ggccttcggc ggctccagcg agccgtgcgc gctctgcagc ctgcacagca tcggcaagat     300
   cggcggcgcg cagaaccgct cctacagcaa gctgctgtgc ggcctgctgg ccgagcgcct     360
   gcgcatcagc ccggacaggg tctacatcaa ctattacgac atgaacgcgg ccaatgtggg     420
   ctggaacaac tccaccttcg cctaagagcc gcagggaccc acgctgtctg cgctggctcc     480
   acccgggaac ccgccgcacg ctgtgttcta ggcccgccca ccccaacctt ctggtgggga     540
   gaaataaacg gtttagagac t                                               561
<210> 20
   <211> 2167
   <212> DNA
   <213> Homo sapiens
<300>
   <308> HUMMIF
   <309> 1994-09-29
   <313> (1)..(2167)
<400> 20
   ctgcaggaac caatacccat aggctatttg tataaatggg ccatggggcc tcccagctgg 60
   aggctggctg gtgccacgag ggtcccacag gcatgggtgt ccttcctata tcacatggcc 120
   ttcactgaga ctggtatatg gattgcacct atcagagacc aaggacagga cctccctgga 180
   aatctctgag gacctggcct gtgatccagt tgctgccttg tcctcttcct gctatgtcat 240
   ggcttatctt ctttcaccca ttcattcatt cattcattca ttcagcagta ttagtcaatg 300
   tctcttgata tgcctggcac ctgctagatg gtccccgagt ttaccattag tggaaaagac 360
   atttaagaaa ttcaccaagg gctctatgag aggccataca cggtggacct gactagggtg 420
   tggcttccct gaggagctga agttgcccag aggcccagag aaggggagct gagcacgttt 480
   gaaccactga acctgctctg gacctcgcct ccttccttcg gtgcctccca gcatcctatc 540
   ctctttaaag agcaggggtt cagggaagtt ccctggatgg tgattcgcag gggcagctcc 600
   cctctcacct gccgcatgac taccccgccc catctcaaac acacaagctc acgcatgcgg 660
   gactggagcc cttgaggaca tgtggcccaa agacaggagg tacaggggct cagtgcgtgc 720
   agtggaatga actgggcttc atctctggaa gggtaagggg ccatcttccg ggttcaccgc 780
   cgcatcccca cccccggcac agcgcctcct ggcgactaac atcggtgact tagtgaaagg 840
   actaagaaag acccgaggcg aggccggaac aggccgattt ctagccgcca agtggagaac 900
   aggttggagc ggtgcgccgg gcttagcggc ggttgctgga ggaacgggcg gagtcgccca 960
   gggtcctgcc ctgcgggggt cgagccgagg caggcggtga cttccccact cggggcggag 1020
   ccgcagcctc gcgggggcgg ggcctggcgc cggcggtggc gtcacaaaag gcgggaccac 1080
   agtggtgtcc gagaagtcag gcacgtagct cagcggcggc cgcggcgcgt gcgtctgtgc 1140
   ctctgcgcgg gtctcctggt ccttctgcca tcatgccgat gttcatcgta aacaccaacg 1200
   tgccccgcgc ctccgtgccg gacgggttcc tctccgagct cacccagcag ctggcgcagg 1260
   ccaccggcaa gcccccccag gtttgccggg aggggacagg aagagggggg tgcccaccgg 1320
   acgaggggtt ccgcgctggg agctggggag gcgactcctg aacggagctg gggggcgggg 1380
   cggggggagg acggtggctc gggcccgaag tggacgttcg gggcccgacg aggtcgctgg 1440
   ggcgggctga ccgcgccctt tcctcgcagt acatcgcggt gcacgtggtc ccggaccagc 1500
   tcatggcctt cggcggctcc agcgagccgt gcgcgctctg cagcctgcac agcatcggca 1560
   agatcggcgg cgcgcagaac cgctcctaca gcaagctgct gtgcggcctg ctggccgagc 1620
   gcctgcgcat cagcccggac aggtacgcgg agtcgcggag gggcggggga ggggcggcgg 1680
   cgcgcggcca ggcccgggac tgagccaccc gctgagtccg gcctcctccc cccgcagggt 1740
   ctacatcaac tattacgaca tgaacgcggc caatgtgggc tggaacaact ccaccttcgc 1800
   ctaagagccg cagggaccca cgctgtctgc gctggctcca cccgggaacc cgccgcacgc 1860
   tgtgttctag gcccgcccac cccaaccttc tggtggggag aaataaacgg tttagagact 1920
   aggagtgcct cggggttcct tggcttgcgg gaggaattgg tgcagagccg ggacattggg 1980
   gagcgaggtc gggaaacggt gttgggggcg ggggtcaggg ccgggttgct ctcctcgaac 2040
   ctgctgttcg ggagcccttt tgtccagcct gtccctccta cgctcctaac agaggagccc 2100
   cagtgtcttt ccattctatg gcgtacgaag ggatgaggag aagttggcac tctgccctgg 2160
   gctgcag 2167
<210> 21
   <211> 105
   <212> PRT
   <213> Homo sapiens
<300>
   <308> P31949
   <309> 2004-10-25
   <313> (1)..(105)
<400> 21
   Met Ala Lys Ile Ser Ser Pro Thr Glu Thr Glu Arg Cys Ile Glu Ser
   1 5 10 15
   Leu Ile Ala Val Phe Gln Lys Tyr Ala Gly Lys Asp Gly Tyr Asn Tyr
   20 25 30
   Thr Leu Ser Lys Thr Glu Phe Leu Ser Phe Met Asn Thr Glu Leu Ala
   35 40 45
   Ala Phe Thr Lys Asn Gln Lys Asp Pro Gly Val Leu Asp Arg Met Met
   50 55 60
   Lys Lys Leu Asp Thr Asn Ser Asp Gly Gln Leu Asp Phe Ser Glu Phe
   65 70 75 80
   Leu Asn Leu Ile Gly Gly Leu Ala Met Ala Cys His Asp Ser Phe Leu
   85 90 95
   Lys Ala Val Pro Ser Gln Lys Arg Thr
   100 105
<210> 22
   <211> 595
   <212> DNA
   <213> Homo sapiens
<300>
   <308> NM_005620
   <309> 2004-10-26
   <313> (1)..(595)
<400> 22
   gggcaaggct gggccgggaa gggcgtgggt tgaggagagg ctccagaccc gcacgccgcg      60
   cgcacagagc tctcagcgcc gctcccagcc acagcctccc gcgcctcgct cagctccaac     120
   atggcaaaaa tctccagccc tacagagact gagcggtgca tcgagtccct gattgctgtc     180
   ttccagaagt atgctggaaa ggatggttat aactacactc tctccaagac agagttccta     240
   agcttcatga atacagaact agctgccttc acaaagaacc agaaggaccc tggtgtcctt     300
   gaccgcatga tgaagaaact ggacaccaac agtgatggtc agctagattt ctcagaattt     360
   cttaatctga ttggtggcct agctatggct tgccatgact ccttcctcaa ggctgtccct     420
   tcccagaagc ggacctgagg accccttggc cctggccttc aaacccaccc cctttccttc    480
   cagcctttct gtcatcatct ccacagccca cccatcccct gagcacacta accacctcat     540
   gcaggcccca cctgccaata gtaataaagc aatgtcactt ttttaaaaca tgaaa          595
<210> 23
   <211> 249
   <212> PRT
   <213> Homo sapiens
<300>
   <308> P00938
   <309> 2003-09-15
   <313> (1)..(249)
<400> 23
   Met Ala Pro Ser Arg Lys Phe Phe Val Gly Gly Asn Trp Lys Met Asn
   1               5                   10                  15
   Gly Arg Lys Gln Ser Leu Gly Glu Leu Ile Gly Thr Leu Asn Ala Ala
               20                  25                  30
   Lys Val Pro Ala Asp Thr Glu Val Val Cys Ala Pro Pro Thr Ala Tyr
           35                  40                  45
   Ile Asp Phe Ala Arg Gln Lys Leu Asp Pro Lys Ile Ala Val Ala Ala
       50                  55                  60
   Gln Asn Cys Tyr Lys Val Thr Asn Gly Ala Phe Thr Gly Glu Ile Ser
   65                  70                  75                  80
   Pro Gly Met Ile Lys Asp Cys Gly Ala Thr Trp Val Val Leu Gly His
                   85                  90                  95
   Ser Glu Arg Arg His Val Phe Gly Glu Ser Asp Glu Leu Ile Gly Gln
               100                 105                 110
   Lys Val Ala His Ala Leu Ala Glu Gly Leu Gly Val Ile Ala Cys Ile
           115                 120                 125
   Gly Glu Lys Leu Asp Glu Arg Glu Ala Gly Ile Thr Glu Lys Val Val
       130                 135                 140
   Phe Glu Gln Thr Lys Val Ile Ala Asp Asn Val Lys Asp Trp Ser Lys
   145                 150                 155                 160
   Val Val Leu Ala Tyr Glu Pro Val Trp Ala Ile Gly Thr Gly Lys Thr
                   165                 170                 175
   Ala Thr Pro Gln Gln Ala Gln Glu Val His Glu Lys Leu Arg Gly Trp
               180                 185                 190
   Leu Lys Ser Asn Val Ser Asp Ala Val Ala Gln Ser Thr Arg Ile Ile
           195                 200                 205
   Tyr Gly Gly Ser Val Thr Gly Ala Thr Cys Lys Glu Leu Ala Ser Gln
       210                 215                 220
   Pro Asp Val Asp Gly Phe Leu Val Gly Gly Ala Ser Leu Lys Pro Glu
   225                 230                 235                 240
   Phe Val Asp Ile Ile Asn Ala Lys Gln
                   245
<210> 24
   <211> 1254
   <212> DNA
   <213> Homo sapiens
<300>
   <308> NM_000365
   <309> 2004-12-18
   <313> (1)..(1254)
<400> 24
   ccttcagcgc ctcggctcca gcgccatggc gccctccagg aagttcttcg ttgggggaaa      60
   ctggaagatg aacgggcgga agcagagtct gggggagctc atcggcactc tgaacgcggc     120
   caaggtgccg gccgacaccg aggtggtttg tgctccccct actgcctata tcgacttcgc    180
   ccggcagaag ctagatccca agattgctgt ggctgcgcag aactgctaca aagtgactaa     240
   tggggctttt actggggaga tcagccctgg catgatcaaa gactgcggag ccacgtgggt     300
   ggtcctgggg cactcagaga gaaggcatgt ctttggggag tcagatgagc tgattgggca     360
   gaaagtggcc catgctctgg cagagggact cggagtaatc gcctgcattg gggagaagct     420
   agatgaaagg gaagctggca tcactgagaa ggttgttttc gagcagacaa aggtcatcgc     480
   agataacgtg aaggactgga gcaaggtcgt cctggcctat gagcctgtgt gggccattgg     540
   tactggcaag actgcaacac cccaacaggc ccaggaagta cacgagaagc tccgaggatg     600
   gctgaagtcc aacgtctctg atgcggtggc tcagagcacc cgtatcattt atggaggctc     660
   tgtgactggg gcaacctgca aggagctggc cagccagcct gatgtggatg gcttccttgt     720
   gggtggtgct tccctcaagc ccgaattcgt ggacatcatc aatgccaaac aatgagcccc     780
   atccatcttc cctacccttc ctgccaagcc agggactaag cagcccagaa gcccagtaac     840
   tgccctttcc ctgcatatgc ttctgatggt gtcatctgct ccttcctgtg gcctcatcca     900
   aactgtatct tcctttactg tttatatctt caccctgtaa tggttgggac caggccaatc     960
   ccttctccac ttactataat ggttggaact aaacgtcacc aaggtggctt ctccttggct    1020
   gagagatgga aggcgtggtg ggatttgctc ctgggttccc taggccctag tgagggcaga    1080
   agagaaacca tcctctccct tcttacaccg tgaggccaag atcccctcag aaggcaggag    1140
   tgctgccctc tcccatggtg cccgtgcctc tgtgctgtgt atgtgaacca cccatgtgag    1200
   ggaataaacc tggcactagg aaaaaaaaaa aaaaaaaaaa aaaaaaaaaa aaaa          1254
<210> 25
   <211> 5005
   <212> DNA
   <213> Homo sapiens
<300>
   <308> X69723
   <309> 1997-03-24
   <313> (1)..(5005)
<400> 25
   ctgcagttcc tgccaggcct tgccagccgg ggcgagggtt gggatgatcc tggcggccta 60
   tgcctgtgtg ggctgcccct cccgctgtga accctgcatt tgtcccgcaa gttttcactc 120
   aggtagactc cctgggtaca agggtgcctg ctcagcagtc gggcatgagc tgctccgatg 180
   ggcgaaggag gttgtctatt ccacagttgg agaggggccc tctctgcccc agtgggcgat 240
   ctgggctacg gccaagttgc caccagctag ttccgcttga aaaccacttc tggccccgtg 300
   ggggactcaa gtcgccaagc gagggttccc ctgagcgccg gagctcacag gtctcgcctt 360
   gtcccgaaag ccccgcaatc gaggcggagg cgaccgagcc cccgactctc ctagaacgtt 420
   gccacaagaa gggggaacgt cggaacagtg catcatcggg cggcggccgg ggcggcggca 480
   ggagggcggg cggggggcag ggctccgggg gactgggcgg gccatggcgg aggacggcga 540
   ggaggcggag ttccacttcg cggcgctcta tataagtggg cagtggccgc gactgcgcgc 600
   agacactgac cttcagcgcc tcggctccag cgccatggcg ccctccagga agttcttcgt 660
   tgggggaaac tggaagatga acgggcggaa gcagagtctg ggggagctca tcggcactct 720
   gaacgcggcc aaggtgccgg ccgacaccgg taagccctcg ccgaggaggg gtctggccgg 780
   gccggggccg ggccggggca ggagtggcag cgcctctccc gaggcccgag gtccgggccg 840
   gtatccgcgc ggacctgatg cagggctgtg ggacgagggc cgctggggtc cgggcagggg 900
   cctcgcagcc gcagccccgt cggtgcgtcg agggggcagg gcggagcaca tgatgcccct 960
   tggactacgg ggcaggtaag gacgttttgg gtctcctgga ggaaggcggc cccggggcgc 1020
   gcactggctg tgcccgccag gcgacggggt taggagccga gcccgaggct ctgcgggaga 1080
   ccgggggagg ctgggccgcg tgggcttccg ctccctgccc tggcctccgc gtgcgcgccg 1140
   ccgcacgtag ccccagactc ctccccctcc tcgccggcgt cgtcccgcgc cgagctgctg 1200
   ctgccctgag cccccagatc tgaacccctt cccttcggca acctgagcga ctcccgcctt 1260
   ccacggaagg gaccgagccc gtgccaaaca ggctgagcga tttgggagtg aggagccatc 1320
   ctaccgcttt ccccaacctg gaaacagcaa agcgcaaggc ctctgagtca gttaggtctc 1380
   tgccacccac gggcaaagga tgctctcctc catcctcctt cctccctcca ccgaaatcgg 1440
   agagccgcgg gcctgatcca aagaggcatc cccttctcgt tcattcccca gaggcctcaa 1500
   tacaaacccc aggagttggc ccctctcctt ttgctacaaa tccttgcctt gcaaagggga 1560
   ggtgaggatg ggctatttta gaagggaagc agggttgctc cctggagaat gctgagtctg 1620
   tgaggtgcct atgccgagaa tagctcgagg aaattggagc cccagctgtt aaaagagcag 1680
   agggcagggt gagggccgtg gctctcaggg gtatctggaa ggctcttcga gttgagtgca 1740
   gacccagcct tgggctggaa aatggacaaa ggtcatcttg ctggggtgaa aagggggaga 1800
   gcagaaccaa gaagaagagg gtgagggctg gggggctcca gggcactggt taggaattgt 1860
   ggggaatgaa ggctttcttt agtctcatcc ccctgtggta ccatcttgtc ctcagaggtg 1920
   gtttgtgctc cccctactgc ctatatcgac ttcgcccggc agaagctaga tcccaagatt 1980
   gctgtggctg cgcagaactg ctacaaagtg actaatgggg cttttactgg ggagatcagg 2040
   tgagatcgag gtggagaggg gtgtgtggga cccttccctc actttcctcg ttgaggggaa 2100
   agccacaggg tgggctccct gctgaacctt ggcttcatct cttcctttag ccctggcatg 2160
   atcaaagact gcggagccac gtgggtggtc ctggggcact cagagagaag gcatgtcttt 2220
   ggggagtcag atgaggttag tagccaagag agaagataag ggatgtcttt ttccaagaag 2280
   gatgtctcac caagtctgtt tctcaacagc tgattgggca gaaagtggcc catgctctgg 2340
   cagagggact cggagtaatc gcctgcattg gggagaagct agatgaaagg gaagctggca 2400
   tcactgagaa ggttgttttc gagcagacaa aggtcatcgc aggtatctct ggagaaaggg 2460
   acctttgagc ctatccaggg ccacagagac tcagagggta gggtcaggcc ctggagcctg 2520
   tcttggtccc catgctgatc cagaaaagga aaaaggggag ggggagtgac aatctttgct 2580
   tggggcctat gacttctcca gccccaaggt agatgccacc tggaaatccc ccaatgtcca 2640
   ctagggggca gtaggccacc gttcttcgta ctccggagaa cctggctgga gagctctttc 2700
   ttgttcaccc ttccctccat ctgtatctct gccctgcaga taacgtgaag gactggagca 2760
   aggtcgtcct ggcctatgag cctgtgtggg ccattggtac tggcaagact gcaacacccc 2820
   aacaggtaac cgggcccagg agccctgccc tcatcccagc ctgcctcaat aggtttggac 2880
   agacacagcc cacatggagc aaccccttat ttcaaagaca cagagacctt gaacccagag 2940
   acagtgactt gtccaagggc atccagtcca gggcctggct tggatcagag ccctggtact 3000
   ctgactcagt cagaaaccac actaagtgtc cactggtgcc agtgattttt cctcttagag 3060
   aggcagaaaa ggtcttactt aggccagctt cttgttctag gcccaggaag tacacgagaa 3120
   gctccgagga tggctgaagt ccaacgtctc tgatgcggtg gctcagagca cccgtatcat 3180
   ttatggaggt gagtggcttt ggttcccggc tgaggtggag tgggctgagg actagactga 3240
   gccctcggac atggaggtgg ggatggggca gactcatccc attcttgacc aagcccttgt 3300
   tctgctccct tcccaggctc tgtgactggg gcaacctgca aggagctggc cagccagcct 3360
   gatgtggatg gcttccttgt gggtggtgct tccctcaagc ccgaattcgt ggacatcatc 3420
   aatgccaaac aatgagcccc atccatcttc cctacccttc ctgccaagcc agggactaag 3480
   cagcccagaa gcccagtaac tgccctttcc ctgcatatgc ttctgatggt gtcatctgct 3540
   ccttcctgtg gcctcatcca aactgtatct tcctttactg tttatatctt caccctgtaa 3600
   tggttgggac caggccaatc ccttctccac ttactataat ggttggaact aaacgtcacc 3660
   aaggtggctt ctccttggct gagagatgga aggcgtggtg ggatttgctc ctgggttccc 3720
   taggccctag tgagggcaga agagaaacca tcctctccct tcttacaccg tgaggccaag 3780
   atcccctcag aaggcaggag tgctgccctc tcccatggtg cccgtgcctc tgtgctgtgt 3840
   atgtgaacca cccatgtgag ggaataaacc tggcactagg tcttgtggtt tgtctgcctt 3900
   cactggactt gcccagataa tcttcctttt tgaggcagct atataaatga tcatttgtgc 3960
   aagaaaaaaa aaaaaacaag aacaggtttc tataacaaca tctcttacta tttttacttg 4020
   aaaaaatgtt ttgcgtagca gactgtcata gccttgaacg ccggctccct ttcttcctcc 4080
   ctccaagtgg ctctggggct gttgatttcc gcagagcttg ggttggggta gggctcagcc 4140
   tcaccagctt tcagcagctg gtctaggcca gcagtgcctc cccacctccc caagggaggg 4200
   tggtggcaag acctcagcac agtctgtggt atcacaggct cactggtaga gcagtagcgc 4260
   ttcatgcagg gggcaagggc agggcagaca cctggccgag cggtatcccc aggttgtggc 4320
   gcacacacag gcggctcagg tgcagaaggg agtgtggctc cgctgggaga gagaaggagg 4380
   ggaatgtaag tatgggtgca gccaccagcc agatgtcctc aaactacggg gtcctcatca 4440
   gatgcctttc tgctttcctg cttcgagtgt gcccacctgg ctgaaagggg aatttgagat 4500
   acccggaagt tctgcctccc agataagatt tcacacatcc ctagtcagag ctgggggtga 4560
   agagctggct aaggccctct aaacaacagg ccaaggtggc tctgacagtg gtggagctgg 4620
   cccaggcttt gactccagag gcttgggagc tggggctgag gtgaggaggg atggccctcc 4680
   actctacagc ccaacacaac tgcagagagc agctccaagc cctggaccca gtcagttcct 4740
   ggggaggctc ctcccctgct gccccaccct aaggcctgcc tcctccactg ctctcctcct 4800
   ccctggtgcc cagggcccca gtgtctccat cctgaggtgt ggctgaggaa ggaagtaggt 4860
   atgtggcaca gagacaggtt agagcccagg gaatccggta tacagcctgg gtacctcgtc 4920
   tgcccatcct tcttttggac ctgtacatca aacccagtac ctaaccgttt gcacctcttg 4980
   cctaggggtg attactcctg aattc 5005
<210> 26
   <211> 938
   <212> PRT
   <213> Homo sapiens
<220>
   <221> MISC_FEATURE
   <223> alfa-1-antitrypsin precursor
<300>
   <308> Q05586
   <309> 2005-01-25
   <313> (1)..(938)
<400> 26
<210> 27
   <211> 3109
   <212> DNA
   <213> Homo sapiens
<300>
   <308> D13515
   <309> 1999-02-20
   <313> (1)..(3109)
<400> 27
   gcttcagcgc cccttccctc ggccgacgtc ccgggaccgc cgctccgggg gagacgtggc      60
   gtccgcagcc cgcggggccg ggcgagcgca ggacggcccg gaagccccgc gggggatgcg     120
   ccgagggccc cgcgttcgcg ccgcgcagag ccaggcccgc ggcccgagcc catgagcacc     180
   atgcgcctgc tgacgctcgc cctgctgttc tcctgctccg tcgcccgtgc cgcgtgcgac     240
   cccaagatcg tcaacattgg cgcggtgctg agcacgcgga agcacgagca gatgttccgc     300
   gaggccgtga accaggccaa caagcggcac ggctcctgga agattcagct caatgccacc     360
   tccgtcacgc acaagcccaa cgccatccag atggctctgt cggtgtgcga ggacctcatc     420
   tccagccagg tctacgccat cctagttagc catccaccta cccccaacga ccacttcact     480
   cccacccctg tctcctacac agccggcttc taccgcatac ccgtgctggg gctgaccacc     540
   cgcatgtcca tctactcgga caagagcatc cacctgagct tcctgcgcac cgtgccgccc     600
   tactcccacc agtccagcgt gtggtttgag atgatgcgtg tctacagctg gaaccacatc     660
   atcctgctgg tcagcgacga ccacgagggc cgggcggctc agaaacgcct ggagacgctg     720
   ctggaggagc gtgagtccaa ggcagagaag gtgctgcagt ttgacccagg gaccaagaac     780
   gtgacggccc tgctgatgga ggcgaaagag ctggaggccc gggtcatcat cctttctgcc     840
   agcgaggacg atgctgccac tgtataccgc gcagccgcga tgctgaacat gacgggctcc     900
   gggtacgtgt ggctggtcgg cgagcgcgag atctcgggga acgccctgcg ctacgcccca     960
   gacggcatcc tcgggctgca gctcatcaac ggcaagaacg agtcggccca catcagcgac    1020
   gccgtgggcg tggtggccca ggccgtgcac gagctcctcg agaaggagaa catcaccgac    1080
   ccgccgcggg gctgcgtggg caacaccaac atctggaaga ccgggccgct cttcaagaga    1140
   gtgctgatgt cttccaagta tgcggatggg gtgactggtc gcgtggagtt caatgaggat    1200
   ggggaccgga agttcgccaa ctacagcatc atgaacctgc agaaccgcaa gctggtgcaa    1260
   gtgggcatct acaatggcac ccacgtcatc cctaatgaca ggaagatcat ctggccaggc 1320
   ggagagacag agaagcctcg agggtaccag atgtccacca gactgaagat tgtgacgatc 1380
   caccaggagc ccttcgtgta cgtcaagccc acgctgagtg atgggacatg caaggaggag 1440
   ttcacagtca acggcgaccc agtcaagaag gtgatctgca ccgggcccaa cgacacgtcg 1500
   ccgggcagcc cccgccacac ggtgcctcag tgttgctacg gcttttgcat cgacctgctc 1560
   atcaagctgg cacggaccat gaacttcacc tacgaggtgc acctggtggc agatggcaag 1620
   ttcggcacac aggagcgggt gaacaacagc aacaagaagg agtggaatgg gatgatgggc 1680
   gagctgctca gcgggcaggc agacatgatc gtggcgccgc taaccataaa caacgagcgc 1740
   gcgcagtaca tcgagttttc caagcccttc aagtaccagg gcctgactat tctggtcaag 1800
   aaggagattc cccggagcac gctggactcg ttcatgcagc cgttccagag cacactgtgg 1860
   ctgctggtgg ggctgtcggt gcacgtggtg gccgtgatgc tgtacctgct ggaccgcttc 1920
   agccccttcg gccggttcaa ggtgaacagc gaggaggagg aggaggacgc actgaccctg 1980
   tcctcggcca tgtggttctc ctggggcgtc ctgctcaact ccggcatcgg ggaaggcgcc 2040
   cccagaagct tctcagcgcg catcctgggc atggtgtggg ccggctttgc catgatcatc 2100
   gtggcctcct acaccgccaa cctggcggcc ttcctggtgc tggaccggcc ggaggagcgc 2160
   atcacgggca tcaacgaccc tcggctgagg aacccctcgg acaagtttat ctacgccacg 2220
   gtgaagcaga gctccgtgga tatctacttc cggcgccagg tggagctgag caccatgtac 2280
   cggcatatgg agaagcacaa ctacgagagt gcggcggagg ccatccaggc cgtgagagac 2340
   aacaagctgc atgccttcat ctgggactcg gcggtgctgg agttcgaggc ctcgcagaag 2400
   tgcgacctgg tgacgactgg agagctgttt ttccgctcgg gcttcggcat aggcatgcgc 2460
   aaagacagcc cctggaagca gaacgtctcc ctgtccatcc tcaagtccca cgagaatggc 2520
   ttcatggaag acctggacaa gacgtgggtt cggtatcagg aatgtgactc gcgcagcaac 2580
   gcccctgcga cccttacttt tgagaacatg gccggggtct tcatgctggt agctgggggc 2640
   atcgtggccg ggatcttcct gattttcatc gagattgcct acaagcggca caaggatgct 2700
   cgccggaagc agatgcagct ggcctttgcc gccgttaacg tgtggcggaa gaacctgcag 2760
   gatagaaaga gtggtagagc agagcctgac cctaaaaaga aagccacatt tagggctatc 2820
   acctccaccc tggcttccag cttcaagagg cgtaggtcct ccaaagacac gagcaccggg 2880
   ggtggacgcg gcgctttgca aaaccaaaaa gacacagtgc tgccgcgacg cgctattgag 2940
   agggaggagg gccagctgca gctgtgttcc cgtcataggg agagctgaga ctccccgccc 3000
   gccctcctct gccccctccc ccgcagacag acagacagac ggacgggaca gcggcccggc 3060
   ccacgcagag ccccggagca ccacggggtc gggggaggag cacccccag 3109
<210> 28
   <211> 15
   <212> PRT
   <213> Homo sapiens
<400> 28
   Leu Leu Thr Leu Leu Ala Leu Leu Phe Ser Cys Ser Val Ala Arg
   1               5                   10                  15
<210> 29
   <211> 12
   <212> PRT
   <213> Homo sapiens
<400> 29
   Ile Thr Met Leu Cys Thr Gly Ser Arg Thr Leu Lys
   1               5                   10
<210> 30
   <211> 418
   <212> PRT
   <213> Homo sapiens
<300>
   <308> ITHU
   <309> 2000-09-15
   <313> (1)..(418)
<400> 30
<210> 31
   <211> 1607
   <212> DNA
   <213> Homo sapiens
<300>
   <308> NM_000295
   <309> 2004-12-20
   <313> (1)..(1607)
<400> 31
   aatgactcct ttcggtaagt gcagtggaag ctgtacactg cccaggcaaa gcgtccgggc      60
   agcgtaggcg ggcgactcag atcccagcca gtggacttag cccctgtttg ctcctccgat     120
   aactggggtg accttggtta atattcacca gcagcctccc ccgttgcccc tctggatcca     180
   ctgcttaaat acggacgagg acagggccct gtctcctcag cttcaggcac caccactgac     240
   ctgggacagt gaatcgacaa tgccgtcttc tgtctcgtgg ggcatcctcc tgctggcagg     300
   cctgtgctgc ctggtccctg tctccctggc tgaggatccc cagggagatg ctgcccagaa     360
   gacagataca tcccaccatg atcaggatca cccaaccttc aacaagatca cccccaacct     420
   ggctgagttc gccttcagcc tataccgcca gctggcacac cagtccaaca gcaccaatat     480
   cttcttctcc ccagtgagca tcgctacagc ctttgcaatg ctctccctgg ggaccaaggc     540
   tgacactcac gatgaaatcc tggagggcct gaatttcaac ctcacggaga ttccggaggc     600
   tcagatccat gaaggcttcc aggaactcct ccgtaccctc aaccagccag acagccagct     660
   ccagctgacc accggcaatg gcctgttcct cagcgagggc ctgaagctag tggataagtt     720
   tttggaggat gttaaaaagt tgtaccactc agaagccttc actgtcaact tcggggacac     780
   cgaagaggcc aagaaacaga tcaacgatta cgtggagaag ggtactcaag ggaaaattgt     840
   ggatttggtc aaggagcttg acagagacac agtttttgct ctggtgaatt acatcttctt     900
   taaaggcaaa tgggagagac cctttgaagt caaggacacc gaggaagagg acttccacgt     960
   ggaccaggtg accaccgtga aggtgcctat gatgaagcgt ttaggcatgt ttaacatcca    1020
   gcactgtaag aagctgtcca gctgggtgct gctgatgaaa tacctgggca atgccaccgc    1080
   catcttcttc ctgcctgatg aggggaaact acagcacctg gaaaatgaac tcacccacga    1140
   tatcatcacc aagttcctgg aaaatgaaga cagaaggtct gccagcttac atttacccaa    1200
   actgtccatt actggaacct atgatctgaa gagcgtcctg ggtcaactgg gcatcactaa    1260
   ggtcttcagc aatggggctg acctctccgg ggtcacagag gaggcacccc tgaagctctc    1320
   caaggccgtg cataaggctg tgctgaccat cgacgagaaa gggactgaag ctgctggggc    1380
   catgttttta gaggccatac ccatgtctat cccccccgag gtcaagttca acaaaccctt    1440
   tgtcttctta atgattgaac aaaataccaa gtctcccctc ttcatgggaa aagtggtgaa    1500
   tcccacccaa aaataactgc ctctcgctcc tcaacccctc ccctccatcc ctggccccct     1560
   ccctggatga cattaaagaa gggttgagct ggtccctgcc tgcaaaa                  1607
<210> 32
   <211> 12222
   <212> DNA
   <213> Homo sapiens
<300>
   <308> K02212
   <309> 1995-08-08
   <313> (1)..(12222)
<400> 32
   gaattccagg ttggaggggc ggcaacctcc tgccagcctt caggccactc tcctgtgcct 60
   gccagaagag acagagcttg aggagagctt gaggagagca ggaaaggtgg aacattgctg 120
   ctgctgctca ctcagttcca caggtgggag gaacagcagg gcttagagtg ggggtcattg 180
   tgcagatggg aaaacaaagg cccagagagg ggaagaaatg cctaggagct accgagggca 240
   ggcgacctca accacagccc agtgctggag ctgtgagtgg atgtagagca gcggaatatc 300
   cattcagcca gctcagggga aggacagggg ccctgaagcc aggggatgga gctgcaggga 360
   agggagctca gagagaaggg gaggggagtc tgagctcagt ttcccgctgc ctgaaaggag 420
   ggtggtacct actcccttca cagggtaact gaatgagaga ctgcctggag gaaagctctt 480
   caagtgtggc ccaccccacc ccagtgacac cagcccctga cacgggggag ggagggcagc 540
   atcaggaggg gctttctggg cacacccagt acccgtctct gagctttcct tgaactgttg 600
   cattttaatc ctcacagcag ctcaacaagg tacataccgt caccatcccc attttacaga 660
   tagggaaatt gaggctcgga gcggttaaac aactcacctg aggcctcaca gccagtaagt 720
   gggttccctg gtctgaatgt gtgtgctgga ggatcctgtg ggtcactcgc ctggtagagc 780
   cccaaggtgg aggcataaat gggactggtg aatgacagaa ggggcaaaaa tgcactcatc 840
   cattcactct gcaagtatct acggcacgta cgccagctcc caagcaggtt tgcgggttgc 900
   acagcggagc gatgcaatct gatttaggct tttaaaggat tgcaatcaag tgggacccac 960
   tagcctcaac cctgtacctc ccctcccctc cacccccagc agtctccaaa ggcctccaac 1020
   aaccccagag tgggggccat gtatccaaag aaactccaag ctgtatacgg atcacactgg 1080
   ttttccagga gcaaaaacag aaacagcctg aggctggtca aaattgaacc tcctcctgct 1140
   ctgagcagcc tagggggcag actaagcaga gggctgtgca gacccacata aagagcctac 1200
   tgtgtgccag gcacttcacc cgaggcactt cacaagcatg cttgggaatg aaacttccaa 1260
   ctctttggga tgcaggtgaa acagttcctg gttcagagag gtgaagcggc ctgcctgagg 1320
   cagcacagct cttctttaca gatgtgcttc cccacctcta ccctgtctca cggcccccca 1380
   tgccagcctg acggttgtgt ctgcctcagt catgctccat ttttccatcg ggaccatcaa 1440
   gagggtgttt gtgtctaagg ctgactgggt aactttggat gagcggtctc tccgctccga 1500
   gcctgtttcc tcatctgtca aacgggctct aacccactct gatctcccag ggcggcagta 1560
   agtcttcagc atcaggcatt ttggggtgac tcagtaaatg gtagatcttg ctaccagtgg 1620
   aacagccact aaggattctg cagtgagagc agagggccag ctaagtggta ctctcccaga 1680
   gactgtctga ctcacgccac cccctccacc ttggacacag gacgctgtgg tttctgagcc 1740
   aggtacaatg actcctttcg gtaagtgcag tggaagctgt acactgccca ggcaaagcgt 1800
   ccgggcagcg taggcgggcg actcagatcc cagccagtgg acttagcccc tgtttgctcc 1860
   tccgataact ggggtgacct tggttaatat tcaccagcag cctcccccgt tgcccctctg 1920
   gatccactgc ttaaatacgg acgaggacag ggccctgtct cctcagcttc aggcaccacc 1980
   actgacctgg gacagtgaat cgtaagtatg cctttcactg cgaggggttc tggagaggct 2040
   tccgagctcc ccatggccca ggcaggcagc aggtctgggg caggaggggg gttgtggagt 2100
   gggtatccgc ctgctgaggt gcagggcaga tggagaggct gcagctgagc tcctattttc 2160
   ataataacag cagccatgag ggttgtgtcc tgtttcccag tcctgcccgg tcccccctcg 2220
   gtacctcctg gtggatacac tggttcctgt aagcagaagt ggatgagggt gtctaggtct 2280
   gcagtcctgg caccccagga tgggggacac cagccaagat acagcaacag caacaaagcg 2340
   cagccatttc tttctgtttg cacagctcct ctgtctgtcg ggggctcctg tctgttgtct 2400
   cctataagcc tcaccacctc tcctactgct tgggcatgca tctttctccc cttctataga 2460
   tgaggaggtt aaggttcaga gaggggtggg gaggaacgcc ggctcacatt ctccatcccc 2520
   tccagatatg accaggaaca gacctgtgcc agcctcagcc ttacatcaaa atgggcctcc 2580
   ccatgcaccg tggacctctg ggccctcctg tcccagtgga ggacaggaag ctgtgagggg 2640
   cactgtcacc cagggctcaa gctggcattc ctgaataatc gctctgcacc aggccacggc 2700
   taagctcagt gcgtgattaa gcctcataac cctccaaggc agttactagt gtgattccca 2760
   ttttacagat gaggaagatg gggacagaga ggtgaataac tggccccaaa tcacacacca 2820
   tccataattc gggctcaggc acctggctcc agtccccaaa ctcttgaacc tggccctagt 2880
   gtcactgttt ctcttgggtc tcaggcgctg gatggggaac aggaaacctg ggctgaactt 2940
   gaggcctctc tgatgctcgg tgacttcaga cagttgctca acctctctgt tctcttgggc 3000
   aaaacatgat aacctttgac ttctgtcccc tcccctcacc ccacccgacc ttgatctctg 3060
   aagtgttgga aggatttaat ttttcctgca ctgagttttg gagacaggtc aaaaagatga 3120
   ccaaggccaa ggtggccagt ttcctataga acgcctctaa aagacctgca gcaatagcag 3180
   caagaactgg tattctcgag aacttgctgc gcagcaggca cttcttggca ttttatgtgt 3240
   atttaatttc acaatagctc tatgacaaag tccacctttc tcatctccag gaaactgagg 3300
   ttcagagagg ttaagtaact tgtccaaggt cacacagcta atagcaagtt gacgtggagc 3360
   aatctggcct cagagccttt aattttagcc acagactgat gctcccctct tcatttagcc 3420
   aggctgcctc tgaagttttc tgattcaaga cttctggctt cagctttgta cacagagatg 3480
   attcaatgtc aggttttgga gcgaaatctg tttaatccca gacaaaacat ttaggattac 3540
   atctcagttt tgtaagcaag tagctctgtg atttttagtg agttatttaa tgctctttgg 3600
   ggctcaattt ttctatctat aaaatagggc taataatttg caccttatag ggtaagcttt 3660
   gaggacagat tagatgatac ggtgcctgta aaacaccagg tgttagtaag tgtggcaatg 3720
   atggtgacgc tgaggctgtg tttgcttagc atagggttag gcagctggca ggcagtaaac 3780
   agttggataa tttaatggaa aatttgccaa actcagatgc tgttcactgc tgagcaggag 3840
   ccccttcctg ctgaaatggt cctggggagt gcagcaggct ctccgggaag aaatctacca 3900
   tctctcgggc aggagctcaa cctgtgtgca ggtacaggga gggcttcctc acctggtgcc 3960
   cactcatgca ttacgtcagt tattcctcat ccctgtccaa aggattcttt tctccattgt 4020
   acagctatga agctagtgct caaagaagtg aagtcattta ccccaggccc cctgccagta 4080
   agtgacaggg cctggtcaca cttgggttta tttattgccc agttcaacag gttgtttgac 4140
   cataggcgag attctcttcc ctgcaccctg ccgggttgct cttggtccct tattttatgc 4200
   tcctgggtag aaatggtgcg agattaggca gggagtggac gcttccctgt ccctggcccc 4260
   gcaaagagtg ctcccacctg ccccgatccc agaaatgtca ccatgaagcc ttcattcttt 4320
   tggtttaaag cttggcctca gtgtccgtac accatggggt ccttggccag atggcgactt 4380
   tctcctctcc agtcgccctc ccaggcacta gcttttagga gtgcagggtg ctgcctctga 4440
   tagaagggcc aggagagagc aggttttgga gacctgatgt tataaggaac agcttgggag 4500
   gcataatgaa cccaacatga tgcttgagac caatgtcaca gcccaattct gacattcatc 4560
   atctgagatc tgaggacaca gctgtctcag ttcatgatct gagtgctggg aaagccaaga 4620
   cttgttccag ctttgtcact gacttgctgt atagcctcaa caaggccctg accctctctg 4680
   ggcttcaaac tcttcactgt gaaaggagga aaccagagta ggtgatgtga caccaggaaa 4740
   gatggatggg tgtgggggaa tgtgctcctc ccagctgtca ccccctcgcc accctccctg 4800
   caccagcctc tccacctcct ttgagcccag aattcccctg tctaggaggg cacctgtctc 4860
   gtgcctagcc atgggaattc tccatctgtt ttgctacatt gaacccagat gccattctaa 4920
   ccaagaatcc tggctgggtg caggggctct cgcctgtaac cccagcactt tgggaggcca 4980
   aggcaggcgg atcaagaggt caggagttca agacctgcct ggccaacacg gtgaaacctc 5040
   agctctacta aaaatacaaa aattagccag gcgtggtggc acacgcctgt aatcccagct 5100
   atttgggaag ctgagacaga agaatttctt gaacccggga ggtggaggtt tcagtgagcc 5160
   gagatcacgc cactgcactc caccctggcg gataaagcga gactctgtct caaaaaaaac 5220
   ccaaaaacct atgttagtgt acagagggcc ccagtgaagt cttctcccag ccccactttg 5280
   cacaactggg gagagtgagg ccccaggacc agaggattct tgctaaaggc caagtggata 5340
   gtgatggccc tgccaggcta gaagccacaa cctctggccc tgaggccact cagcatattt 5400
   agtgtcccca ccctgcagag gcccaactcc ctcctgacca ctgagccctg taatgatggg 5460
   ggaatttcca taagccatga aggactgcac aaagttcagt tgggagtgaa agagaaatta 5520
   aagggagatg gaaatataca gcactaattt tagcaccgtc ttcagttcta acaacactag 5580
   ctagctgaag aaaatacaaa catgtattat gtaatgtgtg gtctgttcca tttggattac 5640
   ttagaggcac gagggccaag gagaaaggtg gtggagagaa accagctttg cacttcattt 5700
   gttgctttat tggaaggaaa cttttaaaag tccaaggggg ttgaagaatc tcaatatttg 5760
   ttatttccag ctttttttct ccagtttttc atttcccaaa ttcaaggaca cctttttctt 5820
   tgtattttgt taagatgatg gttttggttt tgtgactagt agttaacaat gtggctgccg 5880
   ggcatattct cctcagctag gacctcagtt ttcccatctg tgaagacggc aggttctacc 5940
   tagggggctg caggcaggtg gtccgaagcc tgggcatatc tggagtagaa ggatcactgt 6000
   ggggcagggc aggttctgtg ttgctgtgga tgacgttgac tttgaccatt gctcggcaga 6060
   gcctgctctc gctggttcag ccacaggccc caccactccc tattgtctca gccccgggta 6120
   tgaaacatgt attcctcact ggcctatcac ctgaagcctt tgaatttgca acacctgcca 6180
   acccctccct caaaagagtt gccctctcta gatccttttg atgtaaggtt tggtgttgag 6240
   acttatttca ctaaattctc atacataaac atcactttat gtatgaggca aaatgaggac 6300
   cagggagatg aatgacttgt cctggctcat acacctggaa agtgacagag tcagattaga 6360
   tcctaggtct atctgaagtt aaaagaggtg tcttttcact tcccacctcc tccatctact 6420
   ttaaagcagc acaaacccct gctttcaagg agagatgagc gtctctaaag cccctgacag 6480
   caagagccca gaactgggac accattagtg acccagacgg caggtaagct gactgcagga 6540
   gcatcagcct attcttgtgt ctgggaccac agagcattgt ggggacagcc ccgtctcttg 6600
   ggaaaaaaac cctaagggct gaggatcctt gtgagtgttg ggtgggaaca gctcccagga 6660
   ggtttaatca cagcccctcc atgctctcta gctgttgcca ttgtgcaaga tgcatttccc 6720
   ttctgtgcag cagtttccct ggccactaaa tagtgggatt agatagaagc cctccaaggg 6780
   ctccagcttg acatgattct tgattctgat ctgacccgat tctgataatc gtgggcaggc 6840
   ccattcctct tcttgtgcct cattttcttc ttttgtaaaa caatggctgt accatttgca 6900
   tcttagggtc attgcagatg aaagtgttgc tgtccagagc ctgggtgcag gacctagatg 6960
   taggattctg gttctgctac ttcctcagtg acattgaata gctgacctaa tctctctggc 7020
   tttggtttct tcatctgtaa aagaaggata ttagcattag cacctcacgg gattgttaca 7080
   agaaagcaat gaattaacac atgtgagcac ggagaacagt gcttggcata tggtaagcac 7140
   tacgtacatt ttgctattct tctgattctt tcagtgttac tgatgtcggc aagtacttgg 7200
   cacaggctgg tttaataatc cctaggcact ttcacgtggt gtcaatccct gatcactggg 7260
   agtcatcatg tgccttgact cgggcctggc ccccccatct ctgtcttgca ggacaatgcc 7320
   gtcttctgtc tcgtggggca tcctcctgct ggcaggcctg tgctgcctgg tccctgtctc 7380
   cctggctgag gatccccagg gagatgctgc ccagaagaca gatacatccc accatgatca 7440
   ggatcaccca accttcaaca agatcacccc caacctggct gagttcgcct tcagcctata 7500
   ccgccagctg gcacaccagt ccaacagcac caatatcttc ttctccccag tgagcatcgc 7560
   tacagccttt gcaatgctct ccctggggac caaggctgac actcacgatg aaatcctgga 7620
   gggcctgaat ttcaacctca cggagattcc ggaggctcag atccatgaag gcttccagga 7680
   actcctccgt accctcaacc agccagacag ccagctccag ctgaccaccg gcaatggcct 7740
   gttcctcagc gagggcctga agctagtgga taagtttttg gaggatgtta aaaagttgta 7800
   ccactcagaa gccttcactg tcaacttcgg ggacaccgaa gaggccaaga aacagatcaa 7860
   cgattacgtg gagaagggta ctcaagggaa aattgtggat ttggtcaagg agcttgacag 7920
   agacacagtt tttgctctgg tgaattacat cttctttaaa ggtaaggttg ctcaaccagc 7980
   ctgagctgtt tcccatagaa acaagcaaaa atatttctca aaccatcagt tcttgaactc 8040
   tccttggcaa tgcattatgg gccatagcaa tgcttttcag cgtggattct tcagttttct 8100
   acacacaaac actaaaatgt tttccatcat tgagtaattt gaggaaataa tagattaaac 8160
   tgtcaaaact actgacgctc tgcagaactt ttcagagcct ttaatgtcct tgtgtatact 8220
   gtatatgtag aatatataat gcttagaact atagaacaaa ttgtaataca ctgcataaag 8280
   ggatagtttc atggaacata ctttacacga ctctagtgtc ccagaatcag tatcagtttt 8340
   gcaatctgaa agacctgggt tcaaatcctg cctctaacac aattagcttt tgacaaaaac 8400
   aatgcattct acctctttga ggtgctaatt tctcatctta gcatggacaa aataccattc 8460
   ttgctgtcag gtttttttag gattaaacaa atgacaaaga ctgtggggat ggtgtgtggc 8520
   atacagcagg tgatggactc ttctgtatct caggctgcct tcctgcccct gaggggttaa 8580
   aatgccaggg tcctgggggc cccagggcat tctaagccag ctcccactgt cccaggaaaa 8640
   cagcataggg gaggggaggt gggaggcaag gccaggggct gcttcctcca ctctgaggct 8700
   cccttgctct tgaggcaaag gagggcagtg gaggcaagcc aggctgcagt cagcacagct 8760
   aaagtcctgg ctctgctgtg gccttagtgg gggcccaggt ccctctccag ccccagtctc 8820
   ctccttctgt ccaatgagaa agctgggatc aggggtccct gaggcccctg tccactctgc 8880
   atgcctcgat ggtgaagctc tgttggtatg gcagagggga ggctgctcag gcatctgcat 8940
   ttcccctgcc aatctagagg atgaggaaag ctctcaggaa tagtaagcag aatgtttgcc 9000
   ctggatgaat aactgagctg ccaattaaca aggggcaggg agccttagac agaaggtacc 9060
   aaatatgcct gatgctccaa cattttattt gtaatatcca agacaccctc aaataaacat 9120
   atgattccaa taaaaatgca cagccacgat ggcatctctt agcctgacat cgccacgatg 9180
   tagaaattct gcatcttcct ctagttttga attatcccca cacaatcttt ttcggcagct 9240
   tggatggtca gtttcagcac cttttacaga tgatgaagct gagcctcgag ggatgtgtgt 9300
   cgtcaagggg gctcagggct tctcagggag gggactcatg gtttcttatt ctgctacact 9360
   cttccaaacc ttcactcacc cctggtgatg cccaccttcc cctctctcca ggcaaatggg 9420
   agagaccctt tgaagtcaag gacaccgagg aagaggactt ccacgtggac caggtgacca 9480
   ccgtgaaggt gcctatgatg aagcgtttag gcatgtttaa catccagcac tgtaagaagc 9540
   tgtccagctg ggtgctgctg atgaaatacc tgggcaatgc caccgccatc ttcttcctgc 9600
   ctgatgaggg gaaactacag cacctggtaa atgaactcac ccacgatatc atcaccaagt 9660
   tcctggaaaa tgaagacaga aggtgattcc ccaacctgag ggtgaccaag aagctgccca 9720
   cacctcttag ccatgttggg actgaggccc atcaggactg gccagagggc tgaggagggt 9780
   gaaccccaca tccctgggtc actgctactc tgtataaact tggcttccag aatgaggcca 9840
   ccactgagtt caggcagcgc cgtccatgct ccatgaggag aacagtaccc agggtgagga 9900
   ggtaaaggtc tcgtccctgg gaacttccca ctccagtgtg gacactgtcc cttcccaata 9960
   tccagtgccc aaggcaggga cagcagcacc accacacgtt ctggcagaac caaaaaggaa 10020
   cagatgggct tcctggcaaa ggcagcagtg gagtgtggag ttcaagggta gaatgtccct 10080
   ggggggacgg gggaagagcc tgtgtggcaa ggcccagaaa agcaaggttc ggaattggaa 10140
   cagccaggcc atgttcgcag aaggcttgcg tttctctgtc actttatcgg tgctgttaga 10200
   ttgggtgtcc tgtagtaagt gatacttaaa catgagccac acattagtgt atgtgtgtgc 10260
   attcgtgatt atgcccatgc cctgctgatc tagttcgttt tgtacactgt aaaaccaaga 10320
   tgaaaataca aaaggtgtcg ggttcataat aggaatcgag gctggaattt ctctgttcca 10380
   tgccagcacc tcctgaggtc tctgctccag gggttgagaa agaacaaaga ggctgagagg 10440
   gtaacggatc agagagccca gagccagctg ccgctcacac cagaccctgc tcagggtggc 10500
   attgtctccc catggaaaac cagagaggag cactcagcct ggtgtggtca ctcttctctt 10560
   atccactaaa cggttgtcac tgggcactgc caccagcccc gtgtttctct gggtgtaggg 10620
   ccctggggat gttacaggct gggggccagg tgacccaaca ctacagggca agatgagaca 10680
   ggcttccagg acacctagaa tatcagagga ggtggcattt caagcttttg tgattcattc 10740
   gatgttaaca ttctttgact caatgtagaa gagctaaaag tagaacaaac caaagccgag 10800
   ttcccatctt agtgtgggtg gaggacacag gagtaagtgg cagaaataat cagaaaagaa 10860
   aacacttgca ctgtggtggg tcccagaaga acaagaggaa tgctgtgcca tgccttgaat 10920
   ttcttttctg cacgacaggt ctgccagctt acatttaccc aaactgtcca ttactggaac 10980
   ctatgatctg aagagcgtcc tgggtcaact gggcatcact aaggtcttca gcaatggggc 11040
   tgacctctcc ggggtcacag aggaggcacc cctgaagctc tccaaggtga gatcaccctg 11100
   acgaccttgt tgcaccatgg tatctgtagg gaagaatgtg tgggggctgc agcactgtcc 11160
   tgaggctgag gaaggggccg agggaaacaa atgaagaccc aggctgagct cctgaagatg 11220
   cccgtgattc actgacacgg gacggtgggc aaacagcaaa gccaggcagg ggctgctgtg 11280
   cagctggcac tttcggggcc tcccttgagg ttgtgtcact gaccctgaat ttcaactttg 11340
   cccaagacct tctagacatt gggccttgat ttatccatac tgacacagaa aggtttgggc 11400
   taagttgttt caaaggaatt tctgactcct tcgatctgtg agatttggtg tctgaattaa 11460
   tgaatgattt cagctaaagt gacacttatt ttggaaaact aaaggcgacc aatgaacaac 11520
   ctgcagttcc atgaatggct gcattatctt ggggtctggg cactgtgaag gtcactgcca 11580
   gggtccgtgt cctcaaggag cttcaagccg tgtactagaa aggagagagc cctggaggca 11640
   gacgtggagt gacgatgctc ttccctgttc tgagttgtgg gtgcacctga gcagggggag 11700
   aggcgcttgt caggaagatg gacagagggg agccagcccc atcagccaaa gccttgagga 11760
   ggagcaaggc ctatgtgaca gggagggaga ggatgtgcag ggccagggcc gtccaggggg 11820
   agtgagcgct tcctgggagg tgtccacgtg agccttgctc gaggcctggg atcagcctta 11880
   caacgtgtct ctgcttctct cccctccagg ccgtgcataa ggctgtgctg accatcgacg 11940
   agaaagggac tgaagctgct ggggccatgt ttttagaggc catacccatg tctatccccc 12000
   ccgaggtcaa gttcaacaaa ccctttgtct tcttaatgat tgaacaaaat accaagtctc 12060
   ccctcttcat gggaaaagtg gtgaatccca cccaaaaata actgcctctc gctcctcaac 12120
   ccctcccctc catccctggc cccctccctg gatgacatta aagaagggtt gagctggtcc 12180
   ctgcctgcat gtgatctgta aatccctggg atgttttctc tg 12222
<210> 33
   <211> 381
   <212> PRT
   <213> Homo sapiens
<300>
   <308> P12277
   <309> 2005-01-25
   <313> (1)..(381)
<400> 33
   Met Pro Phe Ser Asn Ser His Asn Ala Leu Lys Leu Arg Phe Pro Ala
   1               5                   10                  15
   Glu Asp Glu Phe Pro Asp Leu Ser Ala His Asn Asn His Met Ala Lys
               20                  25                  30
   Val Leu Thr Pro Glu Leu Tyr Ala Glu Leu Arg Ala Lys Ser Thr Pro
           35                  40                  45
   Ser Gly Phe Thr Leu Asp Asp Val Ile Gln Thr Gly Val Asp Asn Pro
       50                  55                  60
   Gly His Pro Tyr Ile Met Thr Val Gly Cys Val Ala Gly Asp Glu Glu
   65                  70                  75                  80
   Ser Tyr Glu Val Phe Lys Asp Leu Phe Asp Pro Ile Ile Glu Asp Arg
                   85                  90                  95
   His Gly Gly Tyr Lys Pro Ser Asp Glu His Lys Thr Asp Leu Asn Pro
               100                 105                 110
   Asp Asn Leu Gln Gly Gly Asp Asp Leu Asp Pro Asn Tyr Val Leu Ser
           115                 120                 125
   Ser Arg Val Arg Thr Gly Arg Ser Ile Arg Gly Phe Cys Leu Pro Pro
       130                 135                 140
   His Cys Ser Arg Gly Glu Arg Arg Ala Ile Glu Lys Leu Ala Val Glu
   145                 150                 155                 160
   Ala Leu Ser Ser Leu Asp Gly Asp Leu Ala Gly Arg Tyr Tyr Ala Leu
                   165                 170                 175
   Lys Ser Met Thr Glu Ala Glu Gln Gln Gln Leu Ile Asp Asp His Phe
               180                 185                 190
   Leu Phe Asp Lys Pro Val Ser Pro Leu Leu Leu Ala Ser Gly Met Ala
           195                 200                 205
   Arg Asp Trp Pro Asp Ala Arg Gly Ile Trp His Asn Asp Asn Lys Thr
       210                 215                 220
   Phe Leu Val Trp Val Asn Glu Glu Asp His Leu Arg Val Ile Ser Met
   225                 230                 235                 240
   Gln Lys Gly Gly Asn Met Lys Glu Val Phe Thr Arg Phe Cys Thr Gly
                   245                 250                 255
   Leu Thr Gln Ile Glu Thr Leu Phe Lys Ser Lys Asp Tyr Glu Phe Met
               260                 265                 270
   Trp Asn Pro His Leu Gly Tyr Ile Leu Thr Cys Pro Ser Asn Leu Gly
           275                 280                 285
   Thr Gly Leu Arg Ala Gly Val His Ile Lys Leu Pro Asn Leu Gly Lys
       290                 295                 300
   His Glu Lys Phe Ser Glu Val Leu Lys Arg Leu Arg Leu Gln Lys Arg
   305                 310                 315                 320
   Gly Thr Gly Gly Val Asp Thr Ala Ala Val Gly Gly Val Phe Asp Val
                   325                 330                 335
   Ser Asn Ala Asp Arg Leu Gly Phe Ser Glu Val Glu Leu Val Gln Met
               340                 345                 350
   Val Val Asp Gly Val Lys Leu Leu Ile Glu Met Glu Gln Arg Leu Glu
           355                 360                 365
   Gln Gly Gln Ala Ile Asp Asp Leu Met Pro Ala Gln Lys
       370                 375                 380
<210> 34
   <211> 1431
   <212> DNA
   <213> Homo sapiens
<300>
   <308> NM_001823
   <309> 2004-10-27
   <313> (1)..(1431)
<400> 34
   gctgttcgcc tgcgtcgctc cgggagctgc cgacggacgg agcgcccccg cccccgcccg    60
   gccgcccgcc cgccgccgcc atgcccttct ccaacagcca caacgcactg aagctgcgct   120
   tcccggccga ggacgagttc cccgacctga gcgcccacaa caaccacatg gccaaggtgc   180
   tgacccccga gctgtacgcg gagctgcgcg ccaagagcac gccgagcggc ttcacgctgg   240
   acgacgtcat ccagacaggc gtggacaacc cgggccaccc gtacatcatg accgtgggct   300
   gcgtggcggg cgacgaggag tcctacgaag tgttcaagga tctcttcgac cccatcatcg   360
   aggaccggca cggcggctac aagcccagcg atgagcacaa gaccgacctc aaccccgaca   420
   acctgcaggg cggcgacgac ctggacccca actacgtgct gagctcgcgg gtgcgcacgg   480
   gccgcagcat ccgtggcttc tgcctccccc cgcactgcag ccgcggggag cgccgcgcca   540
   tcgagaagct cgcggtggaa gccctgtcca gcctggacgg cgacctggcg ggccgatact   600
   acgcgctcaa gagcatgacg gaggcggagc agcagcagct catcgacgac cacttcctct   660
   tcgacaagcc cgtgtcgccc ctgctgctgg cctcgggcat ggcccgcgac tggcccgacg   720
   cccgcggtat ctggcacaat gacaataaga ccttcctggt gtgggtcaac gaggaggacc   780
   acctgcgggt catctccatg cagaaggggg gcaacatgaa ggaggtgttc acccgcttct   840
   gcaccggcct cacccagatt gaaactctct tcaagtctaa ggactatgag ttcatgtgga   900
   accctcacct gggctacatc ctcacctgcc catccaacct gggcaccggg ctgcgggcag   960
   gtgtgcatat caagctgccc aacctgggca agcatgagaa gttctcggag gtgcttaagc  1020
   ggctgcgact tcagaagcga ggcacaggcg gtgtggacac ggctgcggtg ggcggggtct  1080
   tcgacgtctc caacgctgac cgcctgggct tctcagaggt ggagctggtg cagatggtgg  1140
   tggacggagt gaagctgctc atcgagatgg agcagcggct ggagcagggc caggccatcg  1200
   acgacctcat gcctgcccag aaatgaagcc cggcccacac ccgacaccag ccctgctgct  1260
   tcctaactta ttgcctgggc agtgcccacc atgcacccct gatgttcgcc gtctggcgag  1320
   cccttagcct tgctgtagag acttccgtca cccttggtag agtttatttt tttgatggct  1380
   aagatactgc tgatgctgaa ataaactagg gttttggcct gcctgcgtct g           1431
<210> 35
   <211> 4200
   <212> DNA
   <213> Homo sapiens
<300>
   <308> X15334
   <309> 1993-04-24
   <313> (1)..(4200)
<400> 35
   gatcagtttt tttttttaat cgcacttatg cttattgttt attagcgttt cctcccatct    60
   ttgcctgaag tctccgggga ctgcctttgg gggtcgggta aacttgtccc ctgcgaagag   120
   ggcccagggt tggggtctgg aaactccgag gctgcacttg ccagcggcct cttaaggcca   180
   cagcgtcccc gtggtttctg gctcgcagcc ccccgagacc caggacttgt ccaaggtcag   240
   ggcaccgcgg gtgcccccgg gctgggccgc agcagactgc gcttcccgcg cgccttcgct   300
   ttgcaccagg atcgcccagg aaatgcctgc gggcaccttg aggaaggtcg gcggctccgg   360
   gccagctcgc actggccggg gtggggcggg ggccgtacct gctgcggaag ccccgaaagc   420
   tttcgcccgg cccctcgccg ccgccgcggg ggctggctgg actaggcggg caggctcgag   480
   gatgcggatg aacccaagcg tcctcgagtg cccggaggct ctccgcctca gtttcccgcc   540
   cagaggcaag ggcgtgcgag gggatccaga tatccaagga cctgaggttt cggcctcgag   600
   gtcttgggcg ggggactggg caggctgcgc ggggtcccag cgaggggaca gctcgggtgg   660
   gcggccaggg tgttgggggc tgcgggcggc ggacaaagcg gcggcaccac cccgcggcgc   720
   gggccaatgg aatgaatggg ctataaatag ccgccaatgg gcggcccgcg ttgtgcccct   780
   taagagccgc gggagcgcgg agcggccgct gttcgcctgc gtcgctccgg gagctgccga   840
   cggacggagc gcccccgccc ccgcccggcc gcccggtgag tgggcccggg ggccgggggc   900
   gtccgcgccc gggctagggg cgctgcgagc aaagggggcg cgtcgcctgg agcgcgcgcc   960
   ggaccggccg ggggtccccg gcgatgatgg cgctccccgc gcgcgctgcg gaccccgctg  1020
   accttggccg cgtcccgggg ggcgccgggg ggcccggcgg cgggggcctg agtggtacgc  1080
   gggagcccgg gaaccccggc gtgccggtcc cctctgaccc cgcgtctccc cgcagcccgc  1140
   cgccgccatg cccttctcca acagccacaa cgcactgaag ctgcgcttcc cggccgagga  1200
   cgagttcccc gacctgagcg cccacaacaa ccacatggcc aaggtgctga cccccgagct 1260
   gtacgcggag ctgcgcgcca agagcacgcc gagcggcttc acgctggacg acgtcatcca 1320
   gacaggcgtg gacaacccgg gtacgcgacc cctcggggcc ggggtcccgg ccccccctcc 1380
   ccccgcgcag ccgcagggtc ctcagcagcg cgctcgggcc cggcagtgac gtcactgtcc 1440
   ccgtcccgcg ccccctcccc caggccaccc gtacatcatg accgtgggct gcgtggcggg 1500
   cgacgaggag tcctacgaag tgttcaagga tctcttcgac cccatcatcg aggaccggca 1560
   cggcggctac aagcccagcg atgagcacaa gaccgacctc aaccccgaca acctgcaggt 1620
   gcggggctgc gggcgggccg ggcgggcggg gccggggtct tcgggcgctc actcccgtct 1680
   cgcctcccag ggcggcgacg acctggaccc caactacgtg ctgagctcgc gggtgcgcac 1740
   gggccgcagc atccgtggct tctgcctccc cccgcactgc agccgcgggg agcgccgagc 1800
   catcgagaag ctcgcggtgg aaggtagggg ccgggcgggc cgaggggcgg cggcggccgc 1860
   gtccccctcc cggcgcggtc cccgcccgct tttgtttacg tcgcccggga gcggcagccg 1920
   ccgtcgcgct cttatctgcg cgcgcccggg ttcagtttcc cggacccacc gagggacgga 1980
   ggcccagccc ccgcgcccac agcggcctgg ggcccaggga gggcgggtcc tggcgcgggg 2040
   tcaccgcctg ggaccgtcgc ccgggccgtg aggactggac gcccgcagat ccgggcgggt 2100
   ggggccctct gacgtccccc gaggtggggc acgggggcgg gcgggtccgc gctgcgggct 2160
   ggaggggcgg gcgcgggagc ccagcgtcct gagcgcaccc ctcgcagccc tgtccagcct 2220
   ggacggcgac ctggcgggcc gatactacgc gctcaagagc atgacggagg cggagcagca 2280
   gcagctcatc gacgaccact tcctcttcga caagcccgtg tcgcccctgc tgctggcctc 2340
   gggcatggcc cgcgactggc ccgacgcccg cggtatctgg tgcgtgtccc tctgcgccct 2400
   ctcgcggcgt cctccctccc cgctacctcc gctttccctc tcgcccccct cgcgggggtg 2460
   gggcccctcg cggcgaggag gaggaggagg aggagggagg ggccggccgc gctccgggtc 2520
   tgggttccgt gccgcgcctc ctcctgcgcc ggtgaccttg gccgagcagg tgcgttaagg 2580
   gactgggccc cggcccgtgg gggctcagga ctcagcaaca cctccccacc ccgagacgtg 2640
   aggtgggggc ggggctctct ggcgcctctc cccgacggcc ctgggagctg gagctctttg 2700
   ttttcttttc tcactcctcc gccgctggga ttctaccagg ggctggtgac gccaaagctt 2760
   ctccaggggc agggctccta cccccactgt ggggggcggg tcgggctgtc ctggcggtcc 2820
   ctggccccgc cccacctcgg gccacagcgc atgatggcag ctggggttct cctgctgtga 2880
   ggcgtcccgg ttcccccgcc cgccccgtgt tggcgggtgg agtcttggca gcagcctcca 2940
   ctcctgggca tggcagggag cagcacctca gggacttggg aagttccttt ggtctggggg 3000
   cggcctgggg cttttttctg ggtatgccct gagaccagcc ctcccgcagg cacaatgaca 3060
   ataagacctt cctggtgtgg gtcaacgagg aggaccacct gcgggtcatc tccatgcaga 3120
   aggggggcaa catgaaggag gtgttcaccc gcttctgcac cggcctcacc caggtgccag 3180
   ggacggggca ggcccagacc ccagggcccc agcagggatg tgggtgcccc agcatcagtc 3240
   cccccggggg atttccggca ctggggagtc tcagggcctg taggggtttc aggcaggcct 3300
   tctccctcat accctcttct ccgtctgcag attgaaactc tcttcaagtc taaggactat   3360
   gagttcatgt ggaaccctca cctgggctac atcctcacct gcccatccaa cctgggcacc   3420
   gggctgcggg caggtgtgca tatcaagctg cccaacctgg gcaagcatga gaagttctcg   3480
   gaggtgctta agcggctgcg acttcagaag cgaggcacag gtgagcaggg caggtgctgc   3540
   ggcttcccgt ggcctttggg cagccctgtt tcctccgccc tgacttgctg tctccccagg   3600
   cggtgtggac acggctgcgg tgggcggggt cttcgacgtc tccaacgctg accgcctggg   3660
   cttctcagag gtggagctgg tgcagatggt ggtggacgga gtgaagctgc tcatcgagat   3720
   ggaacagcgg ctggagcagg gccaggccat cgacgacctc atgcctgccc agaaatgaag   3780
   cccggcccac acccgacacc agccctgctg cttcctaact tattgcctgg gcagtgccca   3840
   ccatgcaccc ctgatgttcg ccgtctggcg agcccttagc cttgctgtag agacttccgt   3900
   cacccttggt agagtttatt tttttgatgg ctaagatact gctgatgctg aaataaacta   3960
   gggttttggc ctgcctgcgt ctgagtggtg cctctccttt cccagggggg agggggaagg   4020
   gcagcagcca ggccccagga gtcttgagtc ctgggcctgc tgtgggcctc gccttctgtg   4080
   agatgggaca agagccagga ggtggccact ctgttctgcc tgccctacct agtccatggg   4140
   ccccttccct cgtgtctatc gggctgtgca ggcaggaaca tgggagagag cgagggagga   4200
<210> 36
   <211> 531
   <212> PRT
   <213> Homo sapiens
<300>
   <308> P14618
   <309> 2004-10-25
   <313> (1)..(531)
<400> 36
   Met Ser Lys Pro His Ser Glu Ala Gly Thr Ala Phe Ile Gln Thr Gln
   1               5                   10                  15
   Gln Leu His Ala Ala Met Ala Asp Thr Phe Leu Glu His Met Cys Arg
               20                  25                  30
   Leu Asp Ile Asp Ser Pro Pro Ile Thr Ala Arg Asn Thr Gly Ile Ile
           35                  40                  45
   Cys Thr Ile Gly Pro Ala Ser Arg Ser Val Glu Thr Leu Lys Glu Met
       50                  55                  60
   Ile Lys Ser Gly Met Asn Val Ala Arg Leu Asn Phe Ser His Gly Thr
   65                  70                  75                  80
   His Glu Tyr His Ala Glu Thr Ile Lys Asn Val Arg Thr Ala Thr Glu
                   85                  90                  95
   Ser Phe Ala Ser Asp Pro Ile Leu Tyr Arg Pro Val Ala Val Ala Leu
               100                 105                 110
   Asp Thr Lys Gly Pro Glu Ile Arg Thr Gly Leu Ile Lys Gly Ser Gly
   115 120 125
   Thr Ala Glu Val Glu Leu Lys Lys Gly Ala Thr Leu Lys Ile Thr Leu
   130 135 140
   Asp Asn Ala Tyr Met Glu Lys Cys Asp Glu Asn Ile Leu Trp Leu Asp
   145 150 155 160
   Tyr Lys Asn Ile Cys Lys Val Val Glu Val Gly Ser Lys Ile Tyr Val
   165 170 175
   Asp Asp Gly Leu Ile Ser Leu Gln Val Lys Gln Lys Gly Ala Asp Phe
   180 185 190
   Leu Val Thr Glu Val Glu Asn Gly Gly Ser Leu Gly Ser Lys Lys Gly
   195 200 205
   Val Asn Leu Pro Gly Ala Ala Val Asp Leu Pro Ala Val Ser Glu Lys
   210 215 220
   Asp Ile Gln Asp Leu Lys Phe Gly Val Glu Gln Asp Val Asp Met Val
   225 230 235 240
   Phe Ala Ser Phe Ile Arg Lys Ala Ser Asp Val His Glu Val Arg Lys
   245 250 255
   Val Leu Gly Glu Lys Gly Lys Asn Ile Lys Ile Ile Ser Lys Ile Glu
   260 265 270
   Asn His Glu Gly Val Arg Arg Phe Asp Glu Ile Leu Glu Ala Ser Asp
   275 280 285
   Gly Ile Met Val Ala Arg Gly Asp Leu Gly Ile Glu Ile Pro Ala Glu
   290 295 300
   Lys Val Phe Leu Ala Gln Lys Met Met Ile Gly Arg Cys Asn Arg Ala
   305 310 315 320
   Gly Lys Pro Val Ile Cys Ala Thr Gln Met Leu Glu Ser Met Ile Lys
   325 330 335
   Lys Pro Arg Pro Thr Arg Ala Glu Gly Ser Asp Val Ala Asn Ala Val
   340 345 350
   Leu Asp Gly Ala Asp Cys Ile Met Leu Ser Gly Glu Thr Ala Lys Gly
   355 360 365
   Asp Tyr Pro Leu Glu Ala Val Arg Met Gln His Leu Ile Ala Arg Glu
   370 375 380
   Ala Glu Ala Ala Ile Tyr His Leu Gln Leu Phe Glu Glu Leu Arg Arg
   385 390 395 400
   Leu Ala Pro Ile Thr Ser Asp Pro Thr Glu Ala Thr Ala Val Gly Ala
   405 410 415
   Val Glu Ala Ser Phe Lys Cys Cys Ser Gly Ala Ile Ile Val Leu Thr
   420 425 430
   Lys Ser Gly Arg Ser Ala His Gln Val Ala Arg Tyr Arg Pro Arg Ala
   435 440 445
   Pro Ile Ile Ala Val Thr Arg Asn Pro Gln Thr Ala Arg Gln Ala His
   450 455 460
   Leu Tyr Arg Gly Ile Phe Pro Val Leu Cys Lys Asp Pro Val Gln Glu
   465 470 475 480
   Ala Trp Ala Glu Asp Val Asp Leu Arg Val Asn Phe Ala Met Asn Val
   485 490 495
   Gly Lys Ala Arg Gly Phe Phe Lys Lys Gly Asp Val Val Ile Val Leu
   500 505 510
   Thr Gly Trp Arg Pro Gly Ser Gly Phe Thr Asn Thr Met Arg Val Val
   515 520 525
   Pro Val Pro
   530
<210> 37
   <211> 10368
   <212> DNA
   <213> Homo sapiens
<300>
   <308> X56494
   <309> 1998-11-13
   <313> (1)..(10368)
<400> 37
   ggtcttcaca ttttgaatgc gcaacattgt atctgtgaat gaaggcaaga gttaacagct 60
   gtttaattga taactgctcg catcattagt tgctggctaa caactgggaa atcagaaaat 120
   gtcttgtaga aaaatgtaag aaaagttcca acaatactga cttaaacacg agcaaaggtg 180
   aaaacagaaa tgctgactcc tgcataggtt atcggcccta atgttctgac ttgatatttc 240
   cagatgccca gctctgcgct aatatcaaca ccgtctattt actttctact ctgaggcatt 300
   cgctctgcag gattccagac cctactaaat tattcacatg gccccaaccg gtccttcctt 360
   gttccgcggt cctaacacaa tgaatggtcc taagaggaaa acggcctcgg ctcccgctcc 420
   aggcccactt cgcagtccct agttctccct actgccgctc cagtgccaga gcccctccga 480
   aggcggccag gacctccaac cacgcacaag tctgcagctc tccccaactt tccgttcagc 540
   tcagtctccg agggtgcgcc agagcagaca cccggaggag tggggagtgg cagggcgggg 600
   ccgggagaat gctgccccgg aacccataaa ttcggccctg cccaggtagg ccgggacagc    660
   tggggtggcc tgggccgaga gccaagaaaa gagaccccat ctggacgccc aacttggcgg    720
   caacaggtgg ccggcgcccg ggggtctggg aggaaagtcg ctccgggcgg gccccgttgc    780
   cccgccgcgt ccccattggt catcaggttt cttaaaatgt gactctgaat ctgtgtcctt    840
   ccgccgcaga atttagtccc accgaaaggg caacctgccc gcgcgttccg ccaccgccgc    900
   cgcgcttcct cctgaaggtg actcgagccc gcggggacgc agggggcggg gcccgggtcg    960
   cccggagccg ggattgggca gagggcgggg cggcggaggg attgcggcgg cccgcagcgg   1020
   gataaccttg aggctgaggc agtggctcct tgcacagcag ctgcacgcgc cgtggctccg   1080
   gatctcttcg tctttgcagc gtagcccgag tcggtcagca gccggaggtg agcggtgcag   1140
   gcagtacgcc atcagtcccc accaagggcc agtcgcccgg ctagtgcgga atcccggcgc   1200
   gccggccggc cccgggcacg caggcagggc ggcgcaggat ccctgtgcta aatggtatat   1260
   taaccacttc tcagtcttac cactctcttt caatttgtct cgacccagga cctcagcagc   1320
   catgtcgaag ccccatagtg aagccgggac tgccttcatt cagacccagc agctgcacgc   1380
   agccatggct gacacattcc tggagcacat gtgccgcctg gacattgatt caccacccat   1440
   cacagcccgg aacactggca tcatctgtac cattggtgag tgggtgtccc ccttccccca   1500
   aaaagggctt catgggcagt gacctttctc tcctgaaaag agctccatgc actttttaaa   1560
   gacttttgag ctatttggga gaggaaaaat tttcagggaa aaaaattctt taaacttaaa   1620
   gcaaacttaa atgtttttcc ttggttgaat aattaatact tgtggcttta aaacttttcc   1680
   taataggccc agcttcccga tcagtggaga cgttgaagga gatgattaag tctggaatga   1740
   atgtggctcg tctgaacttc tctcatggaa ctcatgaggt gagctgtggc tggaccctat   1800
   cctggcaggg gaattggagc tggattctag tgtgggagca cgcttgtcat cttccttctt   1860
   ttcccccagt accatgcgga gaccatcaag aatgtgcgca cagccacgga aagctttgct   1920
   tctgacccca tcctctaccg gcccgttgct gtggctctag acactaaagg acctgagatc   1980
   cgaactgggc tcatcaaggg cgtgagtatt ctgcggagag cgaggggaag gctcagtagg   2040
   caatatgccc cagagacatg attccttccg aggtgatgct gctactggtg tctccagttt   2100
   ggactcttcc ttactctctt gtccctagag cggcactgca gaggtggagc tgaagaaggg   2160
   agccactctc aaaatcacgc tggataacgc ctacatggaa aagtgtgacg agaacatcct   2220
   gtggctggac tacaagaaca tctgcaaggt ggtggaagtg ggcagcaaga tctacgtgga   2280
   tgatgggctt atttctctcc aggtgaagca gaaaggtacg tatgggagct ggagtccagt   2340
   tgtctaaaac agtcttttgt ctctaaactt ctcgtctctg cctccccaac ttaccctttt   2400
   ttatacaggt gccgacttcc tggtgacgga ggtggaaaat ggtggctcct tgggcagcaa   2460
   gaagggtgtg aaccttcctg gggctgctgt ggacttgcct gctgtgtcgg agaaggacat   2520
   ccaggatctg aagtttgggg tcgagcagga tgttgatatg gtgtttgcgt cattcatccg   2580
   caaggcatct gatgtccatg aagttaggaa ggtcctggga gagaagggaa agaacatcaa   2640
   gattatcagc aaaatcgaga atcatgaggg ggttcggagg caagtccccg ttgtccctgg   2700
   tctactgcca tacttgtggc ctctgttcta tataacctct ctccccccca ctttgtccat 2760
   caggtttgat gaaatcctgg aggccagtga tgggatcatg gtggctcgtg gtgatctagg 2820
   cattgagatt cctgcagaga aggtcttcct tgctcagaag atgatgattg gacggtgcaa 2880
   ccgagctggg aagcctgtca tctgtgctac tcaggcatgt gcccaccctt ccccacattc 2940
   tcatgtgcac actcgcatgt ttgtatggga aagctctgga ggctgtctga tctcttccca 3000
   tggaattgtc gcaacgtaac acacagataa tccccttccc ccatgtacct acacaaagcc 3060
   atactctgtg tacctactca ctatccagag gatcagcttg ctgtcatttg tctctgaaga 3120
   cagctcaagc tacatctcac taatgctctg tcccctccca gatgctggag agcatgatca 3180
   agaagccccc gcccactcgg gctgaaggca gtgatgtggc caatgcagtc ctggatggag 3240
   ccgactgcat catgctgtct ggagaaacag ccaaagggga ctatcctctg gaggctgtgc 3300
   gcatgcagca cctggtgagt tctgggcctg ccccatcccc cagggcttcg gactgggcct 3360
   gggatggatg caagctctgg tgcagagctt tttaggtttc tccatcctct tatgcacagc 3420
   ctttcattat cctccaagtt acagcagcaa gagggtgggg gtggaagtgg aggtggcttt 3480
   ttttttttct cctgttcctg cattcctgcc cacaccccca cccctctcat ttccttctgc 3540
   tctggaggca cctccttcat tggacaccac acagtttatt tcacttctga cttcaaggtt 3600
   gtgaattctt cccatggctt aagtcctggg atacttctgc agtgaaagga ggtcttgtac 3660
   ctcttcctca gagtcagaag ttctgagtac ctttgcccta ttctgaaaag ggctaggggc 3720
   tcctgctccc agctgccctc ttcctttggc ttccaattca gttccctctg ccccgcatcc 3780
   tgcagacagg cgctcccgca gggggccctt gtggacctgc actggagtct gttgccttca 3840
   ctgagctgcc tgtgctggcc ttgcatggtg cctgtagggg gatttgcttt gctgtgccat 3900
   tggggtacag ctgctgctct tactctagac caaaaagtcg ggttgagtga ctggtggcag 3960
   ggccaagata gagacagcgg ggagggtggc tgaccctggc ggccctggac tgagcgtctg 4020
   gaggagtcgt ggaggctctt tcccttcttt ctcctctgag agctcgttct tcaggctctt 4080
   ccagcttgtc atgtcgagtg cctggccact gctcagggtt ggaggctcag tccctttgcc 4140
   ctgtctgttc cagctctgga gctaactcag ggatccctga tcagggttac gtaggtttgg 4200
   taaaatgagt gctggaaatt aactttctcc cagtagtctt aggtctagct cagtgaactt 4260
   aaactttatc cagatatggt ttttccttca gcctttctat tccctttcta gccagtgaaa 4320
   gacccgctgc cctttgacct cagccccctc caagccccca agtttaaaac gccaccccct 4380
   gccaccagaa aaaacagaaa aaaaaaaaaa aaaaaaaact aaaacaccca tctggtctgg 4440
   gcatcttcct tcctttttca ctatgtatcc tgttactggg cttaaacagc tttcagagaa 4500
   gagatgtcat ttctattaaa tgctctttca gtagcgaact gagttcacac ttgactaagg 4560
   atattttccg gactgtctgt catcagcatc cttagtgggt ttccccatat ttaaattggt 4620
   agaggccagg gatggtggct cacacctgta atctcagtac tttgggaggc caaggtaggt 4680
   ggattgcttg agctcagaag accagcctgg gcaacctggt gaaaccctgt ctctactaaa 4740
   aattcaagtt agctagctgg gcatggtgat gcacttctgt agtcccagct acttggagag 4800
   ggggtggtgc tggggcagca ggatcgctta aacccaggag gttaaggttg cagtcagcca 4860
   agatggtacc agcctaggtg acaaagtgac accctgtctc aaaaaagaaa ccaaacaaac 4920
   ataaaaaaaa aaacaaaaaa atcggtagag agtgatttct ctcccaggcc cacttaatgt 4980
   agactgggcc tggctgacac ctcaccattc gtgtgatgtg attgctgttc tgatgcttag 5040
   atactcttgg cgcagtctca caattgccac catggtagga aggtgtccag gagacggtgc 5100
   accttgaacc agtcaccact aaagtggctg cctttctggg tctctccaca catcccctct 5160
   ctctaatttc cctacttaat cgtgtgactt catggtctca aaggaggaac agaggctgat 5220
   cttgacttag atatactgaa ccatgaaatc actgcataga atgtggggac ttgaatgtgt 5280
   ctttgggcaa gtcatttaac ctcttaagac ctcatctgta aaatggatta gatatgttta 5340
   attatagcct tagcattaaa tattcattgc tgttattatt aagtgtctga taagtctctg 5400
   tgtacatgga tgtaatcttc ctaactccca ttacctccat ttatagatga gggttatatg 5460
   gccaataaag cctgggtttg aatctaggtc tactgcctcc aaagccagtc ttctctcctg 5520
   caacatcatg ctctgtctag caggagatga gaacaggtct ccatttggag cctgtcagtg 5580
   gggtcagaga ctaagattca ggctcagggt ctaaattccg tatcctttct tccataccct 5640
   ggtgtttcct atgaacagat agatacttta gggctgcaag gtttggattg catggcactg 5700
   ctcagaagat aagttacagg tctgggctag gctgtagctg cccctccagg tggctagacc 5760
   tttcctttct gtgtcaccag ttaacactgg ccaacagttc cttccattaa ctgttcactg 5820
   ctttctcctg tgtctaactg atgcagttta tgacccataa ctaagagcag taccaggtat 5880
   ggctctgttt cctgttcatg tcccctgtcc tctgggctgc atgcattccg ttcttacaga 5940
   aagaatacct ttaacctagt acatcctgcc acacatctgc ttctactgtg aaattgatga 6000
   gggggtatta ccgattcttc cctctcccat catttactga gatgctggtg attgcattat 6060
   aatcctctta agcttacatt gtctttctga ttcttggtct tatctgagca agtgatctat 6120
   aaataactca gtggctttct catgactgtt ttaattatta gattttaatc aagtgtctta 6180
   ttaaatatat ctgcatgctt ccacaggcat ctgtctcttc acatggctgt tcagtgtgcc 6240
   tctcacaact tagcccaaac tcagttgagc tgccttgctt tggctttgac ccagctttcc 6300
   agcgctgctc aatctgttgc catggcaggc cattggaaag gctcagttca tccccgtgcc 6360
   tgaagccaag tgagcgctca ctccatgcat gcatggaggc tgggcaggag cctgcctaat 6420
   caaccagcca tgtgaggagg gagggcctgt tccttcctgt aagctatgtc atgaggcagc 6480
   gtggtcaagt cctctgccag ggagtggcct ggcccagcct gggcatgttt tcatgccagg 6540
   gtgctagagc ctactgccag attgtctccc tccaccccca atgaaaaaat ccttccagaa 6600
   gggaagagcc aatttcccct gtattggagg ggaagtggca gcacctcctg aagcagttgg 6660
   actttcatca ccctacctct gcatctgcct gaaggacaga tttagccaat taacctaagg 6720
   ttaccttcct ctctgataaa ttccccattc tgtcttccca tgtgttgtgt ctcgtttttt 6780
   tcctcctcct tccctcttcc ttgccccctc ttcccctaaa ccttacagat agctcgtgag 6840
   gctgaggcag ccatgttcca ccgcaagctg tttgaagaac ttgtgcgagc ctcaagtcac 6900
   tccacagacc tcatggaagc catggccatg ggcagcgtgg aggcttctta taagtgttta 6960
   gcagcagctt tgatagttct gacggagtct ggcaggtagg gccctaaggg caggtaacac 7020
   tgttaggata accagcctct tgctgcacct gccccaggag aagagagaag gcccaacctg 7080
   gcatctggga acagagcctc ttctcgtctg taggaacacc gccagggagg tcatggcagg 7140
   gcagaccaaa gggtcctgtg gctcagtagg cacagtagat gtcacaggca cttggtgaag 7200
   gactggtttc tgtggagtct tgatcttggc tcagctcaga atctccagtg attgggctcc 7260
   tcttggcctt tgttcccagg aacatgttcc tcaccagctg tccggtgact cttcccctcc 7320
   ctctcctttt gtgacaaagc tctgacaaag ctctgtcccc ctctcgtccc tctggacgga 7380
   tgttgctccc ctagattgcc cgtgaggcag aggctgccat ctaccacttg caattatttg 7440
   aggaactccg ccgcctggcg cccattacca gcgaccccac agaagccacc gccgtgggtg 7500
   ccgtggaggc ctccttcaag tgctgcagtg gggccataat cgtcctcacc aagtctggca 7560
   ggtaggaggc ggcagcggct ccctggaatg ccctgctcag tggtacctca ccttgggggt 7620
   cctgggagca gtccattgaa caatgctcag gtggcactga gccaaggtaa gacccctctg 7680
   cctgccacct tgggcctgca gggaaggatt gagcagagcc ccttcccagg gcccaaagga 7740
   ctctaggtag cactcataag gaatgtcaga acatttggat caaaagcaaa tttatgctgg 7800
   agatttatta cataacagtg cacaggctga ctacaaatgg ttatttgata ttgaaaattt 7860
   agtcctctaa aattgtaaaa gataccactt ttgcttattc cagttactat gtgctcttta 7920
   aaaatttcag ttgggaaatg aatttattta aatgctgttt actgtgcctc catttggcac 7980
   actagtccct gctgtttttg agccctaaag acaaattggg ttccagctca ggagaggttg 8040
   ctgtgctatc ttggctgaca ttctgtgggc ctggcagcca ggctgaggac tgtgtggcct 8100
   atgctgggcc tccaacttgg gatcccttcc ttggcccagg acattgagtt aatgtccttc 8160
   actctcctag ttagggagta tgctccttgt ccctgtccac aggggagcaa gggtttcctg 8220
   gaagagggga gcaaacaggc agtgcccatg cactgaggag cagcagatgg gcgtgggcag 8280
   cccagagaac caggacacaa gctctgtgca gatccctcag cagagggctc cagcctccca 8340
   ctcttggctg aacagctcca acccgtaggg ttgacctttc ttaaaaggtc cagttcttgc 8400
   tgtttggcta ttttaagctc tagtcttctg gggtttcact cagctggtcc tggcttcagc 8460
   aattgcttcc ctctgaaggc cttgcataga ggccaagcgt gaagtgcagg gacttctctg 8520
   ctgtgatgtg gcttaagttt ccctgacacc tgttgagtgt cctcataact tcccttctgg 8580
   tgcccctccc cagctcctga gaccagctgc agctacaagt gtgcagtgtc agtgttcaag 8640
   aaagtgcctg gcagaggggc tttagaaggg tcccctgcct tccaaaggag ctttggcagg 8700
   cagacgtgct cctgcagcaa cactcccatt tcctgttctt gcctgctgag tagcacctag 8760
   atttctaagc ctcatctaga tactcagatt tgattctggg cctttatagc ccagttgctg 8820
   ggactgtttc aggagctagg ggccatgtgg ggcagggaga gggcacaaaa gtagagaagc 8880
   ctgatgttga ttcccagggg gctggtcagc tctgctactg ctccttgcag atgtcaagag 8940
   tcaggtgcta gtcacgtgct gcttggcttg tcactgtcat tggcagcgag aggaatgggt 9000
   gctggtgaca ttgggccagg gctgcctctc tgtgtcagag ttcagggtgt aggaggggtt 9060
   ctgccaacca tgggctgtgt ggggtaagtg ggttgaggct gatctttctg ggtcaaggtg 9120
   atcctgagcc cttgcctgtg gaatgggggt agagggcaat ggtaacctag ctagcatgct 9180
   gtgggggata taggatgagg ggctgcccga ccctcgggag gggtcctagg gagcagatgt 9240
   tgaagaggcc agagccctca gtgagctgga tgagggggtg agccgtttga actccctgag 9300
   ggtacttcct ggggcctcgt gtaatggtct cttctgtatg tcccccatcc catctcaggt 9360
   ctgctcacca ggtggccaga taccgcccac gtgcccccat cattgctgtg acccggaatc 9420
   cccagacagc tcgtcaggcc cacctgtacc gtggcatctt ccctgtgctg tgcaaggacc 9480
   cagtccagga ggcctgggct gaggacgtgg acctccgggt gaactttgcc atgaatgttg 9540
   gtacgtggct ggagcagggg ctagagccta gaggagcttg gggatgcttg agcattggcc 9600
   accaacctcc cttctcttcc tccaggcaag gcccgaggct tcttcaagaa gggagatgtg 9660
   gtcattgtgc tgaccggatg gcgccctggc tccggcttca ccaacaccat gcgtgttgtt 9720
   cctgtgccgt gatggacccc agagcccctc ctccagcccc tgtcccaccc ccttccccca 9780
   gcccatccat taggccagca acgcttgtag aactcactct gggctgtaac gtggcactgg 9840
   taggttggga caccagggaa gaagatcaac gcctcactga aacatggctg tgtttgcagc 9900
   ctgctctagt gggacagccc agagcctggc tgccccatca tgtggcccca cccaatcaag 9960
   ggaagaagga ggaatgctgg actggaggcc cctggagcca gatggcaaga gggtgacagc 10020
   ttcctttcct gtgtgtactc tgtccagttc ctttagaaaa aatggatgcc cagaggactc 10080
   ccaaccctgg cttggggtca agaaacagcc agcaagagtt aggggtcctt agggcactgg 10140
   gctgttgttc cattgaagcc gactctggcc ctggccctta cttgcttctc tagctctcta 10200
   ggcctctcca gtttgcacct gtccccaccc tccactcagc tgtcctgcag caaacactcc 10260
   accctccacc ttccatttcc cccactactg cagcacctcc aggcctgttg ctatagagcc 10320
   tacctgtatg taataaacaa cagctgaagc acctgtttcc tctctttt 10368
<210> 38
   <211> 201
   <212> PRT
   <213> Homo sapiens
<300>
   <308> Q01995
   <309> 2004-06-15
   <313> (1)..(201)
<400> 38
   Met Ala Asn Lys Gly Pro Ser Tyr Gly Met Ser Arg Glu Val Gln Ser
   1               5                   10                  15
   Lys Ile Glu Lys Lys Tyr Asp Glu Glu Leu Glu Glu Arg Leu Val Glu
               20                  25                  30
   Trp Ile Ile Val Gln Cys Gly Pro Asp Val Gly Arg Pro Asp Arg Gly
           35                  40                  45
   Arg Leu Gly Phe Gln Val Trp Leu Lys Asn Gly Val Ile Leu Ser Lys
       50                  55                  60
   Leu Val Asn Ser Leu Tyr Pro Asp Gly Ser Lys Pro Val Lys Val Pro
   65                  70                  75                  80
   Glu Asn Pro Pro Ser Met Val Phe Lys Gln Met Glu Gln Val Ala Gln
                   85                  90                  95
   Phe Leu Lys Ala Ala Glu Asp Tyr Gly Val Ile Lys Thr Asp Met Phe
               100                 105                 110
   Gln Thr Val Asp Leu Phe Glu Gly Lys Asp Met Ala Ala Val Gln Arg
           115                 120                 125
   Thr Leu Met Ala Leu Gly Ser Leu Ala Val Thr Lys Asn Asp Gly His
       130                 135                 140
   Tyr Arg Gly Asp Pro Asn Trp Phe Met Lys Lys Ala Gln Glu His Lys
   145                 150                 155                 160
   Arg Glu Phe Thr Glu Ser Gln Leu Gln Glu Gly Lys His Val Ile Gly
                   165                 170                 175
   Leu Gln Met Gly Ser Asn Arg Gly Ala Ser Gln Ala Gly Met Thr Gly
               180                 185                 190
   Tyr Gly Arg Pro Arg Gln Ile Ile Ser
           195                 200
<210> 39
   <211> 1822
   <212> DNA
   <213> Homo sapiens
<300>
   <308> D84342
   <309> 2002-12-25
   <313> (1)..(1822)
<400> 39
   ccgggtgaaa gcagagtgct ccctgaccct ctgcccctcc ctcctccacc ctggcctgct     60
   ttagctttcc ccagacatgg ccaacaaggg tccttcctat ggcatgagcc gcgaagtgca    120
   gtccaaaatc gagaagaagt atgacgagga gctggaggag cggctggtgg agtggatcat    180
   agtgcagtgt ggccctgatg tgggccgccc agaccgtggg cgcttgggct tccaggtctg    240
   gctgaagaat ggcgtggtga gtggcaccct gggctagggc gctggggggc tggggtgtga     300
   ccccctgtga gtcctgggcc aatccctgag gactgctaag ctgcgtccta tgccctatgc    360
   ctggtagatt ctgagcaagc tggtgaacag cctgtaccct gatggctcca agccggtgaa    420
   ggtgcccgag aacccaccct ccatggtctt caagcagatg gagcaggtgg ctcagttcct    480
   gaaggcggct gaggactatg gggtcatcaa gactgacatg ttccagactg ttgacctctt     540
   tgaaggtaga gaggagaatg ctgggggagg aggtgggcag gaggacaggg tgctgggaca    600
   gggagagggt atgaccaaat atgccacaac taggggtgtg ctcgcccgca cacagcaggg    660
   atgggatatg ccgagaataa cacgccacgc tcacagggcc cactgagagg cctcccttga    720
   attggggaca actcttggcc ctggtttggc catttttttg tgagagacgg gggcaggccc    780
   tggcttggag tcttgtttat acgttcttga tgttcatctc ctctctcctg tcttctcaca    840
   ggcaaagaca tggcagcagt gcagaggacc ctgatggctt tgggcagctt ggcagtgacc    900
   aagaatgatg ggcactaccg tggagatccc aactggttta tgaagtatgt ggcccccagg    960
   gagcttgagt ctccgcatgg ggtgggaggt ggcttgttct aaggagcttg cgggaaggat   1020
   taggggaagc agatagccaa gaaaggataa agtgagggtc tgggatgggg aataatgggt   1080
   ccttaatact ccttgacccc tccctttcca ccctcctgcg ctcagtctcc ctagcctatg   1140
   aggcaagcta gattagggaa aaaaagtgca acaggaaggc aatgggattg ggctaggacg   1200
   taacagaggg atcagaaaac gggtggaaaa cacacagttc taccaagtct ttatcctgct   1260
   tcctcctctt ctaggaaagc gcaggagcat aagagggaat tcacagagag ccagctgcag   1320
   gagggaaagc atgtcattgg ccttcagatg ggcagcaaca gaggggcctc ccaggccggc   1380
   atgacaggct acggacgacc tcggcagatc atcagttaga gcggagaggg ctagccctga   1440
   gcccggccct cccccagctc cttggctgca gccatcccgc ttagcctgcc tcacccacac   1500
   ccgtgtggta ccttcagccc tggccaagct ttgaggctct gtcactgagc aatggtaact   1560
   gcacctgggc agctcctccc tgtgccccca gcctcagccc aacttcttac ccgaaagcat   1620
   cactgccttg gcccctccct cccggctgcc cccatcacct ctactgtctc ctccctgggc   1680
   taagcagggg agaagcgggc tgggggtagc ctggatgtgg gccaagtcca ctgtcctcct   1740
   tggcggcaaa agcccattga agaagaacca gcccagcctg ccccctatct tgtcctggaa   1800
   tatttttggg gttggaactc tc                                            1822
<210> 40
   <211> 355
   <212> PRT
   <213> Homo sapiens
<300>
   <308> Q14103
   <309> 2004-10-25
   <313> (1)..(355)
<400> 40
   Met Ser Glu Glu Gln Phe Gly Gly Asp Gly Ala Ala Ala Ala Ala Thr
   1               5                   10                  15
   Ala Ala Val Gly Gly Ser Ala Gly Glu Gln Glu Gly Ala Met Val Ala
               20                  25                  30
   Ala Thr Gln Gly Ala Ala Ala Ala Ala Gly Ser Gly Ala Gly Thr Gly
           35                  40                  45
   Gly Gly Thr Ala Ser Gly Gly Thr Glu Gly Gly Ser Ala Glu Ser Glu
   50 55 60
   Gly Ala Lys Ile Asp Ala Ser Lys Asn Glu Glu Asp Glu Gly His Ser
   65 70 75 80
   Asn Ser Ser Pro Arg His Ser Glu Ala Ala Thr Ala Gln Arg Glu Glu
   85 90 95
   Trp Lys Met Phe Ile Gly Gly Leu Ser Trp Asp Thr Thr Lys Lys Asp
   100 105 110
   Leu Lys Asp Tyr Phe Ser Lys Phe Gly Glu Val Val Asp Cys Thr Leu
   115 120 125
   Lys Leu Asp Pro Ile Thr Gly Arg Ser Arg Gly Phe Gly Phe Val Leu
   130 135 140
   Phe Lys Glu Ser Glu Ser Val Asp Lys Val Met Asp Gln Lys Glu His
   145 150 155 160
   Lys Leu Asn Gly Lys Val Ile Asp Pro Lys Arg Ala Lys Ala Met Lys
   165 170 175
   Thr Lys Glu Pro Val Lys Lys Ile Phe Val Gly Gly Leu Ser Pro Asp
   180 185 190
   Thr Pro Glu Glu Lys Ile Arg Glu Tyr Phe Gly Gly Phe Gly Glu Val
   195 200 205
   Glu Ser Ile Glu Leu Pro Met Asp Asn Lys Thr Asn Lys Arg Arg Gly
   210 215 220
   Phe Cys Phe Ile Thr Phe Lys Glu Glu Glu Pro Val Lys Lys Ile Met
   225 230 235 240
   Glu Lys Lys Tyr His Asn Val Gly Leu Ser Lys Cys Glu Ile Lys Val
   245 250 255
   Ala Met Ser Lys Glu Gln Tyr Gln Gln Gln Gln Gln Trp Gly Ser Arg
   260 265 270
   Gly Gly Phe Ala Gly Arg Ala Arg Gly Arg Gly Gly Gly Pro Ser Gln
   275 280 285
   Asn Trp Asn Gln Gly Tyr Ser Asn Tyr Trp Asn Gln Gly Tyr Gly Asn
   290 295 300
   Tyr Gly Tyr Asn Ser Gln Gly Tyr Gly Gly Tyr Gly Gly Tyr Asp Tyr
   305 310 315 320
   Thr Gly Tyr Asn Asn Tyr Tyr Gly Tyr Gly Asp Tyr Ser Asn Gln Gln
   325 330 335
   Ser Gly Tyr Gly Lys Val Ser Arg Arg Gly Gly His Gln Asn Ser Tyr
   340 345 350
   Lys Pro Tyr
   355
<210> 41
   <211> 14983
   <212> DNA
   <213> Homo sapiens
<300>
   <308> AF026126
   <309> 1998-06-13
   <313> (1)..(14983)
<400> 41
   tcgcagaggt gcagccacac cccggcctaa cgtgttgttc cccccgatac tggagtggtg      60
   gggagggtga gtggactcca ggaatcctcg gaagggcggg ggcggaggca gggggcccct     120
   ctagccgcta cttcgaaaca gcattccttg ttctcgatgg tccccgcgcg actgtcttag     180
   ctcacgacac ttccggttcc ttttaaaggc ccccaaggct gtgcaacgcg gagcgtgaga     240
   ggaaggtata aagggtagcg agagggcggg accgaggagg aaagggaaaa aaaaaaaact     300
   agggggatag gggtgggggg acgcgcgaag ggcgcgctct cgcgtcacgt gaccgggacg     360
   cgccgttctt ccgtcggcca ttttaggtgg tccgcggcgg cgccattaaa gcgaggagga     420
   ggcgagagcg gccgccgctg gtgcttattc ttttttagtg cagcgggaga gagcgggagt   480
   gtgcgccgcg cgagagtggg aggcgaaggg ggcaggccag ggagaggcgc aggagccttt     540
   gcagccacgc gcgcgccttc cctgtcttgt gtgcttcgcg aggtagagcg ggcgcgcggc     600
   agcgcgggga ttactttgct gctagtttcg gttcgcggca ggcgggtgta gtctcggcgg     660
   cagcggcgga gacactagca ctatgtcgga ggagcagttc ggcggggacg gggcggcggc     720
   agcggcaacg gcggcggtag gcggctcggc gggcgagcag gagggagcca tggtggcggc     780
   gacacagggg gcagcggcgg cggcgggaag cggagccggg accgggggcg gaaccgcgtc     840
   tggaggcacc gaagggggca gcgccgagtc ggagggggcg aagattgacg ccagtaagaa     900
   cgaggaggat gaagggtgag taaggggcat cccaggatag tcaggcccaa ctagtccccc     960
   tccccctctt tattcccccg ccattagcgc tggttcccgc tctcagcccg ttctccgggc     1020
   cccatgcagc ctccttgcac tggtctccct ttttctatgt tgggttcccc ggaccttcat    1080
   ttccttcttc ctttgcctgc ttatcgcccc cctcccccat cacacacgtt tccaccttta    1140
   gccgtcacca tgctgctcct cggcccgccc tcctttcctc cctcgccatg ggtctcctcc    1200
   caccgactta gccgccagat tttttcccgc ctgtcgtggg gtaccttttt tctttccatg    1260
   ctgtcccctc tttttccctt tttctacagt cttgggcata aaaacacaga cacaaacagc    1320
   ttctctgttt gtattactaa ggtttatttg gtgcttctcc accatcctga aacgatgcga    1380
   ttgtttataa gcacatgttt tggggaacgc gtaggctgtc cacctctgcc tctcccttgt    1440
   cggccttacg ccgactgttt tcttgggatt ttgaataagt ttcgcctaag gatttactca    1500
   ttttctccac catacgctat cgcacaatgg catactcata caggccctac attttgacat 1560
   gcagaccaaa ttgggtctgg tgaaatgctc cgagtttctt gttggtacat tggttttgct 1620
   ccgcgggctc tggttaagtt cttagtcgat cgggcctgca cttgactgga gctgttccta 1680
   tctccggcct agcatctacc cttcccccac cccactgagt tattctaacc gcgcaccctt 1740
   ttcgcgccct cagctattgg gttccccaac tgttagtaca gattgtacct tactttttat 1800
   gtgcaaccta attttttaca acccccagcc cccttttttt cccggtgccc aggatcctat 1860
   tttggtgtct tgatatctgt ttcccgccca ctaagggagg cctgtagtcc tcttaagaga 1920
   aacaaatcac tgtattgtgc tatgggatac tttttttttc tttttggtgc aaatttctta 1980
   gtattaactt gcttcctcca attgaaataa cagttgtata tactacctaa atctgcattt 2040
   agtgtattaa aatgcagcat ttgggattgg gaacataata cgggagttag caggagggac 2100
   taaatcaggt ttcttggttg agttttctta gctccgatta ctgaccgatg atcgtgggtt 2160
   cacgcttcca ttcctttttg taatggggag gggtgtgtgt gtgtctgaat ttttttttaa 2220
   ctgtaaagag aaaggtgttg gtggatataa caagatgtcg ctttaatcta gtattagaaa 2280
   acacgatttc ttttactgag aaagagccca ggatttggag ggaaagttgg gaggggagaa 2340
   gcactttgga atatagggtt tattaagccc agcttaggca gattcttgtc aggctgtttg 2400
   atgatagtgc tggttttggg ggagtggtgg tgggaaggga agcaaacttt taaaaattaa 2460
   acagaaccaa ctcgaattgt atatagctta tttgagaaag gaagatgtta gatatttgga 2520
   aacttaaaac ctttaacatt ttggttttcg tttgctgaat tctgttcttt ggacagaggc 2580
   accaaaatga ttaattgtat aacttcctgt ttggggcagg ctttctgtta gccctgataa 2640
   ttcaaatcgc aaagcagctg actttatagt tacctactgt tgaagtgtaa atgaattaaa 2700
   gtttttaacc ctaacagtgt caaaaactaa aactagaatt ttgattggtt gctgtaccgg 2760
   ttgttaattc cctagccatt caaactcctc cccacgacac tctgaagcag cgacggcaca 2820
   gcgggaagaa tggtaaatct tttaaatttt atttctgtat tataaaggtt tcagtagtca 2880
   taatatgtag aggctgtgta ttggttagtt gcccattgat ccccaggaaa ttctagacca 2940
   tagtacatac agtaggtttg tacctgggat atataccttt tataggcatc atgtgtaatc 3000
   tttgggcaaa attgatgccg ttacatggta tttggtctgt ggtaatgcat gtagttgcag 3060
   tttggcaatt taagctaaac aattagtaac gatattttta tatcgagcaa gaaaaaatat 3120
   taccgtaatc ttcacatttg atttatttta agatgaaaaa tatttttgtt ggaatttaag 3180
   tttaatggct aaatatgaag actccttgat ataaaatatt gtgtttaaaa tgtcatgcat 3240
   gttatactga aatattctgg agatgtctga agcagttctt taagcagcac tgtgtattat 3300
   ggaaaaatgt ttgtctttta cctagcatat ttgtaaatag acctttaata aaatgtgtgt 3360
   gtgtgtgtgt gtgtgtgtgt gtgtatatat atataccatt aaaaggtggg ctaatgtggc 3420
   tttccactac agctcttacc agatttttta ttttcagtgt gatttggctg gaatattaat 3480
   aacagttggt tgacatgtat tcacaattgc atgacttctc tttggaagct ggtaggttga 3540
   gtattgttct ttttatccat cctttcccct cccctaaatg tcagtctttc tgcttttagg 3600
   attatatttg aattttcagg ggtttataca ccaccactgg agagtagaag gccactaact 3660
   tgcaggcagt taacttatat ctttacaagc tacattctat tttaagagcc tatttagggt 3720
   aacatttaaa gatttgctta tcactgtatt ctcatccact gaaatcagtc cagctttacc 3780
   ttggagaaaa agcagatggg aaacaggcaa ggggaaaagc aaaaaagagg aaaaaaaaag 3840
   actggggtca cagtttcgat gaatgttatt ccctgtattt ggtgagtggt gggcactagc 3900
   agctcaattt cactgctagc taatagtggc aaattaagat taaactataa actatttgac 3960
   attactccat tatgtatttt gagtctcatg taattgtttc cagtgatttg aaatgaccat 4020
   aaacttaaat ttgaaggtca gtgttttgac tcttcgaagt aattacactt gacctgctac 4080
   ctaaaacgat gtaatattac aacttttttg aataatctca ggaaaaatgg agaaatgttt 4140
   atattcatag ttaataaaca ttctagatag taaccaactg tcatttactg aaaataaaat 4200
   tttacccagg tttattttat ttttgagacg gagtttagct tttgttgccc aggctggagt 4260
   gcagtggtgt gatcttggct cagcgcaacc tccacctccc aggttcaagc aattctcctg 4320
   cctcagcctc caaagtagtt gggattacag gcatattcca ccatgcctgg ctaattttgt 4380
   atttttagta gagacagggt ttcttcatgt tggtcaggct ggtctcgaac tcccgacctc 4440
   aggtgatccg cctgcctcgg cctccccaag tgctgtaatc acaggcgtga gccactgcgc 4500
   ccggcctagc caggttgatt ttaaaattac acttaaaaat aatgttctca tttttaaggg 4560
   ctctaatatt tgacttttct taactttcca attatgcagc cctatcctgt tccagacgtt 4620
   aaggctttct ttttgaatga aaaactttca gacttttttc tctctctttt tttttttttt 4680
   tggtgtctgc actccccacc gtatctgtcc cttcctcttt ctcccatttt gagggatatt 4740
   ataagggcaa cagttttata gtctcaatat tgagggtaag actggttaat agatagagta 4800
   tccaaattga atttattaaa cacagttctg tgtgcatttc cttctgcaaa tctgagaata 4860
   aagtgattac agtttcatcc taaaatactt taaaaatcag ttggttttag aaataggttt 4920
   ttttcctttt gcatgtaaga atggacagct atttttagga aggcatgcac ctgttatccc 4980
   agctactcag gaggctgagg caggagaatt gcttgaaccc gggaggtgga ggttgcagtg 5040
   agcctagatc atgccattgc actccagccc gggcgacaga gtgagactct gtttaaaaag 5100
   aaaaaaataa gtatttgtaa atgtatatat atatatatat acacacacac acacacatat 5160
   atatataaaa tcattatgcc atcagtttga gggagagcat gtatgccaac attgtaaaga 5220
   ctaagtgccc atttatccat aattaagata agcagacttt tccccattgt actataaagt 5280
   tagagtttgg ggctgggtat ggtacttccg cctgtaatcc cagcagtttg ggaggctgga 5340
   gcgggcggat cacttgaggc caggagttca agaccagcct ggccaacgag gggaaacccc 5400
   gtctctacta aaaatgcaaa aatcaagtca ggcgtggtgg tgcagcctgt aatccctccc 5460
   acggaggctg aggaatgaga atcactttga acctgggagg cagaggttgc agtgagctga 5520
   gattgtgcca ctgcgctcca gcttgggcga cagagtgaga ctgtctcaaa aactaagcaa 5580
   ccagaaacag aaaaagatgg aatggaatta atatgttcca tttagtaaag atggaataga 5640
   attggtccag tttgtgttca ttgtagcata ttcacctatt tggtctgcaa ccattttttt 5700
   ttttttaaag cctaagcata ccctgagaac aacaaccttt tttgattaga acagaagaaa 5760
   aagccagaat gttcttgggc tgccaaaata ttaatacgtt tgtctcaaag acgaaccact 5820
   taggtgcttg cttagagact tctactttac acagaatttg aaaagtttga cagctggcta 5880
   cttactctgc aaaacggaag ggagactttt tagaacagca actggtgatc tggcaaaaat 5940
   gaaaagtaga acttgtgaag aaatgagatg gacttggtgt attaatagct aattttagaa 6000
   gaccctgtgg atattctaaa tcagcataat agtgatgtct gagccatagt gttaaattat 6060
   gattactgta tttgaagtat aagaaggcag aaaagtgtga tgctgttaga aaaaaatctc 6120
   atttcaaatg attgaaggtt aaaaacaatt ggggaaagat tagagagggc aggcggtgct 6180
   ttaagggagg tggaataata cttggctcct tattctccaa aagtgctgga tagctgaagt 6240
   ttttaatatt ctggataatt gatatttgat tattagttta ggagggatag catcttcagt 6300
   gaacccttgt agctctaagg taggcatatt aaacaactac catactaaag gtggggagta 6360
   cgtttggaga gcactttgct gggccagctg atgatatgct taggtgatat ttaagaaaat 6420
   ctgcttcttg tctgaaaaac atttggggct tcagaataat accacatacc tcattcttgg 6480
   tgttttaatt tatttctttg taattgtttt ctgtttctta ataattttaa attaagggta 6540
   ttttcccaat ataaacattg ctttttggtt aaataaaatc aaaactagtt ctgtctccat 6600
   atctgaatag accagacaga gaattttctt gcctttcaac agcttaaaaa atgtttttgt 6660
   tagaactgtt gtgttcaggc tgaaatactt tcaaagtttg ttagttatta ttgagaaatt 6720
   tctgtaataa tcatggaaag ggtaatttaa tagttaaatc tcaacttaat tgaagttatt 6780
   tctgttgtgg aacttatggt catcttagca agaggtcatt gctttatagt cagttctctt 6840
   tctcattaaa aatatagtgt actcaccttt acagggtgaa tgttggtaaa taacttctgt 6900
   ctgtgaagga agtattctgg acctgtaagt taaaaataag gtgtttatag caaattatgt 6960
   aaataaagat tgtatattag aaggtacaca ctattcaaat ttaaagaaaa tgtatattga 7020
   gaaaataact caaattcttc catgaaattg gcaagaagta aacatttcaa atacacaaaa 7080
   cattatgggt atttttgttc atttgattat aaaatgccaa agttgtttta taaatgctac 7140
   ttcaagcctg gaaactttga ggaagtcctg aacattaagc atataaatgg cccagctcta 7200
   gaatacatgt aagttgaaaa gctaacctga agtgggaagc gcagtatata cctaagactt 7260
   actctgcact gaaagtttgc tttgtcacta gaagtaaaac aagactgtgg taggatagta 7320
   agatcagtaa cacctcagtt aatcaggtat cttggaggaa gtgaagaaag accctaattc 7380
   aagggacagt taattggcct tttattccaa gaatgggcct tagtggcagt atcttaaaag 7440
   cccacaagat ggagatgttt cctaatgaaa ggcctttaat ttctttatag agctgagtta 7500
   gtgtcacatc ccagtcccca cccatgaccc ttccccagtt aaaaagaaga gaaaatgttg 7560
   agcaagtctg atttgattcc catggtgaca tttttagcca ttatgtaaca aattctgaca 7620
   gtttaccctt aaaattaaaa acctccagtc ctgtcttttt aaagggtaga aagaaggtta 7680
   ggtataggat agctttttat ttatttattt atttattttt gtacaaaggg agcctatgta 7740
   aagctgccag atctgaactt tctggtgttt tgctgtaatg ttagtaagat ttcgccttaa 7800
   aatattttat tttgagtata tacgtttggt cttagagtgt cttggtggat ttccgcttac 7860
   cacccatcac tttctgcatt ttaaaggctt aaatacttta ttgctggtta actgaggttc 7920
   tactgtaaac gaatcatcta agttaattag tggattgtac ttcaatggat aattttcact 7980
   aaattgttta tattgcacat tacttttgtc ttaaggactc ttagcacatc aaaaaaattg 8040
   gctcacaact taattgtgag atgtagaatt ttccatttta tgtgctaaga gttttgttaa 8100
   tgagagaact gttaaaatag aaaaggagct tcagcataga caccaatgct ggttgctgag 8160
   atcagtgggg aactgcatag cattttaaca agtttaatga acatttggaa gagaaatttg 8220
   aagctaagat tctggttttt ttgctgttaa ctttttaatt ttttaatcta aggaaaactt 8280
   ttatgtacag tatttttact ttgggtatat gtttatcttt tagcaagttg aaagacttaa 8340
   tttgctgctt gctcactatt ttgtaattat ttgggagcag cagtaataag ccagcttttt 8400
   ggaataggat gttcctgatg tgtggttatg taggaagaat gatgttttaa tatactgccc 8460
   agtaaactgg tgcagtttgg aaaaggtgtg ttattgatgt ggataatatt taaggcaatt 8520
   ttttttaagc attttaatac tgctttttgt ctttacagga aaatgtttat aggaggcctt 8580
   agctgggaca ctacaaagaa agatcctaag gaaaactttt atgtacagta tttttacttt 8640
   gggtatatgt ttatctttta gcaagttgaa agacttaatt tgctgcttgc tcactatttt 8700
   gtaattattt gggagcagca gtaataagcc agctttttgg aataggatgt tcctgatgtg 8760
   tggttatgta ggaagaatga tgttttaata tactgcccag taaactggtg cagtttggaa 8820
   aaggtgtgtt attgatgtgg ataatattta aggcaatttt ttttaagcat tttaatactg 8880
   ctttttgtct ttacaggaaa atgtttatag gaggccttag ctgggacact acaaagaaag 8940
   atctgaagga ctacttttcc aaatttggtg aagttgtaga ctgcactctg aagttagatc 9000
   ctatcacagg gcgatcaagg ggttttggct ttgtgctatt taaagaatcg gagagtgtag 9060
   ataaggtagt gtgttacgtg ttctgatcag ttaataatat aaaatattaa catatggata 9120
   gtttgataca atgagtttgc ctatttgtgg ttccccattt tgatagtata ggaaggaaga 9180
   atagttcttg ccccaatacg ttttatgaag atagaggtag gttcaggaat tatttcctga 9240
   ataatttgtg ttccaggctc tgctaaattt tgaaattaac tttaaagata ctatagactt 9300
   aaagatgcct agttaaaagg atgtgtttta gcaattcaca gaagtccata ttttgaaatt 9360
   ttgttaggca agcaactttt aactgaatca ttattttgat cctgggctaa agggaagtag 9420
   cagttatgtt tgtatatagt gctaaaggga agtagcagtc atgtttgtat atattgaagg 9480
   taatggttat ctagtaattg gttaaatttg tgtatgtcct accattctta cctttagatt 9540
   taaacagtat atgttagttg atgttacatc accaccatga cttgacagtt taatcttgag 9600
   caagtcaaca tatgcttggc attatctgta tttatagtta tttttaagta agttaatagt 9660
   ctctgaactt cagttaagat atatattttt taaatgaaaa cttcaatttc cttaggtcat 9720
   ggatcaaaaa gaacataaat tgaatgggaa ggtgattgat cctaaaaggg ccaaagccat 9780
   gaaaacaaaa gagccggtta aaaaaatttt tgttggtggc ctttctccag atacacctga 9840
   agagaaaata agggagtact ttggtggttt tggtgaggta tgttataaat gttttgaccc 9900
   agtttatgtc aaaattagtg tgaatgtgat tgtcccatta tggactcaga gtcacttggc 9960
   ttttcaaagc tgttagggta gattatgtga tctgttttgg aataaggata ttgtaaatac 10020
   ttcattagca ggtctttgaa ggttggataa tgtgtttttc tcattgagca cctactctat 10080
   gcagagtatt gctggggtag agtataccaa agatgaaata gacaaacatt cttaaataca 10140
   gacaattaag aggaagaaat ctagattaga aggagacttt tgttgaaaat aggaaaggaa 10200
   ttaagaatag ggtgtagtgc cttatcagtt gaaatgcatg tgtaagtgca aatttaatga 10260
   gactaatcat tatagactca ttagtgaggc tggacgcgat ggctcatgct gtagtaatcc 10320
   cagcacttgg gaggccgaag tgggtggatt actggagacc aggagatgaa gaccatcctg 10380
   gacaacatag tggacccgtc tcaattaaaa gtaaaacaaa cttagactca ttagtgaaat 10440
   aggtagataa tagaggtttc ttttatgaat taatgaatta atgaaagttg agaaatttgt 10500
   ggccttgggc tccagatacg tcccacagac atttattgct tgattgaaac aataagtgct 10560
   tttttagtgt tgaggatttg agatattgcc cacaaaactc agtatctagc tttttaaaaa 10620
   atatttgcaa gagcacgcaa catgggaact gacgctgccc tcctgtacgg cagcatctct 10680
   caaactgagt agtagcttcc atcttggttt gggcatatgc tctccagttt ttagtagtcc 10740
   tcaccaccct acttcctgtt ttctctcaaa tacatttttt ttctgttttt cttagatttg 10800
   actgttttct tcttgttctt tgtgggcatt tgaatttgtg acccttgagt taggtagtaa 10860
   atgtcagtgc gtggtaaagc ttatttttgt aaatagttgt gaagacctta gatggaatgg 10920
   gtgttctaat ttgaagaatt ccttaaaagg attagaataa atagggagaa acaggagact 10980
   agaacttcag tgccaaatac atgttttctt tgtgtgtttt cccccctcta aacttgtgtt 11040
   tcttttaagg tcaataaaat gcatgttagc atattaaaat ttgtttttta ataccaggtg 11100
   gaatccatag agctccccat ggacaacaag accaataaga ggcgtgggtt ctgctttatt 11160
   acctttaagg aagaagaacc agtgaagaag ataatggaaa agaaatacca caatgttggt 11220
   cttagtaaag taagttaagc atccatttac ttgtagagaa aactagctgt tgtaaagagc 11280
   ttaaccattt atctttctct gtaaaggctt aagttctttg catgctttaa aaacttctca 11340
   ttggttactt accattgacc aactttttgt ggggagcagg atggacacat ttgttagtgt 11400
   ttttgtctag gctttcacaa aaatagtttt tagaacttga caagtaaaat gaagtaacat 11460
   cactagcaag tactcataag tgattactct taagtactaa atattggttt aattaataaa 11520
   ctgactgagg aaagtttcaa attagcctac tctatttaaa catgttggct attctggtta 11580
   ttagaaactt atttagcaac ttttattttc ttgagtcagt ttaataatgt aatttttctc 11640
   ttttagtgtg aaataaaagt agccatgtcg aaggaacaat atcagcaaca gcaacagtgg 11700
   ggatctagag gaggatttgc aggaagagct cgtggaagag gtggtggtaa gctagagcct 11760
   aagtttactc tatcttaagc ttttctgctt tttaattatc ctgaagtaaa gatctttgct 11820
   gatcttctga ctttagtgaa cctattaatg tgctgcaggc cccagtcaaa actggaacca 11880
   gggatatagt aactattgga atcaaggcta tggcaactat ggatataaca gccaaggtta 11940
   cggtggttat ggaggatatg actacactgg ttacaacaac tactatggat atggtgatta 12000
   tagcagtaag tactatactt tttatattaa ctgctatttg acatttattt tgtacaaatt 12060
   tggataggca gaaaggttag tgtagccttg ccaagtgcaa atgtcttcag gtttcaaatt 12120
   cctggaaact tgaaactgca gccattttat tgcttggttc ctcccagcct atatcacaca 12180
   cacattataa gggtagggtg tatgtgtggt tctatatatg tttgctgggc atttgtttta 12240
   cttggattta aaaattttaa gctcagttca gatcttttaa gctcaaggta ttctaaatgt 12300
   cacttcttgg accaaccaat aatcttggca tgatgtgtct taatcctata aaactgaata 12360
   ataccacatg ttgccagtta aaactaatag tatcctcgct ttaggattat taatgtagaa 12420
   actcttaaaa cagatgttga gcttgataga accaaaaaac ttgactttta gacatggaaa 12480
   gccctgactt cattgtgcaa ctaaggtagt tgctctccac ctgatttgta gcaactgttg 12540
   agtcgtcagg taaagggttc tactagaagc aatcttacat ttttttggag gagagtggtt 12600
   gcattggttg cattgtttta agtggttttt cttttccttc cttggttaga ccagttcttg 12660
   gagttatatc ctttcttagg tgactaggcc tgctgcacaa taataggtta attaaagtca 12720
   gaagaaggtc agcaaagatg gattgggtga gattggggcc cttttcttag aagggcagag 12780
   atactaagca ctgattgtgg ttgacaattt gttctaaatt ttaagatatt ttttgctggt 12840
   ggttgtgaaa gggtcagctg tccatccttt gaaacttaaa acttttaaac tgtaagggtg 12900
   aggggattgt ctcccatttt atacaataag tcaagtaatc agctcattct gaatgcctgc 12960
   cattgtatgc attcactaca tatttggtaa attatttgat aaatgattgc tcagggtgaa 13020
   tttttcacac ttgggaatta agctaccctt aattttttga gattgtttaa aattaggtac 13080
   tgttctgatt attagtatgt aaccactacc gttctggttc taacacttgt tttattttag 13140
   accagcagag tggttatggg aaggtatcca ggcgaggtgg tcatcaaaat agctacaaac 13200
   catactaaat tattccattt gcaacttatc cccaacaggt atgttctaaa aatagttttt 13260
   ttttgtcatt tacaatagta gtttttataa tctatattgt tcataaaaca atgcttaatt 13320
   taagagtttc acagcaccca gaagtgctta ccatattata acatagtgac tttcaaaaga 13380
   tatgtaacac aggtgctctt aagcttttgc ctttttgtcc tattattaac aagtcagtaa 13440
   agttaacagg taaagtactg ctaatgggta caaattaagg aattgcagca aaaaagtatt 13500
   gcctactaac tctgacatta taccttgttt gtaccgccag cgggaacttc attgcaggcc 13560
   ctgtgtcgcg ctgacttcag attctcacag gcccgctcaa tgcggacagg gtaacgagat 13620
   gctccacgct ctcgaatgct gccgtttggt atggtctctt ccaacatcct gtatcagcat 13680
   tataaaataa aatggatact tcaagctttg ccttcactta tttctttgct ttttaaaaac 13740
   tatttgtaat gtaattttaa tgcatttttt acaggcccag taatggttaa atacgtcagc 13800
   ttactgaata attttaacta tttattcttc taaggataca gcttgtctct ggattttcca 13860
   gtcttaattt tatattttat taatctattt taatgcttgc ttttcccatt tatagacgtt 13920
   gtagcagtaa ttgcaagaag ttcttgagct gaattcctgt tgtgacaact tcctataatt 13980
   acagtagata actttttctt ttagtcgtat ataacttttc tataacttgt gatggacaag 14040
   agatatgctt atccaataaa ataagcttaa atattagatg ctcttgggtc aaaatgtcct 14100
   tttaccaaat tgaccttttt atgagttctt tgggtaaata ctttaaagct ttttatattt 14160
   taaagaatac ttgtaaaagc atatcacatc ttaaaccagt ggtgcacatg tggatttaca 14220
   gctcatggac tctactgttc agctttaatt tataaaacat atcacacatt taatgttata 14280
   cagtatttac atatagtgga acatagggat aactcagttt tatgtaaatt tttgttaagt 14340
   gttgtagcct gcccagagtg acttctattt tttcttcttt gtctccaggt ggtgaagcag 14400
   tattttccaa tttgaagatt catttgaagg tggctcctgc cacctgctaa tagcagttca 14460
   aactaaattt tttgtatcaa gtccctgaat ggaagtatga cgttgggtcc ctctgaagtt 14520
   taattctgag ttctcattaa aagaaatttg ctttcattgt tttatttctt aattgctatg 14580
   cttcagaatc aatttgtgtt ttatgccctt tcccccagta ttgtagagca agtcttgtgt 14640
   taaaagccca gtgtgacagt gtcatgatgt agtagtgtct tactggtttt ttaataaatc 14700
   cttttgtata aaaatgtatt ggctctttta tcatcagaat aggaaaaaat tgtcatggat 14760
   tcaagttatt aaaagcataa gtttggaaga caggcttgcc gaaattgagg acatgattaa 14820
   aattgcagtg aagtttgaaa tgtttttagc aaaatctaat ttttgccata atgtgtcctc 14880
   cctgtccaaa ttgggaatga cttaatgtca atttgtttgt tggttgtttt aataatactt 14940
   ccttatgtag ccattaagat ttatatgaat attttcccca atg 14983
<210> 42
   <211> 279
   <212> PRT
   <213> Homo sapiens
<300>
   <308> NP_852000
   <309> 2004-10-27
   <313> (1)..(279)
<400> 42
   Met Pro Cys Arg Arg Glu Glu Glu Glu Glu Ala Gly Glu Glu Ala Glu
   1               5                   10                  15
   Gly Glu Glu Glu Glu Glu Asp Ser Phe Leu Leu Leu Gln Gln Ser Val
               20                  25                  30
   Ala Leu Gly Ser Ser Gly Glu Val Asp Arg Leu Val Ala Gln Ile Gly
           35                  40                  45
   Glu Thr Leu Gln Leu Asp Ala Ala Gln His Ser Pro Ala Ser Pro Cys
       50                  55                  60
   Gly Pro Pro Gly Ala Pro Leu Arg Ala Pro Gly Pro Leu Ala Ala Ala
   65                  70                  75                  80
   Val Pro Ala Asp Lys Ala Arg Ser Pro Ala Val Pro Leu Leu Leu Pro
                   85                  90                  95
   Pro Ala Leu Ala Glu Thr Val Gly Pro Ala Pro Pro Gly Val Leu Arg
               100                 105                 110
   Cys Ala Leu Gly Asp Arg Gly Arg Val Arg Gly Arg Ala Ala Pro Tyr
   115 120 125
   Cys Val Ala Glu Leu Ala Thr Gly Pro Ser Ala Leu Ser Pro Leu Pro
   130 135 140
   Pro Gln Ala Asp Leu Asp Gly Pro Pro Gly Ala Gly Lys Gln Gly Ile
   145 150 155 160
   Pro Gln Pro Leu Ser Gly Pro Cys Arg Arg Gly Trp Leu Arg Gly Ala
   165 170 175
   Ala Ala Ser Arg Arg Leu Gln Gln Arg Arg Gly Ser Gln Pro Glu Thr
   180 185 190
   Arg Thr Gly Asp Asp Asp Pro His Arg Leu Leu Gln Gln Leu Val Leu
   195 200 205
   Ser Gly Asn Leu Ile Lys Glu Ala Val Arg Arg Leu His Ser Arg Arg
   210 215 220
   Leu Gln Leu Arg Ala Lys Leu Pro Gln Arg Pro Leu Leu Gly Pro Leu
   225 230 235 240
   Ser Ala Pro Val His Glu Pro Pro Ser Pro Arg Ser Pro Arg Ala Ala
   245 250 255
   Cys Ser Asp Pro Gly Ala Ser Gly Arg Ala Gln Leu Arg Thr Gly Asp
   260 265 270
   Gly Val Leu Val Pro Gly Ser
   275
<210> 43
   <211> 2653
   <212> DNA
   <213> Homo sapiens
<300>
   <308> NM_005479
   <309> 2004-10-26
   <313> (1)..(2653)
<400> 43
   ggattccggc tcccgcggct gcaggcgcgc ggctagagtg cctggcgggc tccggcttcc     60
   gcgtccgccc cggccccggt ccagacttag tcttcagctc cgcgcccgct ccgccgcggc    120
   ccaccgcgcc cgccggcagc cgagccccca gcgacgcccg cacagctccg ggtgcccaga    180
   cagggggcca tgccgtgccg gagggaggag gaagaggaag ccggcgagga ggcggagggg    240
   gaggaagagg aggaggacag cttcctccta ctgcagcagt cagtggcgct gggcagctcg    300
   ggcgaggtgg accggctggt ggcccagatc ggcgagacgc tgcagctgga cgcggcgcag    360
   cacagcccgg cctcgccgtg cgggcccccg ggggcgccgc tgcgggcccc ggggcccctg    420
   gctgcggcgg tgccggcgga caaggccagg tccccggcgg tgccgctgct gctgccgccc 480
   gcgttggcgg agactgtggg cccggcgccc cctggggtcc tgcgctgcgc cctgggggac 540
   cgcggccgcg tgcggggccg cgctgcgccc tactgcgtgg ccgagctcgc cacaggcccc 600
   agcgcgctgt ccccactgcc ccctcaggcc gaccttgatg ggcctccggg agctggcaag 660
   cagggcatcc cgcagccgct gtcgggtccg tgccggcgag gatggctccg gggcgccgcc 720
   gcctcccgcc gcctgcagca gcgacgcggg tcccaaccag aaacccgcac aggcgacgac 780
   gacccgcacc ggcttctgca gcagctagtg ctctctggaa acctcatcaa ggaggccgtg 840
   cgaaggcttc attcgcgacg gctgcagtta cgtgcaaagc ttccccaacg cccgctcctg 900
   ggacctctgt cggccccggt gcatgaaccc ccttcgcctc gcagccctcg cgcggcctgc 960
   agtgaccctg gcgcctccgg gagggcgcag ctcagaactg gcgacggcgt tcttgtgcct 1020
   ggcagctaac acgcccgggg tggccacagc gccagcctca gactggaggg caaggggttc 1080
   ccttgagggc tgcagttcta ctcaggctgg tggagaactc tggcttttgg aagcgagagt 1140
   aaaaagctaa tgacgaggaa ccgaaaaatc gcgagtgttt cgcgggtaac tggggttgag 1200
   ggccaaaata tttggaatga aggacttggc cctatttaag gcagatttta cagagcgcac 1260
   ctcaaacgta caagtcagta ggactcctta tttggcgtga cccgacctgg ccgcggagcc 1320
   tgcatttcct cgcagcctct cagtgccctc cagccccgcg accatgtggc cacaatccac 1380
   gcttctccgg atcgcggtgc gccggaacca cggaggatga tgccagttac ttgctttacc 1440
   ttttcagggc tggctcctga tccactttgg gggaggagaa catgagtaga taatttcagg 1500
   gtgcagccca atctgccaga cttaaaaaaa ccatcttgtg tctttggagg tgctgcttaa 1560
   taccaaacat gcggtgccat gaagggaccc tttgggggtt gaataggagt taacccctgc 1620
   gctctctttg caactgtctc tcttctcaga gtggtggggg aaggctgtac gacacgggtg 1680
   gggaaaggag gtgggggcgg ggagtattga atggtggtgg aagggtagag aggcgcggag 1740
   tgaaccccac gccctgtcta aagtgtattt tcagagccgg cccgcctctc ctcggttcaa 1800
   ggtcactgtt tcctgggcac gcactgggtt gcgggacaga gtagccaggt tctgccggtg 1860
   ctcggagaag agcgcagtgt tttgcaagtg ctggagtctc ctgaggacac gcgcgtcgcc 1920
   gccaccgcgg gtgtgggaaa gcgcggacgt gctgggcggc tgtgcttcgg taggcgacca 1980
   ccgcccctgg ccgcgctccg ggctttcacg gaaactcccg agaccgggcc ctgggttcct 2040
   cctctcctac tcggctctgc agtcctactc aagcgggtgg ctctgggatc ctgggggcct 2100
   gggttggggg ctagggagac gccatgtgat ggacactcca gggacacaca gcctagcaca 2160
   gcagcttata atgggctctc cggggccatt tgcaataaca gctgcaattc cctggataga 2220
   cgagttgatt tcctccctct gcccctcccc cagccatgcc agctggcctt tgtaagtgca 2280
   ggaaaccgag tagaaaatgt gaccctccaa atggagaagc tgcaggcttt gccattgtga 2340
   accatggtga agtgcttgga acatactgtt cactcactct aaaggcgctg agactgtgct 2400
   gttgttctcg tttttatagt caatggcttg ttcatcatcc agatgtggct actgacatat 2460
   ctacacttcg caccggagtg tctggaattg tggctatcct gattatagga ttttaactta 2520
   actgaaatgc ctgctttgaa taaatgtgtt gggttttttg tttggtttta ttttatactt 2580
   gccatcagtg aaaaagatgt acagaacaca tttctctgat ctccataaac atgaaaacac 2640
   ttgaaatctc aaa 2653
<210> 44
   <211> 2203
   <212> DNA
   <213> Homo sapiens
<300>
   <308> NM_181355
   <309> 2004-10-27
   <313> (1)..(2203)
<400> 44
   ggattccggc tcccgcggct gcaggcgcgc ggctagagtg cctggcgggc tccggcttcc 60
   gcgtccgccc cggccccggt ccagacttag tcttcagctc cgcgcccgct ccgccgcggc 120
   ccaccgcgcc cgccggcagc cgagccccca gcgacgcccg cacagctccg ggtgcccaga 180
   cagggggcca tgccgtgccg gagggaggag gaagaggaag ccggcgagga ggcggagggg 240
   gaggaagagg aggaggacag cttcctccta ctgcagcagt cagtggcgct gggcagctcg 300
   ggcgaggtgg accggctggt ggcccagatc ggcgagacgc tgcagctgga cgcggcgcag 360
   cacagcccgg cctcgccgtg cgggcccccg ggggcgccgc tgcgggcccc ggggcccctg 420
   gctgcggcgg tgccggcgga caaggccagg tccccggcgg tgccgctgct gctgccgccc 480
   gcgttggcgg agactgtggg cccggcgccc cctggggtcc tgcgctgcgc cctgggggac 540
   cgcggccgcg tgcggggccg cgctgcgccc tactgcgtgg ccgagctcgc cacaggcccc 600
   agcgcgctgt ccccactgcc ccctcaggcc gaccttgatg ggcctccggg agctggcaag 660
   cagggcatcc cgcagccgct gtcgggtccg tgccggcgag gatggctccg gggcgccgcc 720
   gcctcccgcc gcctgcagca gcgacgcggg tcccaaccag aaacccgcac aggcgacgac 780
   gacccgcacc ggcttctgca gcagctagtg ctctctggaa acctcatcaa ggaggccgtg 840
   cgaaggcttc attcgcgacg gctgcagtta cgtgcaaagc ttccccaacg cccgctcctg 900
   ggacctctgt cggccccggt gcatgaaccc ccttcgcctc gcagccctcg cgcggcctgc 960
   agtgaccctg gcgcctccgg gagggcgcag ctcagaactg gcgacggcgt tcttgtgcct 1020
   ggcagctaac acgcccgggg tggccacagc gccagcctca gactggaggg caaggggttc 1080
   ccttgagggc tgcagttcta ctcaggctgg tggagaactc tggcttttgg aagcgagagt 1140
   aaaaagctaa tgacgaggaa ccgaaaaatc gcgagtgttt cgcgggtaac tggggttgag 1200
   ggccaaaata tttggaatga aggacttggc cctatttaag gcagatttta cagagcgcac 1260
   ctcaaacgta caagtcagta ggactcctta tttggcgtga cccgacctgg ccgcggagcc 1320
   tgcatttcct cgcagcctct cagtgccctc cagccccgcg accatgtggc cacaatccac 1380
   gcttctccgg atcgcggtgc gccggaacca cggaggatga tgccagttac ttgctttacc 1440
   ttttcagggc tggctcctga tccactttgg gggaggagaa catgagtaga taatttcagg 1500
   gtgcagccca atctgccaga cttaaaaaaa ccatcttgtg tctttggagg tgctgcttaa 1560
   taccaaacat gcggtgccat gaagggaccc tttgggggtt gaataggagt taacccctgc 1620
   gctctctttg caactgtctc tcttctcaga gtggtggggg aaggctgtac gacacgggtg 1680
   gggaaaggag gtgggggcgg ggagtattga atggtggtgg aagggtagag aggcgcggag 1740
   tgaaccccac gccctgtcta aagtgtattt tcagagccgg cccgcctctc ctcggttcaa 1800
   ggtcactgtt tcctgggcac gcactgggtt gcgggacaga gtagccaggt tctgccggtg 1860
   ctcggagaag agcgcagtgt tttgcaagtg ctggagtctc ctgaggacac gcgcgtcgcc 1920
   gccaccgcgg gtgtgggaaa gcgcggacgt gctgggcggc tgtgcttcgt caatggcttg 1980
   ttcatcatcc agatgtggct actgacatat ctacacttcg caccggagtg tctggaattg 2040
   tggctatcct gattatagga ttttaactta actgaaatgc ctgctttgaa taaatgtgtt 2100
   gggttttttg tttggtttta ttttatactt gccatcagtg aaaaagatgt acagaacaca 2160
   tttctctgat ctccataaac atgaaaacac ttgaaatctc aaa 2203
<210> 45
   <211> 1845
   <212> PRT
   <213> Homo sapiens
<300>
   <308> NP_444254
   <309> 2004-10-28
   <313> (1)..(1845)
<400> 45
   Met Gly Asp Val Lys Leu Val Ala Ser Ser His Ile Ser Lys Thr Ser
   1               5                   10                  15
   Leu Ser Val Asp Pro Ser Arg Val Asp Ser Met Pro Leu Thr Glu Ala
               20                  25                  30
   Pro Ala Phe Ile Leu Pro Pro Arg Asn Leu Cys Ile Lys Glu Gly Ala
           35                  40                  45
   Thr Ala Lys Phe Glu Gly Arg Val Arg Gly Tyr Pro Glu Pro Gln Val
       50                  55                  60
   Thr Trp His Arg Asn Gly Gln Pro Ile Thr Ser Gly Gly Arg Phe Leu
   65                  70                  75                  80
   Leu Asp Cys Gly Ile Arg Gly Thr Phe Ser Leu Val Ile His Ala Val
                   85                  90                  95
   His Glu Glu Asp Arg Gly Lys Tyr Thr Cys Glu Ala Thr Asn Gly Ser
               100                 105                 110
   Gly Ala Arg Gln Val Thr Val Glu Leu Thr Val Glu Gly Ser Phe Ala
           115                 120                 125
   Lys Gln Leu Gly Gln Pro Val Val Ser Lys Thr Leu Gly Asp Arg Phe
       130                 135                 140
   Ser Ala Ser Ala Val Glu Thr Arg Pro Ser Ile Trp Gly Glu Cys Pro
   145 150 155 160
   Pro Lys Phe Ala Thr Lys Leu Gly Arg Val Val Val Lys Glu Gly Gln
   165 170 175
   Met Gly Arg Phe Ser Cys Lys Ile Thr Gly Arg Pro Gln Pro Gln Val
   180 185 190
   Thr Trp Leu Lys Gly Asn Val Pro Leu Gln Pro Ser Ala Arg Val Ser
   195 200 205
   Val Ser Glu Lys Asn Gly Met Gln Val Leu Glu Ile His Gly Val Asn
   210 215 220
   Gln Asp Asp Val Gly Val Tyr Thr Cys Leu Val Val Asn Gly Ser Gly
   225 230 235 240
   Lys Ala Ser Met Ser Ala Glu Leu Ser Ile Gln Gly Leu Asp Ser Ala
   245 250 255
   Asn Arg Ser Phe Val Arg Glu Thr Lys Ala Thr Asn Ser Asp Val Arg
   260 265 270
   Lys Glu Val Thr Asn Val Ile Ser Lys Glu Ser Lys Leu Asp Ser Leu
   275 280 285
   Glu Ala Ala Ala Lys Ser Lys Asn Cys Ser Ser Pro Gln Arg Gly Gly
   290 295 300
   Ser Pro Pro Trp Ala Ala Asn Ser Gln Pro Gln Pro Pro Arg Glu Ser
   305 310 315 320
   Lys Leu Glu Ser Cys Lys Asp Ser Pro Arg Thr Ala Pro Gln Thr Pro
   325 330 335
   Val Leu Gln Lys Thr Ser Ser Ser Ile Thr Leu Gln Ala Ala Arg Val
   340 345 350
   Gln Pro Glu Pro Arg Ala Pro Gly Leu Gly Val Leu Ser Pro Ser Gly
   355 360 365
   Glu Glu Arg Lys Arg Pro Ala Pro Pro Arg Pro Ala Thr Phe Pro Thr
   370 375 380
   Arg Gln Pro Gly Leu Gly Ser Gln Asp Val Val Ser Lys Ala Ala Asn
   385 390 395 400
   Arg Arg Ile Pro Met Glu Gly Gln Arg Asp Ser Ala Phe Pro Lys Phe
   405 410 415
   Glu Ser Lys Pro Gln Ser Gln Glu Val Lys Glu Asn Gln Thr Val Lys
   420 425 430
   Phe Arg Cys Glu Gly Leu Ala Val Met Glu Val Ala Pro Ser Phe Ser
   435 440 445
   Ser Val Leu Lys Asp Cys Ala Val Ile Glu Gly Gln Asp Phe Val Leu
   450 455 460
   Gln Cys Ser Val Arg Gly Thr Pro Val Pro Arg Ile Thr Trp Leu Leu
   465 470 475 480
   Asn Gly Gln Pro Ile Gln Tyr Ala Arg Ser Thr Cys Glu Ala Gly Val
   485 490 495
   Ala Glu Leu His Ile Gln Asp Ala Leu Pro Glu Asp His Gly Thr Tyr
   500 505 510
   Thr Cys Leu Ala Glu Asn Ala Leu Gly Gln Val Ser Cys Ser Ala Trp
   515 520 525
   Val Thr Val His Glu Lys Lys Ser Ser Arg Lys Ser Glu Tyr Leu Leu
   530 535 540
   Pro Val Ala Pro Ser Lys Pro Thr Ala Pro Ile Phe Leu Gln Gly Leu
   545 550 555 560
   Ser Asp Leu Lys Val Met Asp Gly Ser Gln Val Thr Met Thr Val Gln
   565 570 575
   Val Ser Gly Asn Pro Pro Pro Glu Val Ile Trp Leu His Asn Gly Asn
   580 585 590
   Glu Ile Gln Glu Ser Glu Asp Phe His Phe Glu Gln Arg Gly Thr Gln
   595 600 605
   His Ser Leu Cys Ile Gln Glu Val Phe Pro Glu Asp Thr Gly Thr Tyr
   610 615 620
   Thr Cys Glu Ala Trp Asn Ser Ala Gly Glu Val Arg Thr Gln Ala Val
   625 630 635 640
   Leu Thr Val Gln Glu Pro His Asp Gly Thr Gln Pro Trp Phe Ile Ser
   645 650 655
   Lys Pro Arg Ser Val Thr Ala Ser Leu Gly Gln Ser Val Leu Ile Ser
   660 665 670
   Cys Ala Ile Ala Gly Asp Pro Phe Pro Thr Val His Trp Leu Arg Asp
   675 680 685
   Gly Lys Ala Leu Cys Lys Asp Thr Gly His Phe Glu Val Leu Gln Asn
   690 695 700
   Glu Asp Val Phe Thr Leu Val Leu Lys Lys Val Gln Pro Trp His Ala
   705 710 715 720
   Gly Gln Tyr Glu Ile Leu Leu Lys Asn Arg Val Gly Glu Cys Ser Cys
   725 730 735
   Gln Val Ser Leu Met Leu Gln Asn Ser Ser Ala Arg Ala Leu Pro Arg
   740 745 750
   Gly Arg Glu Pro Ala Ser Cys Glu Asp Leu Cys Gly Gly Gly Val Gly
   755 760 765
   Ala Asp Gly Gly Gly Ser Asp Arg Tyr Gly Ser Leu Arg Pro Gly Trp
   770 775 780
   Pro Ala Arg Gly Gln Gly Trp Leu Glu Glu Glu Asp Gly Glu Asp Val
   785 790 795 800
   Arg Gly Val Leu Lys Arg Arg Val Glu Thr Arg Gln His Thr Glu Glu
   805 810 815
   Ala Ile Arg Gln Gln Glu Val Glu Gln Leu Asp Phe Arg Asp Leu Leu
   820 825 830
   Gly Lys Lys Val Ser Thr Lys Thr Leu Ser Glu Asp Asp Leu Lys Glu
   835 840 845
   Ile Pro Ala Glu Gln Met Asp Phe Arg Ala Asn Leu Gln Arg Gln Val
   850 855 860
   Lys Pro Lys Thr Val Ser Glu Glu Glu Arg Lys Val His Ser Pro Gln
   865 870 875 880
   Gln Val Asp Phe Arg Ser Val Leu Ala Lys Lys Gly Thr Ser Lys Thr
   885 890 895
   Pro Val Pro Glu Lys Val Pro Pro Pro Lys Pro Ala Thr Pro Asp Phe
   900 905 910
   Arg Ser Val Leu Gly Gly Lys Lys Lys Leu Pro Ala Glu Asn Gly Ser
   915 920 925
   Ser Ser Ala Glu Thr Leu Asn Ala Lys Ala Val Glu Ser Ser Lys Pro
   930 935 940
   Leu Ser Asn Ala Gln Pro Ser Gly Pro Leu Lys Pro Val Gly Asn Ala
   945 950 955 960
   Lys Pro Ala Glu Thr Leu Lys Pro Met Gly Asn Ala Lys Pro Ala Glu
   965 970 975
   Thr Leu Lys Pro Met Gly Asn Ala Lys Pro Asp Glu Asn Leu Lys Ser
   980 985 990
   Ala Ser Lys Glu Glu Leu Lys Lys Asp Val Lys Asn Asp Val Asn Cys
   995 1000 1005
   Lys Arg Gly His Ala Gly Thr Thr Asp Asn Glu Lys Arg Ser Glu
   1010 1015 1020
   Ser Gln Gly Thr Ala Pro Ala Phe Lys Gln Lys Leu Gln Asp Val
   1025 1030 1035
   His Val Ala Glu Gly Lys Lys Leu Leu Leu Gln Cys Gln Val Ser
   1040 1045 1050
   Ser Asp Pro Pro Ala Thr Ile Ile Trp Thr Leu Asn Gly Lys Thr
   1055 1060 1065
   Leu Lys Thr Thr Lys Phe Ile Ile Leu Ser Gln Glu Gly Ser Leu
   1070 1075 1080
   Cys Ser Val Ser Ile Glu Lys Ala Leu Pro Glu Asp Arg Gly Leu
   1085 1090 1095
   Tyr Lys Cys Val Ala Lys Asn Asp Ala Gly Gln Ala Glu Cys Ser
   1100 1105 1110
   Cys Gln Val Thr Val Asp Asp Ala Pro Ala Ser Glu Asn Thr Lys
   1115 1120 1125
   Ala Pro Glu Met Lys Ser Arg Arg Pro Lys Ser Ser Leu Pro Pro
   1130 1135 1140
   Val Leu Gly Thr Glu Ser Asp Ala Thr Val Lys Lys Lys Pro Ala
   1145 1150 1155
   Pro Lys Thr Pro Pro Lys Ala Ala Met Pro Pro Gln Ile Ile Gln
   1160 1165 1170
   Phe Pro Glu Asp Gln Lys Val Arg Ala Gly Glu Ser Val Glu Leu
   1175 1180 1185
   Phe Gly Lys Val Thr Gly Thr Gln Pro Ile Thr Cys Thr Trp Met
   1190 1195 1200
   Lys Phe Arg Lys Gln Ile Gln Glu Ser Glu His Met Lys Val Glu
   1205 1210 1215
   Asn Ser Glu Asn Gly Ser Lys Leu Thr Ile Leu Ala Ala Arg Gln
   1220 1225 1230
   Glu His Cys Gly Cys Tyr Thr Leu Leu Val Glu Asn Lys Leu Gly
   1235 1240 1245
   Ser Arg Gln Ala Gln Val Asn Leu Thr Val Val Asp Lys Pro Asp
   1250 1255 1260
   Pro Pro Ala Gly Thr Pro Cys Ala Ser Asp Ile Arg Ser Ser Ser
   1265 1270 1275
   Leu Thr Leu Ser Trp Tyr Gly Ser Ser Tyr Asp Gly Gly Ser Ala
   1280 1285 1290
   Val Gln Ser Tyr Ser Ile Glu Ile Trp Asp Ser Ala Asn Lys Thr
   1295 1300 1305
   Trp Lys Glu Leu Ala Thr Cys Arg Ser Thr Ser Phe Asn Val Gln
   1310 1315 1320
   Asp Leu Leu Pro Asp His Glu Tyr Lys Phe Arg Val Arg Ala Ile
   1325 1330 1335
   Asn Val Tyr Gly Thr Ser Glu Pro Ser Gln Glu Ser Glu Leu Thr
   1340 1345 1350
   Thr Val Gly Glu Lys Pro Glu Glu Pro Lys Asp Glu Val Glu Val
   1355 1360 1365
   Ser Asp Asp Asp Glu Lys Glu Pro Glu Val Asp Tyr Arg Thr Val
   1370 1375 1380
   Thr Ile Asn Thr Glu Gln Lys Val Ser Asp Phe Tyr Asp Ile Glu
   1385 1390 1395
   Glu Arg Leu Gly Ser Gly Lys Phe Gly Gln Val Phe Arg Leu Val
   1400 1405 1410
   Glu Lys Lys Thr Arg Lys Val Trp Ala Gly Lys Phe Phe Lys Ala
   1415 1420 1425
   Tyr Ser Ala Lys Glu Lys Glu Asn Ile Arg Gln Glu Ile Ser Ile
   1430 1435 1440
   Met Asn Cys Leu His His Pro Lys Leu Val Gln Cys Val Asp Ala
   1445 1450 1455
   Phe Glu Glu Lys Ala Asn Ile Val Met Val Leu Glu Ile Val Ser
   1460 1465 1470
   Gly Gly Glu Leu Phe Glu Arg Ile Ile Asp Glu Asp Phe Glu Leu
   1475 1480 1485
   Thr Glu Arg Glu Cys Ile Lys Tyr Met Arg Gln Ile Ser Glu Gly
   1490 1495 1500
   Val Glu Tyr Ile His Lys Gln Gly Ile Val His Leu Asp Leu Lys
   1505 1510 1515
   Pro Glu Asn Ile Met Cys Val Asn Lys Thr Gly Thr Arg Ile Lys
   1520 1525 1530
   Leu Ile Asp Phe Gly Leu Ala Arg Arg Leu Glu Asn Ala Gly Ser
   1535 1540 1545
   Leu Lys Val Leu Phe Gly Thr Pro Glu Phe Val Ala Pro Glu Val
   1550 1555 1560
   Ile Asn Tyr Glu Pro Ile Gly Tyr Ala Thr Asp Met Trp Ser Ile
   1565 1570 1575
   Gly Val Ile Cys Tyr Ile Leu Val Ser Gly Leu Ser Pro Phe Met
   1580 1585 1590
   Gly Asp Asn Asp Asn Glu Thr Leu Ala Asn Val Thr Ser Ala Thr
   1595 1600 1605
   Trp Asp Phe Asp Asp Glu Ala Phe Asp Glu Ile Ser Asp Asp Ala
   1610 1615 1620
   Lys Asp Phe Ile Ser Asn Leu Leu Lys Lys Asp Met Lys Asn Arg
   1625 1630 1635
   Leu Asp Cys Thr Gln Cys Leu Gln His Pro Trp Leu Met Lys Asp
   1640 1645 1650
   Thr Lys Asn Met Glu Ala Lys Lys Leu Ser Lys Asp Arg Met Lys
   1655 1660 1665
   Lys Tyr Met Ala Arg Arg Lys Trp Gln Lys Thr Gly Asn Ala Val
   1670 1675 1680
   Arg Ala Ile Gly Arg Leu Ser Ser Met Ala Met Ile Ser Gly Leu
   1685 1690 1695
   Ser Gly Arg Lys Ser Ser Thr Gly Ser Pro Thr Ser Pro Leu Asn
   1700 1705 1710
   Ala Glu Lys Leu Glu Ser Glu Glu Asp Val Ser Gln Ala Phe Leu
   1715 1720 1725
   Glu Ala Val Ala Glu Glu Lys Pro His Val Lys Pro Tyr Phe Ser
   1730 1735 1740
   Lys Thr Ile Arg Asp Leu Glu Val Val Glu Gly Ser Ala Ala Arg
   1745 1750 1755
   Phe Asp Cys Lys Ile Glu Gly Tyr Pro Asp Pro Glu Val Val Trp
   1760 1765 1770
   Phe Lys Asp Asp Gln Ser Ile Arg Glu Ser Arg His Phe Gln Ile
   1775 1780 1785
   Asp Tyr Asp Glu Asp Gly Asn Cys Ser Leu Ile Ile Ser Asp Val
   1790 1795 1800
   Cys Gly Asp Asp Asp Ala Lys Tyr Thr Cys Lys Ala Val Asn Ser
   1805 1810 1815
   Leu Gly Glu Ala Thr Cys Thr Ala Glu Leu Ile Val Glu Thr Met
   1820 1825 1830
   Glu Glu Gly Glu Gly Glu Gly Glu Glu Glu Glu Glu
   1835 1840 1845
<210> 46
   <211> 5719
   <212> DNA
   <213> Homo sapiens
<300>
   <308> AF069601
   <309> 2000-03-14
   <313> (1)..(5719)
<400> 46
   ccggctgcct ctgctgcagt tcagagcaac ttcaggagct tcccagccga gagcttcagg     60
   acgcctttcc tgtcccactg gcccagttgc cacaacaaac aacagagaag acggtgacca    120
   tgggggatgt gaagctggtt gcctcgtcac acatttccaa aacctccctc agtgtggatc    180
   cctcaagagt tgactccatg cccctgacag aggcccctgc tttcattttg ccccctcgga    240
   acctctgcat caaagaagga gccaccgcca agttcgaagg gcgggtccgg ggttacccag    300
   agccccaggt gacatggcac agaaacgggc aacccatcac cagcgggggc cgcttcctgc    360
   tggattgcgg catccggggg actttcagcc ttgtgattca tgctgtccat gaggaggaca    420
   ggggaaagta tacctgtgaa gccaccaatg gcagtggtgc tcgccaggtg acagtggagt    480
   tgacagtaga aggaagtttt gcgaagcagc ttggtcagcc tgttgtttcc aaaaccttag    540
   gggatagatt ttcagcttca gcagtggaga cccgtcctag catctggggg gagtgcccac    600
   caaagtttgc taccaagctg ggccgagttg tggtcaaaga aggacagatg ggacgattct    660
   cctgcaagat cactggccgg ccccaaccgc aggtcacctg gctcaaggga aatgttccac    720
   tgcagccgag tgcccgtgtg tctgtgtctg agaagaacgg catgcaggtt ctggaaatcc    780
   atggagtcaa ccaagatgac gtgggagtgt acacgtgcct ggtggtgaac gggtcgggga    840
   aggcctcgat gtcagctgaa ctttccatcc aaggtttgga cagtgccaat aggtcatttg    900
   tgagagaaac aaaagccacc aattcagatg tcaggaaaga ggtgaccaat gtaatctcaa    960
   aggagtcgaa gctggacagt ctggaggctg cagccaaaag caagaactgc tccagccccc   1020
   agagaggtgg ctccccaccc tgggctgcaa acagccagcc tcagccccca agggagtcca   1080
   agctggagtc atgcaaggac tcgcccagaa cggccccgca gaccccggtc cttcagaaga   1140
   cttccagctc catcaccctg caggccgcaa gagttcagcc ggaaccaaga gcaccaggcc 1200
   tgggggtcct atcaccttct ggagaagaga ggaagaggcc agctcctccc cgtccagcca 1260
   ccttccccac caggcagcct ggcctgggga gccaagatgt tgtgagcaag gctgctaaca 1320
   ggagaatccc catggagggc cagagggatt cagcattccc caaatttgag agcaagcccc 1380
   aaagccagga ggtcaaggaa aatcaaactg tcaagttcag atgtgaaggg cttgccgtga 1440
   tggaggtggc cccctccttc tccagtgtcc tgaaggactg cgctgttatt gagggccagg 1500
   attttgtgct gcagtgctcc gtacggggga ccccagtgcc ccggatcact tggctgctga 1560
   atgggcagcc catccagtac gctcgctcca cctgcgaggc cggcgtggct gagctccaca 1620
   tccaggatgc cctgccggag gaccatggca cctacacctg cctagctgag aatgccttgg 1680
   ggcaggtgtc ctgcagcgcc tgggtcaccg tccatgaaaa gaagagtagc aggaagagtg 1740
   agtaccttct gcctgtggct cccagcaagc ccactgcacc catcttcctg cagggcctct 1800
   ctgatctcaa agtcatggat ggaagccagg tcactatgac tgtccaagtg tcagggaatc 1860
   caccccctga agtcatctgg ctgcacaatg ggaatgagat ccaagagtca gaggacttcc 1920
   actttgaaca gagaggaact cagcacagcc tttggatcca ggaagtgttc ccggaggaca 1980
   cgggcacgta cacctgcgag gcctggaaca gcgctggaga ggtccgcacc caggccgtgc 2040
   tcacggtaca agagcctcac gatggcaccc agccctggtt catcagtaag cctcgctcag 2100
   tgacagcctc cctgggccag agtgtcctca tctcctgcgc catagctggt gacccctttc 2160
   ctaccgtgca ctggctcaga gatggcaaag ccctctgcaa agacactggc cacttcgagg 2220
   tgcttcagaa tgaggacgtg ttcaccctgg ttctaaagaa ggtgcagccc tggcatgccg 2280
   gccagtatga gatcctgctc aagaaccggg ttggcgaatg cagttgccag gtgtcactga 2340
   tgctacagaa cagctctgcc agagcccttc cacgggggag ggagcctgcc agctgcgagg 2400
   acctctgtgg tggaggagtt ggtgctgatg gtggtggtag tgaccgctat gggtccctga 2460
   ggcctggctg gccagcaaga gggcagggtt ggctagagga ggaagacggc gaggacgtgc 2520
   gaggggtgct gaagaggcgc gtggagacga ggcagcacac tgaggaggcg atccgccagc 2580
   aggaggtgga gcagctggac ttccgagacc tcctggggaa gaaggtgagt acaaagaccc 2640
   tatcggaaga cgacctgaag gagatcccgg ccgagcagat ggatttccgt gccaacctgc 2700
   agcggcaagt gaagccaaag actgtgtctg aggaagagag gaaggtgcac agcccccagc 2760
   aggtcgattt tcgctctgtc ctggccaaga aggggacttc caagaccccc gtgcctgaga 2820
   aggtgccacc gccaaaacct gccaccccgg attttcgctc agtgctgggt ggcaagaaga 2880
   aattaccagc agagaatggc agcagcagtg ccgagaccct gaatgccaag gcagtggaga 2940
   gttccaagcc cctgagcaat gcacagcctt cagggccctt gaaacccgtg ggcaacgcca 3000
   agcctgctga gaccctgaag ccaatgggca acgccaagcc tgccgagacc ctgaagccca 3060
   tgggcaatgc caagcctgat gagaacctga aatccgctag caaagaagaa ctcaagaaag 3120
   acgttaagaa tgatgtgaac tgcaagagag gccatgcagg gaccacagat aatgaaaaga 3180
   gatcagagag ccaggggaca gccccagcct tcaagcagaa gctgcaagat gttcatgtgg 3240
   cagagggcaa gaagctgctg ctccagtgcc aggtgtcttc tgacccccca gccaccatca 3300
   tctggacgct gaatggaaag accctcaaga ccaccaagtt catcatcctc tcccaggaag 3360
   gctcactctg ctccgtctcc atcgagaagg cactgcctga ggacagaggc ttatacaagt 3420
   gtgtagccaa gaatgacgct ggccaggcgg agtgctcctg ccaagtcacc gtggatgatg 3480
   ctccagccag tgagaacacc aaggccccag agatgaaatc ccggaggccc aagagctctc 3540
   ttcctcccgt gctaggaact gagagtgatg cgactgtgaa aaagaaacct gcccccaaga 3600
   cacctccgaa ggcagcaatg ccccctcaga tcatccagtt ccctgaggac cagaaggtac 3660
   gcgcaggaga gtcagtggag ctgtttggca aagtgacagg cactcagccc atcacctgta 3720
   cctggatgaa gttccgaaag cagatccagg aaagcgagca catgaaggtg gagaacagcg 3780
   agaatggcag caagctcacc atcctggccg cgcgccagga gcactgcggc tgctacacac 3840
   tgctggtgga gaacaagctg ggcagcaggc aggcccaggt caacctcact gtcgtggata 3900
   agccagaccc cccagctggc acaccttgtg cctctgacat tcggagctcc tcactgaccc 3960
   tgtcctggta tggctcctca tatgatgggg gcagtgctgt acagtcctac agcatcgaga 4020
   tctgggactc agccaacaag acgtggaagg aactagccac atgccgcagc acctctttca 4080
   acgtccagga cctgctgcct gaccacgaat ataagttccg tgtacgtgca atcaacgtgt 4140
   atggaaccag tgagccaagc caggagtctg aactcacaac ggtaggagag aaacctgaag 4200
   agccgaagga tgaagtggag gtgtcagatg atgatgagaa ggagcccgag gttgattacc 4260
   ggacagtgac aatcaatact gaacaaaaag tatctgactt ctacgacatt gaggagagat 4320
   taggatctgg gaaatttgga caggtctttc gacttgtaga aaagaaaact cgaaaagtct 4380
   gggcagggaa gttcttcaag gcatattcag caaaagagaa agagaatatc cggcaggaga 4440
   ttagcatcat gaactgcctc caccacccta agctggtcca gtgtgtggat gcctttgaag 4500
   aaaaggccaa catcgtcatg gtcctggaga tcgtgtcagg aggggagctg tttgagcgca 4560
   tcattgacga ggactttgag ctgacggagc gtgagtgcat caagtacatg cggcagatct 4620
   cggagggagt ggagtacatc cacaagcagg gcatcgtgca cctggacctc aagccggaga 4680
   acatcatgtg tgtcaacaag acgggcacca ggatcaagct catcgacttt ggtctggcca 4740
   ggaggctgga gaatgcgggg tctctgaagg tcctctttgg caccccagaa tttgtggctc 4800
   ctgaagtgat caactatgag cccatcggct acgccacaga catgtggagc atcggggtca 4860
   tctgctacat cctagtcagt ggcctttccc ccttcatggg agacaacgat aacgaaacct 4920
   tggccaacgt tacctcagcc acctgggact tcgacgacga ggcattcgat gagatctccg 4980
   acgatgccaa ggatttcatc agcaatctgc tgaagaaaga tatgaaaaac cgcctggact 5040
   gcacgcagtg ccttcagcat ccatggctaa tgaaagatac caagaacatg gaggccaaga 5100
   aactctccaa ggaccggatg aagaagtaca tggcaagaag gaaatggcag aaaacgggca 5160
   atgctgtgag agccattgga agactgtcct ctatggcaat gatctcaggg ctcagtggca 5220
   ggaaatcctc aacagggtca ccaaccagcc cgctcaatgc agaaaaacta gaatctgaag 5280
   aagatgtgtc ccaagctttc cttgaggctg ttgctgagga aaagcctcat gtaaaaccct 5340
   atttctctaa gaccattcgc gatttagaag ttgtggaggg aagtgctgct agatttgact 5400
   gcaagattga aggataccca gaccccgagg ttgtctggtt caaagatgac cagtcaatca 5460
   gggagtcccg ccacttccag atagactacg atgaggacgg gaactgctct ttaattatta 5520
   gtgatgtttg cggggatgac gatgccaagt acacctgcaa ggctgtcaac agtcttggag 5580
   aagccacctg cacagcagag ctcattgtgg aaacgatgga ggaaggtgaa ggggaagggg 5640
   aagaggaaga agagtgaaac aaagccagag aaaagcagtt tctaagtcat attaaaagga 5700
   ctatttctct caaaatcca 5719
<210> 47
   <211> 284
   <212> PRT
   <213> Homo sapiens
<300>
   <308> AAH07433
   <309> 2004-06-29
   <313> (1)..(284)
<400> 47
   Met Asp Ala Ile Lys Lys Lys Met Gln Met Leu Lys Leu Asp Lys Glu
   1               5                   10                  15
   Asn Ala Leu Asp Arg Ala Glu Gln Ala Glu Ala Asp Lys Lys Ala Ala
               20                  25                  30
   Glu Asp Arg Ser Lys Gln Leu Glu Asp Glu Leu Val Ser Leu Gln Lys
           35                  40                  45
   Lys Leu Lys Gly Thr Glu Asp Glu Leu Asp Lys Tyr Ser Glu Ala Leu
       50                  55                  60
   Lys Asp Ala Gln Glu Lys Leu Glu Leu Ala Glu Lys Lys Ala Thr Asp
   65                  70                  75                  80
   Ala Glu Ala Asp Val Ala Ser Leu Asn Arg Arg Ile Gln Leu Val Glu
                   85                  90                  95
   Glu Glu Leu Asp Arg Ala Gln Glu Arg Leu Ala Thr Ala Leu Gln Lys
               100                 105                 110
   Leu Glu Glu Ala Glu Lys Ala Ala Asp Glu Ser Glu Arg Gly Met Lys
           115                 120                 125
   Val Ile Glu Ser Arg Ala Gln Lys Asp Glu Glu Lys Met Glu Ile Gln
       130                 135                 140
   Glu Ile Gln Leu Lys Glu Ala Lys His Ile Ala Glu Asp Ala Asp Arg
   145                 150                 155                 160
   Lys Tyr Glu Glu Val Ala Arg Lys Leu Val Ile Ile Glu Ser Asp Leu
                   165                 170                 175
   Glu Arg Ala Glu Glu Arg Ala Glu Leu Ser Glu Gly Gln Val Arg Gln
   180 185 190
   Leu Glu Glu Gln Leu Arg Ile Met Asp Gln Thr Leu Lys Ala Leu Met
   195 200 205
   Ala Ala Glu Asp Lys Tyr Ser Gln Lys Glu Asp Arg Tyr Glu Glu Glu
   210 215 220
   Ile Lys Val Leu Ser Asp Lys Leu Lys Glu Ala Glu Thr Arg Ala Glu
   225 230 235 240
   Phe Ala Glu Arg Ser Val Thr Lys Leu Glu Lys Ser Ile Asp Asp Leu
   245 250 255
   Glu Asp Glu Leu Tyr Ala Gln Lys Leu Lys Tyr Lys Ala Ile Ser Glu
   260 265 270
   Glu Leu Asp His Ala Leu Asn Asp Met Thr Ser Met
   275 280
<210> 48
   <211> 1256
   <212> DNA
   <213> Homo sapiens
<300>
   <308> NM_000366
   <309> 2004-12-18
   <313> (1)..(1256)
<400> 48
   gaggaatgcg gtcgccccct tgggaaagta catatctggg agaagcaggc ggctccgcgc    60
   tcgcactccc gctcctccgc ccgaccgcgc gctcgccccg ccgctcctgc tgcagcccca   120
   gggcccctcg ccgccgccac catggacgcc atcaagaaga agatgcagat gctgaagctc   180
   gacaaggaga acgccttgga tcgagctgag caggcggagg ccgacaagaa ggcggcggaa   240
   gacaggagca agcagctgga agatgagctg gtgtcactgc aaaagaaact caagggcacc   300
   gaagatgaac tggacaaata ttctgaggct ctcaaagatg cccaggagaa gctggagctg   360
   gcagagaaaa aggccaccga tgctgaagcc gacgtagctt ctctgaacag acgcatccag   420
   ctggttgagg aagagttgga tcgtgcccag gagcgtctgg caacagcttt gcagaagctg   480
   gaggaagctg agaaggcagc agatgagagt gagagaggca tgaaagtcat tgagagtcga   540
   gcccaaaaag atgaagaaaa aatggaaatt caggagatcc aactgaaaga ggcaaagcac   600
   attgctgaag atgccgaccg caaatatgaa gaggtggccc gtaagctggt catcattgag   660
   agcgacctgg aacgtgcaga ggagcgggct gagctctcag aaggccaagt ccgacagctg   720
   gaagaacaat taagaataat ggatcagacc ttgaaagcat taatggctgc agaggataag   780
   tactcgcaga aggaagacag atatgaggaa gagatcaagg tcctttccga caagctgaag   840
   gaggctgaga ctcgggctga gtttgcggag aggtcagtaa ctaaattgga gaaaagcatt   900
   gatgacttag aagacgagct gtacgctcag aaactgaagt acaaagccat cagcgaggag   960
   ctggaccacg ctctcaacga tatgacttcc atgtaaacgt tcatccactc tgcctgctta  1020
   caccctgccc tcatgctaat ataagtttct ttgcttcact tctcccaaga ctccctcgtc  1080
   gagctggatg tcccacctct ctgagctctg catttgtcta ttctccagct gaccctggtt  1140
   ctctctctta gcatcctgcc ttagagccag gcacacactg tgctttctat tgtacagaag  1200
   ctcttcgttt cagtgtcaaa taaacactgt gtaagctaaa aaaaaaaaaa aaaaaa      1256
<211> 11
   <212> PRT
   <213> Homo sapiens
<400> 49
<210> 50
   <211> 7
   <212> PRT
   <213> Homo sapiens
<400> 50
   Met Leu Thr Glu Leu Glu Lys
   1               5
<210> 51
   <211> 11
   <212> PRT
   <213> Homo sapiens
<400> 51
   Ala Leu Asn Ser Ile Ile Asp Val Tyr His Lys
   1               5                   10
<210> 52
   <211> 7
   <212> PRT
   <213> Homo sapiens
<400> 52
   Gly Ala Asp Val Trp Phe Lys
   1               5

## Description

### FIELD OF THE INVENTION

The invention relates to methods for screening a subject for endometrial cancer.

### BACKGROUND OF THE INVENTION

The endometrium, the tissue lining the uterus, is a glandular layer of variable thickness that is very sensitive to the hormones estrogen and progesterone. During the menstrual cycle the endometrium undergoes cyclic variation with a proliferation phase where the endometrium grows under the influence of estrogen, an ovulation phase where the endometrium is exposed to estrogen and progesterone, and a secretory or progesterone dominated phase where the endometrium shows signs of increased gland growth and secretion due largely to the influence of progesterone [1]. The secretory phase is followed by the shedding of the endometrium during menstruation. The histologic changes in the endometrium have been used to detect the stage or status of the endometrium, which is important for example, in determining the receptivity of patients to fertility procedures, and in determining responses to estrogen and/or progesterone therapies.

Endometrial carcinoma is a common malignancy in women, being exceeded in incidence only by that of breast, lung, and colorectal cancers [10, 11, 48, 125-131]. The lifetime probability of a Canadian woman developing endometrial carcinoma is 2.2% [10]. Although the case-fatality rate for cancer of the endometrium is lower than that of many other cancer sites, this rate does not fully reflect the health care burden posed by endometrial carcinoma. Investigation of women with perimenopansal and postmenopausal bleeding for the presence of endometrial carcinoma is one of the most common gynecologic investigations and requires invasive endometrial sampling. Yet only a small proportion of these investigations will result in a diagnosis of endometrial carcinoma [12].

It has been suggested that some genes and proteins are differentially expressed in endometrial carcinoma tissue, but their potential as tumor markers has not been established [157]. However, no methods for screening or early detection of endometrial carcinoma are available nor are there any serum tumor markers available for the monitoring of endometrial carcinoma patients [13, 14]. Consequently, patients are diagnosed following the development of symptoms, and patients with recurrent disease are detected only following the development of recurrent symptoms, or abnormalities in imaging assessments. Sensitive and specific tumor marker(s) for endometrial carcinoma are urgently needed for screening and diagnosis. There is also a need for reliable endometrial markers for determining the stage/phase, and status of the endometrium.

### SUMMARY OF THE INVENTION

Applicants have developed a method for identifying markers associated with the endometrium, and in particular with proliferative endometrium, secretory endometrium, and diseased endometrial tissue. Using the method they analyzed normal endometrial tissue homogenates and endometrial tumor tissue homogenates, and identified novel endometrial markers, in particular markers of secretory and proliferative endometrium and endometrial cancer markers.

The method of the invention in its broadest sense is as follows:

A method for screening a subject for endometrial cancer comprising(a) detecting in a sample from the subject an amount of an endometrial cancer marker; (b) comparing the amount of the endometrial cancer marker detected to a standard, (c) detecting a higher level of the endometrial cancer marker compared to the standard, wherein a significantly higher level of the endometrial cancer marker compared to the standard is indicative of endometrial cancer, and wherein the endometrial cancer marker is pyruvate kinase M1 (PKM1) or M2 (PKM2) isozyme comprising SEQ ID NO: 36, or a naturally occurring allelic variant thereof, or a naturally occurring truncated or secreted form thereof.

Further aspects of the invention are set out below in the following items, as defined in the numbered paragraphs:
1. A method of the invention as defined above, wherein the level of the endometrial cancer marker is 1.5, 2, 3, 4, 5, or 6 times higher compared to the standard.
2. A method of the invention as defined above, including item 1, wherein the sample is obtained from tissues, extracts, cell cultures, cell lysates, lavage fluid, or physiological fluids. In some embodiments the sample is obtained from a tumor tissue.
3. A method of the invention as defined above, including items 1 or 2, wherein the endometrial cancer marker is detected by contacting the sample with an antibody that specifically binds to the endometrial cancer marker.
4. A method as defined in item 3, wherein the antibody is used in a radioimmunoassay, enzyme immunoassay, microarray, competitive immunoassay, non-competitive immunoassay, or immunofluorescence, immunoprecipiation, latex agglutination, hemagglutination, histochemical or bioluminescence assay.
5. A method of the invention as defined above, including items 1, 2, 3 or 4, wherein the standard comprises levels of the endometrial cancer marker for control samples from healthy subjects.
6. A method of the invention as defined above, including items 1, 2, 3, 4 or 5, wherein the standard comprises levels of the endometrial cancer marker for another sample or an earlier sample from the subject.

### DESCRIPTION OF THE DRAWINGS

The invention will now be described in relation to the drawings in which:
Figure 1 shows a schematic representation of the ICAT procedure including labeling, digestion, SCX fractionation, affinity isolation, cleavage of the biotin moiety and LC-MS/MS analysis.
Figure 2 shows histologic sections of human endometrium, stained with hematoxylin and eosin. A) proliferative endometrium, sample PRO 2, and B) secretory endometrium, sample SEC2.
Figure 3 shows a NanoLC-MS TIC of SCX fraction 16, ICAT sample A.
Figure 4 shows the distribution of proteins identified from the middle ten SCX fractions of all three samples.
Figure 5 shows A) a mass spectral window of Sample A, SCX fraction 19, showing enhanced expression of glutamate receptor subunit zeta 1 precursor in the secretory endometrium. The line to the left of the isotope envelope of the heavy-labeled version of the protein denotes where the light-labeled version is expected. B) Resulting MS/MS spectra of the heavy labelled series showing sequence coverage.
Figure 6 shows A) a mass spectral window of Sample A, SCX fraction 13, showing enhanced expression of MIF in the secretory endometrium. B) Resulting MS/MS spectra of the heavy labelled series sequence coverage.

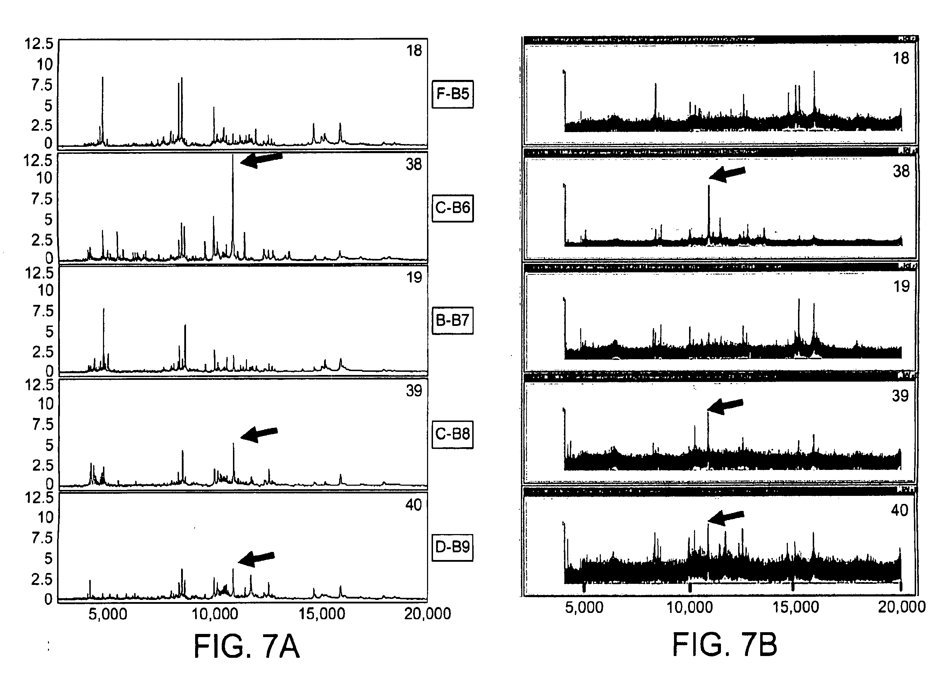

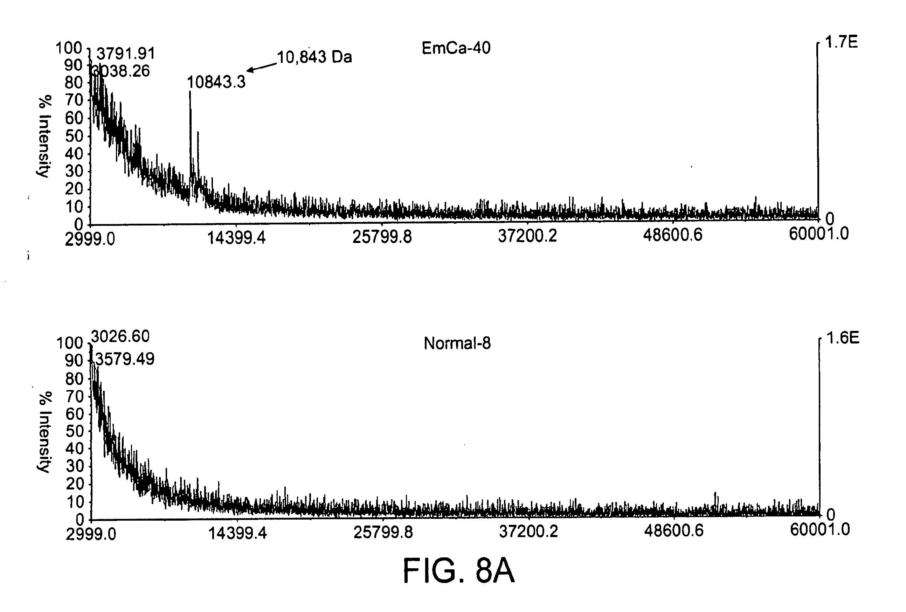

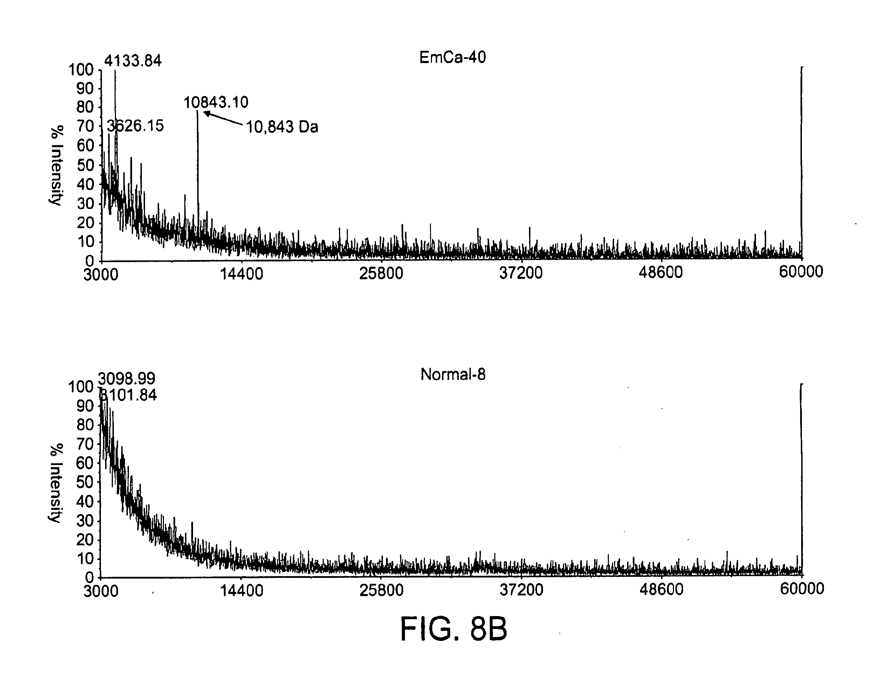

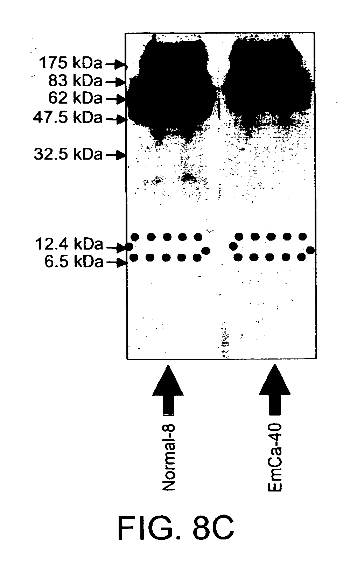

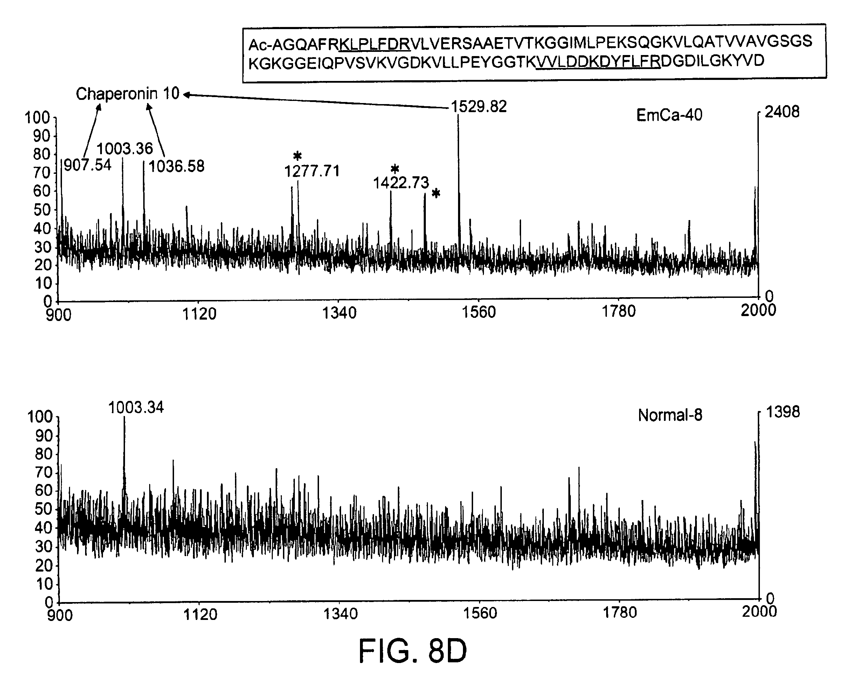

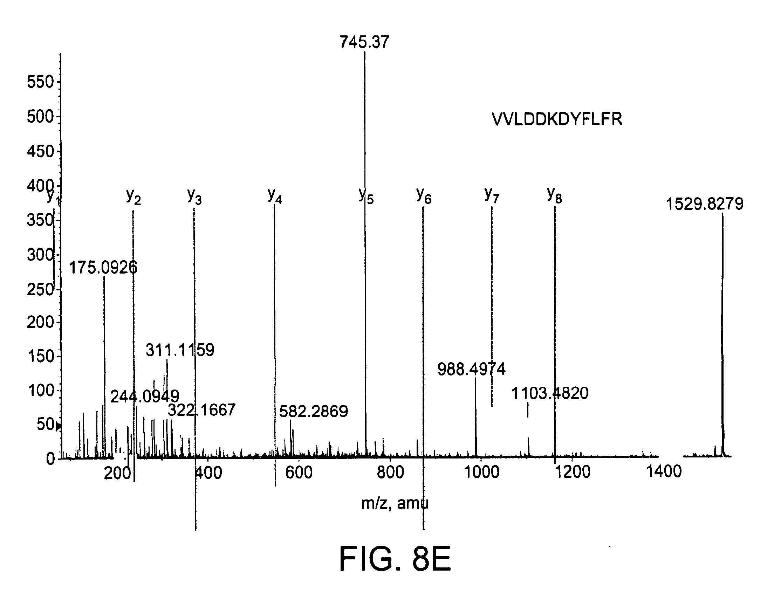

Figure 7 (a+b). STEP ONE Mass spectra obtained from five whole endometrial tissue homogeneates on Proteinchip WCX2 using (1a) SELDI-TOF-MS, PBSIIc, and (1b) QqTOF-MS, QSTAR XL. Note, using either MS method, the three malignant cases (38,39,40) show a distinct protein peak, which is low in the two non-malignant cases [18, 19]. The QSTARXL MS determined the weight of this peak to be 10,843 Da. Figure 7 STEP TWO Outline of protein purification. The target protein eluted in one of the early fractions of the size exclusion HPLC, was concentrated by Proteospin and then subjected to MALDI and SDS-PAGE (see Figure 8).
Figure 8 (A to E). STEP THREE (8A). Molecular weight verification of target protein after SEC. MALDI-TOF MS of samples from Proteospin was used to locate the fraction with the 10,843 Da weight of the target protein in a malignant case (40).The corresponding fraction from normal sample was used in parallel for all the following experiments. (8B) Molecular weight verification of the target protein fraction following DTT treatment. MALDI-TOF analysis of two samples indicates that the 10,843 Da peak remained in the malignant sample even after DTT treatment. (8C) SDS-PAGE analysis of the DTT treated fractions. The gel was stained by colloidal coomassie blue (Gel-Code, Pierce). The region containing the target protein was excised according to the molecular weight marker, and the intact proteins were extracted. (8D) Tryptic peptide profiles of the SDS-PAGE protein extract. MALDI-TOF MS analysis identified six "unique" tryptic peptides in the malignant samples. Three of them with mass of 907.54, 1036.58, and 1529.82 Da matched with the tryptic peptides from chaperonin 10. The remaining three peaks, marked with *, were from Keratin. (insert) Amino acid sequence of chaperonin 10. The locations of the three peptides were underlined. (8E) Fragmentation of peptide by collision induced dissociation. The 1529.82 Da peptide was fragmented. A series ofy ions confirmed that the 1529.82 Da peptide comes from chaperonin 10.
Figure 9. Western blot analysis against chaperonin 10. The malignant endometrial cases (EmCa) demonstrate a higher expression of chaperonin 10 than non-malignant cases (Normal). The level of ERKI was used to demonstrate similar loading among all lanes.
Figure 10. Immunohistochemical staining for chaperonin 10. Note granular cytoplasmic positivity in several malignant endometrioid glands, while adjacent endometrial stroma and a portion of one benign endometrial gland (right) shows much reduced staining.
Figure 11 shows mass spectral windows from isotope-coded affinity tag (ICAT) experiments for three pairs of endometrial cancer / normal samples, demonstrating the over expression of calgizzarin in the cancer samples.
Figure 12: Number of peptides/ protein identified. The first (front) series corresponds to results obtained from cICAT, the second series is from the first iTRAQ run, and the third (rearmost) series is from the second iTRAQ run.
Figure 13: Cytoplasmic actin sequencing and quantification using iTRAQ. *A*: MS/MS spectrum of the doubly protonated peptide, EITALAPSTMK [SEQ ID NO. 49], at 725.4 Th. Residues in black are sequenced from the b-ion series, while those in purple from the y-ion series. Residue 'J' in the y-ion series is the iTRAQ-modified lysine residue. *B*: Expanded view of the low-m/z end of the MS/MS spectrum in '*A*', showing relative abundances of the signature iTRAQ ions at 114.1, 115.1, 116.1 and 117.1 Th.

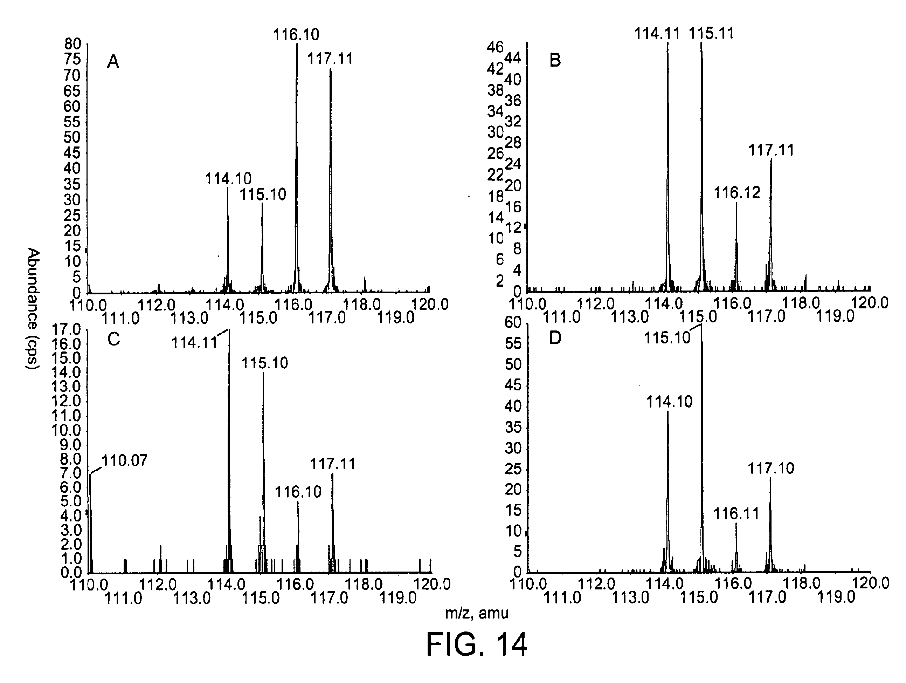

Figure 14: Relative abundances of differentially expressed proteins. The ion assignments are as follows: 114 Th, proliferative endometrium; 115 Th, secretory endometrium; 116 Th, first endometrial carcinoma; and 117 Th, second endometrial carcinoma. *A*: Tryptic peptide from chaperonin 10, showing higher abundances in the 116 and 117 Th ions relative to the 114 and 115 Th ions; *B*: tryptic peptide from alpha-1-antitrypsin precursor, showing lower abundances in the 116 and 117 Th ions relative to the 114 and 115 Th ions; *C*: tryptic peptide from creatine kinase B, showing lower abundances in the 116 and 117 Th ions relative to the 114 and 115 Th ions; *D*: tryptic peptide from transgelin, showing once more lower abundances in the 116 and 117 Th ions relative to the 114 and 115 Th ions.
Figure 15: Relative abundances of calgizzarin. cICAT analysis shows overexpression in all three cancer samples. The dotted line to the left of the heavy-label series, which originates from the cancerous sample, indicates the monoisotopic peak of the light-label series, which originates from the normal sample.
Figure 16: Distribution of proteins identified by *A*: iTRAQ and *B*: cICAT analyses.
Figure 17 shows histologic sections of human endometrium stained with hematoxylin and eosin: (a) proliferative endometrium, sample PRO2, and (b) secretory endometrium, sample SEC2.
Figure 18 is a nano LC/MS total ion chromatogram of strong cation exchange fraction 16 of sample A.
Figure 19 shows the distribution of 119 confirmed proteins. The legend shows the protein categories starting with "Structural" at 18% at the 2 o'clock position and continuing clockwise.
Figure 20 shows the appearance of selected proteins in the strong cation exchange fractions.
Figure 21 shows the (a) mass spectral window of sample A, fraction 19, showing enhanced expression of glutamate receptor subunit zeta 1 precursor in the secretory endometrium. The line to the left of the isotope envelope of the heavy-labeled version of the protein denotes where the light-labeled version is expected; (b) resulting MS/MS spectrum showing a partial sequence for the peptide LLTLALLFSCSVAR [SEQ ID NO. 28].
Figure 22 shows the mass spectral windows of the three sample pairs, fraction 15, showing enhanced expression of FRAT1.
Figure 23 shows the mass spectral windows of sample A, fraction 4, showing enhanced expression of glycodelin in the secretory endometrium.
Figure 24 shows the mass spectral windows showing the isotopic clusters of cathepsin B. Sample A shows enhanced cathepsin B expression in the secretory endometrium; sample B shows the opposite trend.
Figure 25 shows SELDI MS spectral windows acquired on the QqTOF mass spectrometer (QSTAR XL): (a) typical tissue homogenate; (b) homogenate treated with 0 % hydrogen peroxide; (c) homogenate in (b) treated incubated with 3% hydrogen peroxide overnight; (d) laser fluence at 34.6 µJ; and (e) laser fluence at 76.9 µJ.
Figure 26 shows SELDI MS spectra of four malignant (designated by "C") and two endometrial tissue homogenates (designated by "N"): (a) full scan spectra collected on the linear QTOF mass spectrometer (PBS IIc); and (b) mass spectral windows collected on the PBS IIc, left panel, and on the QqTOF mass spectrometer (QSTAR XL), right panel.
Figure 27 shows SELDI MS spectral windows showing binding of both (a) chaperonin 10 and (b) the unknown protein at pH 6.0; and selective binding of the unknown protein at pH 7.0.
Figure 28 shows MS/MS spectra of three tryptic peptides from the unknown protein that identify it as calgranulin A. Peptide identities: (a) MLTELEK [SEQ ID NO. 50], (b) ALNSIIDVYHK [SEQ ID NO. 51], and (c) GADVWFK [SEQ ID NO. 52].
Figure 29 shows Calgranulin A immunohistochemical stain of endometrial TMA (US Biochem 1:150): (A) Endometrioid adenocarcinoma exhibits diffuse, immunostaining of numerous cells (3+) of the glandular component of the adenocarcinoma. Intense immunostaining of macrophages/granulocytes within the stroma and glandular lumina is also evident. No stromal staining is observed. (B) Endometrioid adenocarcinoma shows strong positive immunostaining of occasional malignant glands (I+). Intense individual cell staining is also apparent in macrophages/granulocytes within gland lumina and stroma.
Figure 30 shows Calgranulin A immunohistochemical stain of endometrial TMA (US Biochem 1:150): Note intense (3+) cytoplasmic and nuclear immunostaining in squamous areas of this endometrioid adenocarcinoma. Glandular or columnar areas of the adenocarcinoma and adjacent stroma do not demonstrate any staining in this particular case.
Figure 31 shows Calgranulin A immunohistochemical stain of endometrial TMA (US Biochem 1:150): The glands and stroma of this proliferative endometrium show no immunostaining. However, intense individual macrophage/granulocyte immunostaining is evident within the stroma.

### DETAILED DESCRIPTION OF THE INVENTION

Methods are provided for screening a subject for endometrial cancer. Disclosed are also methods for characterizing the stage or phase of endometrium, detecting the presence of an endometrial disease (e.g. endometrial cancer) in a sample, the absence of a disease (e.g. endometrial cancer) in a sample, the stage of a disease, the stage or grade of the disease, and other characteristics of endometrial diseases that are relevant to prevention, diagnosis, characterization, and therapy of endometrial diseases such as cancer in a patient, for example, the benign or malignant nature of an endometrial cancer, the metastatic potential of an endometrial cancer, assessing the histological type of neoplasm associated with an endometrial cancer, the indolence or aggressiveness of an endometrial cancer, and other characteristics of endometrial diseases that are relevant to prevention, diagnosis, characterization, and therapy of endometrial diseases such as cancer in a patient. Methods are also disclosed for assessing the efficacy of one or more test agents for inhibiting an endometrial disease, assessing the efficacy of a therapy for an endometrial disease, monitoring the progression of an endometrial disease, selecting an agent or therapy for inhibiting an endometrial disease, treating a patient afflicted with an endometrial disease, inhibiting an endometrial disease in a patient, and assessing the disease (e.g. carcinogenic) potential of a test compound.

### Glossary

"Endometrial disease" refers to any disorder, disease, condition, syndrome or combination of manifestations or symptoms recognized or diagnosed as a disorder ofthe endometrium, including but not limited to hyperplasia and cancer precursors, endometrial cancer or carcinoma, endometriosis, reproductive disorders, and infertility.

"Endometrial cancer" or "endometrial carcinoma" includes malignant endometrial disease including but not limited to endometrioid, mucinous, and serous adenocarcinomas, adenosquamous carcinomas, clear cell carcinomas, uterine sarcomas including stromal sarcomas, malignant mixed Mullerian tumors (carcinosarcomas), and leiomyosarcomas.

The terms "sample", "biological sample", and the like mean a material known or suspected of expressing or containing one or more endometrial polynucleotide markers or one or more endometrial markers. A test sample can be used directly as obtained from the source or following a pretreatment to modify the character of the sample. The sample can be derived from any biological source, such as tissues, extracts, or cell cultures, including cells (e.g. tumor cells), cell lysates, and physiological fluids, such as, for example, whole blood, plasma, serum, saliva, ocular lens fluid, cerebral spinal fluid, sweat, urine, milk, ascites fluid, synovial fluid, peritoneal fluid, lavage fluid, and the like. The sample can be obtained from animals, preferably mammals, most preferably humans. The sample can be treated prior to use, such as preparing plasma from blood, diluting viscous fluids, and the like. Methods of treatment can involve filtration, distillation, extraction, concentration, inactivation of interfering components, the addition of reagents, and the like.

In embodiments of the invention the sample is a mammalian tissue sample. In a particular embodiment, the tissue is endometrial tissue.

In another embodiment the sample is a human physiological fluid. In a particular embodiment, the sample is human serum.

The samples that may be analyzed in accordance with the disclosure include polynucleotides from clinically relevant sources, preferably expressed RNA or a nucleic acid derived therefrom (cDNA or amplified RNA derived from cDNA that incorporates an RNA polymerase promoter). The method of the invention is limited to the polypeptide markers specified in claim 1. The target polynucleotides can comprise RNA, including, without limitation total cellular RNA, poly(A)⁺ messenger RNA (mRNA) or fraction thereof, cytoplasmic mRNA, or RNA transcribed from cDNA (i.e., cRNA; see, for example., Linsley & Schelter, U.S. patent application Ser. No. 09/411,074, or U.S. Pat. Nos. 5,545,522, 5,891,636, or 5,716,785). Methods for preparing total and poly(A)⁺ RNA are well known in the art, and are described generally, for example, in Sambrook et al., (1989, Molecular Cloning- A Laboratory Manual (2nd Ed.), Vols. 1-3, Cold Spring Harbor Laboratory, Cold Spring Harbor, N.Y.) and Ausubel et al, eds. (1994, Current Protocols in Moelcular Biology, vol. 2, Current Protocols Publishing, New York). RNA may be isolated from eukaryotic cells by procedures involving lysis of the cells and denaturation of the proteins contained in the cells. Additional steps may be utilized to remove DNA. Cell lysis may be achieved with a nonionic detergent, followed by microcentrifugation to remove the nuclei and hence the bulk of the cellular DNA. (See Chirgwin et al., 1979, Biochemistry 18:5294-5299). Poly(A)+RNA can be selected using oligo-dT cellulose (see Sambrook et al., 1989, Molecular Cloning-A Laboratory Manual (2nd Ed), Vols. 1-3, Cold Spring Harbor Laboratory, Cold Spring Harbor, N.Y.). In the alternative, RNA can be separated from DNA by organic extraction, for example, with hot phenol or phenol/chloroform/isoamyl alcohol.

It may be desirable to enrich mRNA with respect to other cellular RNAs, such as transfer RNA (tRNA) and ribosomal RNA (rRNA). Most mRNAs contain a poly(A) tail at their 3' end allowing them to be enriched by affinity chromatography, for example, using oligo(dT) or poly(U) coupled to a solid support such as cellulose or Sephadex™ (see Ausubel et al., eds., 1994, Current Protocols in Molecular Biology, vol. 2, Current Protocols Publishing, New York). Bound poly(A)-mRNA is eluted from the affinity column using 2 mM EDTA/0.1% SDS.

A sample of RNA can comprise a plurality of different mRNA molecules each with a different nucleotide sequence. In an aspect of the disclosure the mRNA molecules in the RNA sample comprise at least 100 different nucleotide sequences.

Target polynucleotides can be detectably labeled at one or more nucleotides using methods known in the art. The label is preferably uniformly incorporated along the length of the RNA, and more preferably, is carried out at a high degree of efficiency. The detectable label can be a luminescent label, fluorescent label, bioluminescent label, chemi-luminescent label, radiolabel, and colorimetric label. In a particular embodiment, the label is a fluorescent label, such as a fluorescein, a phosphor, a rhodamine, or a polymethine dye derivative. Commercially available fluorescent labels include, for example, fluorescent phosphoramidites such as FluorePrime (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.).

Target polynucleotides from a patient sample can be labeled differentially from polynucleotides of a standard. The standard can comprise target polynucleotides from normal individuals (i.e., those not afflicted with or pre-disposed to endometrial disease, in particular pooled from samples from normal individuals. The target polynucleotides can be derived from the same individual, but taken at different time points, and thus indicate the efficacy of a treatment by a change in expression of the markers, or lack thereof, during and after the course of treatment.

The terms "subject", "individual" or "patient" refer to a warm-blooded animal such as a mammal. In particular, the terms refer to a human. A subject, individual or patient may be afflicted with or suspected of having or being pre-disposed to endometrial disease or a condition as described herein. The term also includes domestic animals bred for food or as pets, including horses, cows, sheep, poultry, fish, pigs, cats, dogs, and zoo animals.

Methods disclosed herein for administering an agent or composition to subjects/individuals/patients contemplate treatment as well as prophylactic use. Typical subjects for treatment include persons susceptible to, suffering from or that have suffered a condition or disease described herein. In particular, suitable subjects for treatment in accordance with the invention are persons that are susceptible to, suffering from or that have suffered endometrial cancer.

The term "endometrial marker" includes a marker associated with normal or diseased endometrial tissue identified using a method of the invention. The term includes native-sequence polypeptides isoforms, chimeric polypeptides, complexes, all homologs, fragments, precursors, and modified forms and derivatives of the markers.

An endometrial marker may be associated with a stage or phase of endometrial tissue such as the secretory or proliferative phase. Examples of endometrial markers associated with the secretory phase are glutamate receptor subunit zeta 1 [SEQ ID NO. 26] and parts thereof (e.g. a tryptic fragment of SEQ ID NO. 28), macrophage migration inhibitory factor [SEQ ID NO. 18], FRAT1 [SEQ ID NO. 42], myosin light chain kinase 2 [SEQ ID NO. 45], and tropomyosin 1 alpha chain [SEQ ID NO. 47].

An endometrial marker may be associated with an endometrial disease, in particular it may be an endometrial cancer marker. The term "endometrial cancer marker" includes a marker associated with endometrial cancer, in particular a marker listed in Table 1.

In an aspect disclosed herein an endometrial cancer marker is chaperonin 10. The term "chaperonin 10", "chaperonin 10 polypeptide" or "chaperonin protein" includes human chaperonin 10, in particular the native-sequence polypeptide, isoforms, chimeric polypeptides, all homologs, fragments, precursors, complexes, and modified forms and derivatives of human chaperonin 10. The amino acid sequence for native human chaperonin 10 includes the sequences of GenBank Accession No. Q04984 and AAH23518 shown in SEQ ID NO. 1.

A "native-sequence polypeptide" comprises a polypeptide having the same amino acid sequence of a polypeptide derived from nature. Such native-sequence polypeptides can be isolated from nature or can be produced by recombinant or synthetic means. The term specifically encompasses naturally occurring truncated or secreted forms of a polypeptide, polypeptide variants including naturally occurring variant forms (e.g. alternatively spliced forms or splice variants), and naturally occurring allelic variants.

An allelic variant may also be created by introducing substitutions, additions, or deletions into a polynucleotide encoding a native polypeptide sequence such that one or more amino acid substitutions, additions, or deletions are introduced into the encoded protein. Mutations may be introduced by standard methods, such as site-directed mutagenesis and PCR-mediated mutagenesis. In an embodiment, conservative substitutions are made at one or more predicted non-essential amino acid residues. A "conservative amino acid substitution" is one in which an amino acid residue is replaced with an amino acid residue with a similar side chain. Amino acids with similar side chains are known in the art and include amino acids with basic side chains (e.g. Lys, Arg, His), acidic side chains (e.g. Asp, Glu), uncharged polar side chains (e.g. Gly, Asp, Glu, Ser, Thr, Tyr and Cys), nonpolar side chains (e.g. Ala, Val, Leu, Iso, Pro, Trp), beta-branched side chains (e.g. Thr, Val, Iso), and aromatic side chains (e.g. Tyr, Phe, Trp, His). Mutations can also be introduced randomly along part or all ofthe native sequence, for example, by saturation mutagenesis. Following mutagenesis the variant polypeptide can be recombinantly expressed and the activity of the polypeptide may be determined.

A naturally occurring allelic variant may contain conservative amino acid substitutions from the native polypeptide sequence or it may contain a substitution of an amino acid from a corresponding position in a polypeptide homolog, for example, a murine polypeptide.

Endometrial markers include chimeric or fusion proteins. A "chimeric protein" or "fusion protein" comprises all or part (preferably biologically active) of an endometrial marker operably linked to a heterologous polypeptide (i.e., a polypeptide other than an endometrial marker). Within the fusion protein, the term "operably linked" is intended to indicate that an endometrial marker and the heterologous polypeptide are fused in-frame to each other. The heterologous polypeptide can be fused to the N-terminus or C-terminus of an endometrial marker. A useful fusion protein is a GST fusion protein in which an endometrial marker is fused to the C-terminus of GST sequences. Another example of a fusion protein is an immunoglobulin fusion protein in which all or part of an endometrial marker is fused to sequences derived from a member of the immunoglobulin protein family. Chimeric and fusion proteins can be produced by standard recombinant DNA techniques.

A modified form of a polypeptide referenced herein includes modified forms of the polypeptides and derivatives of the polypeptides, including post-translationally modified forms such as glycosylated, phosphorylated, acetylated, methylated or lapidated forms of the polypeptides. For example, an N-terminal methionine may be cleaved from a polypeptide, and a new N-terminal residue may or may not be acetylated. In particular, for chaperonin 10 the first residue, methionine, can be cleaved and the second first residue, alanine can be N-acetylated.

Endometrial markers identified in accordance with a method of the invention may be prepared by recombinant or synthetic methods, or isolated from a variety of sources, or by any combination of these and similar techniques.

Polynucleotide markers are disclosed but not claimed.

"Endometrial polynucleotide marker(s)", polynucleotides encoding the marker(s)", and polynucleotides encoding endometrial markers" refer to polynucleotides that encode endometrial markers including native-sequence polypeptides, polypeptide variants including a portion of a polypeptide, an isoform, precursor, complex, a chimeric polypeptide, or modified forms and derivatives of the polypeptides. An endometrial polynucleotide marker includes the polynucleotides encoding the polypeptides listed in Table 1, 4, 5, or 6. In particular, the invention includes the polynucleotides encoding glutamate receptor subunit zeta 1 [e.g. see Accession No. D13515 and SEQ ID NO. 27], a tryptic peptide thereof [SEQ ID NO. 28], macrophage migration inhibitory factor [e.g. see Accession Nos. NM_002415 and L19686 and SEQ ID NOs. 19 and 20], FRAT1 [e.g. see Accession Nos. NM_005479 and NM_181355 and SEQ ID NOs. 43 and 44], myosin light chain kinase 2 [Accession No. A069601 and SEQ ID NO. 46], tropomyosin 1 alpha chain e.g. see Accession No. NM_000366 and BC007433 and SEQ ID NO. 48], and the endometrial markers listed in Table 1 [in particular, GenBank Accession Nos. NM_002157 and U07550, A12027, NM_002964, X06233, M21064, NM_002644, NM_002567, NM_006305, NM_002155, X51757, NM_002415, L19686, NM_005620 and D38583, NM_000365, X69723, NM_000295, K02212, NM_001823, X15334, X56494, D84342, A026126, and SEQ ID NOs. 2, 4, 5, 7, 8, 10, 12, 14, 16, 17, 19, 20, 22, 24, 25, 31, 32, 34, 35, 37, 39, 41]. In an embodiment, a polynucleotide of the invention encodes chaperonin 10, more particularly a polynucleotide sequence of GenBank Accession No. NM_002157 and U07550 [SEQ ID NO 2], or a fragment thereof.

Endometrial polynucleotide markers include complementary nucleic acid sequences, and nucleic acids that are substantially identical to these sequences (e.g. having at least about 45%, 50%, 55%, 60%, 65%, 70%, 75%, 80%, 85%, 90%, 95%, 97%, 98%, or 99% sequence identity).

Endometrial polynucleotide markers also include sequences that differ from a native sequence [e.g. see GenBank Accession Nos. NMR_002157 and U07550, A12027, NM_002964, X06233, M21064, NM_002644, NM_002567, NM_006305, NM_002155, X51757, NM_002415, L19686, NM_005620 and D38583, NM_000365, X69723, D13515, NM_000295, K02212, NMR_001823, X15334, X56494, D84342, A026126, NM_005479, NM_181355, AF069601, NM_000366, and BC007433, and SEQ ID NOs.2, 4, 5, 7, 8, 10, 12, 14, 16, 17, 19, 20, 22, 24, 25, 27, 31, 32, 34, 35, 37, 39, 41, 43, 44, 46, and 48] due to degeneracy in the genetic code. As one example, DNA sequence polymorphisms within the nucleotide sequence of an endometrial marker may result in silent mutations that do not affect the amino acid sequence. Variations in one or more nucleotides may exist among individuals within a population due to natural allelic variation: DNA sequence polymorphisms may also occur which lead to changes in the amino acid sequence of a polypeptide.

Endometrial polynucleotide markers also include nucleic acids that hybridize under stringent conditions, preferably high stringency conditions to an endometrial polynucleotide marker, in particular an endometrial cancer polynucleotide marker [e.g. see GenBank Accession Nos. NM_002157 and U07550, A12027, NM_002964, X06233, M21064, NM_002644, NM_002567, NM_006305,NM_002155, X51757, NM_002415, L19686, NM_005620 and D38583, NM_000365, X69723, D13515, NM_000295, K02212, NM_001823, X15334, X56494, D84342, AF026126, NM_005479, NM_181355, AF069601, NM_000366, and BC007433, and SEQ ID NOs. 2,4,5,7,8,10,12,14,16,17,19,20,22,24,25,27,31,32,34,35,37,39,41,43,44,46, and 48]. Appropriate stringency conditions which promote DNA hybridization are known to those skilled in the art, or can be found in Current Protocols in Molecular Biology, John Wiley & Sons, N.Y. (1989), 6.3.1-6.3.6. For example, 6.0 x sodium chloride/sodium citrate (SSC) at about 45°C, followed by a wash of 2.0 x SSC at 50°C may be employed. The stringency may be selected based on the conditions used in the wash step. By way of example, the salt concentration in the wash step can be selected from a high stringency of about 0.2 x SSC at 50°C. In addition, the temperature in the wash step can be at high stringency conditions, at about 65°C.

Endometrial polynucleotide markers also include truncated nucleic acids or nucleic acid fragments and variant forms of the nucleic acids that arise by alternative splicing of an mRNA corresponding to a DNA.

The endometrial polynucleotide markers are intended to include DNA and RNA (e.g. mRNA) and can be either double stranded or single stranded. A polynucleotide may, but need not, include additional coding or non-coding sequences, or it may, but need not, be linked to other molecules and/or carrier or support materials. The polynucleotides for use in the methods of the disclosure may be of any length suitable for a particular method. In certain applications the term refers to antisense polynucleotides (e.g. mRNA or DNA strand in the reverse orientation to sense cancer polynucleotide markers).

"Statistically different levels", "significantly altered levels", or "significant difference" in levels of markers in a patient sample compared to a control or standard (e.g. normal levels or levels in other samples from a patient) may represent levels that are higher or lower than the standard error of the detection assay. In particular embodiments, the levels may be 1.5, 2, 3, 4, 5, or 6 times higher or lower than the control or standard.

"Microarray" and "array," refer to nucleic acid or nucleotide arrays or protein or peptide arrays that can be used to detect biomolecules associated with endometrium or phase threof or endometrial disease, for instance to measure gene expression. A variety of arrays are made in research and manufacturing facilities worldwide, some of which are available commercially. By way of example, spotted arrays and *in situ* synthesized arrays are two kinds of nucleic acid arrays that differ in the manner in which the nucleic acid materials are placed onto the array substrate. A widely used in situ synthesized oligonucleotide array is GeneChip™ made by Affymetrix, Inc. Oligonucleotide probes that are 20- or 25-base long can be synthesized in silico on the array substrate. These arrays can achieve high densities (e.g., more than 40,000 genes per cm²). Generally spotted arrays have lower densities, but the probes, typically partial cDNA molecules, are much longer than 20- or 25-mers. Examples of spotted cDNA arrays include LifeArray made by Incyte Genomics and DermArray made by IntegriDerm (or Invitrogen). Pre-synthesized and amplified cDNA sequences are attached to the substrate of spotted arrays. Protein and peptide arrays also are known (see for example, Zhu et al., Science 293:2101 (2001).

"Binding agent" refers to a substance such as a polypeptide or antibody that specifically binds to one or more endometrial markers. A substance "specifically binds" to one or more endometrial markers if is reacts at a detectable level with one or more endometrial markers, and does not react detectably with peptides containing an unrelated or different sequence. Binding properties may be assessed using an ELISA, which may be readily performed by those skilled in the art (see for example, Newton et al, Develop. Dynamics 197: 1-13,1993).

A binding agent may be a ribosome, with or without a peptide component, an aptamer, an RNA molecule, or a polypeptide. A binding agent may be a polypeptide that comprises one or more endometrial marker sequence, a peptide variant thereof, or a non-peptide mimetic of such a sequence. By way of example, a chaperonin 10 sequence may be a peptide portion of a chaperonin 10 that is capable of modulating a function mediated by chaperonin 10.

An aptamer includes a DNA or RNA molecule that binds to nucleic acids and proteins. An aptamer that binds to a protein (or binding domain) of an endometrial marker or an endometrial polynucleotide marker can be produced using conventional techniques, without undue experimentation. (For example, see the following publications describing *in vitro* selection ofaptamers: Klug et al., Mol. Biol. Reports 20:97-107 (1994); Wallis et al., Chem. Biol. 2:543-552 (1995); Ellington, Curr. Biol. 4:427-429 (1994); Lato et al., Chem. Biol. 2:291-303 (1995); Conrad et al., Mol. Div. 1:69-78 (1995); and Uphoff et al., Curr. Opin. Struct. Biol. 6:281-287 (1996)).

Antibodies for use in the present invention include but are not limited to monoclonal or polyclonal antibodies, immunologically active fragments (e.g. a Fab or (Fab)₂ fragments), antibody heavy chains, humanized antibodies, antibody light chains, genetically engineered single chain Fᵥ molecules (Ladner et al, U.S. Pat. No. 4,946,778), chimeric antibodies, for example, antibodies which contain the binding specificity ofmurine antibodies, but in which the remaining portions are of human origin, or derivatives, such as enzyme conjugates or labeled derivatives.

Antibodies including monoclonal and polyclonal antibodies, fragments and chimeras, may be prepared using methods known to those skilled in the art. Isolated native or recombinant endometrial markers may be utilized to prepare antibodies. (See, for example, Kohler et al. (1975) Nature 256:495-497; Kozbor et al. (1985) J. Immunol Methods 81:31-42; Cote et al. (1983) Proc Natl Acad Sci 80:2026-2030; and Cole et al. (1984) Mol Cell Biol 62:109-120 for the preparation of monoclonal antibodies; Huse et al. (1989) Science 246:1275-1281 for the preparation of monoclonal Fab fragments; and, Pound (1998) Immunochemical Protocols, Humana Press, Totowa, N.J for the preparation of phagemid or B-lymphocyte immunoglobulin libraries to identify antibodies). Antibodies specific for an endometrial marker may also be obtained from scientific or commercial sources.

In an embodiment of the invention, antibodies are reactive against an endometrial marker if they bind with a kₐ of greater than or equal to 10⁻⁷ M.

### Markers

The disclosure provides a set of markers correlated with endometrium or phase thereof, or endometrial disease. The invention uses at least PKM1 or PKMa as defined in claim 1 as markers. A subset of these markers identified as useful for detection, diagnosis, prevention and therapy of endometrial disease is listed in Table I or 5. A subset of these markers identified as useful for detection and diagnosis of endometrium phase is listed in Table 4 or 6. The invention also provides a method of using these markers to distinguish endometrium phase or to distinguish endometrial disease.

The invention provides marker sets that distinguish endometrium phase or endometrial disease and uses therefor. Disclosed is also a method for classifying an endometrium phase or endometrial disease comprising detecting a difference in the expression of a first plurality of endometrial markers or endometrial polynucleotide markers relative to a control, the first plurality of endometrial markers or endometrial polynucleotide markers consisting of at least 5 of the markers listed in Table 1, 4, 5, or 6. In specific aspects, the plurality of markers consists of at least 10 of the markers listed in Table 1, 4, 5, or 6. In another specific aspect, the control comprises markers derived from a pool of samples from individual patients with no endometrial disease, or individuals with a known endometrium phase.

Any of the markers disclosed herein may be used alone or with other markers of endometrium phase or endometrial disease, or with markers for other phenotypes or conditions.

### Identification of Endometrial Markers

Markers associated with the endometrium or a phase thereof, or associated with a disease of the endometrium may be identified by:
(a) obtaining a sample of endometrium from a subject;
(b) extracting proteins from the sample and producing a profile of the proteins by subjecting the proteins to mass spectrometry; and
(c) comparing the profile with a profile for normal endometrial tissue or for a known endometrium phase to identify proteins associated with the endometrium phase or an endometrial disease.

Proteins may be extracted from the samples in a manner known in the art. For example, proteins may be extracted by first digesting or disrupting cell membranes by standard methods such as detergents or homogenization in an isotonic sucrose solution, followed by ultra-centrifugation or other standard techniques.

The separated proteins may be digested into peptides, in particular using proteolytic enzymes such as trypsin, pepsin, subtilisin, and proteinase. For example, proteins may be treated with trypsin which cleaves at the sites of lysine and arginine, to provide doubly-charged peptides with a length of from about 5 to 50 amino acids. Such peptides may be particularly appropriate for mass spectrometry analysis, especially electrospray ionization mass spectrometry. Chemical reagents including cyanogens bromide may also be utilized to digest proteins.

Mass spectrometers that may be used to analyze the peptides or proteins include a Matrix-Assisted Laser Desorption/Ionisation Time-of-Flight Mass Spectrometer ("MALDI-TOF") (e.g. from PerSeptive Biosystems, Framingham, Mass.); an Electrospray Ionization ("ESI") ion trap spectrometer, (e.g. from Finnigan MAT, San Jose, Calif.), an ESI quadrupole mass spectrometer (e.g. from Finnigan or Perkin-Elmer Corporation, Foster City, Calif.), a quadrupole/TOF hybrid tandem mass spectrometer, QSTAR XL (Applied Biosystems/MDS Sciex), or a Surface Enhanced Laser Desorption/Ionization (SELDI-TOF) Mass Spectrometer (e.g. from Ciphergen Biosystems Inc.).

### Detection Methods

A variety of methods can be employed for the diagnostic and prognostic evaluation of endometrial disease or endometrial status involving one or more endometrial markers and polynucleotides encoding the markers, and the identification of subj ects with a predisposition to endometrial diseases or that are receptive to *in vitro* fertilization and embryo transfer procedures. Such methods may, for example, utilize endometrial polynucleotide markers, and fragments thereof, and binding agents (e.g. antibodies) against one or more endometrial markers, including peptide fragments. In particular, the polynucleotides and antibodies may be used, for example, for (1) the detection of the presence of endometrial polynucleotide marker mutations, or the detection of either over- or under-expression of endometrial marker mRNA relative to a non-disorder state or different endometrium phase, or the qualitative or quantitative detection of alternatively spliced forms of endometrial polynucleotide marker transcripts which may correlate with certain conditions or susceptibility toward such conditions; and (2) the detection of either an over- or an under-abundance of one or more endometrial markers relative to a non- disorder state or a different endometrium phase or the presence of a modified (e.g., less than full length) endometrial marker which correlates with a disorder state or a progression toward a disorder state, or a particular endometrium phase.

Contemplated is further a method for detecting the phase of an endometrial tissue, in particular a secretory endometrial tissue, comprising producing a profile of levels of one or more endometrial marker associated with a known endometrium phase and/or polynucleotides encoding the markers, and optionally other markers associated with the endometrium phase in cells from a patient, and comparing the profile with a reference to identify a profile for the test cells indicative of the endometrium phase. In an aspect, the endometrial markers are one or more of glutamate receptor subunit zeta 1, macrophage migration inhibitory factor, FRAT1, myosin light chain kinase 2, tropomyosin 1 alpha chain, and fragments thereof.

Contemplated is also a method for detecting an endometrial disease, in particular an endometrial cancer, comprising producing a profile of levels of one or more endometrial marker associated with an endometrial disease and/or polynucleotides encoding the markers, and other markers associated with endometrial disease in cells from a patient, and comparing the profile with a reference to identify a profile for the test cells indicative of disease. In an aspect, the endometrial markers are one or more of chaperonin 10, calgranulin A, calgranulin B, polymeric-immunoglobulin receptor (precursor), phosphatidylethanolamine-binding protein, acidic leucine-rich nuclear phosphoprotein 32 family member A, heat shock 70 kDa protein 6, macrophage migration inhibitory factor, calgizzarin (S100C protein), triosephosphate isomerase, Alpha-1-antitrypsin precursor, creatine kinase, B chain, (B-CK), pyruvate, M1 or M2 isozyme, transgelin (smooth muscle protein 22-alpha), and heterologous nuclear ribonucleoprotein D0.

The methods described herein may be used to evaluate the probability of the presence of malignant or pre-malignant cells, for example, in a group of cells freshly removed from a host. Such methods can be used to detect tumors, quantitate their growth, and help in the diagnosis and prognosis of endometrial disease. The methods can be used to detect the presence of cancer metastasis, as well as confirm the absence or removal of all tumor tissue following surgery, cancer chemotherapy, and/or radiation therapy. They can further be used to monitor cancer chemotherapy and tumor reappearance.

The methods described herein can be adapted for diagnosing and monitoring endometrial tissue status or an endometrial disease by detecting one or more endometrial markers or polynucleotides encoding the markers in biological samples from a subject. These applications require that the amount of markers or polynucleotides quantitated in a sample from a subject being tested be compared to a predetermined standard or cut-off value. The standard may correspond to levels quantitated for another sample or an earlier sample from the subject, or levels quantitated for a control sample. Levels for control samples from healthy subjects, different endometrial tissue phases, or subjects with an endometrial disease may be established by prospective and/or retrospective statistical studies. Healthy subjects who have no clinically evident disease or abnormalities may be selected for statistical studies. Diagnosis may be made by a finding of statistically different levels of detected endometrial markers associated with disease or polynucleotides encoding same, compared to a control sample or previous levels quantitated for the same subject.

The methods described herein may also use multiple markers for an endometrial disease, in particular endometrial cancer. Therefore, the invention contemplates a method for anaylzing a biological sample for the presence ofone or more endometrial markers and polynucleotides encoding the markers, and other markers that are specific indicators of an endometrial disease. The methods described herein may be modified by including reagents to detect the additional markers, or polynucleotides for the markers.

### Nucleic Acid Methods/Assays (not claimed)

As noted herein an endometrial disease or phase may be detected based on the level of endometrial polynucleotide markers in a sample. Techniques for detecting polynucleotides such as polymerase chain reaction (PCR) and hybridization assays are well known in the art.

Probes may be used in hybridization techniques to detect endometrial polynucleotide markers. The technique generally involves contacting and incubating nucleic acids (e.g. recombinant DNA molecules, cloned genes) obtained from a sample from a patient or other cellular source with a probe under conditions favorable for the specific annealing of the probes to complementary sequences in the nucleic acids. After incubation, the non-annealed nucleic acids are removed, and the presence of nucleic acids that have hybridized to the probe if any are detected.

Nucleotide probes for use in the detection of nucleic acid sequences in samples may be constructed using conventional methods known in the art. Suitable probes may be based on nucleic acid sequences encoding at least 5 sequential amino acids from regions of an endometrial polynucleotide marker, preferably they comprise 10-200, more particularly 10-30, 10-40, 20-50, 40-80, 50-150, 80-120 nucleotides in length.

The probes may comprise DNA or DNA mimics (e.g., derivatives and analogues) corresponding to a portion of an organism's genome, or complementary RNA or RNA mimics. Mimics are polymers comprising subunits capable of specific, Watson-Crick-like hybridization with DNA, or of specific hybridization with RNA. The nucleic acids can be modified at the base moiety, at the sugar moiety, or at the phosphate backbone.

DNA can be obtained using standard methods such as polymerase chain reaction (PCR) amplification of genomic DNA or cloned sequences. (See, for example, in Innis et al., eds., 1990, PCR Protocols: A Guide to Methods and Applications, Academic Press Inc., San Diego, Calif.). Computer programs known in the art can be used to design primers with the required specificity and optimal amplification properties, such as Oligo version 5.0 (National Biosciences). Controlled robotic systems may be useful for isolating and amplifying nucleic acids.

A nucleotide probe may be labeled with a detectable substance such as a radioactive label that provides for an adequate signal and has sufficient half-life such as ³²P, ³H, ¹⁴C or the like. Other detectable substances that may be used include antigens that are recognized by a specific labeled antibody, fluorescent compounds, enzymes, antibodies specific for a labeled antigen, and luminescent compounds. An appropriate label may be selected having regard to the rate of hybridization and binding of the probe to the nucleotide to be detected and the amount of nucleotide available for hybridization. Labeled probes may be hybridized to nucleic acids on solid supports such as nitrocellulose filters or nylon membranes as generally described in Sambrook et al, 1989, Molecular Cloning, A Laboratory Manual (2nd ed.). The nucleic acid probes may be used to detect endometrial polynucleotide markers, preferably in human cells. The nucleotide probes may also be useful in the diagnosis of an endometrial disease involving one or more endometrial polynucleotide markers, in monitoring the progression of such disorder, or monitoring a therapeutic treatment.

The detection of endometrial polynucleotide markers may involve the amplification of specific gene sequences using an amplification method such as polymerase chain reaction (PCR), followed by the analysis of the amplified molecules using techniques known to those skilled in the art. Suitable primers can be routinely designed by one of skill in the art.

By way of example, at least two oligonucleotide primers may be employed in a PCR based assay to amplify a portion of a polynculeotide encoding one or more endometrial marker derived from a sample, wherein at least one of the oligonucleotide primers is specific for (i.e. hybridizes to) a polynucleotide encoding the endometrial marker. The amplified cDNA is then separated and detected using techniques well known in the art, such as gel electrophoresis.

In order to maximize hybridization under assay conditions, primers and probes employed in the methods of the invention generally have at least about 60%, preferably at least about 75%, and more preferably at least about 90% identity to a portion of a polynucleotide encoding an endometrial marker; that is, they are at least 10 nucleotides, and preferably at least 20 nucleotides in length. In an aspect the primers and probes are at least about 10-40 nucleotides in length.

Hybridization and amplification techniques described herein may be used to assay qualitative and quantitative aspects of endometrial polynucleotide marker expression. For example, RNA may be isolated from a cell type or tissue known to express an endometrial polynucleotide marker and tested utilizing the hybridization (e.g. standard Northern analyses) or PCR techniques referred to herein.

The primers and probes may be used in the above-described methods *in situ* i.e directly on tissue sections (fixed and/or frozen) of patient tissue obtained from biopsies or resections.

In an aspect of the disclosure, a method is provided employing reverse transcriptase-polymerase chain reaction (RT-PCR), in which PCR is applied in combination with reverse transcription. Generally, RNA is extracted from a sample tissue using standard techniques (for example, guanidine isothiocyanate extraction as described by Chomcynski and Sacchi, Anal. Biochem. 162:156-159,1987) and is reverse transcribed to produce cDNA. The cDNA is used as a template for a polymerase chain reaction. The cDNA is hybridized to a set of primers, at least one of which is specifically designed against an endometrial marker sequence. Once the pimer and template have annealed a DNA polymerase is employed to extend from the primer, to synthesize a copy of the template. The DNA strands are denatured, and the procedure is repeated many times until sufficient DNA is generated to allow visualization by ethidium bromide staining and agarose gel electrophoresis.

Amplification may be performed on samples obtained from a subject with a suspected endometrial disease and an individual who is not afflicted with an endometrial disease. The reaction may be performed on several dilutions of cDNA spanning at least two orders of magnitude. A statistically significant difference in expression in several dilutions of the subject sample as compared to the same dilutions ofthe non-disease sample may be considered positive for the presence of an endometrial disease.

Disclosed are also methods for determining the presence or absence of an endometrial disease in a subject comprising (a) contacting a sample obtained from the subject with oligonucleotides that hybridize to endometrial polynucleotide markers; and (b) detecting in the sample a level of nucleic acids that hybridize to the polynucleotides relative to a predetermined cut-off value, and therefrom determining the presence or absence of an endometrial disease in the subject. In an aspect, the endometrial disease is cancer and the endometrial markers are one or more of chaperon in 10, calgranulin A, calgranulin B, polymeric-immunoglobulin receptor (precursor), phosphatidylethanolamine-binding protein, acidic leucine-rich nuclear phosphoprotein 32 family member A, heat shock 70 kDa protein 6, macrophage migration inhibitory factor, calgizzarin (S100C protein), triosephosphate isomerase, alpha-1-antitrypsin precursor, creatine kinase, B chain, (B-CK), pyruvate, M1 or M2 isozyme, transgelin (smooth muscle protein 22-alpha), and heterologous nuclear ribonucleoprotein D0.

Disclosed are further methods for determining uterine receptivity ofa subject to *in vitro* fertilization comprising (a) contacting a sample obtained from the subject with oligonucleotides that hybridize to endometrial polynucleotide markers associated with an endometrial tissue phase (e.g. secretory phase); and (b) detecting in the sample a level of nucleic acids that hybridize to the polynucleotides relative to a predetermined cut-off value, wherein the presence or absence of the endometrial marker polynucleotides as compared to non-receptive controls indicates uterine receptivity. In an aspect, the endometrial markers are one or more of glutamate receptor subunit zeta 1, macrophage migration inhibitory factor, FRAT1, myosin light chain kinase 2, tropomyosin 1 alpha chain, and fragments thereof

Endometrial marker mRNA may be detected by (a) isolating mRNA from a sample and combining the mRNA with reagents to convert it to cDNA; (b) treating the converted cDNA with amplification reaction reagents and nucleic acid primers that hybridize to one or more endometrial marker polynucleotides, to produce amplification products; (d) analyzing the amplification products to detect amounts of mRNA encoding endometrial polynucleotide markers; and (e) comparing the amount ofmRNA to an amount detected against a panel of expected values for normal and malignant tissue derived using similar nucleic acid primers.

Endometrial cancer marker-positive samples or alternatively higher levels in patients compared to a control (e.g. non-cancerous tissue) may be indicative of late stage disease, and/or that the patient is not responsive to chemotherapy. Alternatively, negative samples or lower levels compared to a control (e.g. non-cancerous tissue or negative samples) may also be indicative of progressive disease and shorter overall survival.

Determining the presence or absence of endometrial cancer in a subject may comprise (a) contacting a sample obtained from the subject with oligonucleotides that hybridize to one or more endometrial cancer polynucleotide markers; and (b) detecting in the sample levels of nucleic acids that hybridize to the polynucleotides relative to a predetermined cut-off value, and therefrom determining the presence or absence of endometrial cancer in the subject. In an embodiment, the endometrial cancer polynucleotide markers encode one or more polypeptides listed in Table 1. In particular, the endometrial markers are one or more of chaperonin 10, calgranulin A, calgranulin B, polymeric-immunoglobulin receptor (precursor), phosphatidylethanolamine-binding protein, acidic leucine-rich nuclear phosphoprotein 32 family member A, heat shock 70 kDa protein 6, macrophage migration inhibitory factor, calgizzarin (S100C protein), triosephosphate isomerase, alpha-1-antitrypsin precursor, creatine kinase, B chain, (B-CK), pyruvate, M1 or M2 isozyme, transgelin (smooth muscle protein 22-alpha), and heterologous nuclear ribonucleoprotein D0, and fragments thereof.

Chaperonin 10 mRNA may be detected by (a) isolating mRNA from a sample and combining the mRNA with reagents to convert it to cDNA; (b) treating the converted cDNA with amplification reaction reagents and nucleic acid primers that hybridize to a polynucleotide encoding chaperonin 10, to produce amplification products; (d) analyzing the amplification products to detect an amount of mRNA encoding chaperonin 10; and (e) comparing the amount ofmRNA to an amount detected against a panel of expected values for normal and malignant tissue derived using similar nucleic acid primers.

Endometrial cancer marker-positive samples or alternatively higher levels, in particular significantly higher levels of chaperonin 10 polynucleotides in patients compared to a control (e.g. normal or benign) are indicative of endometrial cancer. Negative samples or lower levels compared to a control (e.g. normal or benign) may also be indicative of progressive disease and poor overall survival.

Oligonucleotides or longer fragments derived from an endometrial cancer polynucleotide marker may be used as targets in a microarray. The microarray can be used to simultaneously monitor the expression levels of large numbers of genes and to identify genetic variants, mutations, and polymorphisms. The information from the microarray may be used to determine gene function, to understand the genetic basis of a disorder, to diagnose a disorder, and to develop and monitor the activities of therapeutic agents.

The preparation, use, and analysis of micro arrays are well known to a person skilled in the art. (See, for example, Brennan, T. M. et al. (1995) U.S. Pat. No. 5,474,796; Schena, et al. (1996) Proc. Natl. Acad. Sci. 93:10614-10619; Baldeschweiler et al. (1995), PCT Application WO95/251116; Shalon, D. et al. (1995) PCT application WO95/35505; Heller, R. A. et al. (1997) Proc. Natl. Acad. Sci. 94:2150-2155; and Heller, M.J.et al. (1997) U.S. Pat. No. 5,605,662.)

An array may comprise one or more endometrial polynucleotide markers (in particular the markers listed in Table 1, 4, 5, or 6) and optionally other markers. The array can be used to assay expression of endometrial polynucleotide markers in the array. The invention allows the quantitation of expression of one or more endometrial polynucleotide markers.

Microarrays typically contain at separate sites nanomolar quantities of individual genes, cDNAs, or ESTs on a substrate (e.g.nitrocellulose or silicon plate), or photolithographically prepared glass substrate. The arrays are hybridized to cDNA probes using conventional techniques with gene-specific primer mixes. The target polynucleotides to be analyzed are isolated, amplified and labeled, typically with fluorescent labels, radiolabels or phosphorous label probes. After hybridization is completed, the array is inserted into the scanner, where patterns of hybridization are detected. Data are collected as light emitted from the labels incorporated into the target, which becomes bound to the probe array. Probes that completely match the target generally produce stronger signals than those that have mismatches. The sequence and position of each probe on the array are known, and thus by complementarity, the identity of the target nucleic acid applied to the probe array can be determined.

Microarrays are prepared by selecting polynucleotide probes and immobilizing them to a solid support or surface. The probes may comprise DNA sequences, RNA sequences, copolymer sequences of DNA and RNA, DNA and/or RNA analogues, or combinations thereof. The probe sequences may be full or partial fragments of genomic DNA, or they may be synthetic oligonucleotide sequences synthesized either enzymatically *in vivo,* enzymatically *in vitro* (e.g., by PCR), or non-enzymatically *in vitro.*

The probe or probes used can be immobilized to a solid support or surface which may be either porous or non-porous. For example, the probes can be attached to a nitrocellulose or nylon membrane or filter covalently at either the 3' or the 5' end of the polynucleotide probe. The solid support may be a glass or plastic surface. In an aspect ofthe invention, hybridization levels are measured to microarrays of probes consisting of a solid support on the surface of which are immobilized a population of polynucleotides, such as a population of DNA or DNA mimics, or, alternatively, a population of RNA or RNA mimics. A solid support may be a nonporous or, optionally, a porous material such as a gel.

A microarray may be provided comprising a support or surface with an ordered array of hybridization sites or "probes" each representing one of the markers described herein. The microarrays can be addressable arrays, and in particular positionally addressable arrays. Each probe ofthe array is typically located at a known, predetermined position on the solid support such that the identity of each probe can be determined from its position in the array. In preferred embodiments, each probe is covalently attached to the solid support at a single site.

Microarrays used are preferably (a) reproducible, allowing multiple copies of a given array to be produced and easily compared with each other, (b) made from materials that are stable under hybridization conditions; (c) small, (e.g., between 1 cm² and 25 cm², between 12 cm² and 13 cm², or 3 cm²; and (d) comprise a unique set of binding sites that will specifically hybridize to the product of a single gene in a cell (e.g., to a specific mRNA, or to a specific cDNA derived therefrom). However, it will be appreciated that larger arrays may be used particularly in screening arrays, and other related or similar sequences will cross hybridize to a given binding site.

The microarray may be an array in which each position represents one of the markers described herein. Each position of the array can comprise a DNA or DNA analogue based on genomic DNA to which a particular RNA or cDNA transcribed from a genetic marker can specifically hybridize. A DNA or DNA analogue can be a synthetic oligomer or a gene fragment. In an embodiment, probes representing each of the endometrial markers and endometrial polynucleotide markers is present on the array. In a preferred embodiment, the array comprises at least 5 of the endometrial polynucleotide markers.

Probes for the microarray can be synthesized using N-phosphonate or phosphoramidite chemistries (Froehler et al., 1986, Nucleic Acid Res. 14:5399-5407; McBride et al., 1983, Tetrahedron Lett. 24:246-248). Synthetic sequences are typically between about 10 and about 500 bases, 20-100 bases, or 40-70 bases in length. Synthetic nucleic acid probes can include non-natural bases, such as, without limitation, inosine. Nucleic acid analogues such as peptide nucleic acid may be used as binding sites for hybridization. (see, e.g., Egholm et al., 1993, Nature 363:566-568; U.S. Pat. No. 5,539,083).

Probes can be selected using an algorithm that takes into account binding energies, base composition, sequence complexity, cross-hybridization binding energies, and secondary structure (see Friend et al., International Patent Publication WO 011/05935, published Jan. 25,2001).

Positive control probes, (e.g., probes known to be complementary and hybridizae to sequences in the target polynucleotides), and negative control probes, (e.g., probes known to not be complementary and hybridize to sequences in the target polynucleotides) are typically included on the array. Positive controls can be synthesized along the perimeter of the array or synthesized in diagonal stripes across the array. A reverse complement for each probe can be next to the position of the probe to serve as a negative control.

The probes can be attached to a solid support or surface, which may be made from glass, plastic (e.g., polypropylene, nylon), polyacrylamide, nitrocellulose, gel, or other porous or nonporous material. The probes can be printed on surfaces such as glass plates (see Schena et al.,1995, Science 270:467-470). This method may be particularly useful for preparing microarrays of cDNA (See also, DeRisi et al., 1996, Nature Genetics 14:457-460; Shalon et al., 1996, Genome Res. 6:639-645; and Schena et al., 1995, Proc. Natl. Acad. Sci. U.S.A. 93:10539-11286).

High-density oligonucleotide arrays containing thousands of oligonucleotides complementary to defined sequences, at defined locations on a surface can be produced using photolithographic techniques for synthesis in situ (see, Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat Nos. 5,578,832; 5,556,752; and 5,510,270) or other methods for rapid synthesis and deposition of defined oligonucleotides (Blanchard et al., Biosensors & Bioelectronics 11:687-690). Using these methods oligonucleotides (e.g., 60-mers) of known sequence are synthesized directly on a surface such as a derivatized glass slide. The array produced may be redundant, with several oligonucleotide molecules per RNA.

Microarrays can be made by other methods including masking (Maskos and Southern, 1992, Nuc. Acids. Res. 20:1679-1684). In an embodiment, microarrays of the present invention are preduced by synthesizing polynucleotide probes on a support wherein the nucleotide probes are attached to the support covalently at either the 3' or the 5' end of the polynucleotide.

The microarrays may comprise a disclosed marker set. In one embodiment, the invention provides a microarray for distinguishing endometrial disease samples comprising a positionally-addressable array of polynucleotide probes bound to a support, the polynucleotide probes comprising a plurality of polynucleotide probes of different nucleotide sequences, each of the different nucleotide sequences comprising a sequence complementary and hybridizable to a plurality of genes, the plurality consisting of at least 2, 3, 4, 5, 6, 7, 8, 9, 10, 11, 12, 13, 14, or 15 of the genes corresponding to the markers listed in Table 1, 4, 5, or 6. An aspect of the invention provides microarrays comprising at least 5, 10, or 15 of the polynucleotides encoding the markers listed in Table 1, 4, 5, or 6.

Diclosed are gene marker sets that distinguish endometrium phase or endometrial disease and uses therefor. Further disclosed is a method for classifying an endometrium phase or disease comprising detecting a difference in the expression of a first plurality of genes relative to a control, the first plurality of genes consisting of at least 5 of the genes encoding the markers listed in Table 1, 4, 5, or 6. In specific aspects, the plurality of genes consists of at least 10 or 15 of the genes encoding the markers listed in Table 1, 4, 5, or 6. In another specific aspect, the control comprises nucleic acids derived from a pool of samples from individual control patients.

A method for classifying an endometrium phase or endometrial disease by calculating the similarity between the expression of at least 5 polynucleotides encoding markers listed in Table 1, 4, 5, or 6 in a sample to the expression of the same markers in a control pool, may comprise the steps of:
(a) labeling nucleic acids derived from a sample, with a first fluorophore to obtain a first pool of fluorophore-labeled nucleic acids;
(b) labeling with a second fluorophore a first pool of nucleic acids derived from two or more endometrial disease samples, and a second pool of nucleic acids derived from two or more control samples;
(c) contacting the first fluorophore-labeled nucleic acid and the first pool ofsecond fluorophore-labeled nucleic acid with a first microarray under conditions such that hybridization can occur, and contacting the first fluorophore-labeled nucleic acid and the second pool of second fluorophore-labeled nucleic acid with a second microarray under conditions such that hybridization can occur, detecting at each of a plurality of discrete loci on the first microarray a first flourescent emission signal from the first fluorophore-labeled nucleic acid and a second fluorescent emission signal from the first pool of second fluorophore-labeled genetic matter that is bound to the first microarray and detecting at each of the marker loci on the second microarray the first fluorescent emission signal from the first fluorophore-labeled nucleic acid and a third fluorescent emission signal from the second pool of second fluorophore-labeled nucleic acid;
(d) determining the similarity of the sample to patient and contol pools by comparing the first fluorescence emission signals and the second fluorescence emission signals, and the first emission signals and the third fluorescence emission signals; and
(e) classifying the sample as endometrial disease where the first fluorescence emission signals are more similar to the second fluorescence emission signals than to the third fluorescent emission signals, and classifying the sample as non-endometrial disease where the first fluorescence emission signals are more similar to the third fluorescence emission signals than to the second fluorescent emission signals, wherein the first microarray and the second microarray are similar to each other, exact replicas of each other, or are identical, and wherein the similarity is defined by a statistical method such that the cell sample and control are similar where the p value of the similarity is less than 0.01.

The array can be used to monitor the time course of expression of one or more endometrial polynucleotide markers in the array. This can occur in various biological contexts such as tumor progression.

The array is also useful for ascertaining differential expression patterns of endometrial polynucleotide markers, and optionally other markers, in normal and abnormal cells. This may provide a battery of nucleic acids that could serve as molecular targets for diagnosis or therapeutic intervention.

### Protein Methods

Binding agents may be used for a variety of diagnostic and assay applications. There are a variety of assay formats known to the skilled artisan for using a binding agent to detect a target molecule in a sample. (For example, see Harlow and Lane, Antibodies: A Laboratory Manual, Cold Spring Harbor Laboratory, 1988). In general, the presence or absence of an endometrial disease (e.g. cancer) or an endometrium phase in a subject may be determined by (a) contacting a sample from the subject with a binding agent; (b) detecting in the sample a level of polypeptide that binds to the binding agent; and (c) comparing the level of polypeptide with a predetermined standard or cut-off value.

The binding agent may be an antibody. Antibodies specifically reactive with one or more endometrial marker, or derivatives, such as enzyme conjugates or labeled derivatives, may be used to detect one or more endometrial marker in various samples (e.g. biological materials). They may be used as diagnostic or prognostic reagents and they may be used to detect abnormalities in the level of expression of one or more endometrial marker, or abnormalities in the structure, and/or temporal, tissue, cellular, or subcellular location of one or more endometrial marker. Antibodies may also be used to screen potentially therapeutic compounds *in vitro* to determine their effects on disorders (e.g. endometrial cancer) involving one or more endometrial markers, and other conditions. *In vitro* immunoassays may also be used to assess or monitor the efficacy of particular therapies.

Endometrial cancer may be monitored or diagnosed in a subject by quantitating one or more endometrial markers in a biological sample from the subject comprising reacting the sample with antibodies specific for one or more endometrial markers, which are directly or indirectly labeled with detectable substances and detecting the detectable substances. In a particular embodiment, endometrial markers are quantitated or measured.

A method for detecting an endometrial cancer may comprise:
(a) obtaining a sample suspected of containing one or more endometrial markers associated with an endometrial disease;
(b) contacting said sample with antibodies that specifically bind to the endometrial markers under conditions effective to bind the antibodies and form complexes;
(c) measuring the amount of endometrial markers present in the sample by quantitating the amount of the complexes; and
(d) comparing the amount of endometrial markers present in the samples with the amount of endometrial markers in a control, wherein a change or significant difference in the amount of endometrial markers in the sample compared with the amount in the control is indicative of an endometrial disease.

Contemplated is a method for monitoring the progression of an endometrial disease (e.g. cancer) in an individual, comprising:
(a) contacting antibodies which bind to one or more endometrial markers with a sample from the individual so as to form complexes comprising the antibodies and one or more endometrial markers in the sample;
(b) determining or detecting the presence or amount of complex formation in the sample;
(c) repeating steps (a) and (b) at a point later in time; and
(d) comparing the result of step (b) with the result of step (c), wherein a difference in the amount of complex formation is indicative of disease, disease stage, and/or progression of the disease in said individual.

The amount of complexes may also be compared to a value representative of the amount of the complexes from an individual not at risk of, or afflicted with, an endometrial disease at different stages. A significant difference in complex formation may be indicative of advanced disease e.g. advanced endometrial cancer, or an unfavourable prognosis.

The endometrial markers may be one or more of chaperonin 10, calgranulin A, calgranulin B, polymeric-immunoglobulin receptor (precursor), phosphatidylethanolamine-binding protein, acidic leucine-rich nuclear phosphoprotein 32 family member A, heat shock 70 kDa protein 6, macrophage migration inhibitory factor, calgizzarin (S100C protein), triosephosphate isomerase, alpha-1-antitrypsin precursor, creatine kinase, B chain, (B-CK), pyruvate, M1 or M2 isozyme, transgelin (smooth muscle protein 22-alpha), and heterologous nuclear ribonucleoprotein D0, or fragments thereof.

Chaperonin 10 may be detected in samples and higher levels, in particular significantly higher levels compared to a control (normal or benign) is indicative of endometrial cancer.

In characterizing endometrium phase the endometrial markers may be one or more of glutamate receptor subunit zeta 1, macrophage migration inhibitory factor, FRAT1, myosin light chain kinase 2, tropomyosin I alpha chain, and fragments thereof.

Methods for determining uterine receptivity of a subject to *in vitro* fertilization may comprise (a) contacting a sample obtained from the subject with antibodies that bind to one or more endometrial marker associated with a certain endometrium phase (e.g. secretory phase); and (b) detecting in the sample a level of endometrial marker relative to a predetermined cut-off-value, wherein the presence or absence of the endometrial marker as compared to non-receptive controls indicates uterine receptivity. In a particular embodiment, the marker is one or more of glutamate receptor subunit zeta 1, macrophage migration inhibitory factor, FRAT1, myosin light chain kinase 2, tropomyosin 1 alpha chain, and fragments thereof, more particularly glutamate receptor subunit zeta 1 or a fragment thereof, and/or macrophage migration inhibitory factor.

Antibodies may be used in any known immunoassays that rely on the binding interaction between antigenic determinants of one or more endometrial marker and the antibodies. Immunoassay procedures for *in vitro* detection of antigens in fluid samples are also well known in the art. [See for example, Paterson et al., Int. J. Can. 37:659 (1986) and Burchell et al., Int. J. Can. 34:763 (1984) for a general description of immunoassay procedures]. Qualitative and/or quantitative determinations ofone or more endometrial marker in a sample may be accomplished by competitive or non-competitive immunoassay procedures in either a direct or indirect format. Detection of one or more endometrial marker using antibodies can be done utilizing immunoassays which are run in either the forward, reverse or simultaneous modes. Examples of immunoassays are radioimmunoassays (RIA), enzyme immunoassays (e.g. ELISA), immunofluorescence, immunoprecipitation, latex agglutination, hemagglutination, histochemical tests, and sandwich (immunometric) assays. These terms are well understood by those skilled in the art. A person skilled in the art will know, or can readily discern, other immunoassay formats without undue experimentation.

An immunoassay for detecting one or more endometrial markers in a biological sample may comprise contacting binding agents that specifically bind to endometrial markers in the sample under conditions that allow the formation of first complexes comprising a binding agent and endometrial markers and determining the presence or amount of the complexes as a measure of the amount of endometrial markers contained in the sample. In a particular embodiment, the binding agents are labeled differently or are capable of binding to different labels.

Antibodies may be used to detect and quantify one or more endometrial markers in a sample in order to diagnose and treat pathological states. In particular, the antibodies may be used in immunohistochemical analyses, for example, at the cellular and sub-subcellular level, to detect one or more endometrial markers, to localize them to particular endometrial cells and tissues (e.g. tumor cells and tissues), and to specific subcellular locations, and to quantitate the level of expression.

Immunohistochemical methods for the detection of antigens in tissue samples are well known in the art. For example, immunohistochemical methods are described in Taylor, Arch. Pathol. Lab. Med.102:112 (1978). Briefly, in the context of the present invention, a tissue sample obtained from a subject suspected of having an endometrial-related problem is contacted with antibodies, preferably monoclonal antibodies recognizing one or more endometrial markers. The site at which the antibodies are bound is determined by selective staining of the sample by standard immunohistochemical procedures. The same procedure may be repeated on the same sample using other antibodies that recognize one or more endometrial markers. Alternatively, a sample may be contacted with antibodies against one or more endometrial markers simultaneously, provided that the antibodies are labeled differently or are able to bind to a different label. The tissue sample may be normal endometrial tissue, a cancer tissue or a benign tissue.

An antibody microarray in which binding sites comprise immobilized, preferably monoclonal, antibodies specific to a substantial fraction of marker-derived endometrial markers of interest can be utilized in the present invention. Antibody arrays can be prepared using methods known in the art [(see for example, Zhu et al., Science 293:2101 (2001) and reference 20].

Antibodies specific for one or more endometrial marker may be labelled with a detectable substance and localised in biological samples based upon the presence of the detectable substance. Examples of detectable substances include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), luminescent labels such as luminol; enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or colorimetric methods), predetermined polypeptide epitopes recognized by a secondary reporter (e.g., leucine zipper pair sequences, binding sites for secondary antibodies, metal binding domains, epitope tags). In some embodiments, labels are attached via spacer arms of various lengths to reduce potential steric hindrance. Antibodies may also be coupled to electron dense substances, such as ferritin or colloidal gold, which are readily visualised by electron microscopy.

One of the ways an antibody can be detectably labeled is to link it directly to an enzyme. The enzyme when later exposed to its substrate will produce a product that can be detected. Examples of detectable substances that are enzymes are horseradish peroxidase, beta-galactosidase, luciferase, alkaline phosphatase, acetylcholinesterase, malate dehydrogenase, ribonuclease, urease, catalase, glucose-6-phosphate, staphylococcal nuclease, delta-5-steriod isomerase, yeast alcohol dehydrogenase, alpha-glycerophosphate, triose phosphate isomerase, asparaginase, glucose oxidase, and acetylcholine esterase.

For increased sensitivity in an immunoassay system a fluorescence-emitting metal atom such as Eu (europium) and other lanthanides can be used. These can be attached to the desired molecule by means of metal-chelating groups such as DTPA or EDTA.

A bioluminescent compound may also be used as a detectable substance. Bioluminescence is a type of chemiluminescence found in biological systems where a catalytic protein increases the efficiency of the chemiluminescent reaction. The presence of a bioluminescent molecule is determined by detecting the presence of luminescence. Examples of bioluminescent detectable substances are luciferin, luciferase and aequorin.

Indirect methods may also be employed in which the primary antigen-antibody reaction is amplified by the introduction of a second antibody, having specificity for the antibody reactive against one or more endometrial markers. By way of example, if the antibody having specificity against one or more endometrial markers is a rabbit IgG antibody, the second antibody may be goat anti-rabbit gamma-globulin labelled with a detectable substance as described herein.

Methods for conjugating or labelling the antibodies discussed above may be readily accomplished by one of ordinary skill in the art. (See for example Inman, Methods In Enzymology, Vol. 34, Affinity Techniques, Enzyme Purification: Part B, Jakoby and Wichek (eds.), Academic Press, New York, p. 30, 974; and Wilchek and Bayer, "The Avidin-Biotin Complex in Bioanalytical Applications,"Anal. Biochem. 171:1-32, 1988 re methods for conjugating or labelling the antibodies with enzyme or ligand binding partner).

Cytochemical techniques known in the art for localizing antigens using light and electron microscopy may be used to detect one or more endometrial markers. Generally, antibodies may be labeled with detectable substances and one or more endometrial markers may be localised in tissues and cells based upon the presence of the detectable substances.

In the context of the methods of the invention, the sample, binding agents (e.g. antibodies specific for one or more endometrial markers), or one or more endometrial markers may be immobilized on a carrier or support. Examples of suitable carriers or supports are agarose, cellulose, nitrocellulose, dextran, Sephadex, Sepharose, liposomes, carboxymethyl cellulose, polyacrylamides, polystyrene, gabbros, filter paper, magnetite, ion-exchange resin, plastic film, plastic tube, glass, polyamine-methyl vinyl-ether-maleic acidcopolymer, amino acid copolymer, ethylene-maleic acid copolymer, nylon, silk, etc. The support material may have any possible configuration including spherical (e.g. bead), cylindrical (e.g. inside surface of a test tube or well, or the external surface of a rod), or flat (e.g. sheet, test strip). Thus, the carrier may be in the shape of, for example, a tube, test plate, well, beads, disc, sphere, etc. The immobilized antibody may be prepared by reacting the material with a suitable insoluble carrier using known chemical or physical methods, for example, cyanogen bromide coupling. An antibody may be indirectly immobilized using a second antibody specific for the antibody. For example, mouse antibody specific for an endometrial marker may be immobilized using sheep anti-mouse IgG Fc fragment specific antibody coated on the carrier or support.

Where a radioactive label is used as a detectable substance, one or more endometrial marker may be localized by radioautography. The results of radioautography may be quantitated by determining the density of particles in the radioautographs by various optical methods, or by counting the grains.

Time-resolved fluorometry may be used to detect a signal. For example, the method described in Christopoulos TK and Diamandis EP Anal Chem 1992:64:342-346 may be used with a conventional time-resolved fluorometer.

One or more endometrial marker antibodies may be directly or indirectly labelled with enzymes, substrates for the enzymes are added wherein the substrates are selected so that the substrates, or a reaction product of an enzyme and substrate, form fluorescent complexes with a lanthanide metal (e.g. europium, terbium, samarium, and dysprosium, preferably europium and terbium). A lanthanide metal is added and one or more endometrial cancer markers are quantitated in the sample by measuring fluorescence of the fluorescent complexes. Enzymes are selected based on the ability of a substrate of the enzyme, or a reaction product of the enzyme and substrate, to complex with lanthanide metals such as europium and terbium. Suitable enzymes and substrates that provide fluorescent complexes are described in U.S. Patent No. 5,3112,922 to Diamandis. Examples of suitable enzymes include alkaline phosphatase and β-galactosidase. Preferably, the enzyme is alkaline phosphatase.

Examples of enzymes and substrates for enzymes that provide such fluorescent complexes are described in U.S. Patent No. 5,312,922 to Diamandis. By way of example, when the antibody is directly or indirectly labelled with alkaline phosphatase the substrate employed in the method maybe 4-methylumbelliferyl phosphate, 5-fluorosalicyl phosphate, or diflunisal phosphate. The fluorescence intensity of the complexes is typically measured using a time-resolved fluorometer e.g. a GyberFluor 615 Imunoanalyzer (Nordion International, Kanata, Ontario).

One or more endometrial marker antibodies may also be indirectly labelled with an enzyme. For example, the antibodies may be conjugated to one partner of a ligand binding pair, and the enzyme may be coupled to the other partner of the ligand binding pair. Representative examples include avidin-biotin, and riboflavin-riboflavin binding protein. In an embodiment, the antibodies are biotinylated, and the enzyme is coupled to streptavidin. In another embodiment, an antibody specific for endometrial marker antibody is labeled with an enzyme.

Endometrial markers in a sample may be measured by immunoassay. It will be evident to a skilled artisan that a variety of immunoassay methods can be used to measure one or more endometrial markers. In general, an immunoassay method may be competitive or noncompetitive. Competitive methods typically employ an immobilized or immobilizable antibody to one or more endometrial marker and a labeled form of one or more endometrial marker. Sample endometrial markers and labeled endometrial markers compete for binding to antibodies to endometrial markers. After separation of the resulting labeled endometrial markers that have become bound to antibodies (bound fraction) from that which has remained unbound (unbound fraction), the amount of the label in either bound or unbound fraction is measured and may be correlated with the amount of endometrial markers in the test sample in any conventional manner, e.g., by comparison to a standard curve.

In an aspect, a non-competitive method is used for the determination of one or more endometrial markers, with the most common method being the "sandwich" method. In this assay, two antibodies to endometrial markers are employed. One of the antibodies to endometrial markers is directly or indirectly labeled (sometimes referred to as the "detection antibody") and the other is immobilized or immobilizable (sometimes referred to as the "capture antibody"). The capture and detection antibodies can be contacted simultaneously or sequentially with the test sample. Sequential methods can be accomplished by incubating the capture antibody with the sample, and adding the detection antibody at a predetermined time thereafter (sometimes referred to as the "forward" method); or the detection antibody can be incubated with the sample first and then the capture antibody added (sometimes referred to as the "reverse" method). After the necessary incubation(s) have occurred, to complete the assay, the capture antibody is separated from the liquid test mixture, and the label is measured in at least a portion of the separated capture antibody phase or the remainder of the liquid test mixture. Generally it is measured in the capture antibody phase since it comprises endometrial cancer markers bound by ("sandwiched" between) the capture and detection antibodies. In an embodiment, the label may be measured without separating the capture antibodies and liquid test mixture.

In a typical two-site immunometric assay for endometrial markers, one or both of the capture and detection antibodies are polyclonal antibodies or one or both of the capture and detection antibodies are monoclonal antibodies (i.e. polyclonal/polyclonal, monoclonal/monoclonal, or monoclonal/polyclonal). The label used in the detection antibody can be selected from any of those known conventionally in the art. The label may be an enzyme or a chemiluminescent moiety, but it can also be a radioactive isotope, a fluorophor, a detectable ligand (e.g., detectable by a secondary binding by a labeled binding partner for the ligand), and the like. In a particular aspect, the antibody is labelled with an enzyme which is detected by adding a substrate that is selected so that a reaction product of the enzyme and substrate forms fluorescent complexes. The capture antibody may be selected so that it provides a means for being separated from the remainder of the test mixture. Accordingly, the capture antibody can be introduced to the assay in an already immobilized or insoluble form, or can be in an immobilizable form, that is, a form which enables immobilization to be accomplished subsequent to introduction of the capture antibody to the assay. An immobilized capture antibody may comprise an antibody covalently or noncovalently attached to a solid phase such as a magnetic particle, a latex particle, a microtiter plate well, a bead, a cuvette, or other reaction vessel. An example of an immobilizable capture antibody is antibody which has been chemically modified with a ligand moiety, e.g., a hapten, biotin, or the like, and which can be subsequently immobilized by contact with an immobilized form of a binding partner for the ligand, e.g., an antibody, avidin, or the like. In an embodiment, the capture antibody may be immobilized using a species specific antibody for the capture antibody that is bound to the solid phase.

The above-described immunoassay methods and formats are intended to be exemplary and are not limiting.

### Computer Systems

Analytic methods contemplated herein can be implemented by use of computer systems and methods described below and known in the art. A computer readable media may be provided comprising one or more endometrial markers, and/or polynucleotides encoding one or more endometrial markers, and optionally other markers (e.g. markers of endometrial cancer). "Computer readable media" refers to any medium that can be read and accessed directly by a computer, including but not limited to magnetic storage media, such as floppy discs, hard disc storage medium, and magnetic tape; optical storage media such as CD-ROM; electrical storage media such as RAM and ROM; and hybrids of these categories such as magnetic/optical storage media. Thus, the invention contemplates computer readable medium having recorded thereon markers identified for patients and controls.

"Recorded" refers to a process for storing information on computer readable medium. The skilled artisan can readily adopt any of the presently known methods for recording information on computer readable medium to generate manufactures comprising information on one or more endometrial markers, and optionally other markers.

A variety of data processor programs and formats can be used to store information on one or more endometrial markers, and/or polynucleotides encoding one or more endometrial markers, and other markers on computer readable medium. For example, the information can be represented in a word processing text file, formatted in commercially-available software such as WordPerfect and Microsoft Word, or represented in the form of an ASCII file, stored in a database application, such as DB2, Sybase, Oracle, or the like. Any number of dataprocessor structuring formats (e.g., text file or database) may be adapted in order to obtain computer readable medium having recorded thereon the marker information.

By providing, the marker information in computer readable form, one can routinely access the information for a variety of purposes. For example, one skilled in the art can use the information in computer readable form to compare marker information obtained during or following therapy with the information stored within the data storage means.

A medium may be provided for holding instructions for performing a method for determining uterine endometrial receptivity of a patient, or whether a patient has an endometrial disease (e.g. endometrial cancer) or a pre-disposition to an endometrial disease (e.g. cancer), comprising determining the presence or absence of one or more endometrial markers, and/or polynucleotides encoding one or more endometrial markers, and optionally other markers, and based on the presence or absence of the one or more endometrial markers, and/or polynucleotides encoding one or more endometrial markers, and optionally other markers, determining uterine endometrial receptivity, endometrial disease (e.g. cancer) or a pre-disposition to an endometrial disease (e.g. cancer), and optionally recommending a procedure or treatment.

An electronic system and/or a network may be used in a a method for determining uterine endometrial receptivity of a patient, whether a subject has an endometrial disease (e.g. cancer) or a pre-disposition to an endometrial disease (e.g. cancer), the method comprising determining the presence or absence of one or more endometrial markers, and/or polynucleotides encoding one or more endometrial markers, and optionally other markers (e.g. cancer markers), and based on the presence or absence of the one or more endometrial markers, and/or polynucleotides encoding one or more endometrial markers, and optionally other markers, determining the uterine endometrial receptivity of the patient, whether the subject has an endometrial disease (e.g. cancer) or a pre-disposition to an endometrial disease (e.g. cancer), and optionally recommending a procedure or treatment.

A network may be used in a method for determining whether a subject is receptive to *in vitro* fertilization, has an endometrial disease (e.g. cancer) or a pre-disposition to an endometrial disease (e.g. cancer the method comprising: (a) receiving phenotypic information on the subject and information on one or more endometrial markers, and/or polynucleotides encoding one or more endometrial markers, and optionally other markers associated with samples from the subject; (b) acquiring information from the network corresponding to the one or more endometrial markers, and/or polynucleotides encoding one or more endometrial markers, and optionally other markers; and (c) based on the phenotypic information and information on the one or more endometrial markers, and/or polynucleotides encoding one or more endometrial markers, and optionally other markers, determining whether the subject is receptive to *in vitro* fertilization, has an endometrial disease (e.g. cancer) or a pre-disposition to an endometrial disease (e.g. cancer); and (d) optionally recommending a procedure or treatment.

A system may be used for identifying selected records that identify a diseased endometrial cell or tissue (e.g. cancer cell or tissue) or an endometrium phase. A system generally comprises a digital computer; a database server coupled to the computer; a database coupled to the database server having data stored therein, the data comprising records of data comprising one or more endometrial markers, and/or polynucleotides encoding one or more endometrial markers, and optionally other endometrial markers, and a code mechanism for applying queries based upon a desired selection criteria to the data file in the database to produce reports of records which match the desired selection criteria.

Diclosed is also a method for detecting endometrial cancer tissue or cells using a computer having a processor, memory, display, and input/output devices, the method comprising the steps of
(a) creating records of one or more endometrial cancer markers, and/or polynucleotides encoding one or more endometrial cancer markers, and optionally other markers of cancer identified in a sample suspected of containing endometrial cancer cells or tissue;
(b) providing a database comprising records of data comprising one or more endometrial cancer markers, and/or polynucleotides encoding one or more endometrial cancer markers, and optionally other markers of cancer; and
(c) using a code mechanism for applying queries based upon a desired selection criteria to the data file in the database to produce reports of records of step (a) which provide a match of the desired selection criteria of the database of step (b) the presence of a match being a positive indication that the markers of step (a) have been isolated from cells or tissue that are endometrial cancer cells or tissue.

A method for determining whether a subject is receptive to *in vitro* fertilization, has an endometrial disease (e.g. cancer) or a pre-disposition to endometrial cancer may comprise: (a) receiving phenotypic information on the subject and information on one or more endometrial markers, and/or polynucleotides encoding the markers, and optionally other markers, associated with samples from the subject; (b) acquiring information from a network corresponding to one or more endometrial markers, and/or polynucleotides encoding the markers, and optionally other markers; and (c) based on the phenotypic information, information on one or more endometrial markers, and/or polynucleotides encoding the markers, and optionally other markers, and acquired information, determining whether the subject is receptive to *in vitro* fertilization, has an endometrial disease (e.g. cancer) or a pre-disposition to an endometrial disease (e.g. cancer); and (d) optionally recommending a procedure or treatment.

The computer systems, components, and methods described herein may be used to monitor disease or determine the stage of disease, or determine uterine endometrial receptivity.

### Imaging Methods

Binding agents, in particular antibodies, specific for one or more endometrial markers may also be used in imaging methodologies in the management of an endometrial disease or determining uterine endometrial receptivity.

Tumors associated with one or more endometrial cancer markers may be imaged.

Imaging methods described herein may use multiple markers for an endometrial disease or endometrium phase. Preferably each agent is labeled so that it can be distinguished during the imaging.

In a use a subject or patient may be administered one or more agents that carry an imaging label and that are capable of targeting or binding to one or more endometrial markers. The agent is allowed to incubate *in vivo* and bind to the endometrial markers associated with endometrial cells or tissues of a particular phase or associated with diseased cells or tissues, (e.g. an endometrial tumor). The presence of the label is localized to the endometrial cells or tissues, and the localized label is detected using imaging devices known to those skilled in the art.

The agent may be an antibody or chemical entity that recognizes the endometrial markers. The agent may be a polyclonal antibody or monoclonal antibody, or fragments thereof, or constructs thereof including but not limited to, single chain antibodies, bifunctional antibodies, molecular recognition units, and peptides or entities that mimic peptides. The antibodies specific for the endometrial markers used in the methods of the invention may be obtained from scientific or commercial sources, or isolated native endometrial markers or recombinant endometrial markers may be utilized to prepare antibodies etc. as described herein.

An agent may be a peptide that mimics the epitope for an antibody specific for an endometrial marker and binds to the marker. The peptide may be produced on a commercial synthesizer using conventional solid phase chemistry. By way of example, a peptide may be prepared that includes either tyrosine, lysine, or phenylalanine to which N₂S₂ chelate is complexed (See U.S. Patent No. 4,897,255). An anti-endocrine marker peptide conjugate is then combined with a radiolabel (e.g. sodium ^{99m}Tc pertechnetate or sodium ¹⁸⁸Re perrhenate) and it may be used to locate an endometrial marker producing cell or tissue (e.g. tumor).

The agent carries a label to image the endometrial markers. The agent may be labelled for use in radionuclide imaging. In particular, the agent may be directly or indirectly labelled with a radioisotope. Examples of radioisotopes that may be used in the present invention are the following: ²⁷⁷Ac, ²¹¹At, ¹²⁸Ba, ¹³¹Ba, ⁷Be, ²⁰⁴Bi, ²⁰⁵Bi, ²⁰⁶Bi, ⁷⁶Br, ⁷⁷Br, ⁸²Br, ¹⁰⁹Cd, ⁴⁷Ca, ¹¹C, ¹⁴C, ³⁶Cl , ⁴⁸Cr, ⁵¹Cr, ⁶²Cu, ⁶⁴Cu, ⁶⁷Cu, ¹⁶⁵Dy, ¹⁵⁵Eu, ¹⁸F, ¹⁵³Gd, ⁶⁶Ga, ⁶⁷Ga, ⁶⁸Ga, ⁷²Ga, ¹⁹⁸Au, ³H, ¹⁶⁶Ho, ¹¹¹In, ¹¹³In, ^{115m}In, ¹²³I, ¹²⁵I, ¹³¹I, ¹⁸⁹Ir, ^{191m}Ir, ¹⁹²Ir, ¹⁹⁴Ir, ⁵²Fe, ⁵⁵Fe, ⁵⁹Fe, ¹⁷⁷Lu, ¹⁵O, ^{191m-191}Os, ¹⁰⁹Pd, ³²P, ³³P, ⁴²K, ²²⁶Ra, ¹⁸⁶Re, ¹⁸⁸Re, ^{82m}Rb, ¹⁵³Sm, ⁴⁶Sc, ⁴⁷Sc, ⁷²Se, ⁷⁵Se, ¹⁰⁵Ag, ²²Na, ²⁴Na, ⁸⁹Sr, ³⁵S, ³⁸S, ¹⁷⁷Ta, ⁹⁶Tc, ^{99m}Tc, ²⁰¹Tl, ²⁰²Tl, ¹¹³Sn, ^{117m}Sn, ¹²¹Sn, ¹⁶⁶Yb, ¹⁶⁹Yb, ¹⁷⁵Yb, ⁸⁸Y, ⁹⁰Y, ⁶²Zn and ⁶⁵Zn. Preferably the radioisotope is ¹³¹I, ¹²⁵I, ¹²³I, ¹¹¹I, ^{99m}Tc, ⁹⁰Y, ¹⁸⁶Re, ¹⁸⁸Re, ³²P, ¹⁵³Sm, ⁶⁷Ga, ²⁰¹T1⁷⁷Br, or ¹⁸F, and is imaged with a photoscanning device.

Procedures for labeling biological agents with the radioactive isotopes are generally known in the art. U.S. Pat. No. 4,302,438 describes tritium labeling procedures. Procedures for iodinating, tritium labeling, and ³⁵ S labeling especially adapted for murine monoclonal antibodies are described by Goding, J. W. (supra, pp 124-126) and the references cited therein. Other procedures for iodinating biological agents, such as antibodies, binding portions thereof, probes, or ligands, are described in the scientific literature (see Hunter and Greenwood, Nature 144:945 (1962), David et al., Biochemistry 13:1014-1021 (1974), and U.S. Pat. Nos. 3,867,517 and 4,376,110). Iodinating procedures for agents are described by Greenwood, F. et al., Biochem. J. 89:114-123 (1963); Marchalonis, J., Biochem. J. 113:299-305 (1969); and Morrison, M. et al., Immunochemistry, 289-297 (1971). ^{99m}Tc-labeling procedures are described by Rhodes, B. et al. in Burchiel, S. et al. (eds.), Tumor Imaging: The Radioimmunochemical Detection of Cancer, New York: Masson 111-123 (1982) and the references cited therein. Labelling of antibodies or fragments with technetium-99m are also described for example in U.S. Pat. No. 5,317,091, U.S. Pat. No. 4,478,815, U.S. Pat. No. 4,478,818, U.S. Pat. No. 4,472,371, U.S. Pat. No. Re 32,417, and U.S. Pat. No. 4,311,688. Procedures suitable for ¹¹¹In-labeling biological agents are described by Hnatowich, D. J. et al., J. Immul. Methods, 65:147-157 (1983), Hnatowich, D. et al., J. Applied Radiation, 35:554-557 (1984), and Buckley, R. G. et al., F.E.B.S. 166:202-204 (1984).

An agent may also be labeled with a paramagnetic isotope for purposes of an *in vivo* method of the invention. Examples of elements that are useful in magnetic resonance imaging include gadolinium, terbium, tin, iron, or isotopes thereof. (See, for example, Schaefer et al., (1989) JACC 14, 472-480; Shreve et al., (1986) Magn. Reson. Med. 3, 336-340; Wolf, G L., (1984) Physiol. Chem. Phys. Med. NMR 16, 93-95; Wesbey et al., (1984) Physiol. Chem. Phys. Med. NMR 16, 145-155; Runge et al., (1984) Invest. Radiol. 19, 408-415 for discussions on *in vivo* nuclear magnetic resonance imaging.)

In the case of a radiolabeled agent, the agent may be administered to the patient, it is localized to the cell or tissue (e.g. tumor) having an endometrial marker with which the agent binds, and is detected or "imaged" *in vivo* using known techniques such as radionuclear scanning using e.g., a gamma camera or emission tomography. [See for example, A. R. Bradwell et al., "Developments in Antibody Imaging", Monoclonal Antibodies for Cancer Detection and Therapy, R. W. Baldwin et al., (eds.), pp. 65-85 (Academic Press 1985)]. A positron emission transaxial tomography scanner, such as designated Pet VI located at Brookhaven National Laboratory, can also be used where the radiolabel emits positrons (e.g., ¹¹C, ¹⁸F, ¹⁵O, and ¹³N).

Whole body imaging techniques using radioisotope labeled agents can be used for locating diseased cells and tissues (e.g. primary tumors and tumors which have metastasized). Antibodies specific for endometrial markers, or fragments thereof having the same epitope specificity, are bound to a suitable radioisotope, or a combination thereof, and administered parenterally. For endometrial cancer, administration preferably is intravenous. The bio-distribution of the label can be monitored by scintigraphy, and accumulations of the label are related to the presence of endometrial cancer cells. Whole body imaging techniques are described in U.S. Pat. Nos. 4,036,945 and 4,311,688. Other examples of agents useful for diagnosis and therapeutic use that can be coupled to antibodies and antibody fragments include metallothionein and fragments (see, U.S. Pat. No. 4,732,864). These agents are useful in diagnosis staging and visualization of cancer, in particular endometrial cancer, so that surgical and/or radiation treatment protocols can be used more efficiently.

An imaging agent may carry a bioluminescent or chemiluminescent label. Such labels include polypeptides known to be fluorescent, bioluminescent or chemiluminescent, or, that act as enzymes on a specific substrate (reagent), or can generate a fluorescent, bioluminescent or chemiluminescent molecule. Examples of bioluminescent or chemiluminescent labels include luciferases, aequorin, obelin, mnemiopsin, berovin, a phenanthridinium ester, and variations thereof and combinations thereof. A substrate for the bioluminescent or chemiluminescent polypeptide may also be utilized in a method of the invention. For example, the chemiluminescent polypeptide can be luciferase and the reagent luciferin. A substrate for a bioluminescent or chemiluminescent label can be administered before, at the same time (e.g., in the same formulation), or after administration of the agent.

An imaging agent may comprise a paramagnetic compound, such as a polypeptide chelated to a metal, e.g., a metalloporphyrin. The paramagnetic compound may also comprise a monocrystalline nanoparticle, e.g., a nanoparticle comprising a lanthanide (e.g., Gd) or iron oxide; or, a metal ion comprising a lanthanide. "Lanthanides" refers to elements of atomic numbers 58 to 70, a transition metal of atomic numbers 21 to 29, 42 or 44, a Gd(III), a Mn(II), or an element comprising a Fe element. Paramagnetic compounds can also comprise a neodymium iron oxide (NdFeO₃) or a dysprosium iron oxide (DyFeO₃). Examples of elements that are useful in magnetic resonance imaging include gadolinium, terbium, tin, iron, or isotopes thereof. (See, for example, Schaefer et al., (1989) JACC 14, 472-480; Shreve et al., (1986) Magn. Reson. Med. 3, 336-340; Wolf, G L., (1984) Physiol. Chem. Phys. Med. NMR 16, 93-95; Wesbey et al., (1984) Physiol. Chem. Phys. Med. NMR 16, 145-155; Runge et al., (1984) Invest. Radiol. 19, 408-415 for discussions on *in vivo* nuclear magnetic resonance imaging.)

An image can be generated in a method of the invention by computer assisted tomography (CAT), magnetic resonance spectroscopy (MRS) image, magnetic resonance imaging (MRI), positron emission tomography (PET), single-photon emission computed tomography (SPECT), or bioluminescence imaging (BLI) or equivalent.

Computer assisted tomography (CAT) and computerized axial tomography (CAT) systems and devices well known in the art can be utilized in the practice of the present invention. (See, for example, U.S. Patent Nos. 6,151,377; 5,946,371; 5,446,799; 5,406,479; 5,208,581; 5,109,397). The invention may also utilize animal imaging modalities, such as MicroCAT.TM. (ImTek, Inc.).

Magnetic resonance imaging (MRI) systems and devices well known in the art can be utilized in the practice of the present invention. In magnetic resonance methods and devices, a static magnetic field is applied to a tissue or a body in order to define an equilibrium axis of magnetic alignment in a region of interest. A radio frequency field is then applied to the region in a direction orthogonal to the static magnetic field direction to excite magnetic resonance in the region. The resulting radio frequency signals are then detected and processed, and the exciting radio frequency field is applied. The resulting signals are detected by radio-frequency coils that are placed adjacent to the tissue or area of the body of interest. (For a description of MRI methods and devices see, for example, U.S. Patent Nos. 6,151,377; 6,144,202; 6,128,522; 6,127,825; 6,121,775; 6,119,032; 6,115,446; 6,111,410; 602,891; 5,555,251; 5,455,512; 5,450,010; 5,378,987; 5,214,382; 5,031,624; 5,207,222; 4,985,678; 4,906,931; 4,558,279). MRI and supporting devices are commercially available for example, from Bruker Medical GMBH; Caprius; Esaote Biomedica; Fonar; GE Medical Systems (GEMS); Hitachi Medical Systems America; Intermagnetics General Corporation; Lunar Corp.; MagneVu; Marconi Medicals; Philips Medical Systems; Shimadzu; Siemens; Toshiba America Medical Systems; including imaging systems, by, e.g., Silicon Graphics. The invention may also utilize animal imaging modalities such as micro-MRIs.

Positron emission tomography imaging (PET) systems and devices well known in the art can be utilized in the practice of the present invention. For example, a method of the invention may use the system designated Pet VI located at Brookhaven National Laboratory. For descriptions of PET systems and devices see, for example, U.S. Pat. Nos. 6,151,377; 6,072,177; 5,900,636; 5,608,221; 5,532,489; 5,272,343; 5,103,098. Animal imaging modalities such as micro-PETs (Corcorde Microsystems, Inc.) can also be used in the invention.

Single-photon emission computed tomography (SPECT) systems and devices well known in the art can be utilized in the practice of the present invention. (See, for example, U.S. Patents. Nos. 6,115,446; 6,072,177; 5,608,221; 5,600,145; 5,210,421; 5,103,098.) The methods of the invention may also utilize animal imaging modalities, such as micro-SPECTs.

Bioluminescence imaging includes bioluminescence, fluorescence or chemiluminescence or other photon detection systems and devices that are capable of detecting bioluminescence, fluorescence or chemiluminescence. Sensitive photon detection systems can be used to detect bioluminescent and fluorescent proteins externally; see, for example, Contag (2000) Neoplasia 2:41-52; Zhang (1994) Clin. Exp. Metastasis 12:87-92. The methods of the invention can be practiced using any such photon detection device, or variation or equivalent thereof, or in conjunction with any known photon detection methodology, including visual imaging. By way of example, an intensified charge-coupled device (ICCD) camera coupled to an image processor may be used in the present invention. (See, e.g., U.S. Pat. No. 5,650,135). Photon detection devices are also commercially available from Xenogen, Hamamatsue.

### Screening Methods

Disclosed are also methods for evaluating test agents or compounds for their ability to inhibit an endometrial disease (e.g. cancer), potentially contribute to an endometrial disease (e.g. cancer), or inhibit or enhance an endometrium phase. Test agents and compounds include but are not limited to peptides such as soluble peptides including Ig-tailed fusion peptides, members of random peptide libraries and combinatorial chemistry-derived molecular libraries made of D- and/or L-configuration amino acids, phosphopeptides (including members of random or partially degenerate, directed phosphopeptide libraries), antibodies [e.g. polyclonal, monoclonal, humanized, anti-idiotypic, chimeric, single chain antibodies, fragments, (e.g. Fab, F(ab)₂, and Fab expression library fragments, and epitope-binding fragments thereof)], and small organic or inorganic molecules. The agents or compounds may be endogenous physiological compounds or natural or synthetic compounds.

A method for assessing the potential efficacy of a test agent for inhibiting an endometrial disease (e.g. cancer) in a patient may comprise comparing:
(a) levels of one or more endometrial markers, and/or polynucleotides encoding endometrial markers, and optionally other markers in a first sample obtained from a patient and exposed to the test agent; and
(b) levels of one or more endometrial markers and/or polynucleotides encoding endometrial markers, and optionally other markers, in a second sample obtained from the patient, wherein the sample is not exposed to the test agent, wherein a significant difference in the levels of expression of one or more endometrial markers, and/or polynucleotides encoding one or more endometrial markers, and optionally the other markers, in the first sample, relative to the second sample, is an indication that the test agent is potentially efficacious for inhibiting an endometrial disease (e.g. cancer) in the patient.

The first and second samples may be portions of a single sample obtained from a patient or portions of pooled samples obtained from a patient.

A method of selecting an agent for inhibiting an endometrial disease (e.g. cancer) in a patient may comprise:
(a) obtaining a sample from the patient;
(b) separately maintaining aliquots of the sample in the presence of a plurality of test agents;
(c) comparing one or more endometrial markers, and/or polynucleotides encoding endometrial markers, and optionally other markers, in each of the aliquots; and
(d) selecting one of the test agents which alters the levels of one or more endometrial markers, and/or polynucleotides encoding endometrial markers, and optionally other markers in the aliquot containing that test agent, relative to other test agents.

A method of selecting an agent for inhibiting or enhancing an endometrium phase in a patient may comprise:
(a) obtaining a sample of endometrium in a selected phase (e.g. secretory or proliferative phase);
(b) separately maintaining aliquots of the sample in the presence of a plurality of test agents;
(c) comparing one or more endometrial markers, and/or polynucleotides encoding endometrial markers, and optionally other markers, in each of the aliquots; and
(d) selecting one of the test agents which alters the levels of one or more endometrial markers, and/or polynucleotides encoding endometrial markers, and optionally other markers in the aliquot containing that test agent, relative to other test agents.

A drug discovery method may comprise:
(a) providing one or more methods or assay systems for identifying agents that inhibit an endometrial disease (e.g. endometrial cancer) or affect an endometrium phase in a patient;
(b) conducting therapeutic profiling of agents identified in step (a), or further analogs thereof, for efficacy and toxicity in animals; and
(c) formulating a pharmaceutical preparation including one or more agents identified in step (b) as having an acceptable therapeutic profile.

In certain embodiments, the subject method can also include a step of establishing a distribution system for distributing the pharmaceutical preparation for sale, and may optionally include establishing a sales group for marketing the pharmaceutical preparation.

A method of assessing the potential of a test compound to contribute to an endometrial disease (e.g. endometrial cancer) may comprise:
(a) maintaining separate aliquots of cells or tissues from a patient with an endometrial disease (e.g. cancer) in the presence and absence of the test compound; and
(b) comparing one or more endometrial markers, and/or polynucleotides encoding endometrial markers, and optionally other markers in each of the aliquots.

A significant difference between the levels of the markers in the aliquot maintained in the presence of (or exposed to) the test compound relative to the aliquot maintained in the absence of the test compound, indicates that the test compound possesses the potential to contribute to an endometrial disease (e.g. endometria cancer).

### Kits

Kits may be used for carrying out the methods of the invention. Kits may typically comprise two or more components required for performing a diagnostic assay. Components include but are not limited to compounds, reagents, containers, and/or equipment.

The methods described herein may be performed by utilizing pre-packaged diagnostic kits comprising one or more specific endometrial marker polynucleotide or antibody described herein, which may be conveniently used, e.g., in clinical settings to screen and diagnose patients and to screen and identify those individuals exhibiting a predisposition to developing an endometrial disease.

In an embodiment, a container with a kit comprises a binding agent as described herein. By way of example, the kit may contain antibodies or antibody fragments which bind specifically to epitopes of one or more endometrial markers and optionally other markers, antibodies against the antibodies labelled with an enzyme; and a substrate for the enzyme. The kit may also contain microtiter plate wells, standards, assay diluent, wash buffer, adhesive plate covers, and/or instructions for carrying out a method of the invention using the kit.

The kit may include antibodies or fragments of antibodies which bind specifically to an epitope of the pyruvate kinase and means for detecting binding of the antibodies to their epitope associated with tumor cells, either as concentrates (including lyophilized compositions), which may be further diluted prior to use or at the concentration of use, where the vials may include one or more dosages. Where the kits are for *in vivo* use, single dosages may be provided in sterilized containers, having the desired amount and concentration of agents. Containers that provide a formulation for direct use, usually do not require other reagents, as for example, where the kit contains a radiolabelled antibody preparation for *in vivo* imaging.

A kit may be designed to detect the level of polynucleotides encoding one or more endometrial polynucleotide markers in a sample. In an embodiment, the polynucleotides encode one or more polynucleotides encoding a pyruvate kinase. Such kits generally comprise at least one oligonucleotide probe or primer, as described herein, that hybridizes to a polynucleotide encoding one or more endometrial cancer markers. Such an oligonucleotide may be used, for example, within a PCR or hybridization procedure. Additional components that may be present within the kits include a second oligonucleotide and/or a diagnostic reagent or container to facilitate detection of a polynucleotide encoding one or more endometrial cancer markers.

The may contain a micoarray described herein ready for hybridization to target endometrial polynucleotide markers, plus software for the data analysis of the results. The software to be included with the kit comprises data analysis methods, in particular mathematical routines for marker discovery, including the calculation of correlation coefficients between clinical categories and marker expression. The software may also include mathematical routines for calculating the correlation between sample marker expression and control marker expression, using array-generated fluorescence data, to determine the clinical classification of the sample.

The reagents suitable for applying the screening methods of the invention to evaluate compounds may be packaged into convenient kits described herein providing the necessary materials packaged into suitable containers.

The invention contemplates a kit for assessing the presence of endometrial cells, wherein the kit comprises antibodies specific for one or more endometrial markers, or primers or probes for polynucleotides encoding same, and optionally probes, primers or antibodies specific for other markers associated with an endometrial disease (e.g. cancer).

The invention relates to a kit for assessing the suitability of each of a plurality of test compounds for inhibiting an endometrial disease (e.g. endometrial cancer) in a patient. The kit comprises reagents for assessing one or more endometrial markers or polynucleotides encoding same, and optionally a plurality of test agents or compounds.

Additionally the invention provides a kit for assessing the potential of a test compound to contribute to an endometrial disease (e.g. cancer). The kit comprises endometrial diseased cells (e.g. cancer cells) and reagents for assessing one or more endometrial markers, polynucleotides encoding same, and optionally other markers associated with an endometrial disease.

### Therapeutic Applications

One or more endometrial markers may be targets for immunotherapy. Immunotherapeutic methods include the use of antibody therapy, *in vivo* vaccines, and *ex vivo* immunotherapy approaches.

One or more endometrial marker antibodies disclosed herein may be used systemically to treat an endometrial disease associated with the marker. In particular, the endometrial disease is endometrial cancer and one or more endometrial marker antibodies may be used systemically to treat endometrial cancer. Preferably antibodies are used that target the tumor cells but not the surrounding non-tumor cells and tissue.

A used for treating a patient susceptible to, or having a disease (e.g. cancer) that expresses the endometrial marker pyrivate kinase may comprise administering to the patient an effective amount of an antibody that binds specifically to the endometrial marker.

A use for inhibiting the growth of tumor cells expressing the endometrial cancer marker may comprise administering to a patient an antibody which binds specifically to one or more endometrial cancer markers in an amount effective to inhibit growth of the tumor cells.

One or more endometrial marker antibodies may also be used in a method for selectively inhibiting the growth of, or killing a cell expressing the endometrial marker (e.g. tumor cell expressing one or more endometrial cancer marker) comprising reacting one or more endometrial marker antibody immunoconjugate or immunotoxin with the cell in an amount sufficient to inhibit the growth of, or kill the cell.

By way of example, unconjugated antibodies to the endometrial cancer marker may be introduced into a patient such that the antibodies bind to endometrial cancer marker expressing cancer cells and mediate growth inhibition of such cells (including the destruction thereof), and the tumor, by mechanisms which may include complement-mediated cytolysis, antibody-dependent cellular cytotoxicity, altering the physiologic function of one or more endometrial cancer markers, and/or the inhibition of ligand binding or signal transduction pathways. In addition to unconjugated antibodies to the endometrial cancer marker, one or more endometrial cancer marker antibodies conjugated to therapeutic agents (e.g. immunoconjugates) may also be used therapeutically to deliver the agent directly to one or more endometrial cancer marker expressing tumor cells and thereby destroy the tumor. Examples of such agents include abrin, ricin A, *Pseudomonas* exotoxin, or diphtheriatoxin; proteins such as tumor necrosis factor, alpha-interferon, beta-interferon, nerve growth factor, platelet derived growth factor, tissue plasminogen activator; and biological response modifiers such as lymphokines, interleukin-1, interleukin-2, interleukin-6, granulocyte macrophage colony stimulating factor, granulocyte colony stimulating factor, or other growth factors.

Cancer immunotherapy using one or more endometrial cancer marker antibodies may utilize the various approaches that have been successfully employed for cancers, including but not limited to colon cancer (Arlen et al., 1998, Crit Rev Immunol 18: 133-138), multiple myeloma (Ozaki et al., 1997, Blood 90: 3179-3186; Tsunenati et al.,1997, Blood 90: 2437-2444), gastric cancer (Kasprzyk et al.,1992, Cancer Res 52: 2771-2776), B-cell lymphoma (Funakoshi et al., 1996, J Immunther Emphasis Tumor Immunol 19:93-101), leukemia (Zhong et al.,1996, Leuk Res 20: 581-589), colorectal cancer (Moun et al., 1994, Cancer Res 54:6160-6166); Velders et al., 1995, Cancer Res 55: 4398-4403), and breast cancer (Shepard et al., 1991, J Clin Immunol 11: 117-127).

In the practice of a respective use endometrial cancer marker antibodies capable of inhibiting the growth of cancer cells expressing the endometrial cancer marker are administered in a therapeutically effective amount to cancer patients whose tumors express or overexpress the endometrial cancer marker. The use may provide a specific, effective and long-needed treatment for endometrial cancer. The antibody therapy use may be combined with other therapies including chemotherapy and radiation.

Patients may be evaluated for the presence and level of expression or overexpression of one or more endometrial markers in diseased cells and tissues (e.g. tumors), in particular using immunohistochemical assessments of tissue, quantitative imaging as described herein, or other techniques capable ofreliably indicating the presence and degree of expression of one or more endometrial markers. Immunohistochemical analysis of tumor biopsies or surgical specimens may be employed for this purpose.

Endometrial marker antibodies useful in treating disease (e.g. cancer) include those that are capable of initiating a potent immune response against the disease (e.g. tumor) and those that are capable of direct cytotoxicity. In this regard, endometrial marker antibodies may elicit cell lysis by either complement-mediated or antibody-dependent cell cytotoxicity (ADCC) mechanisms, both of which require an intact Fc portion of the immunoglobulin molecule for interaction with effector cell Fc receptor sites or complement proteins.

Endometrial marker antibodies that exert a direct biological effect on tumor growth may also be useful in the practice of the invention. Such antibodies may not require the complete immunoglobulin to exert the effect. Potential mechanisms by which such directly cytotoxic antibodies may act include inhibition of cell growth, modulation of cellular differentiation, modulation of tumor angiogenesis factor profiles, and the induction of apoptosis. The mechanism by which a particular antibody exerts an anti-tumor effect may be evaluated using any number of *in vitro* assays designed to determine ADCC, antibody-dependent macrophage-mediated cytotoxicity (ADMMC), complement-mediated cell lysis, and others known in the art.

The anti-tumor activity of a particular endometrial cancer marker antibody, or combination of endometrial cancer marker antibodies, may be evaluated *in vivo* using a suitable animal model. Xenogenic cancer models, where human cancer explants or passaged xenograft tissues are introduced into immune compromised animals, such as nude or SCID mice, may be employed.

Contemplated in the administration of single endometrial marker antibodies as well as combinations, or "cocktails", of different individual antibodies such as those recognizing different epitopes of other markers. Such cocktails may have certain advantages inasmuch as they contain antibodies that bind to different epitopes of endometrial markers and/or exploit different effector mechanisms or combine directly cytotoxic antibodies with antibodies that rely on immune effector functionality. Such antibodies in combination may exhibit synergistic therapeutic effects. In addition, the administration of one or more endometrial marker specific antibodies may be combined with other therapeutic agents, including but not limited to chemotherapeutic agents, androgen-blockers, and immune modulators (e.g., IL2, GM-CSF). The endometrial marker specific antibodies may be administered in their "naked" or unconjugated form, or may have therapeutic agents conjugated to them.

The endometrial marker specific antibodies used may be formulated into pharmaceutical compositions comprising a carrier suitable for the desired delivery method. Suitable carriers include any material which when combined with the antibodies retains the function of the antibody and is nonreactive with the subject's immune systems. Examples include any of a number of standard pharmaceutical carriers such as sterile phosphate buffered saline solutions, bacteriostatic water, and the like (see, generally, Remington's Pharmaceutical Sciences 16.sup.th Edition, A. Osal., Ed., 1980).

One or more endometrial marker specific antibody formulations may be administered via any route capable of delivering the antibodies to the a disease (e.g. tumor) site. Routes of administration include, but are not limited to, intravenous, intraperitoneal, intramuscular, intratumor, intradermal, and the like. Preferably, the route of administration is by intravenous injection. Antibody preparations may be lyophilized and stored as a sterile powder, preferably under vacuum, and then reconstituted in bacteriostatic water containing, for example, benzyl alcohol preservative, or in sterile water prior to injection.

Treatment will generally involve the repeated administration of the antibody preparation via an acceptable route of administration such as intravenous injection (IV), at an effective dose. Dosages will depend upon various factors generally appreciated by those of skill in the art, including the type of disease and the severity, grade, or stage of the disease, the binding affinity and half life of the antibodies used, the degree of endometrial marker expression in the patient, the extent of circulating endometrial markers, the desired steady-state antibody concentration level, frequency of treatment, and the influence of any chemotherapeutic agents used in combination with the treatment method of the invention. Daily doses may range from about 0.1 to 100 mg/kg. Doses in the range of 10-500 mg antibodies per week may be effective and well tolerated, although even higher weekly doses may be appropriate and/or well tolerated. A determining factor in defining the appropriate dose is the amount of a particular antibody necessary to be therapeutically effective in a particular context. Repeated administrations may be required to achieve disease inhibition orregression. Direct administration of one or more endometrial marker antibodies is also possible and may have advantages in certain situations.

Patients may be evaluated for serum cancer markers in order to assist in the determination of the most effective dosing regimen and related factors. The endometrial cancer assay methods described herein, or similar assays, may be used for quantitating circulating endometrial marker levels in patients prior to treatment. Such assays may also be used for monitoring throughout therapy, and may be useful to gauge therapeutic success in combination with evaluating other parameters such as serum levels of endometrial markers.

Disclosed are further methods for inhibiting cellular activity (e.g., cell proliferation, activation, or propagation) of a cell expressing one or more endometrial marker. This method comprises reacting immunoconjugates of the invention (e.g., a heterogeneous or homogenous mixture) with the cell so that endometrial markers form complexes with the immunoconjugates. A subject with a neoplastic or preneoplastic condition can be treated when the inhibition of cellular activity results in cell death.

Disclosed are also methods for selectively inhibiting a cell expressing one or more endometrial marker by reacting any one or a combination of the immunoconjugates of the invention with the cell in an amount sufficient to inhibit the cell. Amounts include those that are sufficient to kill the cell or sufficient to inhibit cell growth or proliferation.

Vectors derived from retroviruses, adenovirus, herpes or vaccinia viruses, or from various bacterial plasmids, may be used to deliver polynucleotides encoding endometrial cancer markers to a targeted organ, tissue, or cell population. Methods well known to those skilled in the art may be used to construct recombinant vectors that will express antisense polynucleotides for endometrial markers. (See, for example, the techniques described in Sambrook et al (supra) and Ausubel et al (supra)).

Methods for introducing vectors into cells or tissues include those methods discussed herein and which are suitable for *in* vivo, *in vitro* and ex *vivo* therapy. For *ex vivo* therapy, vectors may be introduced into stem cells obtained from a patient and clonally propagated for autologous transplant into the same patient (See U.S. Pat. Nos. 5,399,493 and 5,437,994). Delivery by transfection and by liposome are well known in the art.

Genes encoding endometrial markers can be turned off by transfecting a cell or tissue with vectors that express high levels of a desired endometrial marker-encoding fragment. Such constructs can inundate cells with untranslatable sense or antisense sequences. Even in the absence of integration into the DNA, such vectors may continue to transcribe RNA molecules until all copies are disabled by endogenous nucleases.

Modifications of gene expression can be obtained by designing antisense molecules, DNA, RNA or PNA, to the regulatory regions of a gene encoding the endometrial marker, i.e., the promoters, enhancers, and introns. Preferably, oligonucleotides are derived from the transcription initiation site, e.g. between -10 and +10 regions of the leader sequence. The antisense molecules may also be designed so that they block translation of mRNA by preventing the transcript from binding to ribosomes. Inhibition may also be achieved using "triple helix" base-pairing methodology. Triple helix pairing compromises the ability of the double helix to open sufficiently for the binding of polymerases, transcription factors, or regulatory molecules. Therapeutic advances using triplex DNA were reviewed by Gee J E et al (In: Huber B E and B I Carr (1994) Molecular and Immunologic Approaches, Futura Publishing Co, Mt Kisco N.Y.).

Ribozymes are enzymatic RNA molecules that catalyze the specific cleavage of RNA. Ribozymes act by sequence-specific hybridization of the ribozyme molecule to complementary target RNA, followed by endonucleolytic cleavage. The invention therefore contemplates engineered hammerhead motif ribozyme molecules that can specifically and efficiently catalyze endonucleolytic cleavage of sequences encoding an endometrial marker.

Specific ribozyme cleavage sites within any potential RNA target may initially be identified by scanning the target molecule for ribozyme cleavage sites which include the following sequences, GUA, GUU and GUC. Once the sites are identified, short RNA sequences of between 15 and 20 ribonucleotides corresponding to the region of the target gene containing the cleavage site may be evaluated for secondary structural features which may render the oligonucleotide inoperable. The suitability of candidate targets may also be determined by testing accessibility to hybridization with complementary oligonucleotides using ribonuclease protection assays.

The, marker and polynucleotides encoding the marker, and fragments thereof, may be used in the treatment of in as subject. The marker or polynucleotides may be formulated into compositions for administration to subjects suffering from an endometrial disease. Disclosed is also a composition comprising one or more endometrial markers or polynucleotides encoding the markers, or a fragment thereof, and a pharmaceutically acceptable carrier, excipient or diluent A use for treating or preventing an endometrial disease in a subject may also comprise administering to a patient in need thereof, the marker or polynucleotides encoding the marker, or a composition as described herein.

A use for inhibiting endometrial cancer in a patient may comprise:
(a) obtaining a sample comprising diseased cells from the patient;
(b) separately maintaining aliquots of the sample in the presence of a plurality of test agents;
(c) comparing levels of one or more endometrial markers, and/or polynucleotides encoding one or more endometrial markers in each aliquot;
(d) administering to the patient at least one of the test agents which alters the levels of the endometrial markers, and/or polynucleotides encoding one or more endometrial markers in the aliquot containing that test agent, relative to the other test agents.

Endometrial markers in uterine biopsy tissue or fluid and sera may vary between known fertile and infertile women during the window of implantation, deviate in women undergoing ovarian hyperstimulation/ovulation induction, and correlate with successful initiation of pregnancy. Therefore, endometrial markers disclosed herein may serve as minimally or noninvasive markers of uterine receptivity for implantation.

A method of determining uterine endometrial receptivity may include first obtaining a serum, uterine fluid or endometrial biopsy sample from a patient and detecting the presence of an endometrial marker associated with a certain endometrium phase, wherein the presence or absence of an endometrial marker as compared to controls indicates uterine receptivity. In an embodiment, the endometrium phase is the secretory phase. Where necessary for the evaluation, repetitive samples may be collected throughout the menstrual cycle. Non-receptive controls are both women who are in the non-fertile stage of the menstrual cycle and women with known uterine dysfunction where an endometrial marker is not present or present on the endometrium throughout the menstrual cycle or certain endometrium phases.

Disclosed is also a method of monitoring the effects of ovarian hyperstimulatlon and/or ovulation induction protocols on uterine receptivity either for individual women receiving the treatment or for the evaluation of new protocols. In an embodiment, the method comprises: (a) obtaining a serum, uterine or fluid or endometrial biopsy sample from a patient receiving the treatments; and (b) detecting the presence of an endometrial marker of the invention present in the endometrium at the time of fertilization, early embryogenesis, and implantation; wherein presence or absence of an endometrial marker indicates receptivity. A disruption of the normal cyclic presence of an endometrial marker indicates that the treatment may adversely affect uterine receptivity. This disruption may include non-cyclic presence of an endometrial marker or an aberrant presence of an endometrial marker as compared to controls.

Further disclosed is a method of determining a probability of successful implantation with an ovarian stimulation *in vitro* fertilization and embryo transfer procedure, comprising:
(a) determining a level of an endometrial marker identified in accordance with a method of the invention in a sample obtained from a patient who has undergone an ovarian stimulation *in vitro* fertilization and embryo transfer procedure; and
(b) determining a probability of successful implantation based on the patient's determined endometrial marker level;
wherein a significantly different endometrial marker level relative to a standard level is associated with a decreased or increased probability of successful implantation.

Disclosed is also a method of contraception by interrupting the cyclic presence of an endometrial marker. The interruption can be to reduce or eliminate a marker present during the uterine receptivity window for implantation ofthe menstrual cycle and to thereby alter the cyclic presence/pattern of a marker. The interruption can utilize an antagonist of a marker. The term antagonist or antagonizing is used in its broadest sense. Antagonism can include any mechanism or treatment that results in inhibition, inactivation, blocking or reduction or alteration of cyclic presence of an endometrial marker.

An active therapeutic substance described herein may be administered in a convenient manner such as by injection (subcutaneous, intravenous, etc.), oral administration, inhalation, transdermal application, or rectal administration. Depending on the route of administration, the active substance may be coated in a material to protect the substance from the action of enzymes, acids and other natural conditions that may inactivate the substance. Solutions of an active compound as a free base or pharmaceutically acceptable salt can be prepared in an appropriate solvent with a suitable surfactant. Dispersions may be prepared in glycerol, liquid polyethylene glycols, and mixtures thereof, or in oils.

The compositions described herein can be prepared by per se known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the compositions include, albeit not exclusively, solutions of the active substances in association with one or more pharmaceutically acceptable vehicles or diluents, and contained in buffered solutions with a suitable pH and iso-osmotic with the physiological fluids.

The compositions are indicated as therapeutic agents either alone or in conjunction with other therapeutic agents or other forms of treatment. The compositions disclosed herein may be administered concurrently, separately, or sequentially with other therapeutic agents or therapies.

The therapeutic activity of compositions and agents/compounds identified using a method described above a may be evaluated *in vivo* using a suitable animal model.

The following non-limiting examples are illustrative of the present invention:

### Example 1

Isotope-coded affinity tag (ICAT) analysis with a cleavable tag was used to examine differentially expressed proteins in proliferative and secretory endometria. Sample complexity ofthe tissue homogenates was reduced by strong cation exchange (SCX) fractionation and subsequent affinity cleanup. Analysis of ten SCX fractions in triplicate by nanobore liquid chromatography - tandem mass spectrometry (nanoLC-MS/MS) resulted in the identification of approximately 400 labeled proteins and the discovery of potential biomarkers for the secretory phase of the endometrial cycle. This study demonstrated the feasibility of using this approach for identifying markers at a proteomics level for different stages of the endometrial cycle.

### Tissue Samples

Endometrium tissue was retrieved from an in-house dedicated, research endometrial tissue bank. All tissues were snap frozen in liquid nitrogen within 15-20 minutes of devitalization at the time of hysterectomy, and were obtained with patient consent. In each case, the endometrium was classified as proliferative or secretory by a pathologist. The histological classification was verified by examination of a histopathologic section from the frozen research tissue. Tissue was taken for proteomic analysis from the mirror-face of the residual block. After addition of 1 ml Hanks' Balanced Salt Solution containing protease inhibitors (leupeptin, aprotinin, pepstatin at 1 µg/mL), the tissue was mechanically homogenized at 30,000 rpms using a Polytron PT 1300D handheld homogenizer (Brinkmann, Westbury, USA). The samples were stored in aliquots at -80°C and/or submitted for protein profiling. These whole tissue homogenates contain endometrial epithelium, supportive stroma and vessels, as well as any secretions. Tissue samples from six different individuals were selected for the study. Three of these tissues were classified as proliferative endometria (PRO1, PRO2 and PR03) and the other three as secretory endometria (SEC1, SEC2, SEC3).

### Chemicals

Acetonitrile, formic acid, potassium chloride, monobasic potassium phosphate, leupeptin, aprotinin, pepstatin and Hanks' Balanced Salt Solution were obtained from Sigma-Aldrich (Oakville, Canada). All reagents and buffers for the cleavable ICAT sample preparation procedure were from Applied Biosystems (Foster City, USA).

### ICAT Sample Preparation Procedure

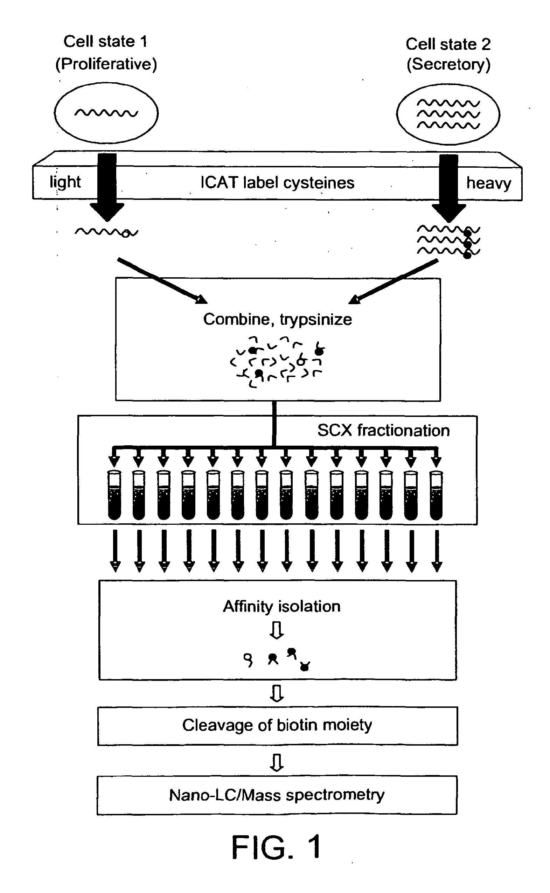

After removal of cell debris by centrifugation, the total protein content for each of the six clarified homogenates was measured using a commercially available Bradford protein assay reagent (Bio-Rad, Hercules, USA). ICAT sample preparation procedure was carried out according to the cleavable ICAT protocol (Applied Biosystems, Foster City, USA) and is illustrated in Figure 1. Following denaturing and reducing steps, 100 µg total protein of each of the samples was labeled with either the light ICAT reagent (proliferative samples) or the heavy reagent (secretory samples). The labeled PRO 1 and SEC I samples were combined to form ICAT Sample A, PRO2 and SEC2 form ICAT Sample B, and lastly PRO3 and SEC3 form ICAT Sample C. Mixing of the labeled proliferative and secretory samples in pairs in this manner ensures that any protein or peptide losses during subsequent processing steps is the same for both samples in a pair. Since the peptides are differentially labeled, they can be traced to specific samples in the pair. Any difference detected in the levels of individual peptides can then be ascribed solely to initial differences in expression level. Chromatographic separation using a strong cation exchange (SCX) column was performed after trypsin digestion to fractionate the ICAT samples. Selected SCX fractions were purified by affinity chromatography according to the ICAT protocol, and subsequently analyzed by nanobore liquid chromatography-tandem mass spectrometry (nanoLC-MS/MS).

### Instrumentation for SCX fractionation

SCX fractionation was performed on an HP 1050 LC system (Agilent, Palo Alto, USA) using a 1.5 ml injection loop and a SF-2120 Super Fraction Collector (Advantec MFS, Dublin, USA).

### LC/MS/MS Instrumentation

The LC system from LC Packings (Amsterdam, The Netherlands) consisted of a Famos autosampler and Ultimate Nano LC system. It was interfaced to an API QSTAR Pulsar QqTOF mass spectrometer (Applied Biosystems/MDS Sciex, Foster City, USA) using a Protana NanoES ion source (Protana Engineering A/S, Odense, Denmark). PicoTip's SilicaTip emitters with a 10 µm tip i.d. (New Objective, Woburn, USA) were used as spray capillaries. All data were acquired using Analyst QS SP5 with Bioanalyst Extension 1.1 and analyzed with ProICAT SP2 software (Applied Biosystems/MDS Sciex, Foster City, USA).

### LC Conditions

Strong cation exchange chromatography (SCX) was performed using a PolyLC Polysulfoethyl A column (The Nest Group, Southborough, USA) equipped with a guard column of the same material with the following dimensions: 5-µm particle size, 300-Å bead, 2.1-mm i.d., 10-mm length (guard column) and 100-mm length (analytical column). Eluent A of the mobile phase consisted of a 10 mM KH₂PO₄ solution in 25% acetonitrile and 75% deionized water (pH = 3.0). Eluent B consisted of a 10 mM KH₂PO₄ and 350 mM KCl solution in 25% acetonitrile and 75% deionized water (pH = 3.0). In each case, 1.5 ml of the total 2.4 ml sample (after acidifying with 2 ml Eluent A) was injected. Fractions were collected every 2 mins at a flow rate of 0.2 ml/min. using a binary gradient with the following profile:

| | | | | |
|---|---|---|---|---|
| *t* [min] | 0.01 | 2 | 58 | 60 |
| *c(Eluent B)* [%] | 0 | 0 | 100 | stop |

All reversed-phase separations were performed using PepMap C18 nano capillary columns (LC Packings, Amsterdam, The Netherlands) with the following dimensions: 3-µm particle size, 100-Å bead, 75-µm i.d. and 150-mm length. Eluent A of the mobile phase consisted of 950 ml deionized water, 50 ml acetonitrile and 1 ml formic acid (pH ≈3). Eluent B consisted of 50 ml deionized water, 950 ml acetonitrile and 1 ml formic acid. A binary gradient at a flow rate of approximately 200 nl/min with the following profile was used:

| | | | | | | | | |
|---|---|---|---|---|---|---|---|---|
| *t* [min] | 0.01 | 5 | 125 | 135 | 157 | 160 | 163 | 190 |
| *c(Eluent B)* [%] | 5 | 5 | 30 | 60 | 80 | 80 | 5 | Stop |

Injections of 1 µl of sample were performed in full loop mode.

### MS Conditions

The source conditions were a curtain-gas setting of 20 and an ionspray voltage (in the range of 2000 - 3800 V) that was optimized daily. All data were obtained in the positive-ion detection mode. In the Q0 region, the instrument parameters were a declustering potential (DP) of 65 V and a focusing potential (FP) of 265 V. Nitrogen was used as the collision gas at a setting of CAD5 for both TOF-MS and MS/MS scans.

All LC-MS/MS data were acquired in information-dependent acquisition (IDA) mode. A TOF-MS survey scan with a mass range of m/z = 400 - 1500 and 1 s scan time was followed by two product ion scans with a mass range of m/z = 70 -2000 and 2 s scan time. The collision energy (CE) was automatically controlled by the IDA CE Parameters script. The switching criteria were set to ions greater than m/z = 400 and smaller than m/z = 1500 with a charge state of 2 to 5 and an intensity of ≥10 counts/s. Former target ions were excluded for 60 s and peaks within a 4 Th window were ignored. In addition, the IDA Extensions II script was set to 2 repetitions before dynamic exclusion and to select a precursor ion nearest to a threshold of 15 count/s every 4 cycles.

### RESULTS AND DISCUSSION

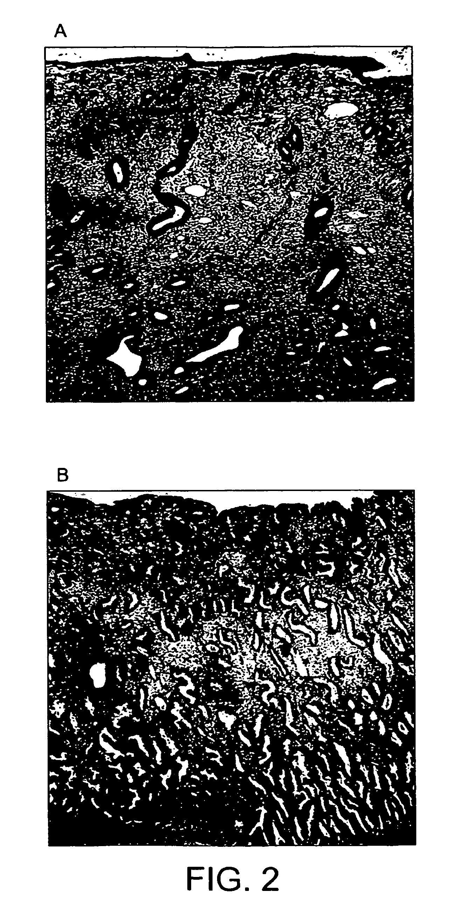

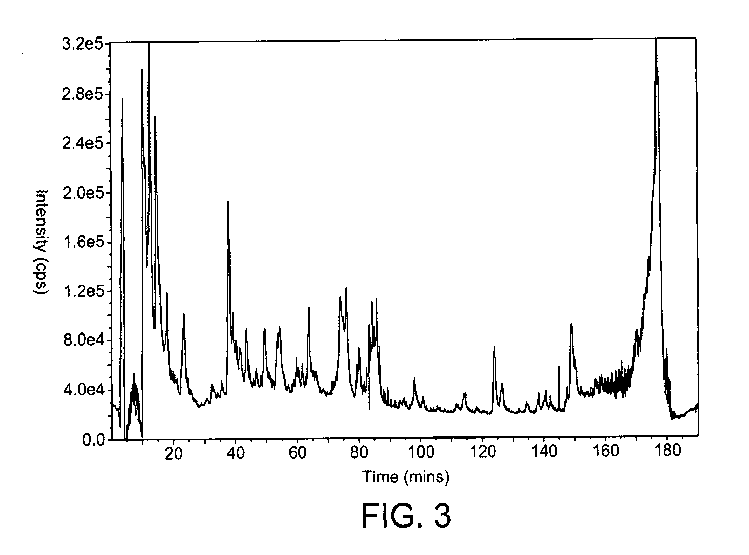

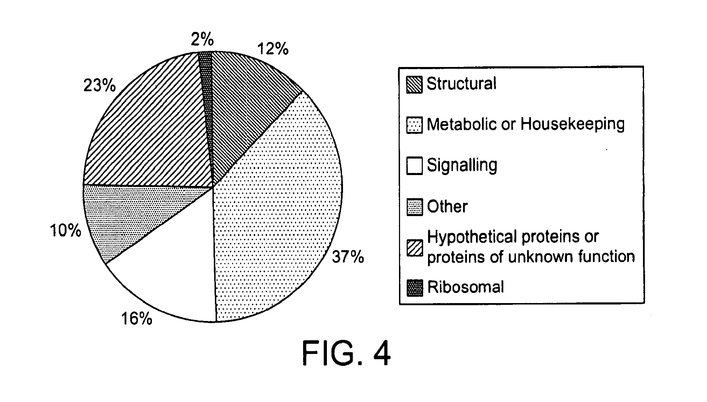

Figure 2 shows examples of the histologic appearance of proliferative (Figure 2A) and secretory (Figure 2B) endometrium. In both endometria the stratum basalis is characterized by a denser stroma than the physiologic responsive stratum functionalis above. Across the top of the stratum functionalis is the surface epithelium, which lines the endometrial cavity. The proliferative endometrium (PRO 2) shows small, coiled glands with lining columnar epithelium reaching to the surface. In contrast, the secretory endometrium (SEC2) is thicker, and contains more tortuous glands with intra-luminal secretions. The endometrium of both types of physiologic phases has abundant supportive stroma and vessels among the epithelial glands. In this study homogenates from the mirror faces of these tissues were used for quantitative analysis. After performing the procedure described above, SCX fractions 11 - 20 (of the 30 fractions) from each of the samples were chosen for further processing. This choice was based on the UV trace generated during fractionation. The 10 chosen fractions from each sample were affinity-purified, cleaved as per the ICAT protocol and analyzed using nanoLC-MS/MS. Figure 3 is an example of a nano LC-MS total ion chromatogram (TIC) from one of the ICAT Sample A SCX fractions. As the samples were run in IDA mode (See above), each such TIC resulted in hundreds of MS/MS spectra. A ProICAT confidence value of 75 was adopted after trial-and-error for reliable protein detection and identification for this initial investigation. This has resulted in identification of approximately 400 distinct proteins. A preliminary classification of these proteins based on function, is seen in Figure 4. As expected the majority of proteins fall under one of the metabolic, housekeeping or structural categories. The proteins classified under "other" are proteins like antibodies, which could not be included with the previously mentioned categories. There were also a significant number ofproteins for which function could not be assigned or were identified from cDNA matches and are therefore classified as hypothetical. This last category often contains the most interesting cases for biologists. Adopting a less stringent confidence value resulted in apparent identification of many more proteins; the reliability of such identifications, however, was judged much poorer after manual inspection. In addition, manual inspection of the spectra was also found necessary to confirm automated quantifications in ProICAT.

The results show that expression levels of the majority of proteins identified were not consistently different between the two phases of the endometrial cycle. This similarity in expression levels of proteins is not surprising since most of the abundant proteins detected tend to be housekeeping or structural in nature. In addition, similar or identical, housekeeping or structural proteins such as actin and tubulin are expressed in many tissue types. This is significant since whole tissue homogenates consist not only of epithelial cells, but also supportive stromal cells and interstitium, blood, vessels and any glandular secretions. These sources provide a major contribution to the protein profile of the overall tissue. These high abundance proteins from these adjacent cellular types may mask differential protein expression by a cellular component of the endometrium. Thirdly the differential expression of low abundance proteins in any one cellular type within proliferative and secretory endometrium may not have been detectable by the methodology used in this study.

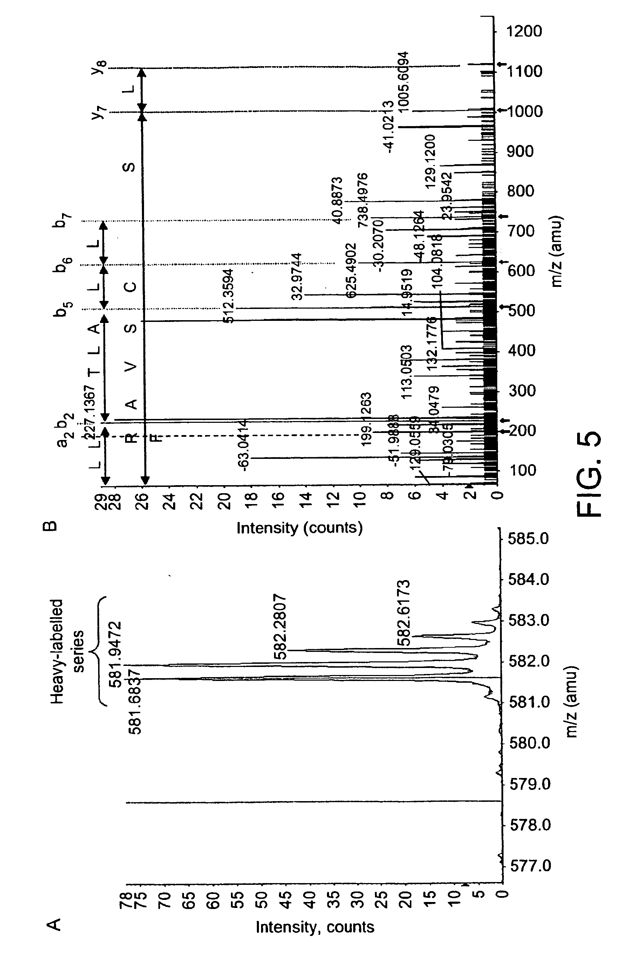

Despite these limitations, some instances of differential protein expression was noted. One such example is given in Figure 5, which shows very significant enhancement of expression of a protein, identified as glutamate receptor subunit zeta 1 precursor [SEQ ID NO. 26], in all three secretory samples. The triply charged series of peaks, starting at 581.6 Th, was MS/MS-analyzed and identified as the heavy-labeled version of the tryptic peptide LLTLALLFSCSVAR [SEQ ID NO. 28], which maps to the N-terminal region of the protein. The absence ofthe light-labeled analogue of this protein, which would have manifested as a series ofpeaks starting at 578.6 Th, suggests a significantly lower level of the same protein in the proliferative samples. This is the first evidence of this protein being a marker for the endometrial secretory phase.

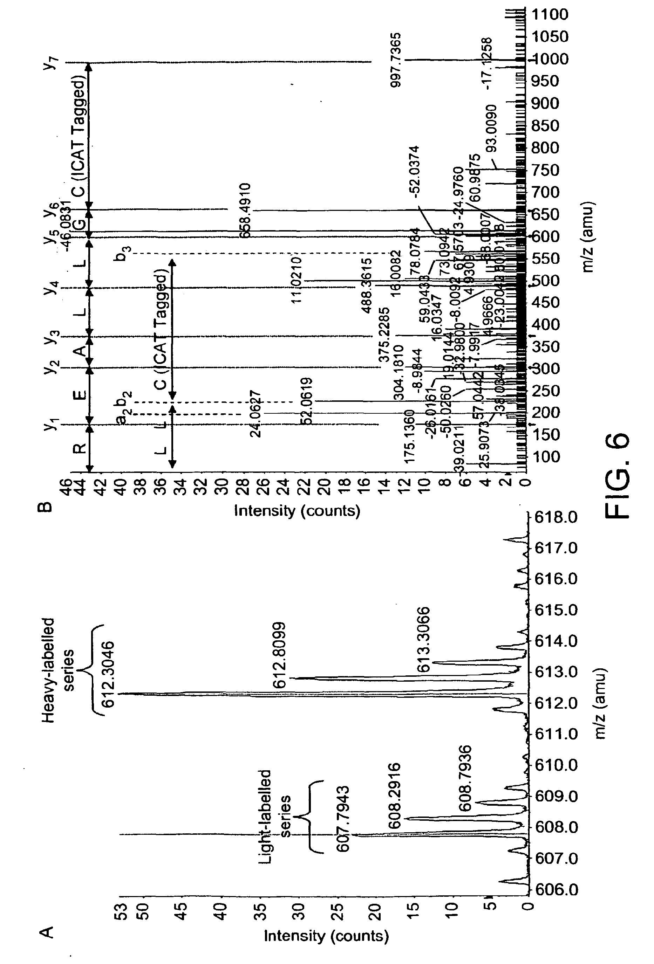

A second example is shown in Figure 6 for the protein, macrophage migration inhibitory factor (MIF). Again, the protein's expression is enhanced in the secretory endometrium (1.71 ± 0.38 times) versus the proliferative endometrium. This relative quantification is based on the three sets of samples and is calculated from the ratios of the total area of the ion peaks within the heavy-labeled series to that ofthe corresponding light-labeled series. Previous studies have demonstrated that MIF is expressed by the human endometrium throughout the menstrual cycle and that this expression is predominantly in the glandular epithelial cells [9]. It was found that MIF localized throughout the glandular epithelial cytoplasm in the proliferative phase, but that this distribution changed during the secretory phase, when it localized to the apical portion of the glandular epithelial cells, and was also detected in glandular secretions. Macrophages are common in female reproductive tissues. In the endometrium, they play an important role in defense. Macrophage degradation of cellular debris and foreign material may play an important role in endometrial shedding and repair. Quantification of MIF levels using ELISA assays suggested a slight increase in the mean concentration in the secretory phase (from approximately 15 to 18 ng / mg of protein based on a total of 25 samples), although the increase was not statistically significant [9]. By contrast, ICAT analysis of the six-sample set shows a significant enhancement (1.71 ± 0.38 times) of MIF expression in the secretory endometrium. The results of this study serve to illustrate the power of the ICAT method for detecting and quantifying gross as well as subtle differences in expression levels over traditional quantitative methods relied on thus far.

### CONCLUSIONS

The ICAT technology employing the new, cleavable ICAT reagent is a powerful tool that can be used for discovering differentially expressed proteins, which could potentially be significant biomarkers of different histological cell states. The results demonstrate that for the human endometrium the expression levels of the majority of proteins do not vary significantly between the proliferative and the secretory phases. Two examples of differentially expressed proteins have been discussed in this study. Further investigation of the remainder of the SCX fractions from each of the three pairs of samples can be expected to yield more markers some of which might have implications for fertility/ infertility.

### Example 2

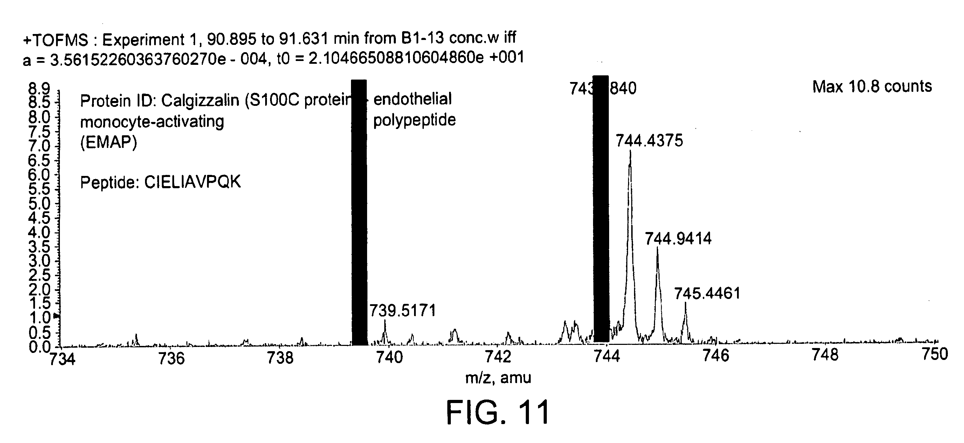

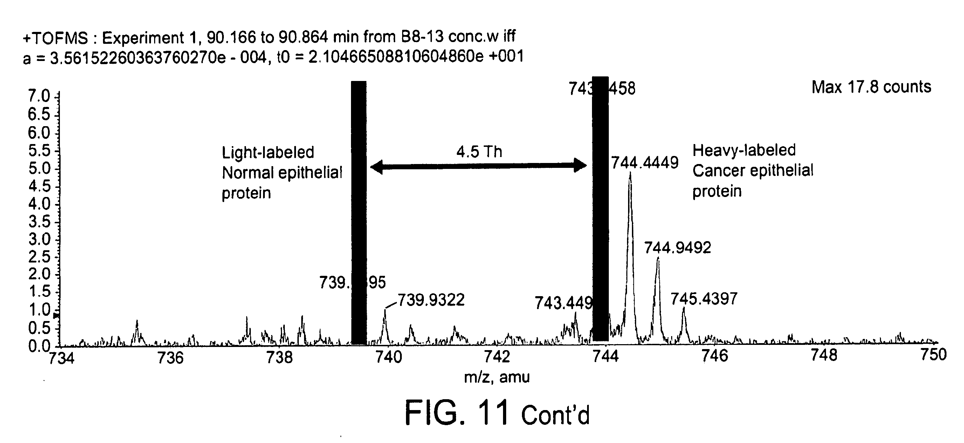

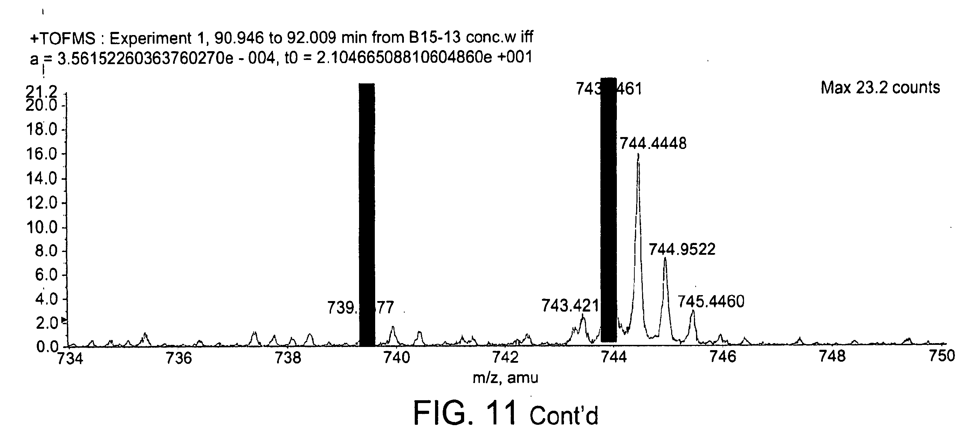

The ICAT analysis as described in Example 1 was used to examine differentially expressed proteins in cancer and normal endometrial tissue. The results of the anaylsis are illustrated in Figure 11 which shows mass spectral windows from the ICAT experiments for three pairs of endometrial cancer/ normal samples, demonstrating the over expression of calgizzarin in the cancer samples.

### Example 3

### Material and Methods:

### i) Tissue preparation and histologic classification

Endometrium and endometrial cancer tissues were retrieved from a dedicated, research in-house endometrial tissue bank. The consenting and tissue banking procedures for this tissue bank were approved by the relevant institutions. All tissues had been snap frozen in liquid nitrogen within 15-20 minutes of devitalization at the time of hysterectomy, and were obtained with patient consent. In each case, the endometrium was classified as non-malignant, or malignant by a pathologist. (26). Non-malignant endometrial cases included both normal physiologic states (atrophic, proliferative, secretory, menstrual) and pathologic states (benign endometrial polyp, disordered proliferative). Malignant endometrial cases included endometrioid, mucinous, and serous adenocarcinomas and malignant mixed Mullerian tumors (carcinosarcomas). This classification was performed using the routine surgical pathology sections. Study cases included only benign or malignant cases; cases of endometrial hyperplasia, some of which could be considered to represent an intermediate phenotype, were not included in this study. The histologic classification was verified by examination of a histopathologic section from the frozen research tissue. Tissue was taken for proteomic analysis from the mirror-face of the residual block.

Tissue was thawed in Hanks' balanced salt solution (HBSS, Sigma) containing protease inhibitors (leupeptine, aprotinin, pepstatin in 1 µg/mL) and followed by mechanical homogenation. The specimens were then stored in aliquots at-80°C and/or submitted for protein profiling. These whole tissue homogenates contain both endometrial epithelium or carcinoma, supportive stroma and vessels, and any secretions.

Immunohistochemical staining of selected malignant endometrial tissue was done using a polyclonal (rabbit) antibody against the putative tumor marker available from Calbiochem (San Diego, CA). Sections were cut from the paraffin embedded tissue, antibody applied in a 1:2000 dilution in Universal Strepavidin System, and immunohistochemical completed using a diaminobenzidine (DAB) chromogen.

### ii) Protein profiling

Tissue lysate was fractionated to reduce the sample complexity before protein profiling. An identical quantity ofproteins was used for all samples within a method; HBSS was used to compensate the initial volume to ensure equal volumes for all samples. For C18 Zip-tip (Millipore) fractionation, 2 µg of proteins from endometrial tissue homogenate in the presence of 0.3% trifluroacetic acid (TFA) were loaded. After washing with water containing 0.3% TFA, 1 µL of 60% acetonitrile with 0.3% TFA were used to elute proteins from C18 directly onto a MALDI target containing pre-dried 1 µL 10 mg/mL sinapinic acid in 60% acetonitrile. The dried protein spots were analyzed by a MALDI-TOF (Voyager DE-STR, Applied Biosystems) mass spectrometer.

For protein profiling using SELDI-TOF MS, 1 µg proteins from endometrial tissue homogenate were incubated with WCX2, SAX2, IMAC, H50 surfaces according to the manufacturer's instructions. In brief, samples were diluted to 55 µL with the corresponding binding buffer, spotted onto the appropriate ProteinChip surface, and incubated in a sealed BioProcessor for one hour at room temperature. The ProteinChip surface was washed twice with the appropriate buffer for five-minutes, briefly rinsed with water and air-dried. Two times 0.5 µL of 50% saturated sinapinic acid in 50% acetonitrile was applied on the samples to form crystals. The ProteinChips were analyzed using a linear TOF analyzer, PBSIIc (Protein Biology System IIc, Ciphergen), or a quadrupole/TOF hybrid tandem mass spectrometer, QSTAR XL (Applied Biosystems/MDS Sciex).

### iii) Protein purification and identification

A normal and an EmCa sample were subject to chromatographic separation in parallel to yield partially purified protein for identification. 500 µg proteins from the whole tissue homogenate were fractionated using size exclusion (BioSep 2000, Phenomenex) at 1mL/min flow with phosphate buffer (pH7.9) and 0.05% (w/v) sodium azide. One-millilitre fractions were collected; the eluates were then concentrated to 50 µL with a silicon carbide-based spincolumn, (ProteoSpin, MDS Sciex). Five microlitres (10%) of concentrate was desalted by C18 zip-tip and analyzed with MALDI-TOF MS to locate the fractions containing the protein marker of interest (10,843 Da) in the EmCa sample. The fractions with the enriched 10,843 Da protein were diluted to 100 µL with freshly prepared dithiothreitol (DTT) (5 mM final) in 150 mM Tris pH 8.5 buffer, and incubated at 60 Cfor one hour. Ten microlitres (10%) of the reaction mixture was desalted by C18 zip-tip and analyzed with MALDI-TOF MS to assess the effect of DTT on the protein of interest (see Result for detail). The remaining 90 µL was precipitated by acetone (80%(v/v) final), resuspended in SDS sample buffer, and the proteins were resolved by sodium dodecyl sulfate polyacrylamide gel electrophoresis (SDS-PAGE). Protein molecular weight markers (New England Biolabs) and cytochrome C (C-2506 Sigma) were included to guide the excision of gel portions containing the protein of interest. Intact proteins were extracted from the gel by 50 µL extraction solution (fonnic acid/acetonitrile/isopropanol/water in a ratio of 50/25/15/10) at room temperature for four hours. The extracts were completely dried by SpeedVac and resuspended in 40 µL 100 mM ammonium bicarbonate. Half of the resuspended proteins was desalted by C18 zip-tip and analyzed with MALDI-TOF, the other half was digested in solution with 100 ng trypsin (Promega). The resulted tryptic peptides were analyzed by MALDI-QqTOF MS. The identity of the 10,843Da protein, chaperonin 10, was determined using amino acid sequence-tag analysis (Mascot, Matrix Science).

Identification of chaperonin 10 was verified by western blot, 12 µg of proteins was resolved by 8-16% gradient SDS-PAGE and electrophoretically transferred to nitrocellulose membrane (MSI). Membranes were incubated for two hours with 1:1000 dilution of anti-chaperonin 10 antiserum (Stressgen) or ERKI antibodies (Santa Cruz) diluted in 5% milk with 0.2% NP-40 and one hour with secondary antibodies. Chaperonin 10 and ERK1 were detected by chemiluminescence reagent (NEN) and X-ray film according to the manufactures's instructions.

### Results:

A total of 44 malignant and non-malignant endometrial tissue samples were submitted for proteomic analysis. Twenty-three of these cases were non-malignant, and the remaining 21 cases were malignant. The exact histopathologic diagnoses are shown in Tables 2 and 3.

### Protein profiling

Both C18 zip-tip purification and retentive separation on ProteinChip WCX2 were effective in generating protein profiles that permit differentiation between normal and tumorous epithelium samples. Distinguishing features include both appearances and disappearances of proteins. One protein at approximately 10,840 ± 10 Da was present in all EmCa samples and absent or greatly diminished in all normal samples tested. Figure 7 shows the mass spectra obtained on the ProteinChip WCX2 using (a) the linear TOF mass spectrometer, PBSIIc, and (b) the QqTOF mass spectrometer, QSTAR XL. The superior mass accuracy and resolution of the latter afforded datennination of the molecular weight of the marker protein as 10,843 Da.

### Marker protein purification and identification

As detailed above and outlined in Figure 8 size-exclusion LC was employed to fractionate the tissue homogeneates. The target 10,843 Da protein was found to elute in one ofthe early fractions from size exclusion column which suggests that this protein is a part of a large protein complex (Figure 8A). After concentration on the spin column, the proteins were treated with DTT to break the tertiary structure and reduce any disulfide bonds. The molecular weight ofthe target protein was verified to remain as 10,843 Da after DTT treatment and before SDS-PAGE (Figure 8B). This result suggests that the 10,843 Da target protein contains no intra- or inter-polypeptide disulfide bonds. The remaining protein concentrates were further separated by SDS-PAGE, and the gel was stained with colloidal Coomassie Blue (Figure 8C). There were, however, no visible bands at the region around 10-11 kDa, probably a consequence of low protein concentration and relatively low sensitivity of the Coomassie stain. The gel portions covering approximate 7,000-16,000 Da were excised as guided by the molecular weight markers (Figure 8C). After protein extraction, digestion with trypsin and MALDI analysis, six "unique" tryptic peptides were detected in the EmCa sample vs. the control (Figure 8D). All six peptides were sequenced by MALDI-QqTOF MS; three were traced to keratin and three to chaperonin 10 (Figure 8D and 8E). The average molecular weight of chaperonin 10 was calculated to be 10842.5 Da after considering two putative posttranslational modifications, the removal of the N-terminal methionine and acetylation of the alanine residue. This is consistent with the measurement of the target protein and the reported molecular weight of chaperonin 10 purified from human platelet (18).

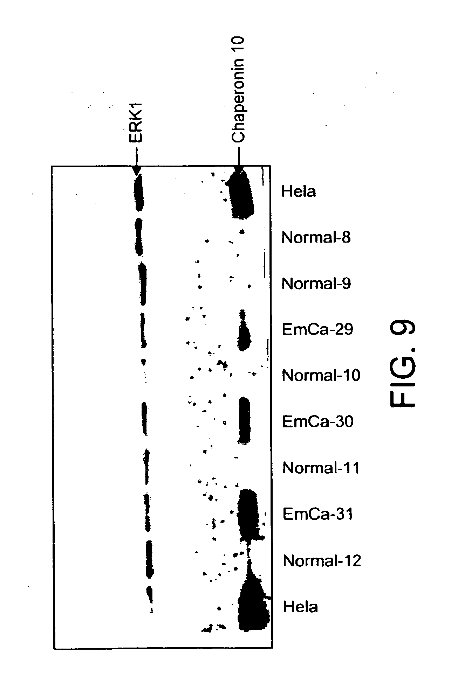

The differential expression of chaperonin 10 among cancerous and normal tissue was re-tested by western blot analysis. The signal of chaperonin 10 is higher in all EmCa specimens that also display a relative high 10,843 Da peak in their corresponding protein profiles (Figure 9).

Tables 2 and 3 summarizes the results in identifying chaperonin 10 by MS and western blot analyses in non-malignant and malignant endometrial tissue respectively. The results for both MS and western blotting have been reported using a semi-quantitative system ranging from absent (0) to 5+ (for high intensity). These two independent methodologies demonstrate consistency in the detection of chaperonin 10.

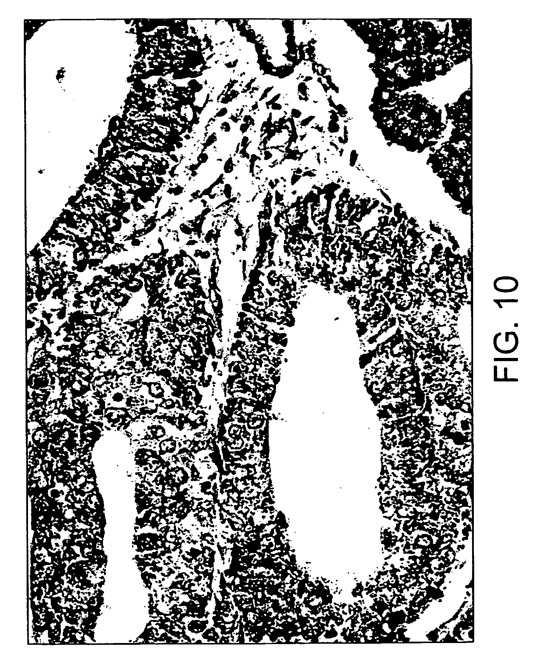

Moderately strong immunohistochemical staining for chaperonin 10 was noted in the cytoplasm of an endometrioid adenocarcinoma, as compared to adjacent stroma and benign endometrial gland. (Figure 10). This result demonstrates the association of chaperonin 10 with malignant endometrial tissues, but much less with normal endometrial epithelium or supportive stroma and vessels, which were also present in the whole tissue homogeneates.

### Discussion:

Genomic studies of endometrial carcinoma have revealed that there are two, and possibly three, types of endometrial carcinoma that are each characterized by multistep pathogenetic pathways characterized by different molecular profiles [28]. A wide variety of genetic and enzymatic markers characterize the initiation, promotion and progression toward each type of endometrial carcinoma. Large-scale messenger RNA expression analysis of the endometrioid type of endometrial carcinoma has identified 50 genes that are capable of discriminating normal from malignant endometrial tissues [29]. Many genes that are constitutively expressed in normal endometrium show either diminished or increased expression in endometrial carcinomas. In addition, there is aberrant expression of the one hundred hormonally regulated genes that are variably expressed in normal endometrial tissues, although finally endometrial carcinoma resembles proliferative endometrium more than secretory endometrium [29].

In contrast to the genomic studies of the endometrium, no studies of the endometrial proteome are available, even though proteome analysis may offer information about protein expression, functions, and modifications which might not be fully reflected by gene expression analysis. Thus, protein expression profiling of the endometrium offers a new opportunity to identify and classify endometrial phenotypes, including carcinoma. Among the available methodologies for protein profiling, SELDI-TOF based method has the advantage of requiring only miniscule samples and high throughput capability, although the determination ofany protein identity requires much more work [30].

This proteomic study of endometrium used lysates of whole tissue homogenates from both control endometrial tissues and endometrial carcinomas. Such tissues include not only the epithelial cells of interest, but also supportive stroma (including both endometrial stromal cells or fibroblasts and extracellular matrix), blood vessels (including smooth muscle cells and endothelium), any secretions, and possibly small amounts of adjacent myometrium. While the use of such whole tissue homogenates is technically straight forward, it does have inherent limitations. The heterogeneous nature of the constituent tissue will lead to a similarly heterogeneous protein profiling [17]. The resultant proteomic analysis reflects not only the cells of interest, but also the presence of contaminating cells [15,17,31]. Successful proteomic analysis of some types of tumors (e.g. hepatoma) may still be productive even with the limitation of whole tissue homogenization, since there is an abundance of tumor cells and minimal associated contaminating stroma [32].

In some tumor types, the inherent limitation of whole tissue homogenates must be surmounted by utilizing cell purification techniques. In the endometrium LCM may be optimal in achieving cellular purification for proteomic analysis since there is a relative abundance of stroma in endometrial carcinoma tissues [32]. Studies using laser capture microdissection (LCM) of melanoma have clearly revealed a different protein profile for melanoma than that of the surrounding epithelium, and thus supports the use of LCM in proteomic profiling studies [21, 33-35].

Despite the limitations of using whole tissue homogenates for protein expression profiling, this preliminary study of endometrial carcinoma has shown that proteomic analysis of endometrial carcinoma can detect differences from that of normal endometrium. Furthermore, a specific protein (Chaperonin 10) was strongly associated with endometrial carcinoma cases. This potential marker might be clinically useful on tissue aspirates since its differential expression pattern can be detected without the LCM procedure.

Chaperonin 10 was not identified exclusively in malignant endometrial tissues; low levels were detected in non-malignant endometrial tissues by both mass spectrometry and western blotting techniques (Table 2). Furthermore, there is no apparent association between any particular histopathologic classification and the detection of these low levels of chaperonin 10. In contrast, high levels ofchaperonin 10 were detected in 17 of 22 malignant endometrial tissues by either mass spectrometry and/or western blotting techniques (Table 3). The apparent absence of chaperonin 10 in the remaining five of the 22 malignant cases may be due to either true absence or technical factors in pre-analytic processing or proteomic analysis. In two of these five cases, re-examination of the corresponding mirror image histologic section revealed minimal tumor in one case (case 28), or abundant necrosis of tumor (case 44). Furthermore, specific protein peaks of interest may be obscured in less-than-optimal mass spectromectric analysis or by adjacent protein peaks.

Chaperonin 10 (Cpn 10) is a heat shock protein (HSP) that functions intra-cellularly as a molecular chaperone for nascent proteins [36,37]. HSP's are ubiquitous intra-cellular proteins that ensure homeostasis of metabolism [37]. Aberrations of HSP function, including chaperonin 10, have been described in a variety of pathologic conditions, including neoplasia [37,38]. Furthermore, HSP's are differentially expressed in a variety of neoplasms. For example, immunohistochemical studies of both primary and secondary brain tumours have shown production of other HSP's [39], and that the expression of some HSP's may depend upon proliferating potential. Furthermore, the modulation of HSP expression profile has been shown to reflect the stage ofprostatic carcinoma [37].

Immunohistochemical studies have identified HSP's within the nuclei and cytoplasm of epithelium, stroma, endothelium, and lymphocytes of the endometrium, with certain types of HSP's showing preferential localization to certain cell types [40]. Whereas the expression of some HSP's occurs independent of the stage of the endometrial cycle (e.g. HSP90), the expression of other HSP's is cycle dependent. The expression of chaperonin 10 can be determined among proliferative, secretory, and menstrual endometria using proteomic, western blotting, and immunohistochemical methods. It is known that the amount of HSP27 and 60 is increased during late proliferative and early secretory phases, and subsequently reduced during mid- and late secretory and menstrual phases [40]. Studies of both decidualized endometrium (decidua) and placenta have show that there are striking differences in the cellular localization of HSP's during normal human gestation.

There have been no studies regarding the role ofHSP's or Cpn10 in endometrial carcinoma. The eutopic endometrial glands from women with endometriosis and adenomyosis shows significantly increased expression of HSP's as compared to endometria from control women - regardless of the menstrual phase [41]. The abnormal expression of HSP's may play a role in the pathophysiology of both endometriosis and adenomyosis.

Early pregnancy factor (EPF) is an extra-cellular homologue of Cpn 10 that appears within 24 hours of fertilization and persists throughout the first half of gestation [36]. It is necessary for embryonic development [42-44] and is immunosuppressive [45]. EPF is also detectable in animal models of liver regeneration and in the development of cancer [38,46,47]. An association between cellular growth and the appearance of extracellular EPF has been shown [38, 46,47]. These findings suggest a role for EPF in neoplastic growth and that the detection of Cpn 10 in the serum of endometrial carcinoma has diagnostic potential.

In conclusion, this protein profiling study of non-malignant and malignant endometrial tissues has identified chaperonin 10 as a tumor marker for endometrial malignancies.

### Example 4

The work described in the previous example using solid-phase extraction followed by matrix-assisted laser desorption/ionization mass spectrometry (MALDI MS) as well as selective surface binding and surface-enhanced laser desorption/ionization (SELDI MS, Ciphergen Biosystems Inc, CA, USA) has succeeded in identifying individual markers that show significant enhanced expression in EmCa tissues. The MALDI/SELDI MS strategy relies on side-by-side comparison of spectra from proteins that have been selected via fast separation [120-124]. Using this methodology the identities of the proteins are unknown and identifying a given protein marker typically involves offline multidimensional chromatography, concentration, trypsin digestion and MS/MS. A second available strategy that highlights differentially expressed proteins involves differential tagging of proteins from samples that are being compared using isotope-coded affinity tag (ICAT) in an isotope-dilution mass spectrometry experiment [6]. This strategy has recently been applied to discover differentially expressed proteins between the proliferative and secretory phases of the human endometrium [49] using a cleavable, second-generation ICAT reagent (cICAT, Applied Biosystems Inc, CA, USA) (see above). Recently, a variation of the ICAT technology, iTRAQ (also from the Applied Biosystems Inc, CA, USA), has been introduced. Both cICAT and iTRAQ tagging permit online identification of multiple markers and relative quantification of these proteins. Although similar in their basic concepts, the two tagging reagents and methodologies differ in significant areas. The cICAT method relies on tagging cysteine residues and isolating peptides containing these tagged residues by affinity chromatography. The net result is a reduction in the complexity of peptide pools generated by digestion with proteases including trypsin [6]. In the case of the new iTRAQ method, tagging is on primary amines. This difference in labeling strategy eliminates the dependence on relatively nonabundant cysteine containing peptides intrinsic to ICAT-based methods, thus potentially allowing the tagging of most tryptic peptides. Other noteworthy features of the iTRAQ technology are that relative quantification is performed via MS/MS and that there are four possible tags, which permit multiplexing of up to four samples (tissue states) in a single experiment. Quantification is performed via the differences in abundances of four product ions, 114, 115, 116 and 117 Th that are each cleaved from one of the four possible tags. The tags have an identical mass, a result of differences in other parts of the iTRAQ tag structure, with the consequence that an identical peptide in the four samples will have an identical mass and LC retention time after tagging. This strategy simplifies analysis and will potentially increase analytical accuracy and precision. The multi-sample capability of the iTRAQ technology is ideally suited for this study, as it now provides a means to perform a proteomic analysis of both the major phases of the normal endometrium, while simultaneously comparing them against cancer samples.

This example describes a feasibility study that compares protein expression profiles between normal and cancerous endometria using iTRAQ as well as contrasts it against a similar feasibility study using the cICAT technology. Both iTRAQ and cICAT labeling afforded discovery of a number of differentially expressed proteins that are potential cancer markers (PCMs). There is little overlap in the PCMs discovered and identified, thus pointing to the complementary nature of the two technologies. It is noteworthy that application of the iTRAQ methodology permits confirmation of the overexpression of chaperon in 10 in EmCa tissues [50]; chaperonin 10 is an approximately 10 kDa heat shock protein that does not contain the cysteine residue, which is the tagging site for cICAT. Thus, in addition to an interest in the discovery of markers for endometrial carcinoma, these experiments have also helped to illustrate the relative strengths and differences of the two tagging techniques, when applied to studies of clinical samples.

### Materials and Methods

***Sample Preparation.*** Endometrial tissue was retrieved from an in-house dedicated, research endometrial tissue bank. All tissues were snap frozen in liquid nitrogen within 15-20 minutes of devitalization at the time of hysterectomy, and were obtained with patient consent. The patient consent forms and tissue-banking procedures were approved by the Research Ethics Boards of York University, Mount Sinai Hospital, University Health Network, and North York General Hospital. In each case, the endometrium was classified by a pathologist (TJC). Histological classification was verified by examination of a section from the frozen research tissue. Tissue for proteomic analysis was taken from the mirror-face of the residual block. In the case of samples used for iTRAQ analysis, 0.5 ml phosphate buffered saline (PBS) containing protease inhibitors (1mM AEBSF, 10 µM leupeptin, 1 µg/ml aprotinin and 1 µM pepstatin) was added. The tissue was then mechanically homogenized at 30,000 rpm using a Polytron PT 1300D handheld homogenizer (Brinkmann, Westbury, USA). For samples used for cICAT analysis, tissues were similarly homogenized in 1 ml Hanks' Balanced Salt Solution with the same concentration of protease inhibitors as listed above. The samples were stored in aliquots at -70°C until used for further processing. These whole tissue homogenates contained not only endometrial epithelium, but supportive stroma and vessels, as well as secretions. The iTRAQ analysis involved one normal proliferative, one normal secretory, and two cancer homogenates, while the cICAT analysis combined one normal proliferative homogenate with three different cancer homogenates in pair-wise comparisons.
***Chemicals.*** Reagent grade chemicals were purchased from Sigma Aldrich (Oakville, ON, Canada), or Fisher Scientific (Nepean, ON, Canada). All iTRAQ and cICAT reagents and buffers were obtained from Applied Biosystems (Foster City, CA, USA).
***iTRAQ Sample Preparation Procedure.*** Cell debris from each of the homogenates was removed by centrifugation in a microfuge at 4 °C for 30 min at 14,000 rpm. The clarified supernatant was then transferred to fresh microfuge tubes and the total protein content determined using a commercial Bradford assay reagent (Bio-Rad, Mississauga, ON, Canada). A standard curve for the Bradford assay was made using γ-globulin as a control. 100 µg of each sample was then denatured and the cysteines blocked as described in the iTRAQ protocol (Applied Biosystems, Foster City, CA, USA). Each sample was then digested with 0.2 mL of a 50 µg/mL trypsin (Promega) solution at 37 °C overnight and labeled with the iTRAQ tags as follows: normal proliferative endometrium, iTRAQ 114; normal secretory endometrium, iTRAQ115; and the two EmCa samples, iTRAQ 116 and iTRAQ117. The labeled samples were then pooled and acidified by mixing with Eluent A (see later) to a total volume of 2.0 mL for strong cation exchange (SCX) chromatography. 1.5 mL of this acidified labeled sample was injected into an HP1050 LC system (Agilent, Palo Alto, CA, USA) with a 1.5 mL injection loop and a 2.1 mm internal diameter (ID) x 100 mm length PolyLC Polysulfoethyl A column packed with 5 µm beads with 300 Å pores (The Nest Group, Southborough, MA, USA). A 2.1 mm ID x 10 mm length guard column of the same material was plumbed upstream from the analytical column. Fractionation was effected by a binary mobile-phase gradient at a total flow rate of 0.2 mL/min. Eluent A consisted of a 10 mM KH₂PO₄ solution in 25% acetonitrile and 75% deionized water acidified to a pH of 3.0 with phosphoric acid. Eluent B consisted of a 10 mM KH₂PO₄ and 350 mM KCl solution in 25% acetonitrile and 75% deionized water acidified to a pH of 3.0 with phosphoric acid. Initially, the gradient comprised 100% Eluent A. At the 2^{nd} minute, the % Eluent B was changed linearly from 0 to 100% at the 58^{th} min. The run was terminated at the 60^{th} min. A total of 30 fractions were collected for the sample, one every two minutes, using an SF-2120 Super Fraction Collector (Advantec MFS, Dublin, CA, USA). Following fractionation, the samples were dried by speed vacuuming and stored at -20 °C. Prior to reverse phase nanobore liquid chromatography-tandem mass spectrometric (nanoLC MS/MS) analysis, these fractions were redissolved in an aqueous buffer containing 5% acetonitrile and 0.1% formic acid for nanoLC MS/MS.
***cICAT sample Preparation Procedure.*** Samples were clarified and their total protein content determined as described above. cICAT sample preparation procedure was carried out according to the cleavable ICAT protocol (Applied Biosystems, Foster City, CA, USA). Three 100 µg aliquots of the clarified normal proliferative homogenate were paired with 100 µg each of the three clarified cancer homogenates separately. After denaturing and reduction, the normal homogenates were labeled with the light reagent while the EmCa homogenates were labeled with the heavy reagent. A light labeled sample was then mixed with one of the heavy labeled samples to form in total three ICAT sample pairs: A, B and C. The final volume of each sample was 0.2 mL. The sample pairs were then digested by incubating each pair with 0.2 mL of a 50 µg/mL trypsin solution at 37°C overnight Afterwards each sample pair was mixed with 2.0 mL of Eluent A and fractionated into 30 fractions using SCX chromatography as described above. The fractions were screened by MALDI MS analysis. Those showing the signature ICAT peak pairs separated by 9 Da were further processed by affinity purification using the avidin cartridge provided with the ICAT kit. The affinity-purified sample was then dried, treated with the cleavage reagent to eliminate biotin, and dried again by speed vacuuming. The resulting solids were re-dissolved in an aqueous buffer containing 5% acetonitrile and 0.1% formic acid for nanoLC MS/MS.
***Nanobore LC*/*MS*/*MS.*** The nanobore LC system was from LC Packings (Amsterdam, The Netherlands) and consisted of a Famos autosampler and an Ultimate Nano LC system. It was interfaced to an API QSTAR Pulsar QqTOF mass spectrometer (Applied Biosystems/MDS Sciex, Foster City, CA, USA) using a Protana NanoES ion source (Protana Engineering A/S, Odense, Denmark). The spray capillary was a PicoTip SilicaTip emitter with a 10 µm ID tip (New Objective, Woburn, MA, USA). The nanobore LC column was a 75 µm ID x 150 mm length reverse-phase PepMap C 18 nano capillary column (LC Packings, Amsterdam, The Netherlands) packed with 3 µm beads with 100 Å pores. One µL of sample was injected via the full-loop mode. Separation was performed using a binary mobile-phase gradient at a total flow rate of 200 nL/min. Eluent A consisted of 94.9% deionized water, 5.0% acetonitrile and 0.1 % formic acid (pH ≈ 3). Eluent B consisted of 5.0% deionized water, 94.9% acetonitrile and 0.1% formic acid.

The following binary gradient was used for the iTRAQ experiments:

| | | | | | | | | | |
|---|---|---|---|---|---|---|---|---|---|
| *Time (min)* | 0 | 5 | 10 | 125 | 135 | 155 | 160 | 162 | 188 |
| *% Eluent B* | 5 | 5 | 15 | 35 | 60 | 80 | 80 | 5 | Stop |

While the binary gradient used for cICAT experiments was:

| | | | | | | | | |
|---|---|---|---|---|---|---|---|---|
| *Time (min)* | 0 | 5 | 125 | 135 | 157 | 160 | 163 | 190 |
| % *Eluent B* | 5 | 5 | 30 | 60 | 80 | 80 | 5 | Stop |

For nanospray, the source conditions were a curtain-gas setting of 20 and an ionspray voltage in the range of 1800 - 3000 V that was optimized daily. In the Q0 region, the instrument parameters were a declustering potential (DP) of 65 V and a focusing potential (FP) of 265 V. Nitrogen was used as the collision gas at a setting of CAD = 5 for both TOF-MS and MS/MS scans. All nanoLC MS/MS data were acquired in information-dependent acquisition (IDA) mode in Analyst QS SP8 with Bioanalyst Extension 1.1 (Applied Biosystems / MDS Sciex). Two sets of runs for the iTRAQ fractions were performed. For the first set, MS cycles comprised a TOF MS survey scan with an m/z range of 400 -1500 Th for 2 s, followed by three product ion scans with an m/z range of 70-2000 Th for 10 s each. For the second, a 1 s TOF MS survey scan, followed by three product ion scans of 3 s each were used. For cICAT experiments, the MS cycles consisted of a TOF MS survey scan for 1 s, followed by two product ion scans of 2 s each. The ranges for the TOF MS and product ion scans were the same as those of the iTRAQ experiments. Collision energy (CE) was automatically controlled by the IDA CE Parameters script. Switching criteria were set to ions greater than m/z = 400 Th and smaller than m/z = 1500 Th with a charge state of 2 to 5 and an abundance of ≥ 10 counts/s. Former target ions were excluded for 60 s and ions within a 4 Th window were ignored. In addition, for iTRAQ runs, the IDA Extensions II script was set to no repetitions before dynamic exclusion for the first run, and one repetition for the second. While for the ICAT experiments, the IDA Extensions II script was set to two repetitions before dynamic exclusion. In all three experiments, the script was set to select a precursor ion nearest to a threshold of 15 count/s every 4 cycles. These settings ensured examination of not only high abundance ions, but low abundance ones as well.
***Data Analysis**.* Data analysis for the iTRAQ experiments was performed with ProQUANT 1.0, while that for cICAT experiments was with ProICAT 1.0 SP3. The cut off for the confidence settings for both analyses was at 75; that for the score was at 20. The tolerance set for peptide identification in ProQUANT searches were 0.1 Da for MS and 0.1 Da for MS/MS, while those for ProICAT searches were, 0.2 Da and 0.1 Da, respectively. All identifications were manually inspected for correctness. Relative quantification of proteins in the case of iTRAQ is performed on the MS/MS scans and is the ratio of the areas under the peaks at 114 Da, 115 Da, 116 Da and 117 Da which are the masses of the tags that correspond to the iTRAQ reagents. Relative quantification of proteins in the case of cICAT was performed on the TOF-MS scans by calculating the relative areas between the pairs of light and heavy label series of peaks. In both cases, the results of the quantification were normalized using the overall ratio obtained for all tagged peptide pairs in the sample. On the basis of previous studies utilizing cICAT reagents to compare secretory to proliferative endometrium, which involved paired tissue analysis of samples labeled with heavy and light isotopes, it was found that many proteins were expressed within a standard deviation (SD) of < 0.3 and an expression ratio close to 1.0, when protein expression was normalized against a housekeeping protein (n=6) [49]. Using this SD as an approximate benchmark for variation of protein expression among proteins whose expression is not altered when utilizing this technique, it was considered that a difference of 3 SDs i.e. 1.0 plus 0.9, or about a 2 fold difference, in normalized expression levels would be outside of the variation expected by chance, with a confidence limit of > 95% using a simple Gaussian approximation. This approximation would, therefore, apply to normalized expression levels > 2.0 or in reciprocal form < 0.5, as no assumption was made with regard to which tissue showed greater abundance. Using this approach, therefore, a two-fold expression change was chosen as an initial benchmark for potentially significant changes. In small sample sizes used for these initial studies, i.e. one sample in each group, at an alpha value of 0.05 (two-sided) and a beta value of 0.2 (80% power), the ratio of expression increase to SD must be at least 4 in order to achieve significance. This means that for minimal statistical significance to be achieved using the above alpha and beta parameters for a single paired-tissue comparison, a 2-fold mean increase in expression must be associated with an SD less than 0.5 for replicate measurements in order to satisfy the sample size equation [n = 2 SD²(Z_{α}+Z_{β})²/(expression increase)²] =1, when the power index (Z_{α}+Z_{β})² = 8 (α = 0.05, β = 0.2). For the sample size of three paired comparisons (one normal tissue and three tumor tissues were used in the cICAT experiment), the sample-size approximation of n = 2 in each group was used to compensate for the fact that one normal tissue was used (i.e. n = 1 in the control group, and n = 3 in the tumor group). The ratio of expression increase to SD must be greater than 2.8 in order to satisfy the above equation for an n = 2 (using the same α and β error limits of 0.05 and 0.2, respectively).

### Results

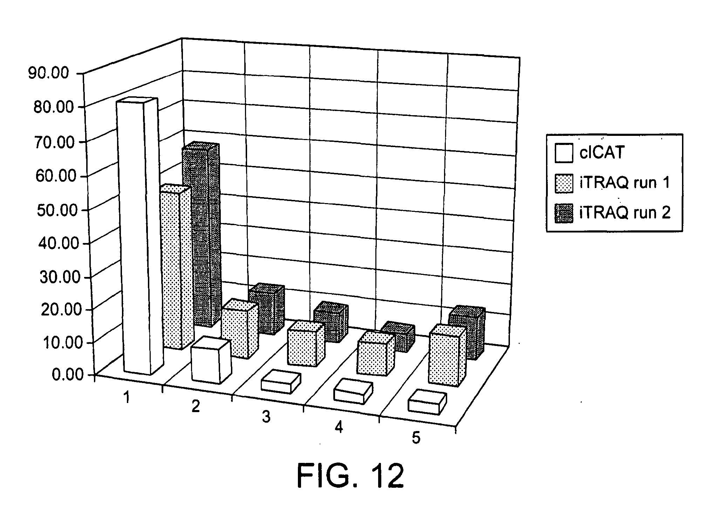

iTRAQ runs 1 and 2 led to sequencing and identification of 645 and 1,026 peptides, respectively. The previous studies (see above) showed that a confidence setting of 75 and a score setting of 20 were optimal. Manual inspection ofhundreds MS/MS spectra concluded that while these conditions were sufficiently stringent in that most identifications were correct, they also did not exclude too many peptides that could have been identified based on spectral quality. The MS/MS spectra of all peptides that scored above the cut offs were manually inspected to verify proper identifications. The shorter MS and MS/MS cycles used for run 2 resulted in a higher number of peptides identified. A significant number of these peptides were identified more than once, thus the number of unique peptides dropped to 292 and 312 for runs 1 and 2, respectively. Many of the more abundant proteins were identified by several peptides (Figure 12); the numbers of proteins identified were 101 and 126, respectively. 63 proteins were identified in both runs. The numbers are modest, which were likely due to the small amount of starting materials.. Five proteins have abundance ratios that show more than a two-fold change (≥ 2.0 or ≤ 0.5) in both cancer samples relative to the proliferative as well as the secretory endometrium, and meet the criteria of differential expression. These are shown in Table 4.

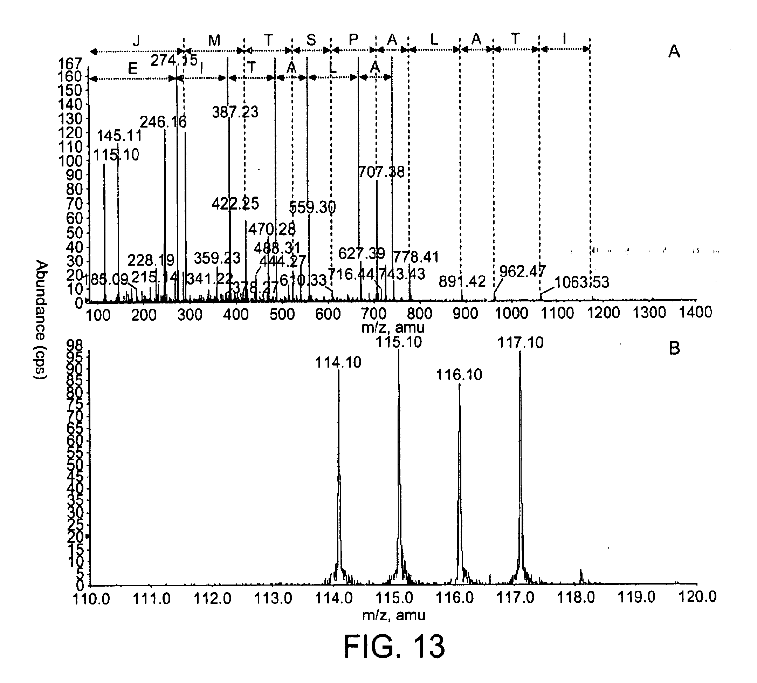

Relative quantification is expressed as three pair-wise ratios against the proliferative endometrium (iTRAQ114) for the secretory endometrium (iTRAQ115) and the two EmCa samples (iTRAQ116 and 117). To account for small differences in protein loadings across the samples, these ratios have been normalized using the overall ratios for all proteins in the samples, as recommended by Applied Biosystems. The rationale for this choice is based on the assumption that the relative abundances for the majority ofproteins are close to one. This assumption is exemplified in this study in terms of the abundance ratios in secretory versus proliferative endometrium for the following abundant proteins: cytoplasmic actin, 0.98; alpha enolase, 0.91; alpha filamin, 1.04; serum albumin precursor, 1.56; and tropomyosin alpha 4 chain, 0.99. Figure 13a shows the MS/MS spectrum for the doubly charged cytoplasmic actin peptide at 725.4 Th in one of the runs. The cluster of peaks around 115 Th is better shown in the mass spectral window 110 - 120 Th in Figure 13b, demonstrating near identical abundances in the four samples: proliferative (iTRAQ114), secretory (iTRAG115), EmCa 1 (iTRAQ116), and EmCa 2 (iTRAQ117).

Typical MS/MS windows (110 - 120 Th) for tryptic peptides in four differentially expressed proteins, (A) chaperonin 10, (B) alpha-1-antitrypsin precursor, (C) creatine kinase B, and (D) transgelin, are shown in Figure 14. Chaperonin 10 is overexpressed in both EmCa samples, whereas alpha-1-antitrypsin precursor, creatine kinase B, and transgelin are underexpressed. The remaining differentially expressed protein, pyruvate kinase M1 or M2 isozyme, is also overexpressed in the two EmCa samples.

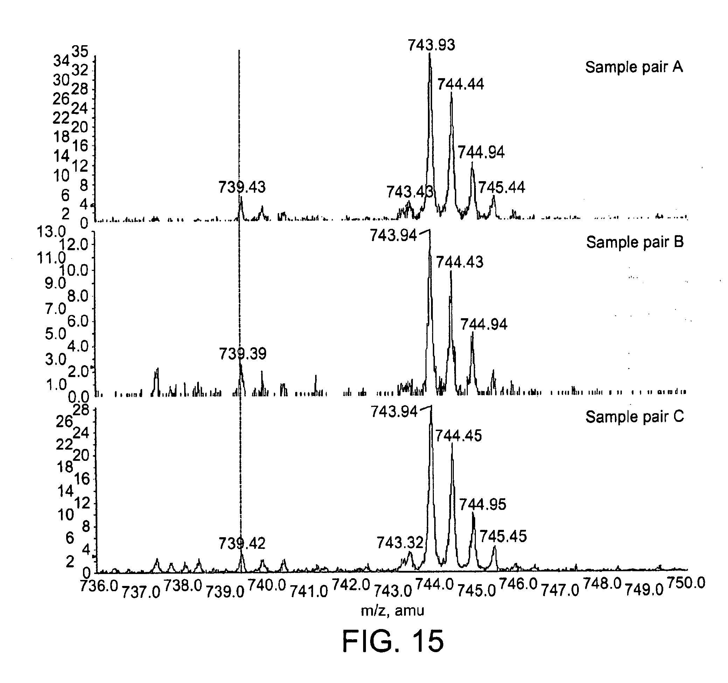

The cICAT experiments led to identification and quantification of 68 proteins, all were manually verified. Again, the modest number stemmed from the small sample size. Fewer proteins were identified by multiple peptides relative to iTRAQ (Figure 12), in accordance with the more selective nature of cICAT labeling on only cysteine residues. Five proteins that meet the two-fold differential expression criterion in all three EmCa versus proliferative endometrium pairs are: calgizzarin, heterogeneous nuclear ribonucleoprotein (hnRNP) D0, macrophage migration inhibitory factor (MIF), polymeric immunoglobulin receptor (PIGR) precursor, and pyruvate kinase M1 or M2 isozyme. These results are summarized in Table 5. All five proteins are overexpressed in EmCa tissues; pyruvate kinase is also shown to be overexpressed with iTRAQ. Figure 15 shows an example of overexpressed protein, calgizzarin A. Again, the relative abundance ratios are normalized to the overall ratio of all proteins in a given sample pair to account for small differences in protein loadings in the two samples. Cytoplasmic actin exhibits a heavy/light label ratio of 0.95 ± 0.24 (standard deviation of three samples).

### Discussion

The combination of iTRAQ and cICAT labeling results in the discovery and identification of nine differentially expressed proteins that are potential cancer markers (PCMs) for EmCa. Six of the nine PCMs are overexpressed, whereas three are underexpressed in EmCa.
***PCMs Overexpressed in EmCa.*** Chaperonin 10 is a heat shock protein that was identified as a PCM using MALDI/SELDI MS and identified by offline separation, preconcentration, trypsinization and MS/MS (see above). Overexpression of chaperonin 10 in EmCa tissues was verified independently by Western analysis; chaperonin 10 was localized to the cancerous epithelium by means of immunohistochemistry (IHC). Elevation of levels of chaperonin 10 has been associated with large bowel and cervical carcinomas [53]. The level of chaperonin 10 in serum was demonstrated to be an indicator of trophoblastic tumor [54]. The observation of chaperonin 10 overexpression in EmCa samples in this study with iTRAQ confirms the previous finding using MALDI/SELDI MS, Western analysis and IHC. Thus, chaperonin 10 may be a serum marker protein for EmCa.

Overexpression of pyruvate kinase MI or M2 isozyme was demonstrated by both the iTRAQ and cICAT applications. Pyruvate kinase was found to be expressed at elevated levels in both plasma and fecal samples in patients of gastrointestinal cancer [55,56]. The ability to detect pyruvate kinase in plasma samples is of particular interest as this might indicate that screening plasma for elevated pyruvate kinase levels in endometrial cancer cases may also be possible. An investigation into the activity of 12 enzymes related to the glycolytic pathway in cervical and endometrial cancers found that only two, pyruvate kinase and phosphoglucose isomerase, were significantly higher in both cancers [57].

Calgizzarin was found to be overexpressed in EmCa tissues using cICAT labeling. Calgizzarin was one of two proteins that exhibited significant upregulation in colorectal and lung carcinoma cell lines over normal colorectal mucosal cells [58]. Calgizzarin was one of three proteins that have been identified as tumor markers in mouse colon cancer [59]. The other two tumor markers were calgranulins A and B, the former of which was also identified as a protein marker in human endometrial carcinoma using SELDI MS. Identification was made possible by size-exclusion chromatography, trypsinization and online nanoLC MS/MS; confirmation of calgranulin A overexpression in EmCa was rendered by IHC in a tissue microarray format.

HnRNP D0, also known as AU-rich element RNA-binding protein I, binds to the AU rich 3' UTR of many proto-oncogenes. One study has shown that this protein is more abundant in murine neoplastic lung epithelial cell lines and that this abundance decreases when non-tumorigenic cells reach confluence or growth arrest [60]. Conversely, it was also found that the abundance was unaffected in spontaneous transformants from this cell line. Another more recent study using transgenic mice showed that overexpression in an isoform of this protein altered mRNA levels of several oncogenes, including c-myc, c-jun, c-fos and TNF-alpha [61]. The mouse line with the highest level of this isoform developed sarcomas [61].

Macrophage Migratory Inhibitory Factor is another protein that has been well documented as being involved with cancer [62]. This ranges from hepatocellular carcinomas [63], to non small cell lung cancer [64], to brain tumors [65] and gliblastomas [66].

PIGR has previously been detected in serum from patients with lung cancer and was shown to be significantly upregulated in the secretory component by an ELISA study involving 45 lung cancer patients compared with 45 control subjects [68]. Another study has also demonstrated a possible linkage between PIGR and bladder carcinoma with protein levels in serum being significantly increased in patients with transitional cell carcinoma [69].
***PCMs Underexpressed in EmCa.*** Alpha-1-antitrypsin precursor is one of the three proteins that are underexpressed in EmCa tissues. Recent studies have shown downregulation of alpha-1-antitrypsin to be associated with malignant lymphoma as well as liver, lung, stomach, bladder and gall bladder cancer [70,71]. There is also evidence for upregulation contributing to enhanced cell migration and metastases of human colon cells in a rat model [72]. Such a contradiction might be explained by the effect of alpha-1-antitrypsin being tissue specific which would prove useful for distinguishing between forms of cancer.

Creatine kinase B shown here as being underexpressed in EmCa has likewise been demonstrated as being downregulated in cervical, colon, and lung cancers but not in hepatocarcinoma [73,74].

Transgelin is another protein observed to be underexpressed in this study. It has also been implicated as being downregulated in breast and colon carcinomas as well as in a lung epithelial cell line [75,76]. Transgelin was isolated and identified as an antigen from renal cell carcinoma; subsequent *in-situ* hybridization experiments, however, found that the malignant cells were negative with respect to transgelin and that the transgelin source was from the mesenchymal cells of the stroma [77]. Thus, downregulation of transgelin appears to be true for renal cell carcinoma as well.
***Proteins Showing Possible Differential Expression.*** There are four proteins that showed differential expression by cICAT, but a smaller than critical (two-fold) change by iTRAQ labeling. One such protein is cyclophilin A, which was observed as being overexpressed by approximately four-fold in the ICAT analysis; however, the iTRAQ experiments showed a smaller overexpression of 1.47 ± 0.29 across both cancer samples in both runs compared with the normal proliferative sample, which is not considered as significant according to our criterion of a two-fold change. Cyclophilin A has recently been reported as overexpressed in lung cancer [76].

Triosephosphate isomerase is another protein showing overexpression by cICAT tagging. However, the relative standard deviation for this protein at ± 72% was also large, suggesting a variable expression level. There is a recent study that suggests triosephosphate isomerase to be upregulated by two-fold in lung cancer [78]. Triosephosphate isomerase level was found to be highly variable in renal cell carcinoma [79]. iTRAQ labeling shows an overexpression of 1.55 ± 0.28.

Superoxide dismutase [Cu-Zn] is another protein that showed a large variation, ± 50%, in the cICAT results. This protein has been implicated in pancreatic adenocarcinoma [80]. iTRAQ results at 1.46 ± 0.08 is below the criterion of overexpression. Phosphatidylethanolamine binding protein showed a cICAT overexpression level of 3.7 ± 1.0 and an iTRAQ overexpression level of 1.39 ± 0.13. There is evidence for upregulation in rat hepatoma cell lines [81].

The use of isotope-coded affinity tags and nanoLC/MS/MS afforded examination of a large number of proteins for differential expression. This method is a lot more efficient and powerful in terms of the number of proteins that can be examined in a given experiment than SELDI MS. However, complex data analysis and the need for manual examination of MS/MS data limit the number of samples that can be examined within a given period of time. As a consequence, iTRAQ tagging involved two normal endometrial tissues versus two EmCa tissues, whereas cICAT pairing involved only one normal endometrial versus three EmCa tissues. By contrast, PCM discovery using SELDI MS involved in excess of 40 samples [see above]. A contribution to the uncertainties of the aforementioned four proteins may simply be individual variations in protein expression, both within the normal group as well as the EmCa group. The experience with the results of chaperonin 10 and calgranulin A shows that, while there is overexpression in EmCa tissues, there are considerable variations in protein abundances, possibly reflecting variability in the cellular subpopulations of the whole tissue homogenates or in the type or nature of the EmCa. In addition, there is variation in the abundances of these proteins across individual normal endometrial samples.
***Selectivity of PCMs.*** Literature data show that the nine differentially expressed proteins discovered and identified here are associated with cancers and are, indeed, potential cancer markers. Individually, these nine PCMs are nonspecific for endometrial carcinoma, as each has been linked with cancers other than EmCa. As the principal concern in screening, diagnosis, and monitoring of EmCa is the exclusion or omission of any malignant endometrial disease, the fact that these PCMs have been noted in other cancers would rarely be a potential clinical drawback. In other words, for envisaged clinical use, the sensitivity is of far more concern than specificity for EmCa. It is probable that different cancers will share similar pathways in tumorigenesis, which will induce over- and underexpression of similar proteins [57]. Nevertheless, it is unlikely that the differential expression pattern involving many proteins will be identical for all or some primary tumor sites. Their collective use as *a panel* may be more effective in indicating the site of origin of a cancer [82,84], despite the association of individual markers with a variety of primary sites.

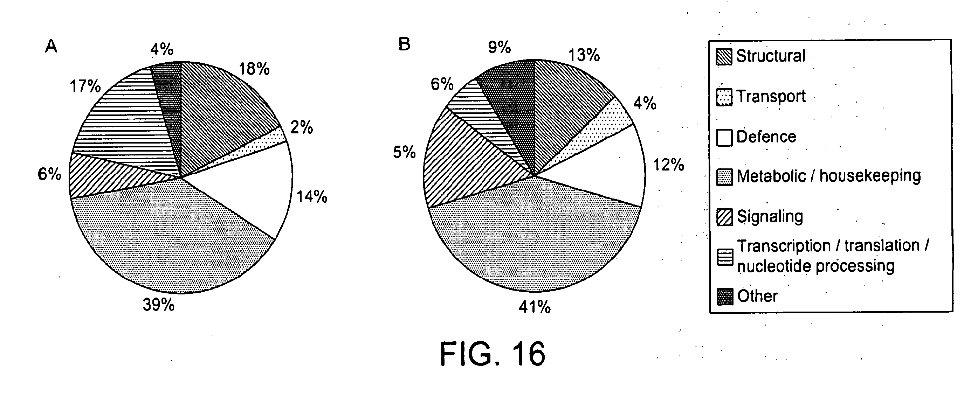

The absolute expression level of PCMs will be an important issue. Knowing protein abundances in normal and diseased states will allow establishment of threshold levels beyond which EmCa is signaled. iTRAQ permits simultaneous investigation of up to four samples, thus facilitating the inclusion of synthetic tryptic peptides of known amounts in absolute isotope-dilution experiments
***iTRAQ versus cIC4T**.* The results obtained by iTRAQ and ICAT analyses suggest that the information generated by the two methods is complementary. There are, however, a few aspects on which each of these methods has advantages over the other. Quantification by cICAT can be compromised by overlapping peaks in the MS spectrum; this complication is resolved in iTRAQ as quantification is performed on the MS/MS spectrum. iTRAQ on the other hand requires processing samples separately until after the tryptic digestion. This increases the potential for errors introduced, as a result of sample handling or different extents of tryptic digestion. Another aspect on which iTRAQ differs is complexity because of the relatively nonspecific nature of labeling. Many more proteins are identified by multiple peptides. This is not necessarily a disadvantage, as it permits quantification of multiple peptides, thereby increasing the confidence in the ratios report. After classifying identified proteins into broad categories (Figure 16), it is apparent that there is a higher proportion of signaling proteins identified by the cICAT method. Conversely, iTRAQ analysis identified a larger percentage of the more abundant ribosomal proteins and transcription factors.

### Example 5

### Summary

Proteomic analyses of the proliferative and secretory phases of the human endometrium were carried out to identify proteins and discover differentially expressed proteins using isotope-coded affinity tags and three stages of chromatographic separation and online MS/MS. From an initial list of 346 proteins identified by ProICAT, manual inspection of MS/MS spectra and confirmatory searches pared the list down to 119 positively identified proteins. Only five of the proteins showed consistent differential expression. The two proteins with unquestionable differential expressions in the secretory endometrium are: glutamate NMDA receptor subunit zeta 1 precursor and FRATI. Some of the proteins that show no differential expression have previously been examined in gene-expression studies with similar conclusions.

Below are reported results of a study to identify proteins in the human endometrium, and especially proteins that are differentially expressed in the proliferative and secretory phases. Differences in protein expression levels are highlighted and determined by the use of the cleavable ICAT reagent [8]. Proteins that are identified are compared with genes that have been examined for regulatory changes.

The following materials and methods were used in the study.

### Materials and Methods

### Tissue samples

Endometrial tissues were retrieved from an in-house dedicated endometrial tissue bank. All tissues had been snap-frozen in liquid nitrogen within 15-20 minutes of devitalization at the time of hysterectomy. All tissue procurements were carried out after informed patient consents. The consent, procurement and banking procedures were approved by the Research Ethics Boards of York University, Mount Sinai Hospital, University Health Network, and North York General Hospital. In every case, the endometrium was classified as proliferative or secretory by a pathologist. The histological classification was verified by examination of a histopathologic section from the frozen tissue. The mirror-face of the residual block was used for proteomic analysis. After addition of 1 ml of Hanks' Balanced Salt Solution containing protease inhibitors (1 mM 4-(2-aminoethyl)benzenesulfonyl fluoride, 10 µM leupeptin, 1 µg/ml aprotinin and 1 µM pepstatin), the tissue sample was mechanically homogenized at 30,000 rpm using a Polytron PT 1300D handheld homogenizer (Brinkmann, Westbury, USA). The whole tissue homogenate contained not only endometrial epithelium, but supportive stroma, vessels as well as secretions. The homogenate was stored in aliquot at -80°C and/or submitted for proteomic analysis. Samples from six different individuals were selected for this study. Three of these tissues had been classified as proliferative endometria (randomly designated as PRO1, PRO2 and PRO3), and the other three as secretory endometria (randomly designated as SEC1, SEC2, SEC3).

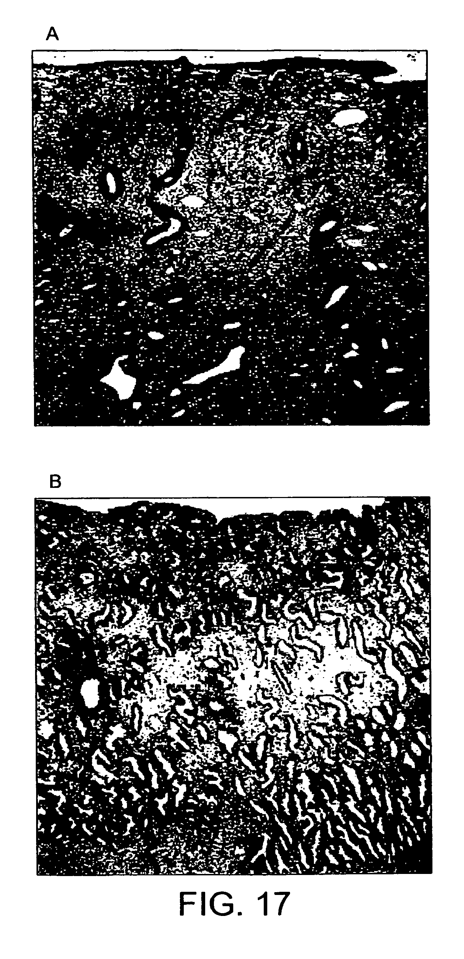

Figure 17 shows an example of the histologic appearances of (a) a proliferative (PRO2) and (b) a secretory (SEC2) endometrium. In both endometria, the stratum basalis is characterized by a denser stroma than the physiologic responsive stratum functionalis above. Across the top ofthe stratum functionalis is the surface epithelium, which lines the endometrial cavity. The proliferative endometrium shows small, coiled glands with lining columnar epithelium reaching to the surface. By contrast, the secretory endometrium is thicker, and contains more tortuous glands with intra-luminal secretions. The endometria of both types ofphysiologic phases have abundant supportive stroma and vessels among the epithelial glands.

### Chemicals

Solvents, chemicals (except otherwise noted), and Hanks' Balanced Salt Solution were obtained from Sigma-Aldrich (Oakville, ON, Canada). All reagents and buffers for the cleavable ICAT sample preparation procedure were from Applied Biosystems (Foster City, CA, USA).

### Sample Preparation

After removal of cell debris by centrifugation, the total protein content for each of the six clarified homogenates was measured using a commercially available Bradford protein assay (Bio-Rad, Mississauga, ON, Canada). ICAT sample preparation procedure was carried out according to the cleavable ICAT protocol (Applied Biosystems, Foster City, CA, USA). One hundred micrograms of proteins was used per sample. The proteins were denatured with the Denaturing Buffer supplied with the ICAT reagent kit. Afterwards, disulfide bonds were cleaved by adding the reducing reagent supplied, which contained 50 mM tris-(carboxyethyl)phosphine, and boiling for 10 min. The proliferative samples were then labeled with the light ICAT reagent and the secretory samples with the heavy reagent by incubating with the respective ICAT label for 2 h at 37°C. The labeled PRO1 and SEC1 samples were combined to form ICAT sample A, PRO2 and SEC2 to form ICAT sample B, and PRO3 and SEC3 to form ICAT sample C. The final volume of each sample was 0.2 ml. Mixing of the labeled proliferative and secretory samples in pairs in this manner ensures that any protein or peptide losses during subsequent processing steps is the same for both samples in a pair. The sample pairs were then digested by incubating each pair with 0.2 ml of a 50 µg/ml trypsin solution at 37°C overnight. The resulting peptides were fractionated by means of strong cation exchange chromatography using an HP 1050 LC system (Agilent, Palo Alto, CA, USA) with 1.5 ml injection loop and a 2.1 mm internal diameter (ID) x 100 mm length PolyLC Polysulfoethyl A column packed with 5 µm beads with 300 Å pores (The Nest Group, Southborough, MA, USA). A 2.1 mm ID x 10 mm length guard column of the same material was plumbed upstream from the analytical column. Fractionation was effected by a binary mobile-phase gradient at a total flow rate of 0.2 ml/min. Eluent A consisted of a 10 mM KH₂PO₄ solution in 25% acetonitrile and 75% deionized water acidified to a pH of 3.0 with phosphorus acid. Eluent B consisted of a 10 mM KH₂PO₄ and 350 mM KCl solution in 25% acetonitrile and 75% deionized water acidified to a pH of 3.0 with phosphorus acid. The trypsinized ICAT sample pair (0.4 ml) was mixed with 2.0 ml ofEluent A. 1.5 ml of the 2.4 ml sample was injected. Initially, the gradient comprised 100% Eluent A. At the 2^{nd} minute, the % Eluent B was changed linearly from 0 to 100% at the 58^{th} min. The run was terminated at the 60^{th} min. A total of 30 fractions were collected, one every two minutes, using an SF-2120 Super Fraction Collector (Advantec MFS, Dublin, CA, USA). UV monitoring ofthe chromatographic eluent revealed abundant peptides in fractions 11-20. These 10 fractions were further purified by affinity chromatography according to the ICAT protocol recommended by Applied Biosystems. Eluted labeled peptides were treated with the Cleaving Reagent, which contains TFA in order to remove the biotin label, and resolved in a third stage of chromatographic separation using reverse-phase nanobore LC with online MS/MS.

### Nanobore LC/MS/MS

The nanobore LC system was from LC Packings (Amsterdam, The Netherlands) and consisted of a Famos autosampler and an Ultimate Nano LC system. It was interfaced to an API QSTAR Pulsar QqTOF mass spectrometer (Applied Biosystems/MDS Sciex, Foster City, CA, USA) using a Protana NanoES ion source (Protana Engineering A/S, Odense, Denmark). The spray capillary was a PicoTip SilicaTip emitter with a 10 µm ID tip (New Objective, Woburn, MA, USA). The nanobore LC column was a 75 µm ID x 150 mm length reverse-phase PepMap C18 nano capillary column (LC Packings, Amsterdam, The Netherlands) packed with 3 µm beads with 100 Å pores. One µl of sample was injected via the full-loop mode. Separation was performed using a binary mobile-phase gradient at a total flow rate of 200 nl/min. Eluent A consisted of 94.9% deionized water, 5.0% acetonitrile and 0.1% formic acid (pH ≈ 3). Eluent B consisted of 5.0% deionized water, 94.9% acetonitrile and 0.1% formic acid. The following binary gradient was used:

| | | | | | | | | |
|---|---|---|---|---|---|---|---|---|
| *Time (min)* | 0 | 5 | 125 | 135 | 157 | 160 | 163 | 190 |
| % *Eluent B* | 5 | 5 | 30 | 60 | 80 | 80 | 5 | Stop |

For nanospray, the source conditions were a curtain-gas setting of 20 and an ionspray voltage in the range of 1800 - 3800 V that was optimized daily. In the Q0 region, the instrument parameters were a declustering potential (DP) of 65 V and a focusing potential (FP) of 265 V. Nitrogen was used as the collision gas at a setting of CAD = 5 for both TOF-MS and MS/MS scans.

All LC-MS/MS data were acquired in information-dependent acquisition (IDA) mode in Analyst QS SP5 with Bioanalyst Extension 1.1 (Applied Biosystems / MDS Sciex). A TOF-MS survey scan with an m/z range of 400 - 1500 and 1 s scan time was followed by two product ion scans with an m/z range of 70-2000 and 2 s scan time. The collision energy (CE) was automatically controlled by the IDA CE Parameters script. The switching criteria were set to ions greater than m/z = 400 and smaller than m/z = 1500 with a charge state of 2 to 5 and an abundance of ≥ 10 counts/s. Former target ions were excluded for 60 s and ions within a 4 Th window were ignored. In addition, the IDA Extensions II script was set to 2 repetitions before dynamic exclusion and to select a precursor ion nearest to a threshold of 15 count/s every 4 cycles. These settings ensured examination of not only high abundance ions, but low abundance ones as well. Each of the three sample pairs, A, B and C, was injected twice, thus yielding a total of six sets of runs.

Data analysis was performed using ProICAT 1.0 SP3 software (Applied Biosystems / MDS Sciex). An initial setting of a confidence value of 75 and a score of 15 was used. Relative quantification ofproteins between the light and heavy labels was performed on the TOF-MS scans by calculating the relative areas under the series of peaks: Differential levels of expression were based upon measured protein expression ratios in proliferative versus secretory endometrium normal ized to glyceraldehyde-3-phosphate dehydrogenase (GAPDH) expression and were considered significant when they exceeded three SDs from the mean expression of housekeeping proteins (actin, tubulin α-chain and triose phosphate isomerase). All expression ratios were based on a mean of two essays. The sample size calculation for determination of minimum numbers of tissue samples for ICAT analysis (N) was based upon the difference between two means, N = 8 (SD / precision) [85]. Based on preliminary experiments, an SD value of 0.3 was observed with a target precision of 0.5, yielding a minimum sample size of 3 for each tissue group. The significance of differential protein expression between proliferative and secretory endometrium was evaluated using the Wilcoxon rank sum test.

### Results

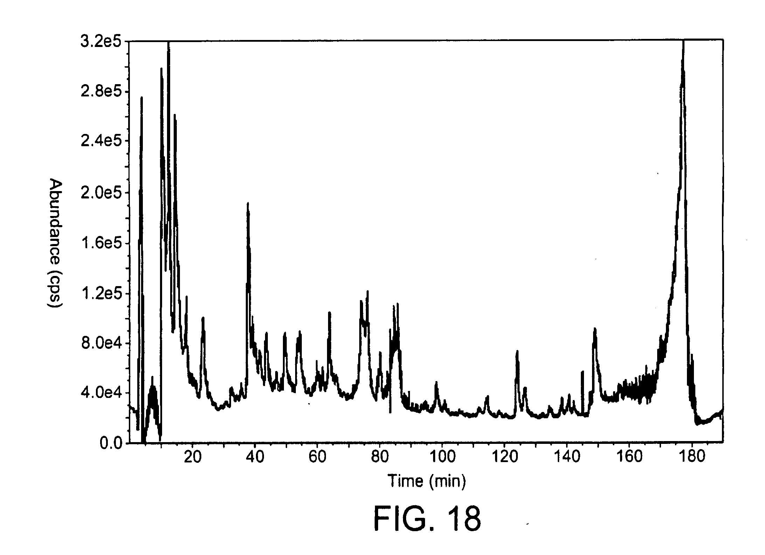

Figure 18 shows a typical nano LC/MS total ion chromatogram (TIC) from fraction 16 of sample A. As a total of 10 fractions were analyzed per sample and each sample was injected twice, there were a total of 60 TICs. The use of the IDA mode generated hundreds of MS/MS spectra per TIC.

Using the initial setting of a confidence value of 75 (ProICAT recommends a lower confidence value of 50) and a score of 15 in ProICAT 1.0 SP3 results in the identification of 346 non-redundant proteins. Manual inspection of the MS/MS spectra reveals that identification of some proteins that score between 15 and 20 may be problematic. A random selection of 50 spectra from proteins scoring between 15 and 20 shows that 15 were analyzed and sequenced correctly, 13 were probably correct, and 22 were incorrect. Manual inspection of the MS/MS spectra of the 145 proteins that score 20 or higher also reveals occasional errors in assignment, but at a rate considerably lower than that between scores 15 and 20. Unfortunately, the use of ICAT-labeling results in a reduced opportunity for corroborating identification based on a second tryptic peptide from the same protein. Although the majority of human proteins do contain more than one cysteine residue per protein, some of these residues may be located within tryptic peptides that are too small or large, others may fall into peptides that have poor ionization efficiencies. Of the 346 proteins, only 23 were identified with more than one peptide; 15 of these 23 proteins were identified with two peptides.

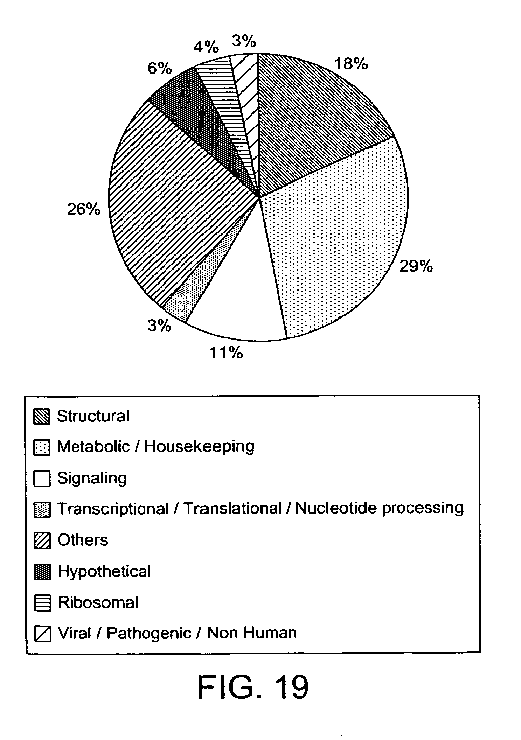

Table 6 lists the 119 proteins that have been identified by ProICAT and verified by manual inspection of MS/MS data. Figure 19 shows the distributions of the proteins in the form of a pie chart. Of the 119 proteins, 15 were added after manual inspection of 50 randomly selected proteins' MS/MS spectra. If the MS/MS spectra of the remaining 151 proteins was inspected, it would have been expected to confirm the identifications of an additional ~ 45 proteins. This, however, was considered impractical, especially after it was known that none of the 15 additional proteins were observed in all three runs and would contribute towards knowledge of differential expression.

The results show that expression levels of the majority of proteins identified are not consistently different between the proliferative and secretory phase of the endometrial cycle. As expected, the majority of identified proteins fall under the metabolic/housekeeping and structural categories. The proteins classified under "Others" include antibodies. A small percentage of the proteins are viral or pathogenic in origin, reflecting the possibility of infection in the biopsied endometria. A few proteins have no known functions or were identified from cDNA matches; these are, therefore, classified as hypothetical.

To normalize small variations of protein amounts, relative quantification of the protein levels is normalized to the ratio of GAPDH. Gene expression studies involving different stages in the endometrial cycle have used GAPDH expression to normalize differential mRNA expressions [88-90]. The assumption is that the constant mRNA level of GAPDH through the endometrial cycle translate to constant protein level (no variation caused by translational controls). The GAPDH ratio of 1.5 ± 0.5 (one SD) before self-normalization in the six runs is in accordance with this assumption.

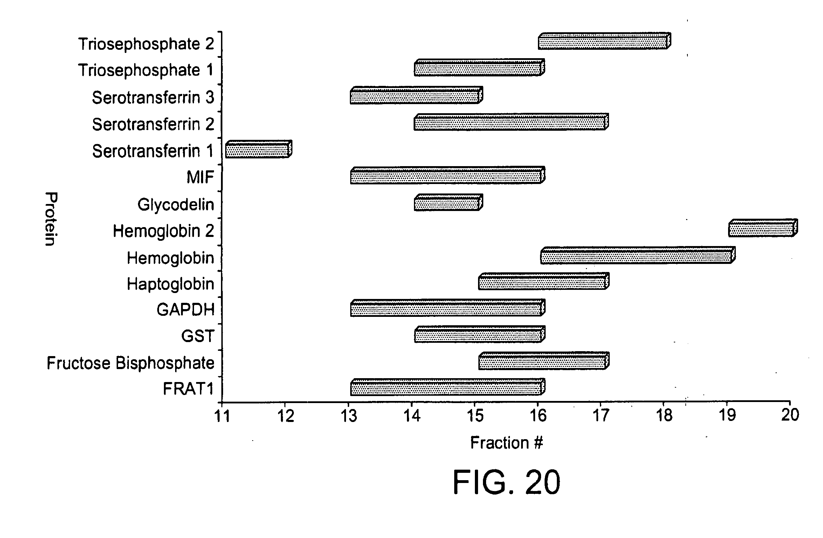

The ratios of the proteins in the secretory / Proliferative phase (heavy / light label) listed in Table 6 are the averages ± SDs of the peptides in all the identified runs. In the case of more abundant proteins, e.g. serum albumin and serotransferrin, individual peptides were detected in as many as 3-4 fractions. Less abundant proteins, however, were frequently detected only in 1-2 fractions (see Figure 20). In cases of multiple fractions, multiple peptides, and/or multiple runs, the ratios listed in Table 6 are the averages and the SDs of all contributing elements. Twenty four proteins show "extreme" differential expression in that only one labeled form was observable (these are noted as "Singleton H or L". However, only five of these 24 proteins were observed in all three samples (and an additional one in two samples). All five proteins were identified by single tryptic peptides. The expression levels of three housekeeping proteins (actin, tubulin α-chain and triose phosphate isomerase) normalized against GADPH were pooled to establish a mean expression level of 1.07~1.1 and an SD of 0.3. The SD of these expression levels was calculated from expression data normalized to a value of 1 for any expression ratios less than 1 to generate linear data. (This was done by taking the reciprocal of expression values less than 1 in the original data set; for example, an expression ratio of 0.7 was converted to its reciprocal 1.42.) This data was then used to calculate the SD of housekeeping protein expression. A significant differential expression value was, therefore, defined as a normalized expression ratio that was three SDs above the ratio of 1.0. On the basis of the observed data, this ratio was set as 2.0 (approximately 1.0 + 3(0.3)). Consequently, only two-fold or larger changes in expression ratios were considered to be of significance, based on the analysis of the variation in measured expression of housekeeping proteins. An additional stringency requirement of this level of expression in all three sample pairs was imposed due to the small initial sample set. No additional proteins in Table 6 meet the differential expression criteria of abundance ratios larger than 2.0 or smaller than 0.5 in all three sample pairs.

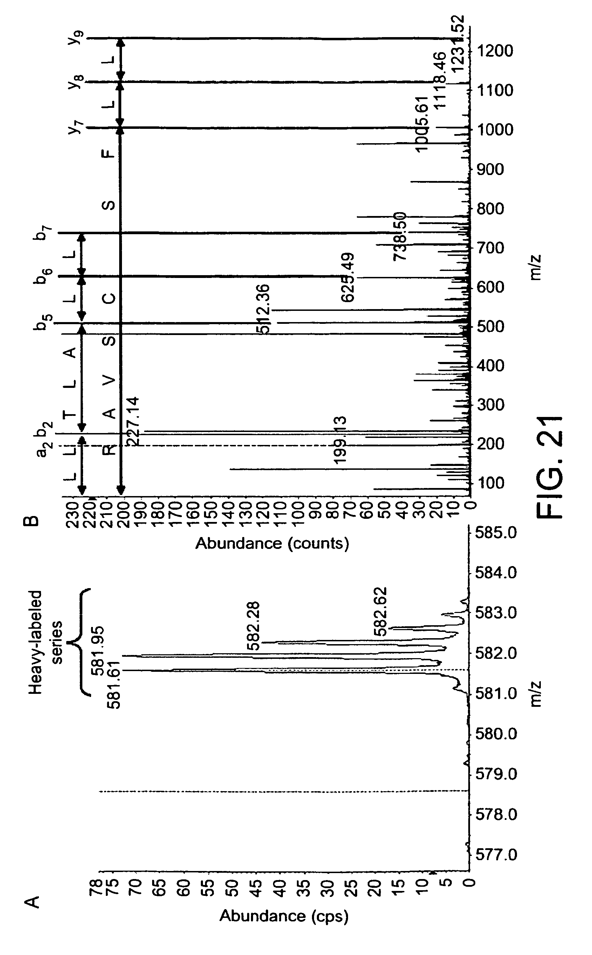

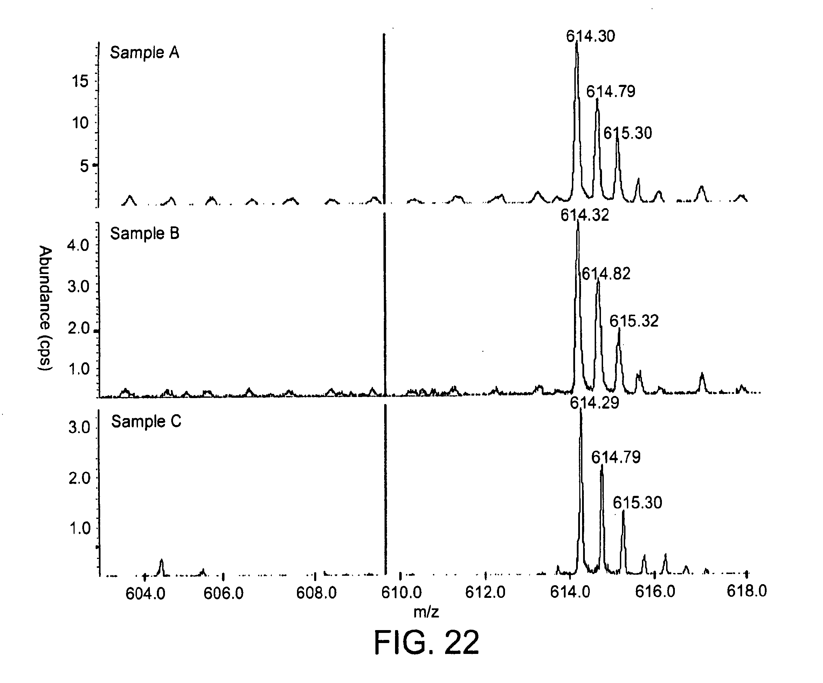

An example of extreme differential expression observable in all three sample pairs is shown in Figure 21, which shows very significant expression enhancement of glutamate NMDA receptor subunit zeta 1 precursor in the secretory endometrium. The absence of the light-labeled analogue, which would have manifested as a cluster of peaks beginning at 578.6 Th, demonstrates the significantly lower expression of this protein in the proliferative endometrium. The triply charged tryptic peptide at 581.6 Th was identified as LLTLALLFSCSVAR [SEQ ID NO.28], which maps to the N-terminal region of the protein. A second example is shown in Figure 22, again showing extreme differential expression in the secretory phase. This protein was identified as FRAT1. For all five proteins that met the differential expression criteria stipulated above, a minimum p value of less than 0.04 was obtained on the basis of the Wilcoxon rank sum test, suggesting that the differences observed were of statistical significance.

### Discussion

The observation of similar expression levels in the majority of proteins shown in Table 6 is not surprising, as many proteins are structural and housekeeping in nature. In addition, structural and housekeeping proteins, e.g. tubulin and actin, are expressed in many tissue types. This is of significance as the biopsies that were examined comprise not only epithelial cells, but also supportive stroma cells, blood and vessels that are not expected to be affected by the endometrial cycle.

In this initial examination of three sample pairs, a conservative strategy was adopted of recognizing proteins as differentially expressed only when their expression ratios are larger than 2.0 or smaller than 0.5 in all three sample pairs. Of the 119 proteins listed in Table 6, five proteins are in this category. In fact, differential expression is in the extreme form that only single labels are apparent. There is one protein (POL polyprotein) that was seen only in the secretory endometrium, but was observed in only two sample pairs and does not meet the criteria of differential expression in this study (see later).

One of the five differentially expressed proteins is glutamate NMDA receptor subunit zeta 1 precursor, which was only observed in the secretory endometrium. This protein is a subunit of a ligand gated ion channel and is known to be involved with synaptic plasticity in neurons. A recent paper suggests that it may also play a role in glutamate-mediated toxicity to mitochondria, eventually leading to apotosis [91]. As stated earlier, the peptide maps to the N-terminal region of the precursor, which is normally cleaved to form the mature protein.

Similarly, FRAT1 is detected only in the secretory endometrial samples under the experimental conditions. There is no direct connection between FRAT1 and the endometrium reported in the literature; however, FRAT1 is expressed in a wide range of human tissues [92]. The gene expressing FRAT1 is a known proto-oncogene that activates the WNT pathway [93,94]. This pathway is important in the endometrial cycle; FRAT1 is known to inhibit c-Jun activity, thereby inhibiting subsequent apotosis [95]. c-Jun, in turn, has been shown to be expressed in the endometrium and this expression is at a higher level during the proliferative phase than the secretory phase [96].

Myosin light chain kinase 2 is another protein that was observed exclusively in the secretory endometrium in all three sample pairs. While there are no studies on the relative amounts of this enzyme during the endometrial cycle, previous studies showed that it is present in the human myometrium at a level that is four-to five-fold higher than that in endometrial stromal cells [97]. It was also demonstrated that the specific activity of myosin light chain kinase, which is believed to be essential for smooth muscle contraction, was 20-fold higher in the myometrium than that in non-muscle cells, e.g. skin fibroblasts [97].

Isopentenyl diphosphate delta-isomerase, was present only in the secretory endometrium. This protein was identified via the peptide ITMLCTGSRTLK [SEQ ID NO. 29]. In addition to this identification with ProICAT, a subsequent search with Mascot (Matrix Science) verified this assignment. This peptide, and therefore the protein, however, is unique and found only in the bacterium *Rickettsia conorii.* BLASTing the human and the *R*. *conorii* isopentenyl disphosphate delta-isomerase proteins showed no region of homology. In addition, MS BLASTing the peptide ITMLCTGSRTLK against non-redundant protein databases returned no other matches.

A protein that appeared in all three sample pairs was initially identified by ProICAT as a simian immunodeficient viral protein, envelope polyprotein GP160 precursor. However, searching Mascot with the same MS/MS spectra returned a different protein from clostridium, putative phosphatidylserine decarboxylase proenzyme (gi 28209853). Endometrial receptors for a clostridium toxin have been documented [98]; thus the identification of a clostridium protein is not entirely surprising.

Another protein initially identified by ProICAT as POL polyprotein from Rous sarcoma virus was observed only in the secretory endometrium in two of the three sample pairs. A subsequent Mascot search, however, returned a positive hit for an unlabeled peptide from serum albumin, and strongly suggested a wrong initial identification.

Tropomyosin I alpha chain was identified in two out of three sample pairs to be selectively expressed in the secretory phase at a ratio of 2.3 ± 0.7. An enhanced expression of this protein in the secretory endometrium is in accordance with the result of a previous gene expression study that reported an up-regulation of 3.7 times [85].

A protein that showed no difference in expression levels (1.2 ± 0.7 in three samples) is macrophage migration inhibitory factor, MIF. This observation is consistent with those in previous studies that demonstrated MIF as being expressed by the human endometrium throughout the menstrual cycle and, in particular, predominantly in the glandular epithelial cells [9]. MIF was found to localize throughout the glandular epithelial cytoplasm in the proliferative phase; however, this distribution changes in the secretory phase, when MIF localizes to the apical portion of the glandular epithelial cells and is also present in glandular secretions. Macrophages are common in female reproductive tissues. In the endometrium, they play an important role in defense. In addition, macrophage degradation of cellular debris and foreign material may play an important role in endometrial shedding and repair [9].

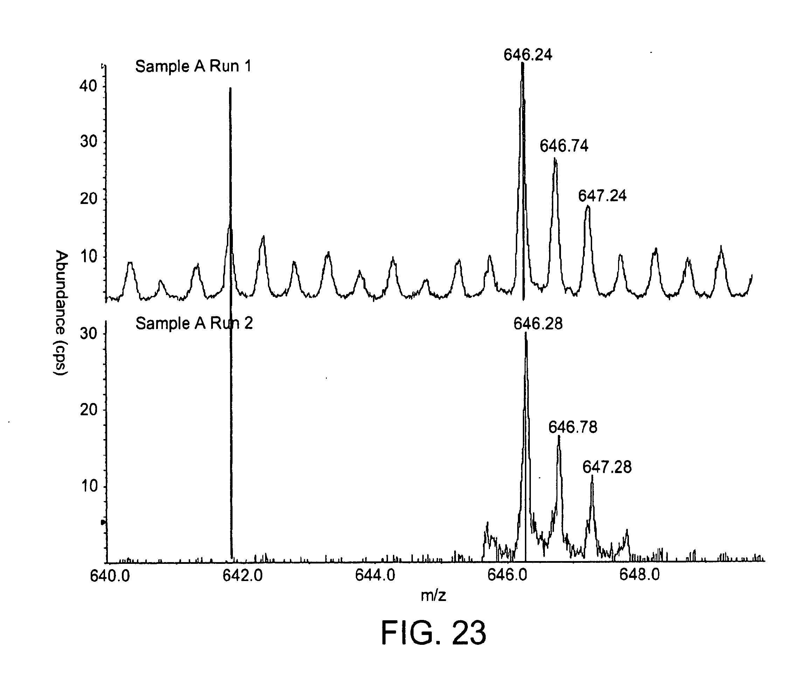

Another protein observed in this study and for which there was precedence in the literature is glycodelin. On the basis of gene expression studies [85], glycodelin was believed to be up-regulated during the secretory phase. In this study, the precursor of this protein was identified in only one sample pair, but it was identified in both runs in only the secretory endometrium (Figure 23).

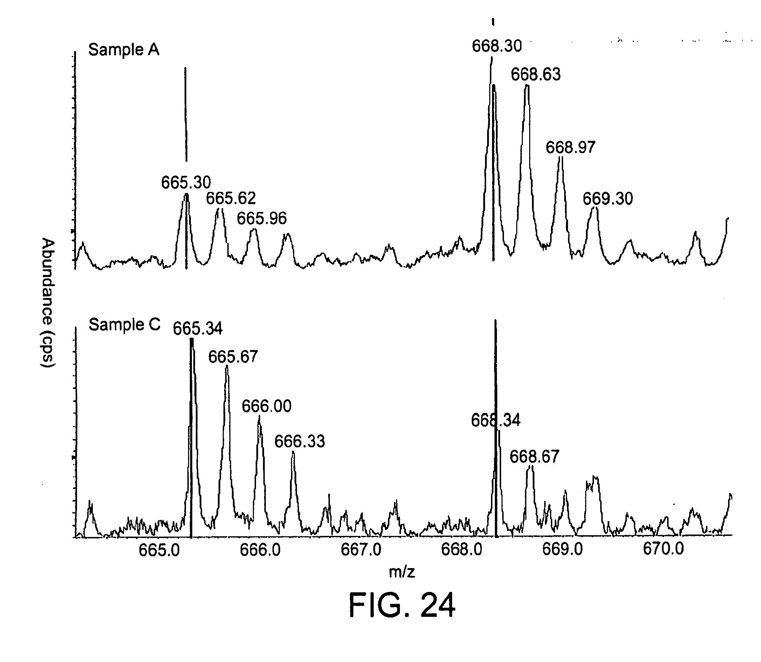

Cathepsin B which, according to a previous study, does not appear to be differentially regulated during the menstrual cycle [99], was seen in two of the sample pairs with opposite trends (Figure 24). Glutathione S transferase is known to show large variability which is believed to result from individual differences, rather than endometrial cycling [100]. The relative expression level observed in this study is 1.3 ± 0.4. Lactate dehydrogenase is another protein not believed to have any cyclic trend [101]; the relative level measured in this study, 1.4 ± 0.3, is in accordance with this expectation. Peptidyl-propyl cis-trans isomerase A or cyclophilin A has been observed in placental and decidual tissues, but there was no study on relative amounts between the phases of the endometrial cycle [102]. The relative expression results of 1.2 ± 0.2 show a constant level of expression. Haptoglobin is thought to be up-regulated during the secretory phase [103]. The average relative expression level of 2.2 ± 1.6 is based on two enhanced secretory expression level measurements in two sample pairs and one reduced secretory expression level measurement in another sample pair that account for the very large SD. Haptoglobin is a liver protein secreted into blood. As the amounts of blood in the biopsied tissues could not be controlled prior to homogenizing, the large SD might simply reflect that the samples contain different amounts of blood as opposed to truly representing increased or decreased haptoglobin expression.

It should perhaps be emphasized that protein identifications using ICAT-labeled peptides are reliable as the identification of one labeled peptide in a pair corroborates the identification of the other in the pair. This offsets somewhat the reduced opportunities in seeing multiple peptides because ICAT targets only cysteine-containing peptides. The chance of a missed identification increases when only one member of the labeled peptide pair is observable in the form of an extreme differential expression and there is uncertainty as to whether the observable peptide is labeled with the light or the heavy ICAT reagent. In addition, the presence of an unlabeled peptide may be mistakenly interpreted as a labeled peptide that is differentially expressed in the extreme. An unlabeled peptide will need to have a combination of "right" chemical properties and very high initial concentrations (e.g. a serum albumin peptide) to "survive" the affinity chromatography stage intended to select only labeled peptides. The above is intended to be a cauti onary note in pointing out potential shortcomings in a powerful technique that readily highlights and quantifies differential protein expressions, and not as a negative opinion on the suitability and utility of ICAT in protein discovery or quantification.

Of the 46 studies published to date that centered on ICAT, the vast majority were on methods development that compared differential protein expressions in two different cell states. Most of these studies were on yeast cultured under different medium conditions and they compared protein levels of one sample pair. Only one recent study [104] targeted clinical samples, and even in this study only one sample pair was analyzed for differential protein expression. The present study is the only work in which multiple sample pairs have been examined. It is, of course, possible that a given observed deviation in protein ratio from 1, i.e. a heavy/light label ratio # 1, is due to individual differences, and that different pairing of the proliferative and secretory samples may produce a different ratio. It is because of this possibility that the criterion for differential expression was stringently set to a ratio of *larger than 2.0* and *smaller than 0.5* in *all three* sample pairs. It turns out that for the five proteins that met the above criterion of differential expression, the differences were in the extreme in that the proteins were observed in only one endometrial phase in a pair. Expression differences at this extreme level in all three sample pairs are highly unlikely to have originated from individual differences.

### Conclusion

From an initial list of 346 proteins identified by ProICAT 1.0 SP3, 119 proteins were identified after manual inspection of MS/MS data and additional searches using Mascot. The expression levels of the majority of proteins do not vary significantly between the proliferative and the secretory phases. Only five proteins satisfy the criteria on differential expression in having an expression ratio larger than 2.0 or smaller than 0.5 in all three sample pairs. In fact, the differential expressions observed are extreme in that the proteins are present in only one of the two endometrial phases. The two proteins with unquestionable differential expressions in the secretory endometrium are: glutamate NMDA receptor subunit zeta 1 precursor and FRAT1. Some of the proteins that show no differential expression have previously been examined in gene-expression studies with similar conclusions.

### Example 6

### Abstract

SELDI MS has conventionally been practiced on linear TOF which has low mass accuracy and resolution. Here in an examination of both malignant and nonmalignant endometrial tissue homogenates it is demonstrated that high mass accuracy and resolution in the MS stage are crucial. Using a commercially available QqTOF, two potential cancer markers were resolved and subsequently identified offline as chaperonin 10 and calgranulin A, that differ by 8 Da in mass. Two offline protein identification protocols were developed: the first was based on SEC, SDS PAGE, protein extraction, trypsin digestion and MALDI-MS/MS; the second on SEC and shotgun nanoLC/MS/MS. Analyses on a cohort of 44 endometrial homogenates showed 22 out of 23 nonmalignant samples had nondetectable to very low abundance of chaperonin 10 and calgranulin A; 17 of the 21 malignant samples had detectable to abundant levels of both proteins. Immunohistochemical staining of a tissue microarray of 32 samples showed that approximately half of malignant endometrial tissues exhibited positive staining for calgranulin A in the malignant epithelium, while 9 out of 10 benign tissues exhibited negative epithelial staining. In addition, macrophages/granulocytes in malignant as well as nonmalignant tissues showed positive staining. No immunostaining occurred in stroma or myometrium.

The following materials and methods were used in the study.

### Materials and methods

### Tissue samples and sample preparation

Endometrium and EmCa tissues were retrieved from a dedicated, research in-house endometrial tissue bank. The consenting and tissue banking procedures for this tissue bank were approved by the Research Ethics Boards of York University, Mount Sinai Hospital, University Health Network, and North York General Hospital. All tissues had been snap frozen in liquid nitrogen within 15-20 minutes of devitalization at the time of hysterectomy, and were obtained with patients' consent. In each case, the endometrium was classified as nonmalignant or malignant (EmCa) by one of the authors (TJC) [Kurman, R.J., Ed., Blaustein's Pathology of the Female Genital Tract, 5^{th} ed. New York, Springer-Verlag, 2002]. Nonmalignant endometrium included both benign physiologic endometrial cases (atrophic, proliferative, secretory, and menstrual) and pathologic states (benign endometrial polyp and disordered proliferative). EmCa cases included adenocarcinomas and rarer malignant mixed Mullerian tumors (carcinosarcomas). This classification was performed using routine surgical pathology sections. The histologic classification of the research tissue was verified by examination of a histopathologic section from the frozen research tissue. Tissue was taken for proteomic analysis from the mirror-face of the residual block. Cases of atypical endometrial hyperplasia, which could be considered to represent an intermediate phenotype, were not available for frozen-tissue banking and MS analysis because all tissue was required for histopathologic examination, but tissue was available for tissue microarray (TMA) preparation.

Tissue was thawed in Hanks' balanced salt solution (HBSS, Sigma) containing protease inhibitors (1 mM 4-(2-aminoethyl)benzenesulfonyl fluoride, 10 µM leupeptide, 1 µg/ml aprotinin and 1 µM pepstatin) and followed by mechanical homogenation at 30,000 rpm using a Polytron PT 1300D handheld homogenizer (Brinkmann, Westbury, USA). These whole tissue homogenates contained not only endometrial epithelium, but supportive stroma, vessels, as well as secretions. The homogenate was then stored in aliquot at -80 °C until protein profiling.

### Chemicals

Solvents, chemicals (except otherwise noted), and Hanks' Balanced Salt Solution were obtained from Sigma-Aldrich (Oakville, ON, Canada).

### Protein profiling using SELD-MS

Tissue lysate was fractionated to reduce sample complexity before protein profiling. An identical quantity of proteins was used for all samples; protein amounts were measured using Bradford Assay (Bio-Rad) on a DU-65 spectrometer (Beckman) at 595 nm. HBSS or urea/thiourea 2-D lysis buffer (7 M urea, 2 M thiourea) was used to compensate the initial volume to ensure equal volumes for all samples. Two micrograms of proteins from endometrial tissue homogenate was incubated with WCX2 or CM10 ProteinChips (Ciphergen) according to the manufacturer's instructions. In brief, samples were diluted to 55 µl with the corresponding binding buffer, spotted onto the appropriate ProteinChip, and incubated in a scaled BioProcessor (Ciphergen) for 1 h at room temperature. The ProteinChip surface was washed twice with the appropriate buffer for 5 minutes, briefly rinsed with water and air-dried. Two times 0.5 µl of 50% saturated sinapinic acid in 50% acetonitrile was applied on the samples to form crystals. The ProteinChips were analyzed using a linear TOF analyzer, PBS IIc (Protein Biology System IIc, Ciphergen), or a hybrid QqTOF mass spectrometer, QSTAR XL (Applied Biosystems / MDS Sciex) fitted with the SELDI interface (Ciphergen). For accurate molecular-weight measurements, on-chip, internal mass calibration was performed using insulin (5,733 Da), cytochrome c (12,361 Da) and myoglobin (16,951 Da) as calibrants.

### Protein purification and identification

Selected EmCa samples were subject to chromatographic separation to yield purified proteins for identification. Five hundred micrograms of proteins from a whole tissue homogenate was first fractionated using SEC (BioSep 2000, Phenomenex) at 1 ml/min flow with phosphate buffer (pH 7.9) containing 0.05% (w/v) sodium azide. One-millilitre fractions were collected; the eluates were then concentrated to 50 µl with a silicon carbide-based spin column (ProteoSpin, MDS Sciex). Five microlitres of a concentrate was desalted by C 18 Zip-Tip and analyzed with MALDI-MS to locate the fractions containing the protein markers of interest in the EmCa sample. Two different approaches were developed and used to identify the target proteins. Some proteins were more amenable to identification with one and others with the second approach. The first approach used SDS PAGE technique to further purify the target proteins. The fractions with the enriched proteins were diluted to 100 µl with freshly prepared DTT (5 mM final) in 150 mM Tris pH 8.5 buffer, and incubated at 60 °C for 1 h. Ten microlitres of the reaction mixture was desalted by C18 Zip-Tip and analyzed with MALDI-MS to assess the effect of DTT on the protein of interest. The remaining 90 µl was precipitated by acetone (80% (v/v) final), resuspended in SDS sample buffer, and the proteins were resolved by SDS PAGE. Protein molecular-weight markers (New England Biolabs) and cytochrome C (C-2506 Sigma) were included to guide the excision of gel portions containing the protein of interest. Intact proteins were extracted from the gel by 50 µl extraction solution (formic acid/acetonitrile/isopropanol/water in a ratio of 50/25155/10) at room temperature for 4 h. The extracts were completely dried by SpeedVac and resuspended in 40 µl 100 mM ammonium bicarbonate. Half of the resuspended proteins was desalted by C18 Zip-Tip and analyzed with MALDI-MS, the other half was digested in solution with 100 ng trypsin (Promega). The identity of the target protein was determined using MS/MS ion search (ProID, Applied Biosystems). The second approach used nanoLC/MS/MS technology. After concentration using ProteoSpin, the fractions with the enriched target proteins were further cleaned up using C18 Zip-Tips. Four C 18 Zip-Tips were used for each 45 µl of concentrated fractions. The sorbed proteins were eluted by 100 µl of 60% acetonitrile with 0.3% TFA. The eluates were completely dried by SpeedVac and resuspended in 100 µl of 100 mM ammonium bicarbonate. The proteins were then digested in solution with 100 ng trypsin (Promega). The resulted tryptic peptides were analyzed by MALDI-MS and online nanoLC/MS/MS. The identity of the target protein was determined using MS/MS ion search (ProID, Applied Biosystems).

### NanoLC/MS/MS

The nanoLC system was from LC Packings (Amsterdam, The Netherlands) and consisted of a Famos autosampler and an Ultimate nanoLC system. It was interfaced to a second QSTAR Pulsar hybrid QqTOF mass spectrometer using a Protana NanoES ion source (Protana Engineering A/S, Odense, Denmark). The spray capillary was a PicoTip SilicaTip emitter with a 10 µm ID tip (New Objective, Woburn, MA, USA). The nanoLC column was a 75 µm ID x 150 mm length reverse-phase PepMap C18 nano capillary column (LC Packings, Amsterdam, The Netherlands) packed with 3 µm beads with 100 Å pores. One microliter of desalted sample was injected via the full-loop mode. Separation was performed using a binary mobile-phase gradient at a total flow rate of 100 nl/min. Eluent A consisted of 94.9% deionized water, 5.0% acetonitrile and 0.1 % formic acid (pH ≈ 3). Eluent B consisted of 5.0% deionized water, 94.9% acetonitrile and 0.1% formic acid. The following binary gradient was used:

| | | | | | | | | |
|---|---|---|---|---|---|---|---|---|
| *Time (min)* | 0 | 10 | 40 | 42 | 44 | 45 | 70 | 72 |
| *% Eluent B* | 5.0 | 5.0 | 30.0 | 80.0 | 80.0 | 5.0 | 5.0 | Stop |

The ion source conditions were a curtain-gas setting of 20 and an ionspray voltage in the range of 1800-3800 V that was optimized daily. In the Q0 region, the instrument parameters were a declustering potential (DP) of 65 V and a focusing potential (FP) of 265 V. Nitrogen was used as the collision gas at a setting of CAD = 5 for both TOF-MS and MS/MS scans.

All LC-MS/MS data were acquired in information-dependent acquisition (IDA) mode in Analyst QS SP5 with Bioanalyst Extension 1.1 (Applied Biosystems / MDS Sciex). A TOF-MS survey scan with an m/z range of 400 - 1500 and 1 s scan time was followed by two product ion scans with an m/z range of 70-2000 and 2 s scan time. The collision energy (CE) was automatically controlled by the IDA CE Parameters script. The switching criteria were set to ions greater than m/z = 400 and smaller than m/z = 1500 with a charge state of 2 to 5 and an abundance of ≥ 10 counts/s. Former target ions were excluded for 60 s and ions within a 4 Th window were ignored. In addition, the IDA Extensions II script was set to 2 repetitions before dynamic exclusion and to select a precursor ion nearest to a threshold of 15 count/s every 4 cycles. These settings ensured examination of not only high abundance ions, but low abundance ones as well.

### Tissue microarray and immunohistochemical protein localization

A 32-core TMA was prepared to confirm the presence of an identified protein marker (10,835 Th, see later sections), and to determine its localization. Following fixation in 10% buffered formalin, benign, hyperplastic and malignant endometrial tissues were histologically processed and embedded in paraffin blocks. A single 2-mm core of tissue was removed from the paraffin block using a Manual Tissue Arrayer (Beecher Instruments). The 26 endometrial study cases included 11 benign endometrium (five secretory, four proliferative, one atrophic and one menstrual), 13 malignant endometrium (12 adenocarcinomas and one carcinosarcoma), and two cases of atypical endometrial hyperplasia. Six other control tissues were also embedded into the TMA, including four non-endometrial carcinomas and 2 benign tumors (salivary gland pleomorphic adenoma and ovarian thecoma). Following MS identification, monoclonal mouse antibodies against the potential cancer marker were procured from two sources: Research Diagnostics, Inc. (Flanders, NJ), labeled as RDI, and US Biological (Swampscott, MA), labeled as USB. Sections were cut from the TMA. The antibodies were applied in an appropriate dilution determined through a pilot study and immunohistochemically visualized using a diaminobenzidine chromogen.

Interpretations of the two inimunohistochemically stained TMA sections were conducted using a standardized method; microscopic reviews were conducted blinded to the findings of the other TMA section. Positive staining (brown) for the potential marker was determined for three cellular components: macrophage/granulocyte, epithelium/carcinoma, and endometrial stroma and myometrium. Immunostaining of these three cell types was semi-quantitatively graded as: 0 (no immunostaining), 1+ (weak positivity and/or positivity in only occasional cells), 2+ (moderate positivity and/or positivity in more abundant cells), and 3+ (strong positivity and/or positivity in numerous cells).

### Results and discussion

### SELDI-MS details

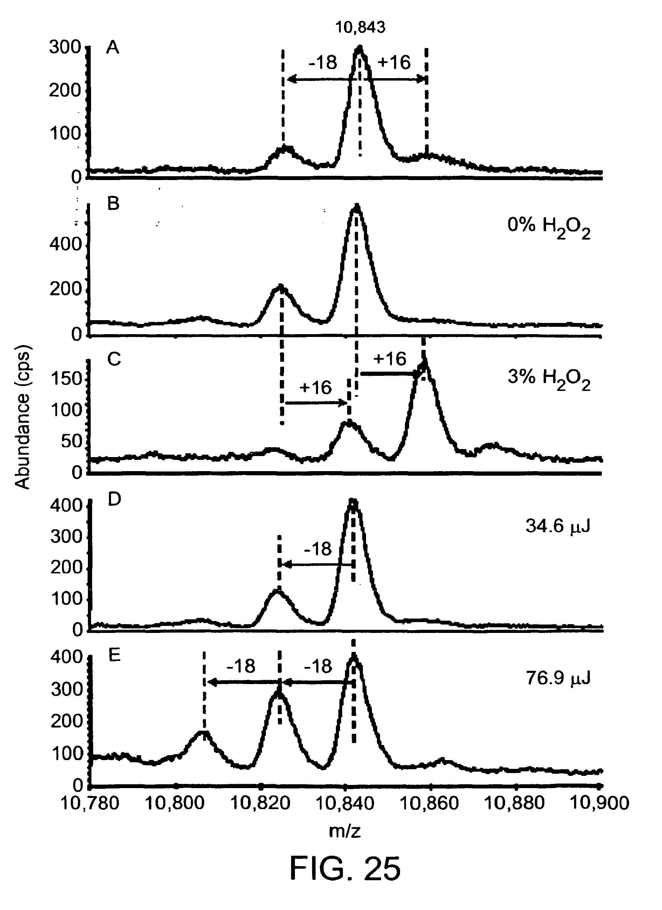

The use of SELDI-MS profiling in discovering a protein marker, chaperonin 10, for EmCa is described above. Chaperonin 10 has a molecular weight of 10,843 Da. Performing SELDI-MS on the higher resolution QqTOF mass spectrometer (QSTAR XL) was revealing: chaperonin 10 frequently appeared as three instead of a single peak (Figure 25a). On the lower resolution PBS IIc TOF mass spectrometer, which is much more widely available and used, the peaks are unresolved. Incubating the tissue homogenate with hydrogen peroxide overnight reduced the abundance of chaperonin 10 while increased the abundance of the protein that is heavier by 16 Da (compare Figure 25b with 25c). Increasing the fluence ofthe MALDI laser (from 34.6 to 76.9 µJ) had the effect of increasing the abundance of the protein peak that is 18 Th lower than chaperonin 10 in m/z value. In addition, a new protein peak that is 36 Th lower in m/z value emerged (compare Figure 25d with 25e). Incubating with hydrogen peroxide enhanced oxidation of chaperonin 10, probably the lone methionine residue; as a result, the molecular weight of chaperonin 10 increased by 16 Da. Increasing the laser intensity probably promoted dehydration of chaperonin 10 within the MALDI source. The sources of the water were likely the five glutamic and eight aspartic acid residues. Laser-induced deamidation (-17 Th) could also occur, but was ruled out after careful and repeated measurements mass-calibrated with cytochrome c. Single measurements for chaperonin 10 on the QSTAR XL with external or internal calibrants carry a typical uncertainty of ± 2 Th.

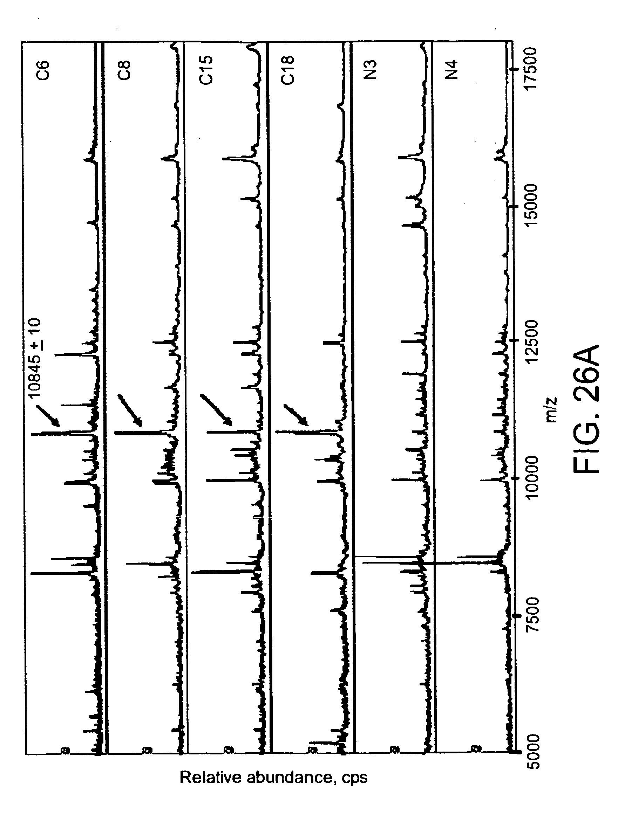

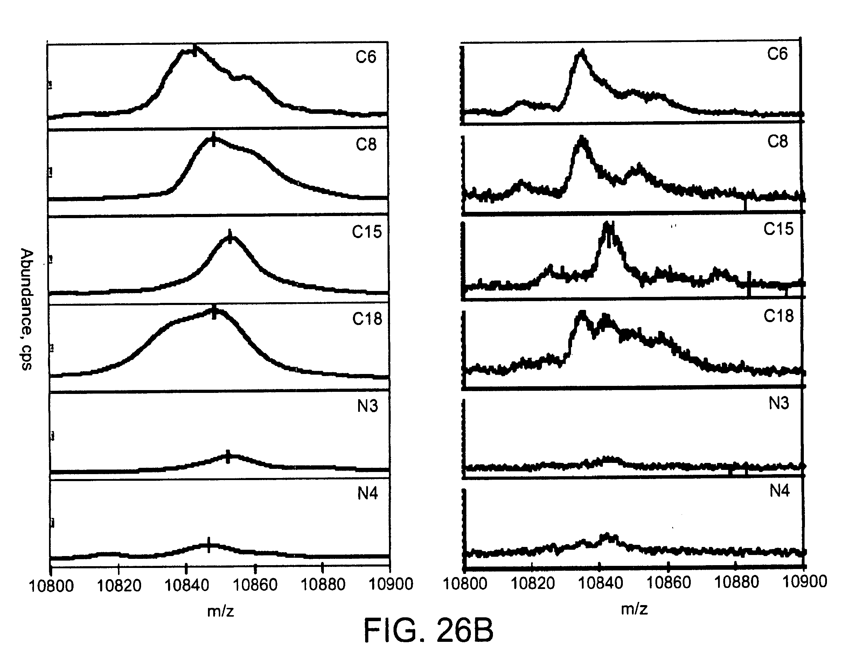

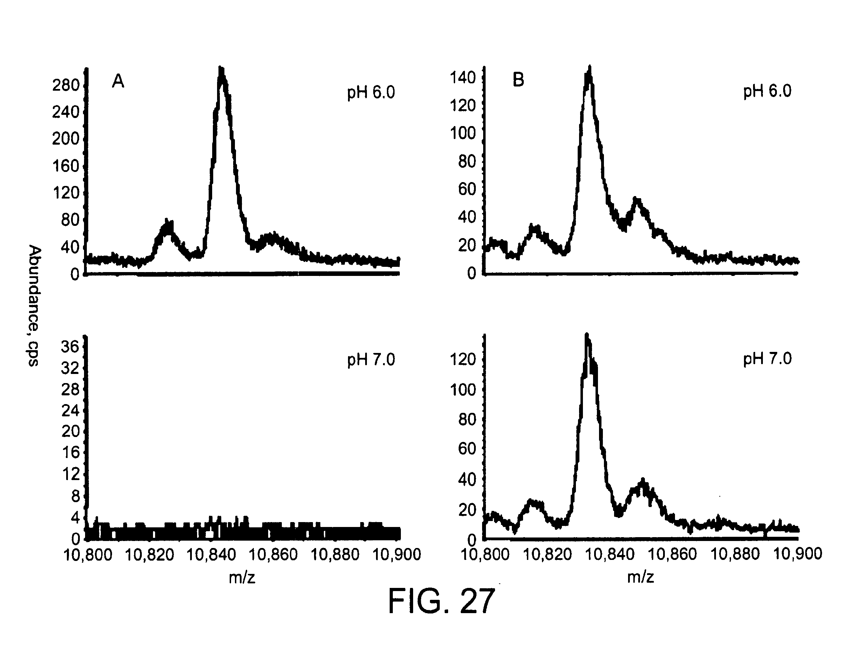

As stated earlier, the aforementioned details were unresolved on the PBS IIc TOF mass spectrometer, which displayed only one broad hump. The poor resolution in this case has no negative consequence as all peaks are different forms of chaperonin 10. However, this deficiency in resolution (and mass accuracy) may be detrimental when the sample contains a second protein of similar molecular weight. Having screened in excess of 40 endometrial homogenates (see later), it was found that a significant fraction of EmCa homogenates carry a protein that is lighter than chaperonin 10 by 8 Da. Figure 26 shows selected SELDI MS profiles, from four EmCa (designated by "C") and two normal endometria (designated by "N"), as collected on the PBS IIc TOF and the QSTAR XL QqTOF mass spectrometers. The pH of the washing buffer was 6.0. The PBS instrument exhibited better sensitivity than the QSTAR However, the profiles generated by the former contain broad humps, while those by the latter exhibit considerable details (Figure 26B). In addition, the mass accuracy of the PBS is considerably inferior to that of the QSTAR. Sample C15 is the simplest of the four EmCa samples and contains only chaperonin 10, its oxidized (10,859 Th) and dehydrated (10,825 Th) forms (compare Figure 25). Sample C8 has a little chaperonin 10 and contains mostly this second protein (10,835 Th), its oxidized (10,851 Th) and dehydrated (10,817 Th) forms. Similarly, sample C6 has some chaperonin 10 admixed with the second protein. In sample C 18, the relative abundance of chaperonin 10 and the unknown protein is approximately 1 to 1. The normal samples show traces of chaperonin 10. The possibility of having two proteins that differ by 8 Da in EmCa samples was confirmed by repeating some experiments, using pH 7.0 washing buffer. In the latter condition, the ProteinChip binds only the unknown protein (Figure 27). The abundances of chaperonin 10 and this second protein in nonmalignant and malignant endometrial tissue homogenates are summarized in Tables 7 and 8, respectively. As SELDI/MALDI analyses on the same sample exhibit considerable variation from shot to shot and from spot to spot, the scales adopted, from "0" (absence) to "5+" (S/N ≥ 10 or higher) are intended to convey semiquantitative rather than quantitative information. For nonmalignant samples, chaperonin 10 and the second protein are absent or present in only very low abundance ("1+") in 22 out of 23 cases; the exception, however, is different for the two proteins. For the malignant samples, two out of 21 cases show absence ofboth chaperonin 10 and the second protein; in addition, two cases show absence of chaperonin 10 but high abundances of the second protein, while another two cases show high abundances of chaperonin 10 but absence of the second protein. The absence of the potential cancer markers in some malignant tissue homogenates may be due to nonoptimal MS conditions and/or absence of tumors in the tissue block homogenized and analyzed.

### Identification of the unknown protein

The vast majority of SELDI-MS applications use spectral signatures from sera to differentiate normal and diseased states without actual protein identification [22, 25, 30, 105-108]. This strategy has come under criticisms and the clinical effectiveness of serum proteomics is currently being questioned [109-119]. The identification of chageronin 10 sin EmCa tissue homogenates as a marker for endometrial cancer is described above. The identification strategy was based on SEC, SDS PAGE isolation, protein extraction, trypsin digestion and MALDI-MS/MS identification. However, the preferred strategy is shotgun nanoLC/MS/MS after initial purification using SEC.

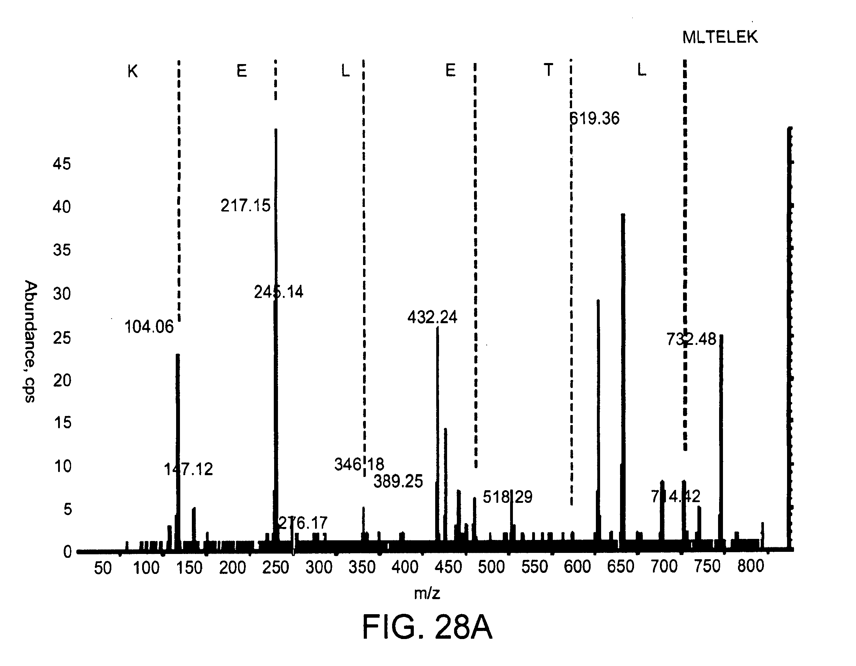

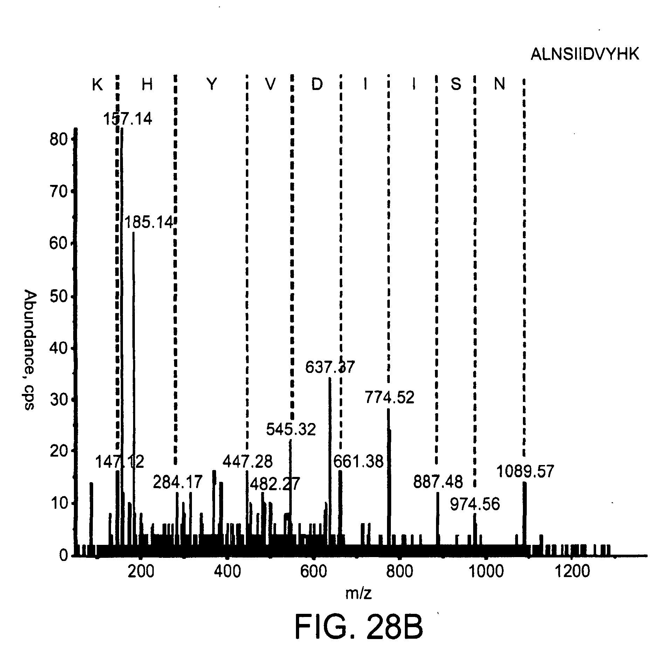

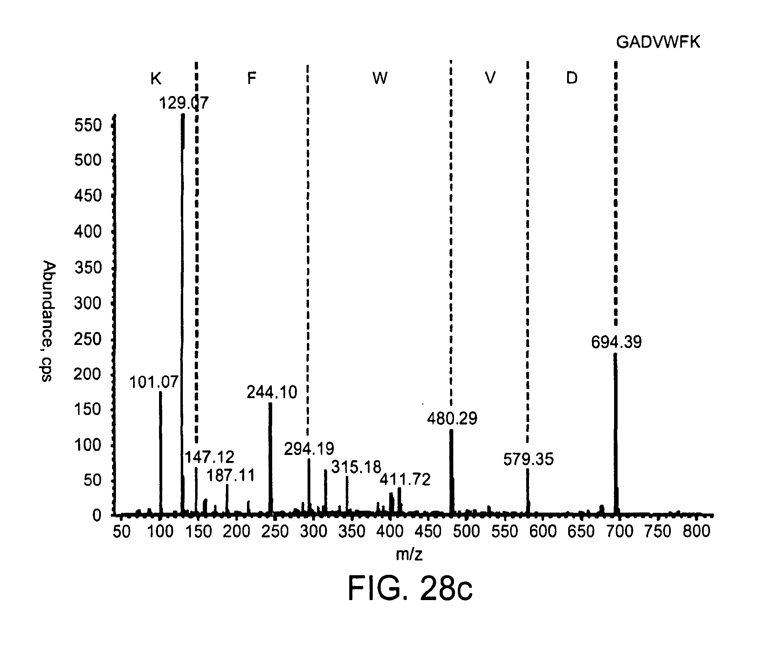

The unknown protein has a molecular weight of 10,835 Da. Using the TagIdent tool available at the ExPASy Website [133] with the following entries: molecular weight, 10,835 ± 2 Da (± 0.02%); pI, 7 ± 5; and organism classification, human to search the Swiss-Prot database that contains 151,047 entries, returned only two proteins, calgranulin A (accession number, P05109) and small nuclear ribonuclear protein D homolog (accession number, Q9Y333). A sample was used that contained the 10,835 Da protein in abundance for SEC. This protein was found to have the highest concentration in SEC fraction 7. NanoLC/MS/MS of the tryptic peptides contained in this fraction identified a number of proteins (Table 9). The only protein that has the correct molecular weight is calgranulin A, 10,834 Da. The other proteins all have higher molecular weights (see later). Figure 28 shows MS/MS spectra of three tryptic peptides, (A) MLTELEK [SEQ ID NO. 50], (B) ALNSIIDVYHK [SEQ ID NO. 51] and (C) GADVWFK [SEQ ID NO. 52], that unambiguously identify calgranulin A. The first two peptides constitute residues 1-18, the third peptide residues 50-56. The three peptides constitute a sequence coverage of 25 out of a total of 93 residues or 27%.

### Calgranulin

Calgranulin A is a member of the S100 family of calcium-binding proteins [134]. It is also known as S100 calcium-binding protein A8, migration inhibitory factor-related protein 8 (MRP-8), cystic fibrosis antigen (CFAG), P8, leukocyte L1 complex light chain, and calprotectin L1L subunit. It has been reported as a specific protein for several diseases [135-156], but has not been implicated in EmCa. Calgranulin A can form homodimers as well as higher-order oligomers [139]. Calgranulin A has been found to be associated with a second protein in the S100 family, calgranulin B (13,242 Da) [137,139]. This heterodimer is thought to be involved in inflammation, including the transmigration process of leukocytes and transport of arachidonic acid to target cells [134,137]. In the present study, calgranulin B was also identified along with calgranulin A, albeit by only one peptide. The possibility that calgranulin A and B coexist as members of a larger unit is in accordance with the observation that calgranulin eluted early in SEC in fraction 7 along with other proteins of much higher molecular weights (Table 9). However, calgranulin B does not appear to be retained on the ProteinChips under conditions that are favorable for calgranulin A.

### Tissue microarray and immunbistochemical localization of calgranulin A

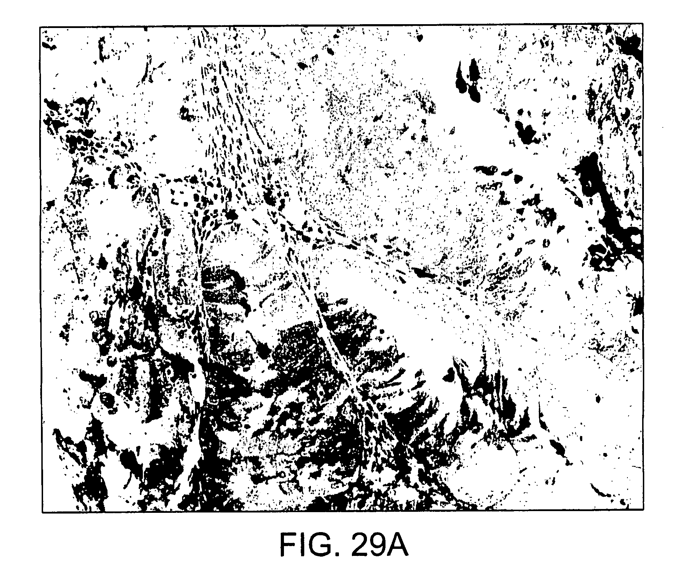

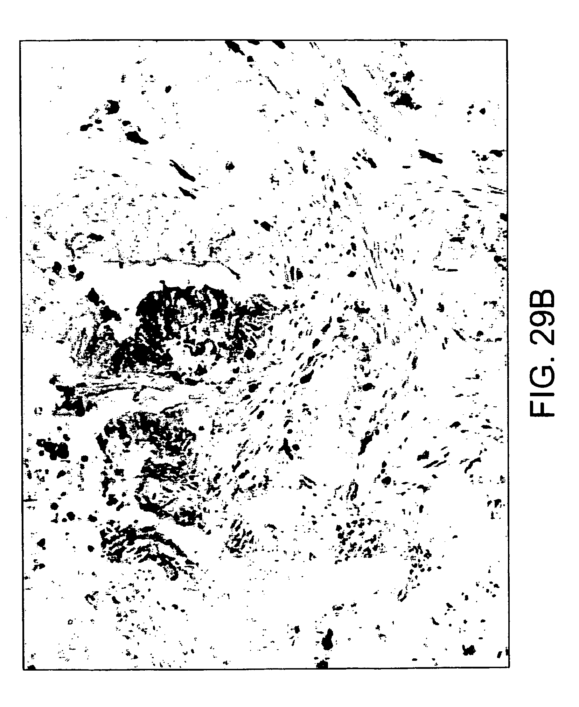

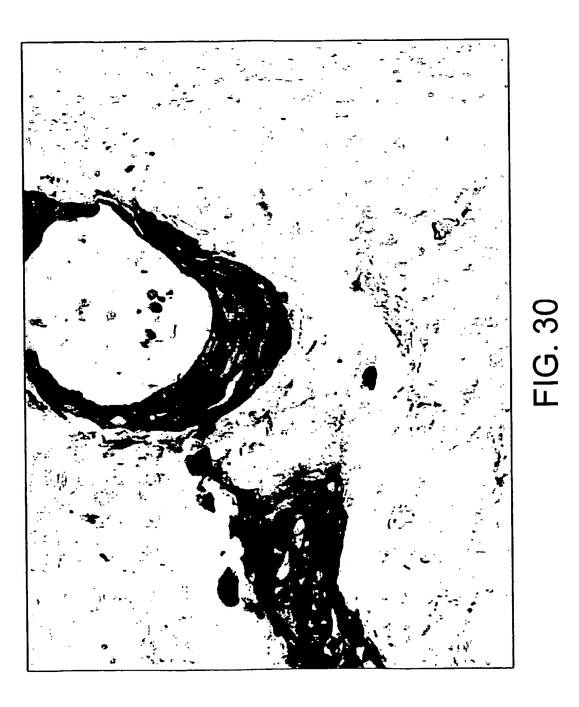

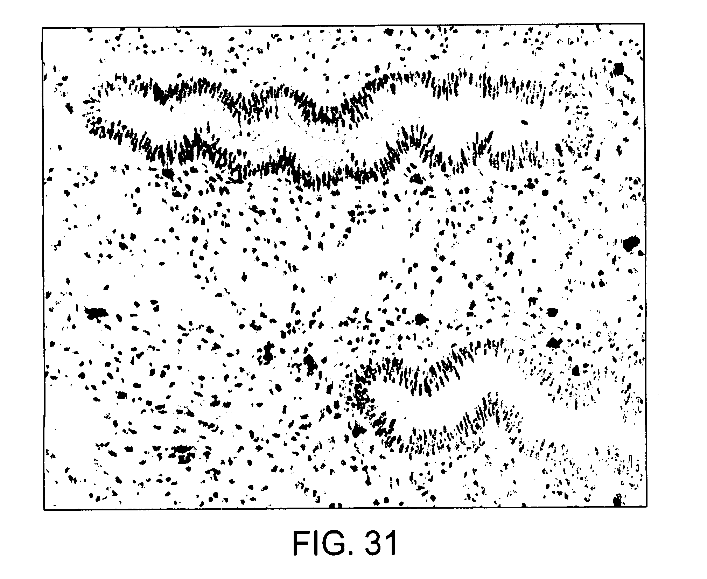

A summary of the TMA immunohistochemical studies using two different monoclonal antibodies (RDI and USB) is shown in Table 10. There is great similarity/identity between the findings from the two immunohistochemical studies. Calgranulin A was localized to the malignant epithelium of endometrial adenocarcinoma and carcinosarcoma. Approximately half of these malignant endometrial tissues showed 1+ to 3+ immunostaining for calgranulin A (Figure 29A and B). The most striking positivity was noted in the squamous areas of three endometrioid carcinomas (Figure 30). Two of four non-endometrial carcinomas also exhibited immunostaining for calgranulin A in the malignant epithelium. Immunohistochemical staining for calgranulin A was absent in 9 of 10 benign endometrial cases. A single case of menstrual endometrium exhibited focal immunostaining (1+). Macrophages/granulocytes in benign endometrium (Figure 31), malignant endometrium (Figure 29), and non-endometrial carcinomas showed discrete strong immunostaining of variable numbers of cells with immunostaining ranging from 1+ to 3+. No immunostaining for calgranulin A was noted in stroma or myometrium.

The MS results presented here show that elevated levels of calgranulin A were detectable in about two-thirds of EmCa homogenate cases, with 14 of 21 cases demonstrating 2+ or greater abundance for this protein (Table 8). By contrast, only minor levels were observed in most nonmalignant endometrium, with 22 of 23 cases demonstrating 1+ or lower abundance (Table 7).

The TMA findings not only confirm the presence of calgranulin A, at least as assessed by immunohistochemistry, but also indicate two cellular sources for the MS findings. In EmCa, the sources are both the malignant epithelium (carcinoma) and macrophage/granulocytes, whereas in nonmalignant endometrium, macrophages seem to be the predominat source, and only rarely benign (menstrual) endometrium (Table 10). The TMA analysis identified calgranulin A not only in a greater proportion of the epithelium in the EmCa cases, but also with greater intensity, than in nonmalignant endometrial cases. Both cases of atypical hyperplasia showed 1+ immunostaining of the affected endometrial glands.

Employment of calganulin A, in combination with other protein markers (including chaperonin 10), may provide a robust endometrial cancer diagnostic and/or screening panel. The utility of any panel will increase with the number of markers employed, and the sensitivity and specificity of individual markers. This study demonstrates the complementary nature of MS and TMA in identifying and localizing sources of potential cancer markers in this search, and a possible utility of TMA in selecting cancer markers.

### Conclusion

High mass accuracy and high resolution in the MS stage are crucial in SELDI MS. Using the QSTAR XL, two potential cancer markers were resolved and subsequently identified offline as chaperonin 10 and calgranulin A, that differ by 8 Da in mass. These two proteins were unresolved on the PBS IIc mass spectrometer. Two offline protein identification protocols were developed: the first was based on SEC, SDS PAGE, protein extraction, trypsin digestion and MALDI-MS/MS; the second on SEC and shotgun nanoLC/MS/MS. Analyses on a cohort of 44 endometrial homogenates showed 22 out of 23 nonmalignant samples had nondetectable to very low abundance of chaperonin 10 and calgranulin A; 17 of the 21 malignant samples had detectable to abundant levels of both proteins. Immunohistochemical staining of a tissue microarray of 32 samples showed that approximately half of malignant endometrial tissues exhibited positive staining for calgranulin A in the malignant epithelium, while 9 out of 10 benign tissues exhibited negative epithelial staining. In addition, macrophages/ganulocytes in malignant as well as nonmalignant tissues showed positive staining. No immunostaining occurred in stroma or myometrium. Calgranulin A, in combination with chaperonin 10 and other proteins, can constitute a panel of markers to permit diagnosis and screening of endometrial cancer.

**Table 1**

| **Differentially Expressed Proteins in Endometrial Malignancies/Cancer** | | | |
|---|---|---|---|
| ***Protein*** | ***Gene name*** | ***Swiss-Prot Acc. No.*** | ***Expression in EmCa*** |
| | | | |
| Chaperonin 10 | HSPE1 | Q04984 and AAH23518 [SEQ ID 1] | Up |
| | | NM_002157 and U07550 [SEQ ID 2] | |
| | | | |
| Calgranulin A | S100A8 | P05109 [SEQ ID 3] | Up |
| | | A12027 [SEQ ID 4] | |
| | | NM_002964 [SEQ ID 5] | |
| | | | |
| Calgranulin B | S100A9 | P06702 [SEQ ID 6] | Up |
| | | X06233 [SEQ ID 7] | |
| | | M21064 [SEQ ID 8] | |
| | | | |
| Polymeric-immunoglobulin Receptor [precursor] | PIGR | P01833 or Q81ZY7 [SEQ ID 9] | Up |
| | | NM_002644 [SEQ ID 10] | |
| | | | |
| Phosphatidylethanolamine-binding protein (PEBP) | PBP | P30086 [SEQ ID 11] | Up |
| | | NM_002567[SEQ ID 12] | |
| | | | |
| Acidic leucine-rich nuclear phosphoprotein 32 family member A | ANP32A | P39687 [SEQ ID 13] | Up |
| | | NM_006305 [SEQ ID 14] | |
| | | | |
| Heat shock 70 kDa protein 6 | HSPA6 | P17066 [SEQ ID 15] | Up |
| | | NM_002155 [SEQ ID 16] | |
| | | X51757 [SEQ ID 17] | |
| | | | |
| Macrophage migration Inhibitory factor (MIF) | MIF | P14174 [SEQ ID 18] | Up |
| | | NM_002415 [SEQ ID 19] | |
| | | L19686 [SEQ ID 20] | |
| | | | |
| Calgizzarin (S100C protein) | S100A11 | P31949 [SEQ ID 21] | Up |
| | | NM_005620 and D38583 [SEQ ID 22] | |
| | | | |
| Triosephosphate isomerase | TPI1 | P00938 and NP_000356 [SEQ ID 23] | Up |
| | | NM_000365 [SEQ ID 24] | |
| | | X69723 [SEQ ID 25] | |
| | | | |
| Alpha-1-antitrypsin precursor | SERPINA1 | gi/1703025 | |
| | | ITHU and P01009 [SEQ ID 30] | Under |
| | | NM_000295[SEQ ID NO. 31] | |
| | | K02212 [SEQ ID 32] | |
| | | | |
| Creatine kinase, B chain (B-CK) | CKB | gi/125294 | Under |
| | | P12277[SEQ ID NO.33] | |
| | | NM_001823 [SEQ ID NO 34] | |
| | | X15334 [SEQ ID NO 35] | |
| Pyruvate kinase, M1 or M2 isozyme | PKM2 | gi/20178296; gi/125604; KPY1_HUMAN (P14618) [SEQ ID NO.36] | Up |
| | | X56494 [SEQ ID 37] | |
| | | | |
| Transgelin (smooth muscle protein 22-alpha) | TAGLN | gi/3123283 | Under |
| | | Q01995 [SEQ ID.38] | |
| | | D84342 [SEQ ID 39] | |
| | | | |
| Heterologous nuclear ribonucleoprotein D | (hnRPD | ROD_HUMAN (Q14103) [SEQ ID NO.40] | Up |
| | | AF026126 [SEQ ID 41] | |

**Table 2**

| **The identification of Chaperon in 10 by mass spectrometry and Western blotting techniques in non-malignant endometrial tissue homogeneates.** | | | |
|---|---|---|---|
| Case | Histopathologic Classification | MS results (relative intensity) | Western Blotting Results (relative intensity) |
| 1 | Proliferative | + | o |
| 2 | Proliferative | + | o |
| 3 | Secretory | + | o |
| 4 | Atrophic | + | + |
| 5 | Benign polyp | o | o |
| 6 | Atrophic | + | + |
| 7 | Secretory | o | o |
| 8 | Disordered proliferative endometrium | o | + |
| 9 | Secretory | o | + |
| 10 | Disordered proliferative | o | + |
| 11 | Secretory | o | + |
| 12 | Secretory | + | + |
| 13 | Secretory | o | + |
| 14 | Secretory | + | o |
| 15 | Atrophic | o | o |
| 16 | Secretory | + | + |
| 17 | Menstrual | + | + |
| 18 | Secretory | + | + |
| 19 | Proliferative | + | o |
| 20 | Proliferative | + | + |
| 21 | Menstrual | o | o |
| 22 | Atrophic | o | o |
| 23 | Atrophic | ++ | + |

**Table 3**

| **The identification of Chaperonin 10 by mass spectrometry and Western blotting techniques in malignant endometrial tissue homogeneates.** | | | |
|---|---|---|---|
| Case | Histopathologic Classification | MS results (relative intensity) | Western Blotting Results (relative intensity) |
| 24 | Em AdCa 1/3 | ++ | ++ |
| 25 | Em AdCa 1/3 | O | + |
| 26 | MMMT | +++++ | +++++ |
| 27 | Em AdCa | ++ | ++ |
| 28 | MMMT* | + | o |
| 29 | Em AdCa 2/3 | + | +++ |
| 30 | Em AdCa 2/3 | + | ++++ |
| 31 | Ser AdCa | + | +++++ |
| 32 | Em AdCa 1/3 | +++++ | +++++ |
| 33 | Em AdCa Grade n.k. | O | ++++ |
| 34 | Em AdCa Grade n.k. | + | +++ |
| 35 | Em AdCa Grade n.k. | O | + |
| 36 | Em AdCa 1/3 | +++++ | ++++ |
| 37 | Em AdCa 1/3 | ++++ | +++ |
| 38 | Em AdCa 1/3 | +++ | +++ |
| 39 | Muc AdCa 1/3 | ++ | o |
| 40 | Em AdCa 1/3 | +++ | +++ |
| 41 | Em AdCa 1/3 | +++ | ++ |
| 42 | Em AdCa - Ser AdCa | +++++ | +++++ |
| 43 | Em** AdCa 1/3 | o | o |
| 44 | Em AdCa 2/3 | ++++ | ++++ |

| | | | |
|---|---|---|---|
| *Mirror section showed minimal tumor. **Mirror image showed necrotic tumor only. Em AdCa = Endometrioid adenocarcinoma MMMT = Malignant Mixed Mullerian Tumor Muc AdCa = Mucinous adenocarcinoma n.k. = not known Ser AdCa = Serous adenocarcinoma | | | |

**Table 4**

| **Differentially expressed proteins quantified by iTRAQ.** | | | | |
|---|---|---|---|---|
| **S: P is the ratio of secretory phase relative to the proliferative phase** | | | | |
| **C1: P and C2: P are the ratios of cancer samples 1 and 2 relative to the proliferative phase, respectively.** | | | | |
| **accession_id** | **name** | **S: P (± SD)** | **C1: P (± SD)** | **C2: P (± SD)** |
| gi\|461730 | 10 kDa heat shock protein, mitochondrial (Hsp10) (10 kDa chaperonin) (CPN10) | 1.06 ± 0.12 | 2.71 ± 0.52 | 2.23 ± 0.29 |
| | | | | |
| gi\|1703025 | Alpha-1-antitrypsin precursor | 1.25 ± 0.39 | 0.34 ± 0.07 | 0.44 ± 0.16 |
| gi\|125294 | Creatine kinase, B chain (B-CK) | 0.96 ± 0.04 | 0.38 ± 0.16 | 0.52 ± 0.06 |
| gi\|20178296 or gi\|125604 | Pyruvate kinase, M 1 or M2 isozyme | 1.03 ± 0.11 | 2.75 ± 0.03 | 2.02 ± 0.08 |
| | | | | |
| gi\|3123283 | Transgelin (Smooth muscle protein 22-alpha) | 1.46 ± 0.26 | 0.26 ± 0.04 | 0.45 ± 0.05 |

**Table 5**

| **Differentially expressed proteins quantified using cICAT. Singleton H signifies extreme Overexpression in the cancer sample relative to the normal proliferative control. Standard deviations were calculated from all three sample pairs.** | | |
|---|---|---|
| **Accession #** | **Name** | **Avg. C: N ratio (± SD)** |
| S111_HUMAN (P31949) | Calgizzarin (S100C protein) (MLN 70). | Singleton H^{a} |
| ROD_HUMAN (Q14103) | Heterogeneous nuclear ribonucleoprotein D0 | 4.62 (± 0.39) |
| MIF_HUMAN (P14174) | Macrophage migration inhibitory factor (MIF) | 2.74 (± 0.92) |
| PEBP_HUMAN (P30086) | Phosphatidylethanolamine-binding protein (PEBP) | 3.72 (± 1.03) |
| KPY1_HUMAN (P14618) | Pyruvate kinase, M1 isozyme (EC 2.7.1.40) | 4.61 (± 1.40) |
| KPY_HUMAN: PIGR_HUMAN (P01833) | Polymeric-immunoglobulin receptor precursor | 9.83 (+/- 6.61) |

| | | |
|---|---|---|
| a: Calculation of SD is not possible | | |

**Table 6**

| **Proteins Identified and Secretory / Proliferative Expression Levels** | | | | |
|---|---|---|---|---|
| **Accession #** | **Description** | **Average H:L ratio** | **SD** | **Frequency** |
| 143T_HUMAN (P27348) | 14-3-3 protein tau (14-3-3 protein theta) | **0.81** | | 1 |
| RS12_ORENI (O13019) | 40S ribosomal protein S12. | **0.52** | | 1 |
| RS21_HUMAN (P35265) | 40S ribosomal protein S21. | **NQ** | | 1 |
| HPPD_MYCGR (042764) | 4-hydroxyphenylpyruvate dioxygenase (EC 1.13.11.27) | **Singleton L** | | 1 |
| RL36_VIBCH (P78001) | 50S ribosomal protein L36. | **0.84** | | 1 |
| ACTA_HUMAN (P03996) or ACTS_HUMAN (P02568) | Actin, aortic smooth muscle or Actin alpha skeletal muscle | **1.86** | | 1 |
| ACTB_HUMAN (P02570) | Actin, cytoplasmic | **0.93** | 0.04 | 3 |
| SAH1_XENLA (P51893) | Adenosylhomocysteinase 1 (EC 3.3.1.1) | **0.70** | | 1 |
| AFAM_HUMAN (P43652) | Afamin precursor (Alpha-albumin) (Alpha-Alb). | **NQ** | | 1 |
| ADHL_HUMAN (P11766) | Alcohol dehydrogenase class III L chain | **0.67** | | 1 |
| DHA1_HUMAN (P00352) | Aldehyde dehydrogenase 1A1 (EC 1.2.1.3) | **0.60** | | 1 |
| A1AG_HUMAN (P02763) | Alpha-1-acid glycoprotein 1 precursor (AGP 1) | **1.1** | 0.5 | 3 |
| A2HS_HUMAN (P02765) | Alpha-2-HS-glycoprotein precursor (Fetuin-A) | **0.82** | 0.54 | 2 |
| ALU5_HUMAN (P39192) | Alu subfamily | **1.1** | | 1 |
| AOP2_HUMAN (P30041) | Antioxidant protein 2 (1-Cys Peroxiredoxin) | **NQ** | | 1 |
| TRAI_AGRT5 (P33907) | Autoinducer synthesis protein traI. | **0.55** | | 1 |
| APOH_HUMAN (P02749) | Beta-2-glycoprotein I precursor (Apolipoprotein H) | **0.58** | | 1 |
| ARK1_HUMAN (P25098) | Beta-adrenergic receptor kinase 1 (EC 2.7.1.126) | **Singleton L** | | 1 |
| BPEA_HUMAN (O94833) | Bullous pemphigoid antigen 1, isoforms 6/9/10 | **0.99** | | 1 |
| CLP1_HUMAN (P51911) | Calponin H1, smooth muscle (Basic calponin) | **1.8** | | 1 |
| KAP3_HUMAN (P31323) | cAMP-dependent protein kinase type II-beta | **Singleton L** | | 1 |
| CABA_MOUSE (Q99020) | CARG-binding factor-A (CBF-A). | **0.97** | 0.33 | 3 |
| CATB_HUMAN (P07858) | Cathepsin B precursor (EC 3.4.22.1) (Cathepsin B1) | **0.88** | 0.70 | 2 |
| CB32_YEAST (P40969) | Centromere DNA-binding protein complex CBF3 subunit | **NQ** | | 1 |
| BPHB_BURCE (P47227) | Cis-2,3-dihydrobiphenyl-2,3-diol dehydrogenase (EC | **Singleton L** | | 1 |
| CLH_BOVIN (P49951) | Clathrin heavy chain. | **0.62** | | 1 |
| COF1_HUMAN (P23528) | Cofilin, non-muscle isoform (18 kDa phosphoprotein) | **0.95** | 0.15 | 3 |
| CO3_HUMAN (P01024) | Complement C3 precursor | **1.1** | 0.1 | 2 |
| CFAH_HUMAN (P08603) | Complement factor H precursor (H factor 1). | **NQ** | | 1 |
| AXO1_CHICK (P28685) | Contactin 2 precursor (Axonin-1). | **0.85** | | 1 |
| KCRB_HUMAN (P12277) | Creatine kinase, B chain (EC 2.7.3.2) (B-CK). | **6.0** | | 1 |
| CRBH_HUMAN (P82279) | Crumbs protein homolog 1 precursor. | **NQ** | | 1 |
| PYRG_STAAM (Q99SD1) | CTP synthase (EC 6.3.4.2) (UTP-ammonia ligase) (C | **Singleton H** | | 1 |
| CYSR_HUMAN (P21291) | Cysteine-rich protein 1 (CRP1) (CRP). | **4.4** | | 1 |
| DDX4_HUMAN (Q9NQ10) | DEAD-box protein 4 (VASA homolog). | **Singleton H** | | 1 |
| DEST_HUMAN (P 18282) | Destrin (Actin-depolymerizing factor) (ADF). | **2.4** | | 1 |
| EF2_HUMAN (P13639) | Elongation factor 2 | **0.60** | | 1 |
| XYNA_PIRSP (Q12667) | Endo-1,4-beta-xylanase A precursor (EC 3.2.1.8) (1 | **0.32** | | 1 |
| ENOA_HUMAN (P06733) or MPB1_HUMAN (P22712) | Enolase (EC 4.2.1.11) (2-phosphoglycerate dehydrat.) or c-myc promoter binding protein (MBP-1) | **2.6** | 1.9 | |
| ENV_SIVM1^{a} (P05885) | Envelope polyprotein GP160 precursor | **Singleton L** | | 3 |
| LEM2_CANFA (P33730) | E-selectin precursor (Endothelial leukocyte adhesi | **Singleton H** | | 1 |
| FIBG_HUMAN (P02679) | Fibrinogen gamma chain precursor | **1.2** | | 1 |
| FLNA_HUMAN (P21333) | Filamin A (Alpha-filamin) (Filamin 1) | **1.6** | 0.8 | 3 |
| ALFA_HUMAN (P04075) | Fructose-bisphosphate aldolase A (EC 4.1.2.13) (Mu | **1.2** | 0.2 | 2 |
| G25P_HUMAN (P25763) or RAC1_HUMAN (P15154) or RHOJ_HUMAN (Q9H4E5) | G25K GTP binding protein (CDC 42 homolog) or Ras related C3 botulinum toxin substrate 1 or 2 or 3 or Rho-related GTP-binding protein Rho G or RhoJ | **NQ** | | 1 |
| | | | | |
| LEG1_CRIGR (P48538) | Galectin-1 (Beta-galactoside-binding lectin L-14-I) | **0.67** | 0.52 | 3 |
| GELS_HUMAN (P06396) | Gelsolin (Actin-depolymerizing factor) (ADF) | **1.4** | | 2 |
| NMZ1_HUMAN (Q05586) | Glutamate [NMDA] receptor subunit zeta 1 precursor | **Singleton H** | | 3 |
| GTP_HUMAN (P09211) | Glutathione S-transferase P (EC 2.5.1.18) | **1.3** | 0.4 | 3 |
| G3P2_HUMAN^{b} (P04406) | Glyceraldehyde 3-phosphate dehydrogenase | **1.0** | 0.0 | 3 |
| PAEP_HUMAN (P09466) | Glycodelin precursor (GD) (Pregnancy-associated en | **Singleton H** | | 1 |
| HPT1_HUMAN (P00737) | Haptoglobin-1 precursor. | **2.2** | 1.6 | 2 |
| HS70_HUMAN (08107) | Heat shock cognate 71 kDa protein. | **1.3** | 1.1 | 3 |
| HS9A_HUMAN (P07900) | Heat Shock Protein HSP90-alpha | **NQ** | | 1 |
| HBB_HUMAN (P02023) | Hemoglobin beta chain. | **0.60** | 0.45 | 3 |
| HEMO_HUMAN (P02790) | Hemopexin precursor (Beta-1B-glycoprotein). | **1.1** | 0.3 | 3 |
| ROD_HUMAN (Q14103) | Heterogeneous nuclear ribonucleoprotein D0 (hnRNP D0) | **1.0** | 0.2 | 3 |
| ROK_HUMAN (Q07244) | Heterogeneous nuclear ribonucleoprotein K (hnRNP K) | **0.68** | | 1 |
| HNT1_HUMAN (P49773) | Histidine triad nucleotide binding protein 1 | **1.4** | | 1 |
| YF48_HUMAN (Q9HCM4) | Hypothetical protein KIAA1548 | **0.85** | | 1 |
| Y885_MYCTU (Q10546) | Hypothetical protein Rv0885. | **NQ** | | 1 |
| GC1_HUMAN (P01857) | Ig gamma-1 chain C region. | **0.76** | 0.23 | 3 |
| GC2_HUMAN (P01859) or GC4_HUMAN (P01861) | Ig gamma-2 chain C region. Or Ig gamma-4 chain C region | **1.0** | 0.5 | 3 |
| GC3_HUMAN (P01860) | Ig gamma-3 chain C region | **0.85** | 0.22 | 3 |
| KAC_HUMAN (P01834) | Ig kappa chain C region. | **0.88** | 0.37 | 3 |
| HUMAN (P01876) or (P01877) | Ig alpha - 1 chain C region or Ig alpha-2 chain C region | **1.4** | | 1 |
| IDI2_RICGN^{c} (Q92HM7) | Isopentenyl-diphosphate delta-isomerase (EC 5.3.3. | **Singleton H** | | 3 |
| TRFL_MOUSE (P08071) | Lactotransferrin precursor (Lactoferrin). | **Singleton H** | | 1 |
| LGUL_HUMAN (Q04760) | Lactoylglutathione lyase (EC 4.4.1.5) (Methylglyox | **1.0** | 0.2 | 2 |
| CSR3_HUMAN (P50461) | LIM domain protein, cardiac (Muscle LIM protein) ( | **4.2** | | 1 |
| LDH_HUMAN (P00338) or (P07195) | L-lactate dehydrogenase (EC 1.1.1.27) (LDH). | **1.4** | 0.3 | 3 |
| MIF_HUMAN (P14174) | Macrophage migration inhibitory factor (MIF) | **1.2** | 0.7 | 3 |
| MSRE_MOUSE (P30204) | Macrophage scavenger receptor types I and II | **Singleton H** | | 1 |
| MDHM_HUMAN (P40926) | Malate dehydrogenase, mitochondrial precursor | **NQ** | | 2 |
| MSP1_PLAF3 (P19598) | Merozoite surface protein 1 precursor | **Singleton L** | | 1 |
| MU5A_HUMAN (P98088) | Mucin 5AC (Mucin 5 subtype AC, tracheobronchial) | **NQ** | | 1 |
| MYH9_HUMAN (P35579) or MYHA_HUMAN (P35580) | Myosin heavy chain non muscle type A or B | **0.29** | | 1 |
| KML2_RABIT (P07313) | Myosin light chain kinase 2 | **Singleton H** | | 3 |
| NIR_NEUCR (P38681) | Nitrite reductase [NAD(P)H] (EC 1.7.1.4). | **1.3** | | 1 |
| NOG3_BRARE (Q9YHV3) | Noggin 3 precursor. | **1.3** | | 1 |
| NDKA_HUMAN (P15531) or NDKB_HUMAN (P22392) | Nucleotide diphosphate kinase A or B | **1.2** | | 1 |
| CYPH_HUMAN (P05092) | Peptidyl-prolyl cis-trans isomerase A (EC 5.2.1.8) | **1.2** | 0.2 | 3 |
| PEBP_BOVIN (P13696) | Phosphatidylethanolamine-binding protein (PEBP) (B | **1.0** | 0.2 | 2 |
| PGK_HUMAN (P00559) | Phosphoglycerate kinase (EC 2.7.2.3). | **0.99** | | 1 |
| PMG1_HUMAN (P18669) | Phosphoglycerate mutase 1 (EC 5.4.2.1) (EC 5.4.2.4 | **0.73** | | 1 |
| POL_RSVP^{d} (P03354) | POL polyprotein | **Singleton H** | | 2 |
| PIGR_HUMAN (P01833) | Polymeric-immunoglobulin receptor precursor (Poly- | **1.1** | | 1 |
| AR34_DROME (Q9VIM5) | Probable ARP2/3 complex 34 kDa subunit (P34-ARC). | **3.5** | | 1 |
| AMPA_CAMJE (Q9PP04) | Probable cytosol aminopeptidase (EC 3.4.11.1) (Leu | **0.93** | | 1 |
| PRO1_HUMAN (P07737) | Profilin I | **1.1** | | 1 |
| PROP_HUMAN (P27918) | Properdin precursor (Factor P). | **Singleton H** | | 1 |
| PRTH_PORGI (P46071) | Protease prtH (EC 3.4.22.-). | **Singleton L** | | 1 |
| PSA5_HUMAN (P28066) | Proteasome subunit alpha type 5 (EC 3.4.25.1) (Pro | **2.1** | | 1 |
| VN02_VACCC (P20641) | Protein N2. | **Singleton L** | | 1 |
| FRT1_HUMAN (Q92837) | Proto-oncogene FRAT1 | **Singleton H** | | 3 |
| O22C_DROME (P81911) | Putative odorant receptor 22c. | **0.90** | 0.31 | 3 |
| KPY1_HUMAN (P14618) | Pyruvate kinase, M1 isozyme (EC 2.7.1.40) (Pyruvat | **1.2** | 0.4 | 3 |
| CUT1_SCHPO (P18296) | Separin (EC 3.4.22.-). | **NQ** | | 1 |
| TRFE_HUMAN (P02787) | Serotransferrin precursor (Transferrin) (Siderophi | **1.3** | 0.4 | 3 |
| ALBU_HUMAN (P02768) | Serum albumin precursor. | **1.1** | 0.3 | 3 |
| STC_DROME (P40798) | Shuttle craft protein. | **1.1** | | 1 |
| SLI1_HUMAN (Q13642) | Skeletal muscle LIM protein 1 | **2.0** | | 1 |
| PCP1_SCHPO (Q92351) | Spindle pole body protein pcp1. | **Singleton H** | | 1 |
| SODC_HUMAN (P00441) | Superoxide dismutase [Cu-Zn] | **0.92** | 0.33 | 3 |
| THIO_HUMAN (P10599) | Thioredoxin. | **0.87** | | 1 |
| TPIS_HUMAN (P00938) | Triosephosphate isomerase (EC 5.3.1.1) (TIM). | **1.1** | 0.5 | 3 |
| TPM1_HUMAN (P09493) | Tropomyosin 1 alpha chain (Alpha-tropomyosin). | **2.3** | 0.7 | 2 |
| TPM4_HUMAN (P07226) or TPM2_HUMAN (P07951) | Tropomyosin alpha 4 chain or beta 2 chain | **0.75** | | 1 |
| TBA_HUMAN (P05209) | Tubulin alpha chain. | **1.2** | 0.5 | 3 |
| TBB_HUMAN (P07437 or P05217)) | Tubulin beta chain (Fragment). | **0.77** | 0.25 | 3 |
| PTK7_HUMAN (Q13308) | Tyrosine-protein kinase like 7 precursor | **0.35** | | 1 |
| SPK1_DUGTI (P42687) | Tyrosine-protein kinase SPK-1 (EC 2.7.1.112). | **Singleton L** | | 1 |
| VSI1_TRYBB (P26326) | Variant surface glycoprotein ILTAT 1.21 precursor | **Singleton L** | | 1 |
| PGCV_MACNE (Q28858) | Versican core protein | **4.3** | | 1 |
| VIME_HUMAN (P08670) | Vimentin (Fragment). | **1.6** | 2.2 | 3 |
| VINC_HUMAN (P18206) | Vinculin (Metavinculin). | **3.1** | | 2 |
| VTDB_HUMAN (P02774) | Vitamin D-binding protein precursor (DBP) (Group-s | **1.3** | 0.4 | 2 |
| WDR1_HUMAN (075083) | WD-repeat protein 1 (Actin interacting protein 1) | **1.6** | 1.0 | 2 |
| Z363_HUMAN (Q96PM5) | Zinc finger protein 363 | **Singleton L** | | 1 |

| | | | | |
|---|---|---|---|---|
| Note: Frequency is the number of sample pairs in which the protein is observed NQ = Not quantified Singleton L = Only observed in Proliferative phase Singleton H = Only observed in Secretory phase ^{a} : Subsequently identified by Mascot as Clostridium putative phosphatidylserine decarboxylase proenzyme (gi: 28209853) ^{b} : Internal standard to which other proteins' peak areas are normalized ^{c} : *Rickettsia conorii* protein ^{d} : Subsequently identified by Mascot as unlabeled serum albumin | | | | |

**Table 7**

| **Protein abundances in nonmalignant endometrial tissue homogenates.** | | | |
|---|---|---|---|
| Case | Histopathologic Classification | Chaperonin 10 (relative abundance)^{†} | Second protein^{‡} (relative abundance)^{†} |
| 1 | Proliferative | 1+ | 0 |
| 2 | Proliferative | 1+ | 0 |
| 3 | Secretory | 1+ | 0 |
| 4 | Atrophic | 1+ | 0 |
| 5 | Benign polyp | 0 | 0 |
| 6 | Atrophic | 1+ | 1+ |
| 7 | Secretory | 0 | 1+ |
| 8 | Disordered proliferative | 0 | 0 |
| 9 | Secretory | 0 | 1+ |
| 10 | Disordered proliferative | 0 | 1+ |
| 11 | Secretory | 0 | 1+ |
| 12 | Secretory | 1+ | 0 |
| 13 | Secretory / menstrual | 0 | 5+* |
| 14 | Secretory | 1+ | 0 |
| 15 | Atrophic | 0 | 1+ |
| 16 | Secretory | 1+ | 0 |
| 17 | Menstrual | 1+ | 1+ |
| 18 | Secretory | 1+ | 0 |
| 19 | Proliferative | 1+ | 0 |
| 20 | Proliferative | 1+ | 0 |
| 21 | Menstrual | 0 | 0 |
| 22 | Atrophic | 0 | 1+ |
| 23 | Atrophic | 2+ | 1+ |

| | | | |
|---|---|---|---|
| ^{†} 0 = absence, 1 + = S/N 2-3, 2+ = S/N 3-5, 3+ = S/N 5-7, 4+ = S/N 7-10, 5+ = S/N ≥ 10. ^{‡} calgranulin A. * cervical cancer present several centimeters away. | | | |

**Table 8**

| **Protein abundances in malignant endometrial tissue homogenates.** | | | |
|---|---|---|---|
| Case | Histopathologic Classification | Chaperonin 10 (relative abundance)^{†} | Second protein^{‡} (relative abundance)^{†} |
| 24 | Em AdCa 1/3 | 2+ | 1+ |
| 25 | Em AdCa 1/3 | 0 | 5+ |
| 26 | MMMT | 5+ | 1+ |
| 27 | Em AdCa | 2+ | 5+ |
| 28 | MMMT | 1+ | 4+ |
| 29 | Em AdCa 2/3 | 1+ | 3+ |
| 30 | Em AdCa 2/3 | 1+ | 4+ |
| 31 | Ser AdCa | 1+ | 3+ |
| 32 | Em AdCa 1/3 | 5+ | 2+ |
| 33 | Em AdCa Grade n.k. | 0 | 0 |
| 34 | Em AdCa Grade n.k. | 1+ | 3+ |
| 35 | Em AdCa Grade n.k. | 0 | 0 |
| 36 | Em AdCa 1/3 | 5+ | 0 |
| 37 | Em AdCa 1/3 | 4+ | 2+ |
| 38 | Em AdCa 1/3 | 3+ | 5+ |
| 39 | Muc AdCa 1/3 | 2+ | 3+ |
| 40 | Em AdCa 1/3 | 2+ | 2+ |
| 41 | Em AdCa 1/3 | 3+ | 1+ |
| 42 | Em AdCa - Ser AdCa | 5+ | 0 |
| 43 | Em AdCa 1/3 | 0 | 5+ |
| 44 | Em AdCa 2/3 | 4+ | 4+ |

| | | | |
|---|---|---|---|
| † 0 = absence, + = S/N 2-3, 2+ = S/N 3-5, 3+ = S/N 5-7, 4+ = S/N 7-10, 5+ = S/N ≥ 10. ‡ calgranulin A. Em AdCa = Endometrioid adenocarcinoma MMMT = Malignant Mixed Mullerian Tumor Muc AdCa = Mucinous adenocarcinoma n.k. = not known Ser AdCa = Serous adenocarcinoma | | | |

**Table 9**

| **Proteins in SEC fraction 7 of Endometrial Cancer Tissue Identified by nanoLC/MS/MS** | | |
|---|---|---|
| Name | Confidence^{a} | Averaged mol. weight (Da) |
| | | |
| Calgranulin A | 99 | 10,834 |
| Calgranulin B | 99 | 13,242 |
| Heat shock protein 10 | 99 | 10,800 |
| Hemoglobin alpha chain | 90 | 15,126 |
| Hemoglobin beta chain | 99 | 15,867 |
| Putative nucleoside diphosphate kinase | 75 | 15,529 |
| Nucleoside diphosphate kinase A | 75 | 17,149 |
| Nucleoside diphosphate kinase B | 75 | 17,298 |
| Cofilin, non-muscle isoform | 90 | 18,502 |
| Cofilin, muscle isoform | 90 | 18,736 |
| Translationally controlled tumor protein | 90 | 19,595 |
| Peroxiredoxin 1 | 99 | 22,110 |
| Peroxiredoxin 2 | 99 | 21,892 |
| Neutrophil gelatinase-associated lipocalin | 99 | 22,588 |
| Glutathione S-transferase P | 99 | 23,224 |
| Triosephosphate isomerase | 90 | 26,538 |
| Peroxiredoxin 4 | 90 | 30,540 |
| NADPH dehydrogenase | 90 | 30,867 |
| Ig gamma-3 chain C region | 75 | 32,331 |
| Ig gamma-2 chain C region | 75 | 35,884 |
| Ig gamma-4 chain C region | 75 | 35,940 |
| Ig gamma-1 chain C region | 75 | 36,106 |
| C-myc promoter-binding protein | 99 | 37,087 |
| Hetero. nuclear ribonucleoproteins A2 | 99 | 37,429 |
| Cathepsin B precursor | 99 | 37,807 |
| Beta enolase | 75 | 46,855 |
| Alpha enolase | 99 | 47,037 |
| Gamma enolase | 75 | 47,137 |
| Alpha enolase, lung specific | 99 | 49,477 |
| Serum albumin precursor | 99 | 69,366 |
| Polymeric-immunoglobulin receptor precursor | 75 | 83,313 |
| Cone cGMP-specific 3',5'-cyclic phosphodiesterase | 75 | 99,102 |

| | | |
|---|---|---|
| ^{a} "Confidence" value in ProID: the highest possible score is 99. | | |

**Table 10**

| **Immunohistochemical identification of calgranulinA in tissue microarray using two monoclonal mouse antibodies (RDI & USB)** | | | | | | |
|---|---|---|---|---|---|---|
| **Histologic Classification** | **Macrophage/ Granulocytic** | | **Epithelium/ Carcinoma** | | **Stroma/ Myometrium** | |
| | **RDI** | **USB** | **RDI** | **USB** | **RDI** | **USB** |
| | | | | | | |
| Benign endometrium, n = 10‡ | *0*(1) | 0(0) | *0* (9) | 0 (9) | *0* (10) | 0 (10) |
| | *1*+ (6) | 1+ (10) | *1*+ (1) | 1+ (1) | *1*+ (0) | 1+ (0) |
| | *2*+ (3) | 2+ (0) | *2*+ (0) | 2+ (0) | *2*+ (0) | 2+ (0) |
| | *3*+ (0) | 3+ (0) | *3*+ (0) | 3+ (0) | *3*+ (0) | 3+ (0) |
| | | | | | | |
| Atypical endometrial hyperplasia, n = 2 | *1*+ (2) | 1+ (2) | *1*+ (2) | 1+ (2) | *0* (2) | 0 (2) |
| | | | | | | |
| EmCa, n = 13† | *0* (1) | 0 (1) | *0* (6) | 0 (7) | *0* (12) | 0 (13) |
| | *1*+ (7) | 1+ (8) | *1*+ (4) | 1+ (2) | *1*+ (0) | 1+ (0) |
| | *2*+ (3) | 2+ (3) | *2*+ (2) | 2+ (2) | *2*+ (0) | 2+ (0) |
| | *3*+ (1) | 3+ (1) | *3*+ (0) | 3+ (2) | *3*+(0) | 3+ (0) |
| | | | | | | |
| Non-endometrial carcinomas, n = 4 | *0* (0) | 0 (1) | *0* (2) | 0 (2) | *0* (4) | 0 (4) |
| | *1*+ (1) | 1+ (1) | *1*+ (1) | 1+ (1) | *1*+ (0) | 1+ (0) |
| | *2*+ (3) | 2+ (2) | *2*+ (1) | 2+ (1) | *2*+ (0) | 2+ (0) |
| | *3*+ (0) | 3+ (1) | *3*+ (0) | 3+ (0) | *3*+ (0) | 3+ (0) |
| | | | | | | |
| Benign non-endometrial neoplasms, n = 2 | *0* (0) | 0 (1) | *0* (0) | 0 (0) | *0* (0) | 0 (0) |
| | | | | | | |

| | | | | | | |
|---|---|---|---|---|---|---|
| ( ) = Number of samples *0* = No immunostaining *1*+ = Positivity in occasional or few cells *2*+ = Positivity in more abundant cells *3*+ = Strong positivity in numerous cells † = In TMA- RDI, one core of EmCa was not displayed ‡ = I of 11 benign endometrial cores (secretory) did not display in either TMA. | | | | | | |

It must be noted that as used herein and in the appended claims, the singular forms "a", "an", and "the" include plural reference unless the context clearly dictates otherwise. Thus, for example, reference to "a cell" includes a plurality of such cells, reference to the "antibody" is a reference to one or more antibodies and equivalents thereof known to those skilled in the art, and so forth.

Below full citations are set out for references.

### Full Citations for References

1. Buckley CH: Normal endometrium and non-proliferative conditions of the endometrium. In Obstetrical and Gynaecological Pathology. Fox H, Wells M. Eds. Churchill-Livingstone. 5th Ed. London, 2003, Pgs.391-442.
2. Andersen JS, Mann M (2000) FEBS Lett 480: 25-31.
3. Yao X, Freas A, Ramirez J, Demirev PA, Fenselau C (2001) Anal Chem 73: 2836-2842.
4. Geng M, Ji J, Regnier F (2000) J Chromoatogr A 870: 295-313.
5. Münchbach M, Quadroni M, Miotto G, James P (2000) Anal Chem 72: 4047-4057.
6. Gygi SP, Rist B, Gerber SA, Turecek F, Gelb MH, Aebersold R (1999) Nat Biotechnol 17: 994-999.
7. Goshe MB, Conrads TP, Panisko EA, AngelI NH, Veenstra TD, Smith RD (2001) Anal Chem 73: 2578-2586.
8. Williamson B, Marchese J, Juhasz P, Hatten S, Patterson D, Graber A, Khainovski N, Romeo A, Martin S (2002) Proceedings of the 50th ASMS Conference on Mass Spectrometry and Allied Topics, Orlando.
9. Arcuri F, Ricci C, Ietta F, Cintorino M, Tripodi SA, Cetin I, Garzia E, Schatz F, Klemi P, Santopietro R, Paulesu L (2001) Biol Reprod 64: 1200 - 1205.
10. Canadian Cancer Statistics 2003. Canadian Cancer Society. 2003. 7-28-0003. Ref Type: Electronic Citation
11. Cancer Facts and Figures. American Cancer Society. 2003. 7-25-0003. Ref Type: Electronic Citation
12. Rose PG: Endometrial carcinoma. N Engl J Med 1996, 335:640-649.
13. Agboola OO, Grunfeld E, Coyle D, Perry GA: Costs and benefits of routine follow-up after curative treatment for endometrial cancer. CMAJ 1997, 157:879-886.
14. Duffy MJ: Clinical uses of tumor markers: a critical review. Crit Rev Clin Lab Sci 2001, 38:225-262.
15. Chambers G, Lawrie L, Cash P, Murray GI: Proteomics: a new approach to the study of disease. J Pathol 2000,192:280-288.
16. Peng J, Gygi SP: Proteomics: the move to mixtures. J Mass Spectrom 2001, 36:1083-1091.
17. Wu W, Hu W, Kavanagh JJ: Proteomics in cancer research. Int J Gynecol Cancer 2002, 12:409-423.
18. Bryant-Greenwood PK, Petricoin 3rd EF, Abati A, Liotta L: High-Throughput Proteomic Analysis of Microdissected Cytological Specimens Yields Distinct Expression Profiles of Follicular vs Papillary Thyroid Carcinomas. Mod Pathol 2000, 13:30A.
19. Jones MB, Krutzsch H, Shu H, Zhao Y, Liotta LA, Kohn EC, Petricoin EF, III: Proteomic analysis and identification of new biomarkers and therapeutic targets for invasive ovarian cancer. Proteomics 2002, 2:76-84.
20. Knezevic V, Leethanakul C, Bichsel VE, Worth JM, Prabhu VV, Gutkind JS, Liotta LA, Munson PJ, Petricoin EF, III, Krizman DB: Proteomic profiling of the cancer microenvironment by antibody arrays. Proteomics 2001, 1:1271-1278.
21. Paweletz CP, Gillespie JW, Ornstein DK, Simone NL, Brown MR, Cole KA, Wang Q-H, Huang J, Hu N, Yip T-T, Rich WE, Kohn EC, Linehan WM, Weber T, Taylor P, Emmert-Buck MR, Liotta LA, Petricoin EF, III: Rapid Protein Display Profiling of Cancer Progression Directly From Human Tissue Using a Protein Biochip. Drug Dev Res 2000, 49:34-42.
22. Petricoin EF, Ardekani AM, Hitt BA, Levine PJ, Fusaro VA, Steinberg SM, Mills GB, Simone C, Fishman DA, Kohn EC, Liotta LA: Use of proteomic patterns in serum to identify ovarian cancer. Lancet 2002, 359:572-577.
23. Vlahou A, Schellhammer PF, Mendrinos S, Patel K, Kondylis FI, Gong L, Nasim S, Wright JG, Jr.: Development of a novel proteomic approach for the detection of transitional cell carcinoma of the bladder in urine. Am J Pathol 2001, 158:1491-1502.
24. Wright Jr GL, Cazares LH, Leung S-M, Nasim S, Adam B-L, Yip T-T, Schellhammer PF, Gong L, Vlahou A: Proteinchip® surface enhanced laser desorption/ionization (SELDI) mass spectrometry: a novel protein biochip technology for detection of prostate cancer biomarkers in complex protein mixtures. Prostate Cancer and Prostatic Diseases 1999, 2:264-276.
25. Wulfkuhle JD, McLean KC, Paweletz CP, Sgroi DC, Trock BJ, Steeg PS, Petricoin EF, III: New approaches to proteomic analysis of breast cancer. Proteomics 2001, 1:1205-1215.
26. Blaustein's Pathology of the Female Genital Tract. New York, Springer-Verlag, 2002.
27. Cavanagh AC, Morton H: The purification of early-pregnancy factor to homogeneity from human platelets and identification as chaperonin 10. Eur J Biochem 1994, 222:551-560.
28. Inoue M: Current molecular aspects of the carcinogenesis of the uterine endometrium. Int J Gynecol Cancer 2001, 11:339-348.
29. Mutter GL, Baak JP, Fitzgerald JT, Gray R, Neuberg D, Kust GA, Gentleman R, Gullans SR, Wei LJ, Wilcox M: Global expression changes of constitutive and hormonally regulated genes during endometrial neoplastic transformation. Gynecol Oncol 2001, 83:177-185.
30. Wulfkuhle JD, Liotta LA, Petricoin EF: Proteomic applications for the early detection of cancer. Nat Rev Cancer 2003, 3:267-275.
31. Walch A, Komminoth P, Hutzler P, Aubele M, Hofler H, Werner M: Microdissection of tissue sections: application to the molecular genetic characterisation of premalignant lesions. Pathobiology 2000, 68:9-17.
32. Sugiyama Y, Sugiyama K, Hirai Y, Akiyama F, Hasumi K: Microdissection is essential for gene expression profiling of clinically resected cancer tissues. Am J Clin Pathol 2002, 117:109-116.
33. Batorfi J, Ye B, Mok SC, Cseh I, Berkowitz RS, Fulop V: Protein profiling of complete mole and normal placenta using ProteinChip analysis on laser capture microdissected cells. Gynecol Oncol 2003, 88:424-428.
34. Jr GW, Cazares LH, Leung SM, Nasim S, Adam BL, Yip TT, Schellhammer PF, Gong L, Vlahou A: Proteinchip(R) surface enhanced laser desorption/ionization (SELDI) mass spectrometry: a novel protein biochip technology for detection of prostate cancer biomarkers in complex protein mixtures. Prostate Cancer Prostatic Dis 1999, 2:264-276.
35. von Eggeling F, Davies H, Lomas L, Fiedler W, Junker K, Claussen U, Ernst G: Tissue-specific microdissection coupled with ProteinChip array technologies: applications in cancer research. BioTechniques 2000,29:1066-1070.
36. Somodevilla-Torres MJ, Hillyard NC, Morton H, Alewood D, Halliday JA, Alewood PF, Vesey DA, Walsh MD, Cavanagh AC: Preparation and characterization of polyclonal antibodies against human chaperonin 10. Cell Stress Chaperones 2000, 5:14-20.
37. Cornford PA, Dodson AR, Parsons KF, Desmond AD, Woolfenden A, Fordham M, Neoptolemos JP, Ke Y, Foster CS: Heat shock protein expression independently predicts clinical outcome in prostate cancer. Cancer Res 2000, 60:7099-7105.
38. Quinn KA, Morton H: Effect of monoclonal antibodies to early pregnancy factor (EPF) on the in vivo growth of transplantable murine tumours. Cancer Immunol Immunother 1992, 34:265-271.
39. Kato S, Kato M, Hirano A, Takikawa M, Ohama E: The immunohistochemical expression of stress-response protein (srp) 60 in human brain tumours: relationship of srp 60 to the other five srps, proliferating cell nuclear antigen and p53 protein. Histol Histopathol 2001, 16:809-820.
40. Tabibzadeh S, Kong QF, Satyaswaroop PG, Babaknia A: Heat shock proteins in human endometrium throughout the menstrual cycle. Hum Reprod 1996, 11:633-640.
41. Ota H, Igarashi S, Hatazawa J, Tanaka T: Distribution of heat shock proteins in eutopic and ectopic endometrium in endometriosis and adenomyosis. Fertil Steril 1997, 68:23-28.
42. Athanasas-Platsis S, Quinn KA, Wong TY, Rolfe BE, Cavanagh AC, Morton H: Passive immunization of pregnant mice against early pregnancy factor causes loss of embryonic viability. J Reprod Fertil 1989, 87:495-502.
43. Athanasas-Platsis S, Morton H, Dunglison GF, Kaye PL: Antibodies to early pregnancy factor retard embryonic development in mice in vivo. J Reprod Fertil 1991, 92:443-451.
44. Athanasas-Platsis S, Corcoran CM, Kaye PL, Cavanagh AC, Morton H: Early pregnancy factor is required at two important stages of embryonic development in the mouse. Am J Reprod Immunol 2000, 43:223-233.
45. Noonan FP, Halliday WJ, Morton H, Clunie GJ: Early pregnancy factor is immunosuppressive. Nature 1979, 278:649-651.
46. Quinn KA, Cavanagh AC, Hillyard NC, McKay DA, Morton H: Early pregnancy factor in liver regeneration after partial hepatectomy in rats: relationship with chaperonin 10. Hepatology 1994, 20:1294-1302.
47. Quinn KA: Active immunization with EPF suppresses the formation of immune ascites in BALB/c mice. Immunol Cell Biol 1991, 69 (Pt 1):1-6.
48. National Cancer Institute of Canada: Canadian Cancer Statistics 2003, Toronto, Canada, 2003 [Serial online] April 2003; Available from: URL: http://www.bc.cancer.ca/vgn/images/portal/cit 776/61/38/56158640niw stats en.pdf
49. DeSouza, L.; Diehl, G.; Yang, E.C.C.; Guo, J.; Rodrigues, M.J.; Romaschin, A.D.; Colgan, T.J.; Siu, K.W.M. Proteomic Analysis of the Proliferative and Secretory Phases of the Human Endometrium: Protein Identification and Differential Protein Expression. Proteomics. 2004, in press.
50. Yang, E.C.C.: Guo, J.; Diehl, G.; DeSouza, L.; Rodrigues, M.J.; Romaschin, A.D.; Colgan, T.J.; Siu, K.W.M. Expression Profiling of Endometrial Malignancies Reveals a New Tumor Marker: Chaperonin 10.J. Proteome Res. 2004 3, G36-643.
51. Guo, J.; Yang, E.C.C.; DeSouza, L.; Diehl, G.; Rodrigues, M.J.; Romaschin, A.D.; Colgan, T.J.; Siu, K.W.M. A Strategy for High-Resolution and Protein Identification in Surface- Enhanced Laser Desorption / Ionization (SELDI) Mass Spectrometry: Calgranulin A and Chaperonin 10 as Protein Markers for Endometrial Carcinoma. Proteomics.
52. Li, J.; Steen, H.; Gygi, S.P. Protein profiling with cleavable isotope-coded affinity tag (cICAT) reagents. Mol. Cell. Proteomics 2003, 2, 1198 - 1204.
53. Cappello, F.; Bellafiore, M.; David, S.; Anzalone, R.; Zummo, G. Ten kilodalton heat shock protein (HSP10) is overexpressed during carcinogenesis of large bowel and uterine exocervix. Cancer Lett. 2003, 196, 35 - 81.
54. Fan, X.; Yan, L.; Jia, S.; Ma, A.; Qiao, C. A study of early pregnancy factor activity in the sera of women with trophoblastic tumour. Am. J. Reprod. Immunol. 1999, 41, 204 -208.
55. Kim, C.W.; Kim, J.I.; Park, S.H.; Han, J.Y.; Kim, J.K.; Chung, K.W.; Sun, H.S. Usefulness of plasma tumor M2-pyruvate kinase in the diagnosis of gastrointestinal cancer. Korean J Gastroenterol. 2003,42, 387 -393.
56. Hardt, P.D.; Toepler, M.; Ngoumou, B.; Rupp, J.; Kloer, H.U. Fecal pyruvate kinase concentrations (ELISA based on a combination of clone 1 and clone 3 antibodies) for gastric cancer screening. Anticancer Res. 2003, 23, 855 - 857.
57. Marshall, M.J.; Goldberg, D.M.; Neal, F.E.; Millar, D.R. Enzymes of glucose metabolism in carcinoma of the cervix and endometrium of the human uterus. Br J Cancer. 1978, 37, 990 - 1001.
58. Tanaka, M.; Adzuma, K.; Iwami, M.; Yoshimoto, K.; Monden, Y.; Itakura, M. Human calgizzarin; one colorectal cancer-related gene selected by a large scale random cDNA sequencing and northern blot analysis. Cancer Lett. 1995, 89, 195 - 200.
59. Chaurand, P.; DaGue, B.B.; Pearsall, R.S.; Threadgill, D.W.; Caprioli, R.M. Profiling proteins from azoxymethane-induced colon tumors at the molecular level by matrix-assisted laser desorption/ionization mass spectrometry. Proteomics 2001, 1, 1320 - 1326.
60. Blaxall, B.C.; Dwyer-Nield, L.D.; Bauer, A.K.; Bohlmeyer, T.J.; Malkinson, A.M.; Port, J.D. Differential expression and localization of the mRNA binding proteins, AU-rich element mRNA binding protein (AUF1) and Hu antigen R (HuR), in neoplastic lung tissue. Mol. Carcinog. 2000, 28, 76 - 83.
61. Gouble, A.; Grazide, S.; Meggetto, F.; Mercier, P.; Delsol, G.; Morello, D. A new player in oncogenesis: AUF1/hnRNPD overexpression leads to tumorigenesis in transgenic mice. Cancer Res. 2002, 62, 1489 - 1495.
62. Nishihira, J.; Ishibashi, T.; Fukushima, T.; Sun, B.; Sato, Y.; Todo, S. Macrophage migration inhibitory factor (MIF): Its potential role in tumor growth and tumor-associated angiogenesis. Ann. New York Acad. Sci. 2003, 995, 171 -182.
63. Ren, Y.; Tsui, H.T.; Poon, R.T.; Ng, I.O.; Li, Z.; Chen, Y.; Jiang, G.; Lau, C.; Yu, W.C.; Bacher, M.; Fan, S.T. Macrophage migration inhibitory factor: roles in regulating tumor cell migration and expression of angiogenic factors in hepatocellular carcinoma. Int. J. Cancer. 2003, 107, 22 - 29.
64. Campa, M.J.; Wang, M.Z.; Howard, B.; Fitzgerald, M.C.; Patz, E.F. Jr. Protein expression profiling identifies macrophage migration inhibitory factor and cyclophilin a as potential molecular targets in non-small cell lung cancer. Cancer Res. 2003, 63, 1652 - 1656.
65. Markert, J.M.; Fuller, C.M.; Gillespie, G.Y.; Bubien, J.K.; McLean, L.A.; Hong, R.L.; Lee, K.; Gullans, S.R.; Mapstone, T.B.; Benos, D.J. Differential gene expression profiling in human brain tumors. Physiol. Genomics. 2001, 555, 21- 33.
66. Bacher, M.; Schrader, J.; Thompson, N.; Kuschela, K.; Gemsa, D.; Waeber, G.; Schlegel, J. Up-regulation of macrophage migration inhibitory factor gene and protein expression in glial tumor cells during hypoxic and hypoglycemic stress indicates a critical role for angiogenesis in glioblastoma multiforme. Am. J. Pathol. 2003, 162, 11 - 17.
67. Mahutte, N.G.; Matalliotakis, I.M.; Goumenou, A.G.; Koumantakis, G.E.; Vassiliadis, S.; Arici, A. Elevations in peritoneal fluid macrophage migration inhibitory factor are independent of the depth of invasion or stage of endometriosis. Fertil. Steril. 2004, 82, 97 - 101.
68. Rossel, M.; Brambilla, E.; Billaud, M.; Vuitton, D.A.; Blanc-Jouvan, F.; Biichle, S.; Revillard, J.P. Nonspecific increased serum levels of secretory component in lung tumors: relationship to the gene expression of the transmembrane receptor form. Am. J. Respir. Cell. Mol. Biol. 1993, 9, 341 - 346.
69. Rossel, M.; Billerey, C.; Bittard, H.; Ksiazek, P.; Alber, D.; Revillard, J.P.; Vuitton, D.A. Alterations in polymeric immunoglobulin receptor expression and secretory component levels in bladder carcinoma. Urol. Res. 1991, 19, 361- 366.
70. Sun, Z.; Yang, P. Role of imbalance between neutrophil elastase and alpha 1-antitrypsin in cancer development and progression. Lancet Oncol. 2004, 5, 182 - 190.
71. Ryu, J.W.; Kim, H.J.; Lee, Y.S.; Myong, N.H.; Hwang, C.H.; Lee, G.S.; Yom, H.C. The proteomics approach to find biomarkers in gastric cancer. J. Korean Med. Sci. 2003, 18, 505 - 509.
72. Nejjari, M.; Berthet, V.; Rigot, V.; Laforest, S.;.Jacquier, M.F.; Seidah, N.G.; Remy, L.; Bruyneel, E.; Scoazec, J.Y.; Marvaldi, J.; Luis, J. Inhibition of proprotein convertases enhances cell migration and metastases development of human colon carcinoma cells in a rat model. Am. J. Pathol. 2004, 164, 1925 - 1933.
73. Choi, H.; Park, C.S.; Kim, B.G.; Cho, J.W.; Park, J.B.; Bae, Y.S.; Bae, D.S. Creatine kinase B is a target molecule of reactive oxygen species in cervical cancer. Mol. Cells. 2001, 12, 412 - 417.
74. Joseph, J.; Cardesa, A.; Carreras, J. Creatine kinase activity and isoenzymes in lung, colon and liver carcinomas. Br. J. Cancer. 1997, 7776, 600 - 605.
75. Shields, J.M.; Rogers-Graham, K.; Der, C.J. Loss of transgelin in breast and colon tumors and in RIE-1 cells by Ras deregulation of gene expression through Raf-independent pathways. J. Biol. Chem. 2002, 277, 9790 - 9799.
76. Chang, J.W.; Jeon, H.B.; Lee, J.H.; Yoo, J.S.; Chun, J.S.; Kim, J.H.; Yoo, Y.J. Augmented expression of peroxiredoxin 1 in lung cancer. Biochem. Biophys. Res. Commun. 2001, 289, 507 - 512.
77. Klade, C.S.; Voss, T.; Krystek, E.; Ahorn, H..; Zatloukal, K.; Pummer, K.; Adolf, G.R. Identification of tumor antigens in renal cell carcinoma by serological proteome analysis. Proteomics 2001, 1, 890 - 898.
78. Chen, G.; Gharib, T.G.; Huang, C.C.; Thomas, D.G.; Shedden, K.A.; Taylor, J.M.; Kardia, S.L.; Misek, D.E.; Giordano, T.J.; Iannettoni, M.D.; Orringer, M.B.; Hanash, S.M.; Beer, D.G. Proteomic analysis of lung adenocarcinoma: identification of a highly expressed set of proteins in tumors. Clin. Cancer Res. 2002, 8, 2298 - 2305.
79. Lichtenfels R, Kellner R, Atkins D, Bukur J, Ackermann A, Beck J, Brenner W, Melchior S, Seliger B. Identification of metabolic enzymes in renal cell carcinoma utilizing PROTEOMEX analyses. Biochim. Biophys. Acta. 2003, 1646, 21 - 31.
80. Crnogorac-Jurcevic, T.; Efthimiou, E.; Nielsen, T.; Loader, J.; Terris, B.; Stamp, G.; Baron, A.; Scarpa, A.; Lemoine, N.R. Expression profiling of microdissected pancreatic adenocarcinomas. Oncogene 2002, 21, 4587 - 4594.
81. Kuramitsu, Y.; Fujimoto, M.; Tanaka, T.; Ohata, J.; Nakamura, K. Differential expression of phosphatidylethanol-amine-binding protein in rat hepatoma cell lines: analyses of tumor necrosis factor-alpha-resistant cKDH-8/11 and -sensitive KDH-8/YK cells by two-dimensional gel electrophoresis. Electrophoresis 2000, 21, 660- 664.
82. Srivastava,S.; Verma,M.; Henson,D.E. Biomarkers for early detection of colon cancer. Clin. Cancer Res. 2001, 7, 1118-1126.
83. Vlahou,A.; Schellhammer,P.F.; Mendrinos,S.; Patel,K.; Kondylis,F.I.; Gong,L.; Nasim,S.; Wright,J.G., Jr. Development of a novel proteomic approach for the detection of transitional cell carcinoma of the bladder in urine. Am. J. Pathol. 2001, 158, 1491-1502.
84. Bundred,N.J. Prognostic and predictive factors in breast cancer. Cancer Treat. Rev. 2001, 27, 137-142.
85. Kao, L.C., Tulac, S., Lobo, S., Imani, B., Yang, J.P., Germeyer, A., Osteen, K., Taylor, R.N., Lessey, B.A., Guidice, L.C., Endocrinology 2002, 143, 2119-2138.
86. Hathout, Y., Riordan, K., Gehrmann, M., Fenselau, C., J. Prot. Res. 2002, 1, 35 - 442.
87. Rautajoki, K., Nyman, T.A., Lahesmaa, R, Proteomics 2004, 4, 84 - 92.
88. Bigonnesse, F., Labelle, Y., Akoum, A., Fertility and Sterility 2001, 75, 79 - 87.
89. Fujiwaki, R., Iida, K., Nakayama, K., Kanasaki, H., Ozaki, T., Hata, K., Sakai, E., Miyazaki, K., Virchows Arch. 2003, 443, 672 - 677.
90. von Wolff, M., Ursel, S., Hahn, U., Steldinger, R., Strowitzki, T., J. Clin. Endocrin. Met. 2003, 88, 3885 - 3892.
91. Duchen, M.R., Diabetes 2004, 53, Suppl. 1, S96 - S 102.
92. Freemantle, S.J., Portland, H.B., Ewings, K., Dmitrovsky, F., DiPetrillo, K., Spinella, M.J., Dmitrovsky, E., Gene 2002, 291, 17 -27.
93. Jonkers, J., Korswagen, H.C., Acton, D., Breuer, M., Berns, A., EMBO J. 1997, 16,441 - 450.
94. Saitoh, T., Mine, T., Katoh, M., Int. J. Oncol. 2002, 20, 785 - 795
95. Hongisto, V., Smeds, N., Brecht, S., Herdegen, T., Courtney, M.J., Coffey, E.T., Mol. Cell Biol. 2003, 23, 6027-6036.
96. Maldonado, V., Castilla, J.A., Martinez, L., Herruzo, A., Concha, A., Fontes, J., Mendoza, N., Garcia-Pena, M.L., Mendoza, J.L., Magan, R., Ortiz, A., Gonzalez, E., J. Assist. Reprod. Genet. 2003, 20, 474 - 481.
97. Richardson, M.R., Taylor, D.A., Casey, M.L., MacDonald, P.C., Stull, J.T. In Vitro Cell Dev. Biol. 1987, 23, 21 - 28.
98. Carson, D.D., Lagow, E., Thathiah, A., Al-Shami, R., Farach-Carson, M.C., Vernon, M., Yuan, L., Fritz, M.A., Lessey, B. Mol. Hum. Reprod. 2002, 8, 871 - 879.
99. Jokimaa, V., Oksjoki, S., Kujari, H., Vuorio, E., Anttila, L., Mol. Hum. Reprod. 2001, 7, 73 -78.
100. Barnette, K.G., Sarkar, M.A., Glover, D.D., Li, P., Boyd, C., Lalka, D., Gynecol. Obstet. Invest. 1999, 47, 114 - 119.
101. Herrmann, U, Jr., Degiampietro, P., Peheim, E., Bachmann, C., Arch. Gynecol. 1987, 240, 233 - 240.
102. Meier, U., Beier-Hellwig, K., Klug, J., Linder, D., Beier, H.M., Hum. Reprod. 1995, 10, 1305 - 1310.
103. Berkova, N., Lemay, A., Dresser, D.W., Fontaine, J.-Y., Kerizit, J., Goupil, S., Mol. Hum. Reprod. 2001, 7, 747 - 754.
104. Li, C., Hong, Y., Tan, Y.-X., Zhou, H., Ai, J.-H., Li, S.-J., Zhang, L., Xia, Q.-C., Wu, J.-R., Wang, H.-Y., Zeng, R., Mol. Cell Proteomics 2004, 3, 399 - 409.
105. Issaq, H.J., Conrads, T.P., Prieto, D.A., Tirumalai, R., Veenstra, T.D., Anal. Chem. 2003, 75, 149A -155A.
106. Petricoin III, E.F., Ornstein, D.K., Pawletz, C.P., Ardekani, A., Hackett, P.S., Hitt, B.A., Valassco, A., Trucco, C., Wiegand, L., Wood, K., Simone, C.V., Leving, P.J., Linehan, W.M., Emmert-Buck, M.R., Steinberg, S.M., Kohn, E.C., Liotta, L.A. J. Natl. Cancer Inst. 2002, 94, 1576 - 1578.
107. Kozak, K.R., Amneus, M.W., Pusey, S.M., Su, F., Luong, M.N., Luong, S.A., Reddy, S.T., Farias-Eisner, R., Proc. Natl. Acad. Sci. USA 2003, 100, 12343 - 12348.
108. Zhu, W., Wang, X., Ma, Y., Rao, M., Glimm, J., Kovach, J.S., Proc. Natl. Acad. Sci. USA 2003, 100, 14666 - 14671.
109. Diamandis, E.P., Lancet 2002, 360, 170.
110. Diamandis, E.P., J. Natl. Cancer Inst. 2003, 95, 489 - 490.
111. Diamandis, E.P., Clin. Chem. 2003, 49, 1271 -1278.
112. Sorace, J.M., Zhan, B. BMC Bioinformatics 2003, 4, 24.
113. Cottingham, K., Anal. Chem. 2003, 75, 472A - 476A.
114. Marshall, J., Kupchak, P., Zhu, W., Yantha, J., Vrees, T., Furesz, S., Jacks, K., Smith, C., Kireeva, I., Zhang, R., Takahashi, M., Stanton, E., Jackowski, G., J. Proteome Res. 2003, 2, 361 - 372.
115. Diamandis, E.P., Mol. Cell Proteomics 2004, 3, 367-378.
116. Baggerly, K.A., Morris, J.S., Coombes, K.R. Bioinformatics 2004, 20, 777 - 785.
117. Check, E., Nature 2004, 429, 496 - 497.
118. Marshall, J., Jankowski, A., Furesz, S., Kireeva, I., Barker, L., Dombrovsky, M., Zhu, W., Jacks, K., Ingratta, L., Bruin, J., Kristensen, E., Zhang, R., Stanton, E., Takahashi, M., Jackowski, G. J. Proteome Res. 2004, 3, 364 - 382.
119. Wagner, L., J. Natl. Cancer Inst. 2004, 96, 500 - 501.
120. He, Q.Y., Chen, J., Kung, H.F., Yuen, A.P., Chiu, J.F., Proteomics 2004, 4, 271 - 278.
121. Zhang, Z., Bast Jr., R.C., Fung, E.T., Yu, Y., Li, J., Rosenzweig, J. Proc. AACR Abstract No. 5739, 2003.
122. Ye, B., Cramer, D.W., Skates, S.J., Gygi, S.P., Pratomo, V., Fu, L., Hornick, N.K., Licklider, L.J., Schorge, J.O., Berkowitz, R.S., Mok, S.C., Clin. Cancer Res. 2003, 9, 2904 -2911.
123. Chan, D. HUPO 2nd Annual & IUBIMB XIX World Congress, Montreal, Quebec, October 8 - 11, 2003.
124. Guo, J., Yang, E.C.C., DeSouza, L., Rodrigues, M.J., Romaschin, A.D., Colgan, T.J., Siu, K.W.M., Proc. 52nd American Society for Mass Spectrometry Conference, Nashville, Tennessee, May 23 - 27,2004.
125. Creasman, W.T., Endometrial Carcinoma Available from: URL: http://www.emedicine.com/med/topic674.htm
126. National Cancer Institute of Canada: Canadian Cancer Statistics 2003, Toronto, Canada, 2003 [Serial online] April 2003; Available from: URL: http://www.bc.cancer.ca/vgn/images/portal/cit_776/61/38/56158640niw_stats_en.pdf
127. American Cancer Society, Statistics Available from: URL: http://www.cancer.org/docroot/STT/stt_0.asp
128. Bernstein, L., Deapen, D., Cerhan, J.R., Schwartz, S.M., J. Natl Cancer Inst. 1999, 91, 1654 -1662.
129. Grady, G., Ernster V.L., Endometrial cancer In: Schottenfeld, D., Fraumeni Jr., J., Ed., Cancer epidemiology and prevention 2nd ed. New York, Saunders, 1996. p. 1058 - 1089.
130. Endometrial cancer Available from: URL: http://health.allrefer.com/health/endometrial-cancer-info.html
131. Uterine Cancer (Endometrial carcinima) Available from: URL: http://alwaysyourchoice.com/ayc/adult/womens/uterine_cancer.php
132. Kurman, R.J., Ed., Blaustein's Pathology of the Female Genital Tract, 5th ed. New York, Springer-Verlag, 2002.
133. ExPASy, URL: http://ca.expasy.org/
134. Odink, K., Cerletti, N., Bruggen, J., Clerc, G.R., Tarcsay, L., Zwadlo, G., Gerhards, G., Schlegel, R., Sorg, C., Nature 1987, 330, 80 - 82.
135. Stulik, J., Osterreicher, J., Koupilova, K., Knizek, J., Macela, A., Bures, J., Jandik, P., Langr, F., Dedic, K., Jungblut, P.R., Electrophoresis 1999, 20, 1047 - 1054.
136. Kerkhoff, C., Eue, I., Sorg, C., Pathobiology 1999, 67, 230 - 232.
137. Kerkhoff, C., Klempt, M., Sorg, C., Biochim. Biophys. Acta. 1998, 1448, 200 - 211.
138. Schluesene, H.J., Kremsner, P.G., Meyermann, R., Acta Neuropathol. (Berl.) 1998, 96, 575 -580.
139. Hunter, M.J., Chazin, W.J., J. Biol. Chem. 1998, 273,12427-12435.
140. Bordmann, G., Burmeister, G., Saladin, S., Urassa, H., Mwankyusye, S., Weiss, N., Tanner, M., Clin. Diagn. Lab. Immunol. 1997, 4, 435 - 439.
141. Lugering; N., Stoll, R., Kucharzik, T., Burmeister, G., Sorg, C., Domschke, W., Clin. Exp. Immunol. 1995, 101, 249 -253.
142. Fanjul, M., Renaud, W., Merten, M., Guy-Crotte, O., Hollande E., Figarella, C., Am. J. Physio. 1995, 268, C1241 - C1251.
143. Schmid, K.W., Lugering, N., Stoll, R., Brinkbaumer, P., Winde, G., Domschke, W., Bocker, W., Sorg, C., Hum. Pathol. 1995, 26, 334-337.
144. Uchida, T., Fukawa, A., Uchida, M., Fujita, K., Saito, K., J. Proteome Res. 2002, 1, 495 -499.
145. Lundy, F.T., Chalk, R., Lamey, P.-J., Shaw, C., Linden, G.J., J. Pathol. 2000, 1992, 540 - 544.
146. Bhardwaj, R.S., Zotz, C., Zwadlo-Kkarwasser, G., Roth, J., Geobeler, M., Mahnke, K., Falk, M., Meinardus-Hager, G., Sorg, C., Eur. J. Immunol. 1992, 22, 1891 - 1897.
147. Burkhardt, K., Radespiel-Troger, M., Rupprecht, H.D., Goppelt-Strube, M., Riess, R., Renders, L., Hauser, I.A., Kunzendorf, U., J. Am. Soc. Nephrol. 2001, 12, 1947 - 1957.
148. Roth, J., Teigelkamp, S., Weike, M., Grun, L., Tummler, B., Sorg, C., Immunobiology 1992, 186, 304 - 314.
149. Munaron, L., Antoniotti, S., Pla, A.F., Lovisolo, D. Curr. Med. Chem. 2004, 11, 1533 - 1543.
150. Lin, J., Black, M., Tang, C., Zimmer, D., Rustandi, R.R., Weber, D.J., Carrier, F., J. Biol. Chem. 2001, 276, 35037 - 35041.
151. Lin, J., Yang, Q., Yan, Z., Markowitz, J., Wilder, P.T., Carrier, F., Weber, D.J. J. Biol. Chem. 2004, 279, 34071-34077.
152. Gebhardt, C., Breitenbach, U., Tuckermann, J.P., Dittrich, B.T., Richter, K.H., Angel, P. Oncogene 2002, 21, 4266 - 4276.
153. Kristinsson, J., Nygaard, K., Aadland, E., Barstad, S., Sauar, J., Hofstad, B., Stray, N., Stallemo, A., Haug, B., Ugstad, M., Ton, H., Fuglerud, P. Digestion 2001, 64, 104 - 110.
154. Tibble, J., Sigthorsson, G., Foster; R., Sherwood, R., Fagerhol, M., Bjarnason, I. Gut 2001, 49, 402 -408.
155. Nacken, W., Roth, J., Sorg, C., Kerkhoff, C. Microsc. Res. Tech. 2003, 60, 569 - 580.
156. Donato, R. Int. J. Biochem. Cell Biol. 2001, 33, 637-668.
157. Desmond, J.C., Chien, W., Said, J., Koeffler, P., Cellular, Molecular, and Tumor Biology, 2003, 44, 1198.

### Sequence Listing

SEQ ID NO. 1
   Q04984 and AAH23518
   Chaperonin 10
   1 magqafrkfl plfdrvlver saaetvtkgg imlpeksqgk vlqatvvavg sgskgkggei 61 qpvsvkvgdk vllpeyggtk vvlddkdyfl frdgdilgky vd
SEQ ID NO. 2
   NM_002157 and U07550
   Human chaperonin 10 mRNA, complete cds
SEQ ID NO. 3
   P05109
Calgranulin A
**SEQ ID NO. 4**
   A12027
   Macrophage migration inhibition factor (MRP-14) cDNA from Human placenta (formula v)
**SEQ ID NO. 5**
   NM_002964
   Homo sapiens S100 calcium binding protein A8 (calgranulin A) S100A8, mRNA
**SEQ ID NO. 6**
   P06702
   Calgranulin B/MRP-14
**SEQ ID NO. 7**
   X06233
   Human mRNA for calcium-binding protein in macrophages (MRP-14)
macrophage migration inhibitory factor (MIF)-related protein
**SEQ ID NO. 8**
   M21064
   Human migration inhibitory factor-related protein 14 (MRP14) gene, complete cds
**SEQ ID NO. 9**
   P01833 and Q81ZY7
   Polymeric-immunoglobulin receptor (precursor)
**SEQ ID NO. 10**
   NM_002644
   Homo sapiens polymeric immunoglobulin receptor (PIGR), mRNA
**SEQ ID NO. 11**
   P30086 - Homo sapiens
   Phosphatidylethanolamine binding protein (PEBP)
**SEQ ID NO. 12**
   NM_002567
   Homo sapiens prostatic binding protein (PBP), mRNA
**SEQ ID NO. 13**
   P39687 - Homo sapiens
   Acidic leucine-rich nuclear phosphoprotein 32 family member A
**SEQ ID NO. 14**
   NM_006305
   Homo sapiens acidic (leucine-rich) nuclear phosphoprotein 32 family, member A (ANP32A), mRNA
**SEQ ID NO. 15**
   P17066 - Homo sapiens
   Heat shock 70kDa protein
**SEQ ID NO. 16**
   NM_002155
   Homo sapiens heat shock 70kDa protein 6 (HSP70B') (HSPA6), mRNA.
**SEQ ID NO. 17**
   X51757
   Human heat-shock protein HSP70B gene
SEQ **ID NO. 18**
   P14174
   macrophage migration inhibitory factor - Homo Sapiens
**SEQ ID NO. 19**
   NM_002415 - Homo Sapiens
   Homo sapiens macrophage migration inhibitory factor (glycosylation-inhibiting factor) (MIF), mRNA
**SEQ ID NO. 20**
   L19686
   Homo sapiens macrophage migration inhibitory factor (MIF) gene, complete cds
**SEQ ID NO. 21**
   P31949
   Calgizzarin - Homo sapiens
**SEQ ID NO. 22**
   NM_005620 and D38583 - Homo sapiens
   Homo sapiens S100 calcium binding protein A11 (calgizzarin)(S100A11), mRNA
**SEQ ID NO. 23**
   P00938 and NP_000356 - Homo sapiens
   Triosephosphate isomerase
**SEQ ID NO. 24**
   NM_000365
   Homo sapiens triosephosphate isomerase 1 (TPI1), mRNA
**SEQ ID NO. 25**
   X69723
   H.sapiens TPI1 gene for triosephosphate isomerase.
**SEQ ID NO. 26**
   Q05586 - Homo sapiens
   Glutamate [NMDA] receptor subunit zeta 1 precursor
**SEQ ID NO. 27**
   D13515
   Homo sapiens mRNA for key subunit of N-methyl-D-aspartate receptor, complete cds
**SEQ ID NO. 28**
   LLTLLALLFSCSVAR
**SEQ ID NO. 29**
   ITMLCTGSRTLK
**SEQ ID NO. 30**
   ITHU and P01009 - Homo sapiens
   α-1-antitrypsin precursor
**SEQ ID NO. 31**
   NM_000295
   Homo sapiens serine (or cysteine) proteinase inhibitor, clade A(alpha-1 antiproteinase, antitrypsin), member 1 (SERPINA1),transcript variant 1, mRNA
**SEQ ID NO. 32**
   K02212
   Human alpha-1-antitrypsin gene (S variant), complete cds
SEQ **ID NO. 33**
   gi/125294, P12277 - Homo sapiens
   Creatine kinase, B chain (B-CK)
**SEQ ID NO. 34**
   NM_001823 Homo sapiens creatine kinase, brain (CKB), mRNA Creatine kinase, B chain (B-CK)
**SEQ ID NO. 35**
   X15334
   Human gene for creatine kinase B (EC 2.7.3.2).
**SEQ ID NO. 36**
   P14618 - Homo sapiens
   Pyruvate kinase M1 or M2 isozyme
**SEQ ID NO. 37**
   X56494
   H.sapiens M gene for M1-type and M2-type pyruvate kinase
**SEQ ID NO. 38**
   Q01995 - Homo sapiens
   Transgelin
**SEQ ID NO. 39**
   D84342
   Homo sapiens DNA for SM22 alpha, complete cds
**SEQ ID NO. 40**
   Q14103 - human
   Heterogeneous nuclear ribonucleoprotein
**SEQ ID NO. 41**
   AF026126
   Homo sapiens heterogeneous nuclear ribonucleoprotein D (HNRPD) gene, complete cds
**SEQ ID NO. 42**
   NP_852000
   GSK-3 Binding Protein - FRAT1
**SEQ ID NO. 43**
   NM_005479
   Homo sapiens frequently rearranged in advanced T-cell lymphomas FRAT1), transcript variant 1, mRNA
**SEQ ID NO. 44**
   NM_181355
   Homo sapiens frequently rearranged in advanced T-cell lymphomas FRAT1), transcript variant 2, mRNA
**SEQ ID NO. 45**
   NP_444254
   myosin light chain isform kinase 2
**SEQ ID NO. 46**
   AF069601
   Homo sapiens myosin light chain kinase isoform 2 (MLCK) mRNA, complete cds
**SEQ ID NO. 47**
   AAH07433 and P09493
   tropomyosin 1 alpha chain.
**SEQ ID NO. 48**
   NM_000366 and BC007433
   Homo sapiens tropomyosin 1 (alpha), mRNA (cDNA clone), complete cds
**SEQ ID NO. 49**
   EITALAPSTMK
**SEQ ID NO. 50**
   MLTELEK
**SEQ ID NO. 51**
   ALNSIIDVYHK
**SEQ ID NO. 52**
   GADVWFK

## Claims

1. A method for screening a subject for endometrial cancer comprising
(a) detecting in a sample from the subject an amount of an endometrial cancer marker;
(b) comparing the amount of the endometrial cancer marker detected to a standard,
(c) detecting a higher level of the endometrial cancer marker compared to the standard, wherein a significantly higher level of the endometrial cancer marker compared to the standard is indicative of endometrial cancer, and
wherein the endometrial cancer marker is pyruvate kinase M1 (PKM1) or M2 (PKM2) isozyme comprising SEQ ID NO: 36, or a naturally occurring allelic variant thereof, or a naturally occurring truncated or secreted form thereof.

2. The method of claim 1 wherein the level of the endometrial cancer marker is 1.5, 2, 3, 4, 5, or 6 times higher compared to the standard.

3. A method as claimed in any preceding claim wherein the sample is obtained from tissues, extracts, cell cultures, cell lysates, lavage fluid, or physiological fluids.

4. The method of claim 3 wherein the sample is obtained from a tumor tissue.

5. A method as claimed in any one of claims 1 to 4 wherein the endometrial cancer marker is detected by contacting the sample with an antibody that specifically binds to the endometrial polypeptide marker.

6. The method of claim 5 wherein the antibody is used in a radioimmunoassay, enzyme immunoassay, microarray, competitive immunoassay, non-competitive immunoassay, or immunofluorescence, immunoprecipiation, latex agglutination, hemagglutination, histochemical or bioluminescence assay.

7. A method as claimed in any preceding claim wherein the standard comprises levels of the endometrial cancer marker for control samples from healthy subjects.

8. A method as claimed in any preceding claim wherein the standard comprises levels of the endometrial cancer marker for another sample or an earlier sample of the subject.

## Patentansprüche

1. Verfahren zum Durchmustern eines Subjekts auf Endometriumkrebs, aufweisend
(a) in einer Probe des Subjekts eine Menge eines Endometriumkrebs-Markers nachweisen,
(b) die nachgewiesene Menge des Endometriumkrebs-Markers mit einem Bezugswert vergleichen,
(c) eine höhere Menge des Endometriumkrebs-Markers im Vergleich zum Bezugswert feststellen, wobei ein signifikant höherer Spiegel des Endometriumkrebs-Markers im Vergleich zum Bezugswert Endometriumkrebs indiziert und
wobei der Endometriumkrebs-Marker das Isoenzym Pyruvatkinase M1 (PKM1) oder M2 (PKM2) ist und die SEQ ID NO: 36 aufweist oder eine natürlich vorkommende Allelvariante davon oder eine natürlich vorkommende verkürzte oder sezernierte Form davon.

2. Verfahren nach Anspruch 1, wobei der Spiegel des Endometriumkrebs-Markers im Vergleich zum Bezugswert 1,5-fach, 2-fach, 3-fach, 4-fach, 5-fach oder 6-fach höher ist.

3. Verfahren wie in einem der vorangehenden Ansprüche beansprucht, wobei die Probe aus Geweben, Extrakten, Zellkulturen, Zell-Lysaten, Spülfluid oder physiologischen Fluiden erhalten ist.

4. Verfahren nach Anspruch 3, wobei die Probe aus einem Tumorgewebe erhalten ist.

5. Verfahren nach einem der Ansprüche 1 bis 4, wobei der Endometriumkrebs-Marker durch Kontaktieren der Probe mit einem Antikörper nachgewiesen wird, der spezifisch an den Endometriumkrebs-Polypeptidmarker bindet.

6. Verfahren nach Anspruch 5, wobei der Antikörper in einem Radioimmunoassay, einem Enzym-Immunoassay, einem Mikro-Array, einem kompetitiven Immunoassay, einem nicht-kompetitiven Immunoassay oder in Immunfluoreszenz, Immunpräzipitation, Latex-Agglutination, Hämagglutination, einem histochemischen oder einem Bioluminesenz-Assay verwendet wird.

7. Verfahren wie in einem der vorangehenden Ansprüche beansprucht, wobei der Bezugswert Spiegel des Endometriumkrebs-Markers für Kontrollproben gesunder Subjekte aufweist.

8. Verfahren wie in einem der vorangehenden Ansprüche beansprucht, wobei der Bezugswert Spiegel des Endometriumkrebs-Markers für eine andere Probe oder eine frühere Probe des Subjekts aufweist.

## Revendications

1. Procédé de criblage d'un sujet pour le cancer endométrial comprenant
(a) détecter dans un échantillon provenant du sujet une quantité d'un marqueur de cancer endométrial ;
(b) comparer la quantité du marqueur de cancer endométrial détectée à un étalon,
(c) détecter un niveau supérieur du marqueur de cancer endométrial par rapport à l'étalon,
dans lequel un niveau nettement supérieur du marqueur de cancer endométrial par rapport à l'étalon est indicateur de cancer endométrial, et
dans lequel le marqueur de cancer endométrial est l'isozyme de pyruvate kinase M1 (PKM1) ou M2 (PKM2) comprenant la SEQ ID N° : 36, ou une variante allélique se produisant naturellement de celui-ci, ou une forme tronquée ou secrétée se produisant naturellement de celui-ci.

2. Procédé selon la revendication 1, dans lequel le niveau du marqueur de cancer endométrial est 1,5, 2, 3, 4, 5 ou 6 fois supérieur par rapport à l'étalon.

3. Procédé selon l'une quelconque des revendications précédentes, dans lequel l'échantillon est obtenu à partir de tissus, d'extraits, de cultures cellulaires, de lysats cellulaires, de fluide de lavage ou de fluides physiologiques.

4. Procédé selon la revendication 3, dans lequel l'échantillon est obtenu à partir d'un tissu tumoral.

5. Procédé selon l'une quelconque des revendications 1 à 4, dans lequel le marqueur de cancer endométrial est détecté en mettant en contact l'échantillon avec un anticorps qui se fixe spécifiquement au marqueur de polypeptide endométrial.

6. Procédé selon la revendication 5, dans lequel l'anticorps est utilisé dans un radio-immunoessai, un immunoessai enzymatique, une puce à ADN, un immunoessai compétitif, un immunoessai non compétitif ou un dosage par immunofluorescence, immunoprécipitation, réaction au latex, hémagglutination, histochimique ou par bioluminescence.

7. Procédé selon l'une quelconque des revendications précédentes, dans lequel l'étalon comprend des niveaux du marqueur de cancer endométrial pour des échantillons témoins provenant de sujets sains.

8. Procédé selon l'une quelconque des revendications précédentes, dans lequel l'étalon comprend des niveaux du marqueur de cancer endométrial pour un autre échantillon ou un échantillon antérieur du sujet.
